# EUROPEAN PATENT APPLICATION

(11) **EP 4 610 261 A1**
(43) Date of publication of application: **03.09.2025**
(21) Application number: 23881983.3
(22) Date of filing: 27.10.2023
(51) Int. Cl.: C07D 487/04, C07D 471/04, A61K 31/437, A61P 25/00, A61P 25/16, A61P 25/18, A61P 25/28

(54) **NITROGEN-CONTAINING HETEROCYCLIC COMPOUND, PHARMACEUTICALLY ACCEPTABLE SALT THEREOF, PREPARATION METHOD THEREFOR AND USE THEREOF**

(30) Priority: 28.10.2022 CN 202211339268; 26.12.2022 CN 202211686868; 08.02.2023 CN 202310099794; 01.03.2023 CN 202310186197; 17.03.2023 CN 202310263463; 16.06.2023 CN 202310719553; 23.10.2023 CN 202311378324
(71) Applicant: Neushen Therapeutics (Shanghai) Co., Ltd, Shanghai 201210 (CN)
(72) Inventor: CHEN, Deheng, Shanghai 201210 (CN); JIA, Haifei, Shanghai 201210 (CN); SHEN, Huaqiong, Shanghai 201210 (CN); ZHAO, Lele, Shanghai 201210 (CN)
(74) Representative: Murgitroyd & Company
(86) International application number: PCT/CN2023/127298
(87) International publication number: WO 2024/088408

(57) **Abstract**

Disclosed are a nitrogen-containing heterocyclic compound, a pharmaceutically acceptable salt thereof, a preparation method therefor and the use thereof. Provided is a compound as shown in formula (I) or a pharmaceutically acceptable salt thereof. The compound of the present invention can be used as a positive allosteric modulator of a muscarinic receptor, and the compound of the present invention can treat M receptor-mediated (or M receptor-related) diseases.

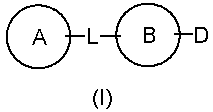

## Description

The present application claims the right of the priority of Chinese patent application No. 202211339268.9 filed on October 28, 2022. The contents of the above Chinese patent application are incorporated herein by reference in its entirety.

The present application claims the right of the priority of Chinese patent application No. 202211686868.2 filed on December 26, 2022. The contents of the above Chinese patent application are incorporated herein by reference in its entirety.

The present application claims the right of the priority of Chinese patent application No. 202310099794.0 filed on February 08, 2023. The contents of the above Chinese patent application are incorporated herein by reference in its entirety.

The present application claims the right of the priority of Chinese patent application No. 202310186197.1 filed on March 01, 2023. The contents of the above Chinese patent application are incorporated herein by reference in its entirety.

The present application claims the right of the priority of Chinese patent application No. 202310263463.6 filed on March 17, 2023. The contents of the above Chinese patent application are incorporated herein by reference in its entirety.

The present application claims the right of the priority of Chinese patent application No. 202310719553.1 filed on June 16, 2023. The contents of the above Chinese patent application are incorporated herein by reference in its entirety.

The present application claims the right of the priority of Chinese patent application No. 202311378324.4 filed on October 23, 2023. The contents of the above Chinese patent application are incorporated herein by reference in its entirety.

### TECHNICAL FIELD

The present disclosure relates to a nitrogen-containing heterocyclic compound, a pharmaceutically acceptable salt thereof, a preparation method therefor, and a use thereof.

### BACKGROUND

Muscarinic receptor (M receptor) belongs to G protein-coupled receptors and is one of acetylcholine receptors. There are five subtypes of M receptors, wherein M1, M3, and M5 are coupled to a Gq protein, activating phospholipase C and increasing intracellular calcium levels. M2 and M4 are coupled to a Gi protein, inhibiting adenylate cyclase and reducing cAMP levels. M1 receptors are distributed in the central nervous system, digestive tract, and lymphoid tissues, which can simultaneously regulate cell excitability and cholinergic transmission as main subtypes of M receptors. M4 receptors are mainly located in the cortex, hippocampus, and striatum, which play an important role in controlling dopamine release and motor activity, but do not modulate important peripheral physiological functions *(*Neuropharmacology 2018, 136, 362).

The use of M receptor agonists as therapeutic agents for mental diseases has been extensively studied by numerous research institutions and pharmaceutical research and development companies. For example, the M1/M4 receptor agonist Xanomeline underwent a phase II clinical trial for the treatment of Alzheimer's disease in the 1990s, which showed an improvement in the cognitive function of patients treated with the drug, but with serious toxic and side effects in the peripheral nervous system and digestive tract. KarXT, consisting of Xanomeline and the M1 receptor antagonist trospium chloride, is a drug developed by Karuna for the treatment of schizophrenia. Recent phase II clinical studies have indicated a significant improvement in the Positive and Negative Syndrome Scale (PANSS) scores in the KarXT treatment group compared to the placebo group, achieving the primary endpoint, but with peripheral cholinergic side effects (N Engl J Med 2021, 384, 717).

A positive allosteric modulator (PAM) of M receptors is one of the research hotspots in the field of mental diseases in recent years. In a genetic mouse model of schizophrenia, the M1 receptor positive allosteric modulator TAK-071 significantly ameliorates the deficits in memory cognition, social competence, and sensory-motor gating in mice (Neurosci Lett 2021, 764, 136240). The compound is undergoing a phase II clinical trial for the treatment of Parkinsonism. Another M1 receptor positive allosteric modulator MK-7622 is undergoing a phase II clinical trial to assess its efficacy for the treatment of Alzheimer's disease (ACS Med Chem Lett 2018, 9, 652). In a phase Ib clinical trial involving patients with schizophrenia, the M4 receptor positive allosteric modulator CVL-231, developed by Cerevel, was able to significantly reduce the PANSS total score in patients, while common adverse reactions were similar to those in the placebo group, and no extrapyramidal adverse reactions were reported. Compared to traditional M receptor orthosteric agonists, positive allosteric modulators pose lower potential risks to both the peripheral and central nervous systems, and may result in fewer toxic and side effects while maintaining efficacy. Therefore, acting on allosteric modulation pockets is a new direction for targeting M receptors to treat mental diseases. M receptor positive allosteric modulators with good druggability, *in vivo* efficacy, and safety profiles have great development value and market prospect.

### CONTENT OF THE PRESENT INVENTION

The present disclosure provides a nitrogen-containing heterocyclic compound, a pharmaceutically acceptable salt thereof, a preparation method therefor, and a use thereof. The compound of the present disclosure can be used as a positive allosteric modulator of a muscarinic receptor. The compound of the present disclosure can be used for the treatment of an M receptor-mediated (or M receptor-related) disease.

The present disclosure provides a compound of formula I or a pharmaceutically acceptable salt thereof: 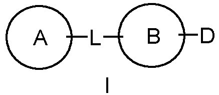 wherein 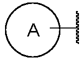 is 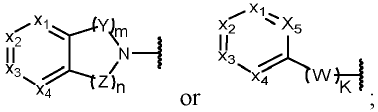
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, O, S, 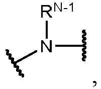 or a chemical bond;
R^{N-1} is hydrogen, C₁-C₆ alkyl, C₁-C₆ alkoxy, or 3- to 7-membered cycloalkyl, wherein the C₁-C₆ alkyl and C₁-C₆ alkoxy are optionally and independently substituted by 1, 2, 3, or 4 R^{N-2};
each R^{N-2} is independently halogen;
Y and Z are independently carbonyl (CO), -(CR²R³)ᵣ-, or a chemical bond; r is a natural number of 0 to 5;
each W is independently carbonyl (CO), -O-, -(CR⁴R⁵)-*,* -NR⁶-, or a chemical bond;
each R¹ is independently hydrogen, halogen, cyano, hydroxyl, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a}; or two R¹ together with the atom to which they are attached form a 3- to 7-membered cycloalkyl group or a 3- to 7-membered heterocycloalkyl group;
each R^{a} is independently halogen, cyano, hydroxyl, C₁-C₃ alkyl, C₁-C₆ alkoxy, or - NR¹⁻⁴R¹⁻⁵; or two R^{a} together with the atom to which they are attached form a 3- to 7-membered cycloalkyl group or a 3- to 7-membered heterocycloalkyl group;
R^{l-1} and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently halogen, cyano, hydroxyl, C₁-C₆ alkoxy, 3- to 7-membered cycloalkyl, 3- to 7-membered heterocycloalkyl, C₁-C₃ alkyl, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently halogen, hydroxyl, or cyano;
each R^{b-2} is independently halogen, C₁-C₆ alkyl, or cyano;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl, wherein the C₁-C₃ alkyl is optionally and independently substituted by 1, 2, 3, or 4 R¹⁻¹⁻¹⁻¹;
R¹⁻⁴⁻¹ and R¹⁻¹⁻¹⁻¹ are each independently halogen, hydroxyl, or cyano;
R² and R³ are independently hydrogen, halogen, cyano, hydroxyl, C₁-C₆ alkoxy, or NR²⁻¹R²⁻²; or R² and R³ together with the atom to which they are attached form a 3- to 7-membered cycloalkyl group or a 3- to 7-membered heterocycloalkyl group;
R²⁻¹ and R²⁻² are independently hydrogen or C₁-C₆ alkyl, wherein the C₁-C₆ alkyl is optionally and independently substituted by 1, 2, 3, or 4 R^{d}; or R²⁻¹ and R²⁻² together with the atom to which they are attached form a 3- to 7-membered heterocycloalkyl group;
each R^{d} is independently halogen, cyano, hydroxyl, C₁-C₆ alkoxy, 3- to 7-membered cycloalkyl, 3- to 7-membered heterocycloalkyl, or C₁-C₃ alkyl;
R⁴, R⁵, and R⁶ are independently hydrogen, halogen, cyano, hydroxyl, C₁-C₆ alkoxy, C₁-C₆ alkyl, or NR⁴⁻¹R⁴⁻², wherein the C₁-C₆ alkyl is optionally and independently substituted by 1, 2, 3, or 4 R^{e};
each R^{e} is independently halogen, cyano, hydroxyl, C₁-C₃ alkyl, C₁-C₃ alkoxy, or - NR⁴⁻⁴R⁴⁻⁵;
R⁴⁻¹ and R⁴⁻² are independently hydrogen or C₁-C₆ alkyl, wherein the C₁-C₆ alkyl is optionally and independently substituted by 1, 2, 3, or 4 R^{f}; or R⁴⁻¹ and R⁴⁻² together with the atom to which they are attached form a 3- to 7-membered heterocycloalkyl group;
each R^{f} is independently halogen, cyano, hydroxyl, 3- to 7-membered cycloalkyl, 3-to 7-membered heterocycloalkyl, C₁-C₃ alkyl, C₁-C₃ alkoxy, or -NR⁴⁻¹⁻¹R⁴⁻²⁻¹;
R⁴⁻⁴ and R⁴⁻⁵ are independently hydrogen or C₁-C₃ alkyl; or R⁴ and R⁵ together with the atom to which they are attached form a 3- to 7-membered cycloalkyl group or a 3- to 7-membered heterocycloalkyl group;
R⁴⁻¹⁻¹ and R⁴⁻²⁻ are independently hydrogen or C₁-C₃ alkyl;
L is 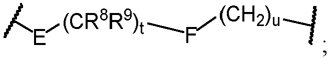
t is a natural number of 0 to 3;
u is a natural number of 0 to 3;
E is carbonyl, 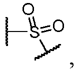 -NHCO-, or a chemical bond;
F is carbonyl, 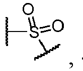 -O-, -NH-, or a chemical bond;
R⁸ and R⁹ are independently hydrogen, halogen, cyano, hydroxyl, or C₁-C₆ alkyl, wherein the C₁-C₆ alkyl is optionally and independently substituted by 1, 2, 3, or 4 R⁸; or R⁸ and R⁹ together with the atom to which they are attached form a 3- to 7-membered cycloalkyl group or a 3- to 7-membered heterocycloalkyl group;
each R^{g} is independently halogen, cyano, hydroxyl, C₁-C₃ alkyl, or C₁-C₃ alkoxy;
B is 3- to 7-membered cycloalkyl, 4- to 6-membered heterocycloalkyl, 6- to 10-membered aryl, or 5- to 12-membered heteroaryl; wherein the 3- to 7-membered cycloalkyl, 4-to 6-membered heterocycloalkyl, 6- to 10-membered aryl, and 5- to 12-membered heteroaryl are optionally and independently substituted by 1, 2, or 3 Rⁱ;
each Rⁱ is independently hydrogen, halogen, hydroxyl, C₁-C₃ alkyl, 3- to 7-membered cycloalkyl, 3- to 7-membered heterocycloalkyl, cyano, -NR¹¹⁻¹R¹¹⁻², -OR¹¹⁻³, or -SR¹¹⁻⁴; wherein the C₁-C₃ alkyl is optionally and independently substituted by 1, 2, 3 or 4 Rⁱ⁻¹, or two Rⁱ on the same atom together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3-to 7-membered heterocycloalkyl group together with B forms a fused ring);
each Rⁱ⁻¹ is independently C₁-C₃ alkyl, halogen, cyano, or hydroxyl;
R¹¹⁻¹ and R¹¹⁻² are independently hydrogen, C₁-C₃ alkyl, 3- to 7-membered cycloalkyl, or 3- to 7-membered heterocycloalkyl; or R¹¹⁻¹ and R¹¹⁻² together with the atom to which they are attached form a 3- to 7-membered cycloalkyl group or a 3- to 7-membered heterocycloalkyl group;
R¹¹⁻³ and R¹¹⁻⁴ are independently C₁-C₃ alkyl, 3- to 7-membered cycloalkyl, or 3- to 7-membered heterocycloalkyl;
in R¹¹⁻¹, R¹¹⁻², R¹¹⁻³, and R¹¹⁻⁴, the C₁-C₃ alkyl, 3- to 7-membered cycloalkyl, and 3- to 7-membered heterocycloalkyl are optionally and independently substituted by 1, 2, or 3 Rⁱ⁻²;
each Rⁱ⁻² is independently C₁-C₃ alkyl, 3- to 7-membered cycloalkyl, 3- to 7-membered heterocycloalkyl, halogen, cyano, or hydroxyl;
D is hydrogen, halogen, cyano, hydroxyl, C₁-C₆ alkyl, 3- to 7-membered cycloalkyl, 3- to 7-membered heterocycloalkyl, 6- to 10-membered aryl, or 5- to 12-membered heteroaryl; wherein the C₁-C₆ alkyl, 3- to 7-membered cycloalkyl, 3- to 7-membered heterocycloalkyl, 6-to 10-membered aryl, and 5- to 12-membered heteroaryl are optionally and independently substituted by 1, 2, or 3 R^{j};
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻³, and R¹²⁻⁴ are independently hydrogen, C₁-C₃ alkyl, 3- to 7-membered cycloalkyl, or 3- to 7-membered heterocycloalkyl; wherein the C₁-C₃ alkyl, 3- to 7-membered cycloalkyl, and 3- to 7-membered heterocycloalkyl are optionally and independently substituted by 1, 2, or 3 R^{k-1}; or R¹²⁻¹ and R¹²⁻² together with the atom to which they are attached form a 3- to 7-membered heterocycloalkyl group;
each R^{k} is independently halogen, cyano, hydroxyl, C₁-C₃ alkyl, 3- to 7-membered cycloalkyl, or 3- to 7-membered heterocycloalkyl;
each R^{k-1} is independently halogen, cyano, hydroxyl, C₁-C₃ alkyl, 3- to 7-membered cycloalkyl, or 3- to 7-membered heterocycloalkyl;
when A is 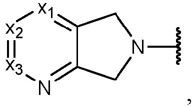 and X₁, X₂, and X₃ are CR¹, then the compound of formula I satisfies any one of the following conditions:
   (1) E is -NHCO- or a chemical bond, and F is -O-, -NH-, or a chemical bond,
   (2) E is carbonyl, and F is -NH-,
   (3) L is -(CH₂)-, -(CH₂)₂-, 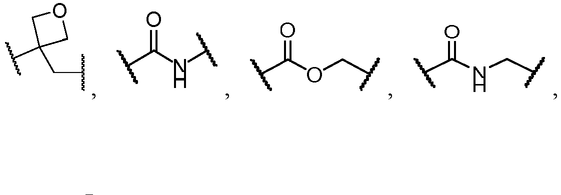 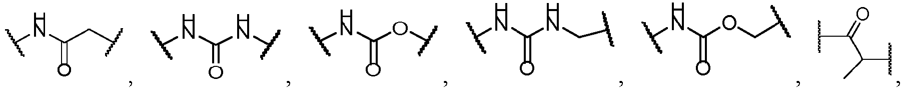 or 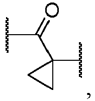
   (4) when E or F is carbonyl, B is 4- to 7-membered cycloalkyl, 6- to 10-membered aryl, 5- to 12-membered heteroaryl, or 4- to 6-membered heterocycloalkyl substituted by 1, 2, or 3 Rᵢ, wherein two Rᵢ on the same atom together with the atom to which they are attached form a 3- to 7-membered cycloalkyl group or a 3- to 7-membered heterocycloalkyl group; or, two adjacent Rᵢ together with the atom to which they are attached form a 3- to 7-membered cycloalkyl group or a 3- to 7-membered heterocycloalkyl group (wherein the 3- to 7-membered cycloalkyl group or the 3- to 7-membered heterocycloalkyl group forms a fused ring with B); wherein the 4- to 7-membered cycloalkyl, 6- to 10-membered aryl, and 5- to 12-membered heteroaryl are optionally and independently substituted by 1, 2 or 3 Rᵢ,
   (5) 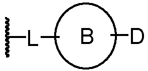 is 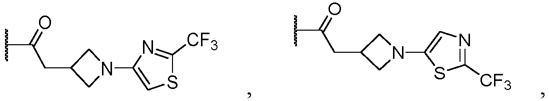 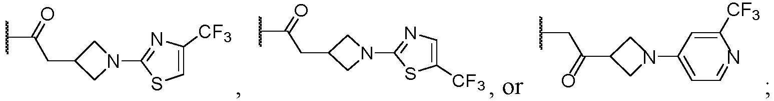
when A is 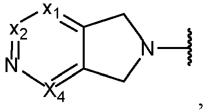 the number of heteroatoms in X₁, X₂, and X₄ is 1 or 2;
when A is 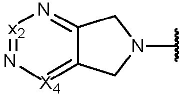 and L is carbonyl, then u is a natural number of 1 to 3, B is 4- to 6-membered heterocycloalkyl, and the 4- to 6-membered heterocycloalkyl is optionally and independently substituted by 1, 2, or 3 Rⁱ;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 5.

In a certain embodiment, in the compound of formula I or the pharmaceutically acceptable salt thereof, certain groups have the following definitions, and the definitions of the groups not mentioned are as described in any embodiment of the present disclosure (hereinafter referred to as "in a certain embodiment", "in some embodiments", "in a certain embodiment" or "in a certain preferred embodiment" in this paragraph).

In a certain embodiment, 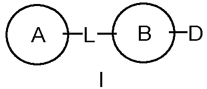 wherein 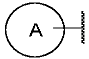 is 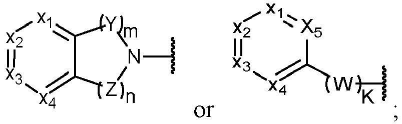
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, O, S, 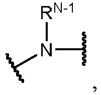 or a chemical bond;
R^{N-1} is hydrogen, C₁-C₆ alkyl, C₁-C₆ alkoxy, or 3- to 7-membered cycloalkyl, wherein the C₁-C₆ alkyl and C₁-C₆ alkoxy are optionally and independently substituted by 1, 2, 3, or 4 R^{N-2};
each R^{N-2} is independently halogen;
Y and Z are independently carbonyl (CO), -(CR²R³)ᵣ-, or a chemical bond; r is a natural number of 0 to 5;
each W is independently carbonyl (CO), -O-, -(CR⁴R⁵)-*,* -NR⁶-, or a chemical bond;
each R¹ is independently hydrogen, halogen, cyano, hydroxyl, C₁-C₆ alkyl, C₁-C₆ alkoxy, 3- to 7-membered cycloalkyl, 3- to 7-membered heterocycloalkyl, or -NR¹⁻¹R¹⁻², wherein the C₁-C₆ alkyl and C₁-C₆ alkoxy are optionally and independently substituted by 1, 2, 3, or 4 R^{a}; or two R¹ together with the atom to which they are attached form a 3- to 7-membered cycloalkyl group or a 3- to 7-membered heterocycloalkyl group;
each R^{a} is independently halogen, cyano, hydroxyl, C₁-C₃ alkyl, C₁-C₆ alkoxy, or - NR¹⁻⁴R¹⁻⁵; or two R^{a} together with the atom to which they are attached form a 3- to 7-membered cycloalkyl group or a 3- to 7-membered heterocycloalkyl group; R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3-to 7-membered heterocycloalkyl group;
each R^{b} is independently halogen, cyano, hydroxyl, C₁-C₆ alkoxy, 3- to 7-membered cycloalkyl, 3- to 7-membered heterocycloalkyl, C₁-C₃ alkyl, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently halogen, hydroxyl, or cyano;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl, wherein the C₁-C₃ alkyl is optionally and independently substituted by 1, 2, 3, or 4 R¹⁻¹⁻¹⁻¹;
R¹⁻⁴⁻¹ and R¹⁻¹⁻¹⁻¹ are each independently halogen, hydroxyl, or cyano;
R² and R³ are independently hydrogen, halogen, cyano, hydroxyl, C₁-C₆ alkoxy, or NR²⁻¹R²⁻²; or R² and R³ together with the atom to which they are attached form a 3- to 7-membered cycloalkyl group or a 3- to 7-membered heterocycloalkyl group;
R²⁻¹ and R²⁻² are independently hydrogen or C₁-C₆ alkyl, wherein the C₁-C₆ alkyl is optionally and independently substituted by 1, 2, 3, or 4 R^{d}; or R²⁻¹ and R²⁻² together with the atom to which they are attached form a 3- to 7-membered heterocycloalkyl group;
each R^{d} is independently halogen, cyano, hydroxyl, C₁-C₆ alkoxy, 3- to 7-membered cycloalkyl, 3- to 7-membered heterocycloalkyl, or C₁-C₃ alkyl;
R⁴, R⁵, and R⁶ are independently hydrogen, halogen, cyano, hydroxyl, C₁-C₆ alkoxy, C₁-C₆ alkyl, or NR⁴⁻¹R⁴⁻², wherein the C₁-C₆ alkyl is optionally and independently substituted by 1, 2, 3, or 4 R^{e};
each R^{e} is independently halogen, cyano, hydroxyl, C₁-C₃ alkyl, C₁-C₃ alkoxy, or - NR⁴⁻⁴R⁴⁻⁵;
R⁴⁻¹ and R⁴⁻² are independently hydrogen or C₁-C₆ alkyl, wherein the C₁-C₆ alkyl is optionally and independently substituted by 1, 2, 3, or 4 R^{f}; or R⁴⁻¹ and R⁴⁻² together with the atom to which they are attached form a 3- to 7-membered heterocycloalkyl group;
each R^{f} is independently halogen, cyano, hydroxyl, 3- to 7-membered cycloalkyl, 3-to 7-membered heterocycloalkyl, C₁-C₃ alkyl, C₁-C₃ alkoxy, or -NR⁴⁻¹⁻¹R⁴⁻²⁻¹;
R⁴⁻⁴ and R⁴⁻⁵ are independently hydrogen or C₁-C₃ alkyl; or R⁴ and R⁵ together with the atom to which they are attached form a 3- to 7-membered cycloalkyl group or a 3- to 7-membered heterocycloalkyl group;
R⁴⁻¹⁻¹ and R⁴⁻²⁻ are independently hydrogen or C₁-C₃ alkyl;
L is 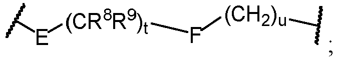
t is a natural number of 0 to 3;
u is a natural number of 0 to 3;
E is carbonyl, -NHCO-, or a chemical bond;
F is carbonyl, -O-, -NH-, or a chemical bond;
R⁸ and R⁹ are independently hydrogen, halogen, cyano, hydroxyl, or C₁-C₆ alkyl, wherein the C₁-C₆ alkyl is optionally and independently substituted by 1, 2, 3, or 4 R⁸; or R⁸ and R⁹ together with the atom to which they are attached form a 3- to 7-membered cycloalkyl group or a 3- to 7-membered heterocycloalkyl group;
each R^{g} is independently halogen, cyano, hydroxyl, C₁-C₃ alkyl, or C₁-C₃ alkoxy;
B is 3- to 7-membered cycloalkyl, 4- to 6-membered heterocycloalkyl, 6- to 10-membered aryl, or 5- to 12-membered heteroaryl; wherein the 3- to 7-membered cycloalkyl, 4-to 6-membered heterocycloalkyl, 6- to 10-membered aryl, and 5- to 12-membered heteroaryl are optionally and independently substituted by 1, 2, or 3 Rⁱ;
each Rⁱ is independently hydrogen, halogen, hydroxyl, C₁-C₃ alkyl, 3- to 7-membered cycloalkyl, 3- to 7-membered heterocycloalkyl, cyano, -NR¹¹⁻¹R¹¹⁻², -OR¹¹⁻³, or -SR¹¹⁻⁴; wherein the C₁-C₃ alkyl is optionally and independently substituted by 1, 2, 3 or 4 Rⁱ⁻¹, or two Rⁱ on the same atom together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3-to 7-membered heterocycloalkyl group together with B forms a fused ring);
each Rⁱ⁻¹ is independently C₁-C₃ alkyl, halogen, cyano, or hydroxyl;
R¹¹⁻¹ and R¹¹⁻² are independently hydrogen, C₁-C₃ alkyl, 3- to 7-membered cycloalkyl, or 3- to 7-membered heterocycloalkyl; or R¹¹⁻¹ and R¹¹⁻² together with the atom to which they are attached form a 3- to 7-membered cycloalkyl group or a 3- to 7-membered heterocycloalkyl group;
R¹¹⁻³ and R¹¹⁻⁴ are independently C₁-C₃ alkyl, 3- to 7-membered cycloalkyl, or 3- to 7-membered heterocycloalkyl;
in R¹¹⁻¹, R¹¹⁻², R¹¹⁻³, and R¹¹⁻⁴, the C₁-C₃ alkyl, 3- to 7-membered cycloalkyl, and 3- to 7-membered heterocycloalkyl are optionally and independently substituted by 1, 2, or 3 Rⁱ⁻²;
each Rⁱ⁻² is independently C₁-C₃ alkyl, 3- to 7-membered cycloalkyl, 3- to 7-membered heterocycloalkyl, halogen, cyano, or hydroxyl;
D is hydrogen, halogen, cyano, hydroxyl, C₁-C₆ alkyl, 3- to 7-membered cycloalkyl, 3- to 7-membered heterocycloalkyl, 6- to 10-membered aryl, or 5- to 12-membered heteroaryl; wherein the C₁-C₆ alkyl, 3- to 7-membered cycloalkyl, 3- to 7-membered heterocycloalkyl, 6-to 10-membered aryl, and 5- to 12-membered heteroaryl are optionally and independently substituted by 1, 2, or 3 R^{j};
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻³, and R¹²⁻⁴ are independently hydrogen, C₁-C₃ alkyl, 3- to 7-membered cycloalkyl, or 3- to 7-membered heterocycloalkyl; wherein the C₁-C₃ alkyl, 3- to 7-membered cycloalkyl, and 3- to 7-membered heterocycloalkyl are optionally and independently substituted by 1, 2, or 3 R^{k-1}; or R¹²⁻¹ and R¹²⁻² together with the atom to which they are attached form a 3- to 7-membered heterocycloalkyl group;
each R^{k} is independently halogen, cyano, hydroxyl, C₁-C₃ alkyl, 3- to 7-membered cycloalkyl, or 3- to 7-membered heterocycloalkyl;
each R^{k-1} is independently halogen, cyano, hydroxyl, C₁-C₃ alkyl, 3- to 7-membered cycloalkyl, or 3- to 7-membered heterocycloalkyl;
when A is 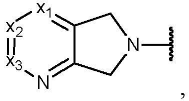 and X₁, X₂, and X₃ are CR¹, then the compound of formula I satisfies any one of the following conditions:
   (1) E is -NHCO- or a chemical bond, and F is -O-, -NH-, or a chemical bond,
   (2) E is carbonyl, and F is -NH-,
   (3) L is -(CH₂)-, -(CH₂)₂-, 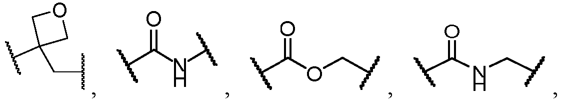 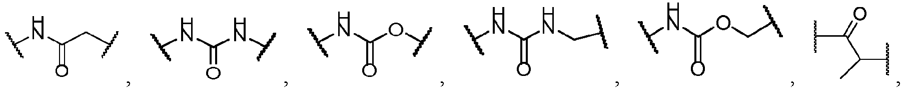 or 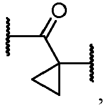
   (4) when E or F is carbonyl, B is 4- to 7-membered cycloalkyl, 6- to 10-membered aryl, 5- to 12-membered heteroaryl, or 4- to 6-membered heterocycloalkyl substituted by 1, 2, or 3 Rᵢ, wherein two Rᵢ on the same atom together with the atom to which they are attached form a 3- to 7-membered cycloalkyl group or a 3- to 7-membered heterocycloalkyl group; or, two adjacent Rᵢ together with the atom to which they are attached form a 3- to 7-membered cycloalkyl group or a 3- to 7-membered heterocycloalkyl group (wherein the 3- to 7-membered cycloalkyl group or the 3- to 7-membered heterocycloalkyl group forms a fused ring with B); wherein the 4- to 7-membered cycloalkyl, 6- to 10-membered aryl, and 5- to 12-membered heteroaryl are optionally and independently substituted by 1, 2 or 3 Rᵢ,
   (5) 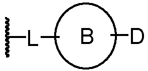 is 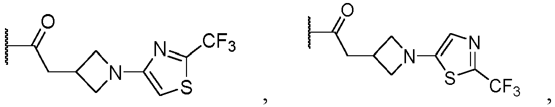 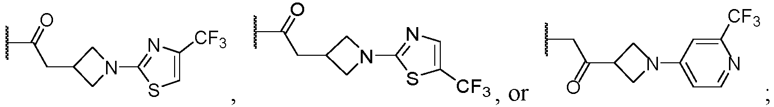
when A is 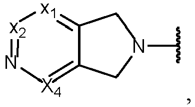 the number of heteroatoms in X₁, X₂, and X₄ is 1 or 2;
when A is 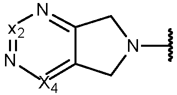 and L is carbonyl, then u is a natural number of 1 to 3, B is 4- to 6-membered heterocycloalkyl, and the 4- to 6-membered heterocycloalkyl is optionally and independently substituted by 1, 2, or 3 Rⁱ;
the heteroatom in the 3- to 7-membered heterocycloalkyl, 4- to 6-membered heterocycloalkyl, and 5- to 12-membered heteroaryl is N, O, or S, and the number of heteroatoms is 1 to 5; or, the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 5.

In a certain embodiment, m is preferably a natural number of 0 to 3, for example, 0, 1, 2, or 3; for another example, 1, 2, or 3.

In a certain embodiment, n is preferably a natural number of 1 to 3, for example, 1, 2, or 3; for another example, 1 or 2.

In a certain embodiment, n is 1.

In a certain embodiment, when X₁ is N, and X₂, X₃, and X₄ are -CR¹-, then Y and Z are independently -(CH₂)-, m and n are 2, and R^{j} is trifluoromethyl.

In a certain embodiment, 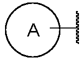 is

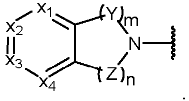

In a certain embodiment, 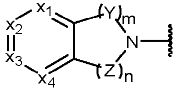 is

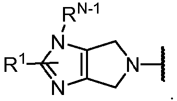

In a certain embodiment, m is 1.

In a certain embodiment, k is preferably a natural number of 0 to 3, for example, 0, 1, 2, or 3; for another example, 1, 2, or 3.

In a certain embodiment, r is preferably a natural number of 0 to 5, for example, 0, 1, 2, 3, 4, or 5; for another example, 1 or 2.

In a certain embodiment, r is 1.

In a certain embodiment, r is a natural number of 0 to 3, for example, 1 or 2.

In a certain embodiment, R^{N-1} is C₁-C₆ alkyl.

In a certain embodiment, R^{N-1} is hydrogen, C₁-C₆ alkyl, or 3- to 7-membered cycloalkyl.

In a certain embodiment, each W is independently -(CR⁴R⁵)-*,* -O-, -NR⁶-, or a chemical bond.

In a certain embodiment, each W is independently -(CR⁴R⁵)-*,* -NR⁶-, or a chemical bond.

In a certain embodiment, Y and Z are independently carbonyl (CO) or -(CR²R³)ᵣ-.

In a certain embodiment, Y and Z are independently -(CR²R³)ᵣ-.

In a certain embodiment, each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻².

In a certain embodiment, each R¹ is independently C₁-C₆ alkyl, C₁-C₆ alkoxy, C₁-C₆ alkylthio, or -NR¹⁻¹R¹⁻².

In a certain embodiment, each R¹ is independently hydrogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², for example, C₁-C₆ alkyl, C₁-C₆ alkoxy, C₁-C₆ alkylthio, or -NR¹⁻¹R^{1-2a}.

In a certain embodiment, each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a}.

In a certain embodiment, each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻².

In a certain embodiment, each R¹ is independently C₁-C₆ alkyl.

In a certain embodiment, each R¹ is independently C₁-C₆ alkyl or C₁-C₆ alkoxy.

In a certain embodiment, R¹⁻¹ and R¹⁻² are independently hydrogen or C₁-C₆ alkyl; or R¹⁻¹ and R¹⁻² together with the atom to which they are attached form a 3- to 7-membered heterocycloalkyl group; for example, R¹⁻¹ and R¹⁻² together with the atom to which they are attached form a 3- to 7-membered heterocycloalkyl group.

In a certain embodiment, when X₁ and X₃ are independently N; and X₄ and X₂ are independently -CR¹-, then each R¹ is independently C₁-C₆ alkyl, C₁-C₆ alkoxy, C₁-C₆ alkylthio, 3- to 7-membered heterocycloalkyl, or -NR¹⁻¹R¹⁻².

In a certain embodiment, when X₂ is independently -CR¹-, R¹ (at X₂) is C₁-C₆ alkyl or C₁-C₆ alkoxy.

In a certain embodiment, when D is pyrimidinyl, each R¹ is independently halogen or 3- to 7-membered cycloalkyl.

In a certain embodiment, when X₂ and X₃ are independently N; and X₄ and X₁ are independently -CR¹-, then each R¹ is independently C₁-C₆ alkyl or 3- to 7-membered heterocycloalkyl.

In a certain embodiment, each R^{a} is independently hydroxyl, C₁-C₃ alkoxy, or NR¹⁻⁴R¹⁻⁵.

In a certain embodiment, each R^{a} is independently halogen, hydroxyl, C₁-C₆ alkoxy, or -NR¹⁻⁴R¹⁻⁵; for example, each R^{a} is independently hydroxyl, C₁-C₆ alkoxy, or -NR¹⁻⁴R¹⁻⁵; for another example, hydroxyl.

In a certain embodiment, each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹.

In a certain embodiment, each R^{b} is independently hydroxyl, 3- to 7-membered cycloalkyl, or C₁-C₃ alkyl; for example, each R^{b} is independently hydroxyl or 3- to 7-membered cycloalkyl.

In a certain embodiment, each R^{b} is independently 3- to 7-membered cycloalkyl.

In a certain embodiment, R^{b-1} is independently hydroxyl.

In a certain embodiment, each R^{b-1} is independently hydroxyl.

In a certain embodiment, each R^{b-2} is independently halogen or C₁-C₆ alkyl.

In a certain embodiment, R¹⁻⁴ and R¹⁻⁵ are independently C₁-C₃ alkyl.

In a certain embodiment, each R¹⁻⁴⁻¹ is independently halogen.

In a certain embodiment, R¹⁻⁴⁻¹ is halogen.

In a certain embodiment, R² and R³ are independently hydrogen or NR²⁻¹R²⁻².

In a certain embodiment, R² and R³ are independently hydrogen.

In a certain embodiment, R²⁻¹ and R²⁻² are independently hydrogen or C₁-C₆ alkyl, for example, hydrogen.

In a certain embodiment, R⁴, R⁵, and R⁶ are independently hydrogen.

In a certain embodiment, R⁴, R⁵, and R⁶ are independently hydrogen or NR⁴⁻¹R⁴⁻².

In a certain embodiment, R⁴⁻¹ and R⁴⁻² are independently hydrogen.

In a certain embodiment, R^{e} is -NR⁴⁻⁴R⁴⁻⁵.

In a certain embodiment, R⁴⁻⁴ and R⁴⁻⁵ are independently hydrogen or C₁-C₃ alkyl.

In a certain embodiment, E is carbonyl, 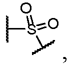 -NHCO-, or a chemical bond.

In a certain embodiment, E is carbonyl or a chemical bond.

In a certain embodiment, E is carbonyl.

In a certain embodiment, t is 0.

In a certain embodiment, F is -NH-, -O-, or a chemical bond.

In a certain embodiment, F is carbonyl, 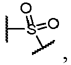 -O-, -NH-, or a chemical bond.

In a certain embodiment, F is a chemical bond.

In a certain embodiment, R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group.

In a certain embodiment, R⁸ and R⁹ are independently hydrogen or C₁-C₆ alkyl; or R⁸ and R⁹ together with the atom to which they are attached form a 3- to 7-membered cycloalkyl group.

In a certain embodiment, R⁸ and R⁹ are independently C₁-C₆ alkyl; or R⁸ and R⁹ together with the atom to which they are attached form a 3- to 7-membered cycloalkyl group or a 3- to 7-membered heterocycloalkyl group.

In a certain embodiment, B is 4- to 6-membered heterocycloalkyl, 6- to 10-membered aryl, or 5- to 12-membered heteroaryl; wherein the 5- to 12-membered heteroaryl, 6- to 10-membered aryl, and 4- to 6-membered heterocycloalkyl are optionally and independently substituted by 1, 2, or 3 Rⁱ.

In a certain embodiment, B is 4- to 6-membered heterocycloalkyl or 5- to 12-membered heteroaryl, wherein the 5- to 12-membered heteroaryl and 4- to 6-membered heterocycloalkyl are optionally and independently substituted by 1, 2, or 3 Rⁱ.

In a certain embodiment, B is 4- to 6-membered heterocycloalkyl; wherein the 4- to 6-membered heterocycloalkyl is optionally and independently substituted by 1, 2, or 3 Rⁱ.

In a certain embodiment, when B is 6- to 10-membered aryl or 5- to 12-membered heteroaryl, D is hydrogen, halogen, cyano, hydroxyl, C₁-C₆ alkyl, 3- to 7-membered cycloalkyl, or 3- to 7-membered heterocycloalkyl, wherein the C₁-C₆ alkyl, 3- to 7-membered cycloalkyl, and 3- to 7-membered heterocycloalkyl are optionally and independently substituted by 1, 2, or 3 R^{j}; preferably, D is hydrogen or C₁-C₆ alkyl.

In a certain embodiment, when X₂ and X₃ are independently N; and X₄ and X₁ are independently -CR¹-, then B is azetidinyl (e.g., 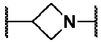 ).

In a certain embodiment, when X₁ and X₃ (or X₂ and X₄) are -CR¹-, and R¹ is C₁-C₆ alkyl, then each R^{j} is independently -OR¹²⁻³ or -SR¹²⁻⁴.

In a certain embodiment, Rⁱ is hydrogen, halogen, hydroxyl, or C₁-C₃ alkyl; or two Rⁱ together with the atom to which they are attached form a 3- to 7-membered cycloalkyl group or a 3- to 7-membered heterocycloalkyl group.

In a certain embodiment, each Rⁱ is independently hydrogen, halogen, hydroxyl, or C₁-C₃ alkyl; or two Rⁱ together with the atom to which they are attached form a 3- to 7-membered cycloalkyl group; for example, Rⁱ is hydrogen or C₁-C₃ alkyl, for example, C₁-C₃ alkyl. In a certain embodiment, Rⁱ is hydrogen.

In a certain embodiment, D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j}.

In a certain embodiment, D is hydrogen, 3- to 7-membered cycloalkyl, C₁-C₆ alkyl, or 5- to 6-membered heteroaryl; wherein the C₁-C₆ alkyl and 5- to 6-membered heteroaryl are optionally and independently substituted by 1, 2, or 3 R^{j}.

In a certain embodiment, D is 5- to 12-membered heteroaryl; wherein the 5- to 12-membered heteroaryl is optionally and independently substituted by 1, 2, or 3 R^{j}.

In a certain embodiment, D is 5- to 6-membered heteroaryl; wherein the 5- to 6-membered heteroaryl is optionally and independently substituted by 1, 2, or 3 R^{j}.

In a certain embodiment, when X₂ and X₃ are independently N; and X₄ and X₁ are independently -CR¹-, then D is 6-membered heteroaryl (e.g., 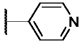 ); wherein the 6-membered heteroaryl is optionally and independently substituted by 1, 2, or 3 R^{j}.

In a certain embodiment, when D is 5-membered heteroaryl, the 5-membered heteroaryl is 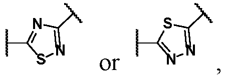 and each R¹ is independently C₁-C₆ alkyl or C₁-C₆ alkoxy.

In a certain embodiment, R^{j} is halogen, C₁-C₃ alkyl, OR¹²⁻³, or SR¹²⁻⁴.

In a certain embodiment, each R^{j} is independently C₁-C₃ alkyl, OR¹²⁻³, or SR¹²⁻⁴, and the C₁-C₃ alkyl is optionally and independently substituted by 1, 2, or 3 R^{k}; preferably, each R^{j} is independently OR¹²⁻³ or SR¹²⁻⁴.

In a certain embodiment, each R^{j} is independently halogen, C₁-C₃ alkyl, OR¹²⁻³, or SR¹²⁻⁴.

In a certain embodiment, each R^{j} is independently hydrogen, halogen, C₁-C₃ alkyl, 3-to 7-membered cycloalkyl, -OR¹²⁻³, or -SR¹²⁻⁴.

In a certain embodiment, each R^{j} is independently hydrogen, halogen, C₁-C₃ alkyl, 3-to 7-membered cycloalkyl, -OR¹²⁻³, or -SR¹²⁻⁴; the C₁-C₃ alkyl and 3- to 7-membered cycloalkyl are optionally and independently substituted by 1, 2, or 3 R^{k}.

In a certain embodiment, R^{j} is OR¹²⁻³.

In a certain embodiment, each R^{j} is independently -OR¹²⁻³.

In a certain embodiment, R^{j} is halogen or C₁-C₃ alkyl.

In a certain embodiment, each R^{j} is independently halogen or C₁-C₃ alkyl.

In a certain embodiment, when X₁ and X₃ are independently N; X₄ and X₂ are independently -CR¹-, and each R¹ is independently C₁-C₆ alkyl or C₁-C₆ alkoxy, then each R^{j} is independently halogen, 3- to 7-membered cycloalkyl, OCH₂CF₃, 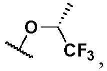 or SR¹²⁻⁴.

In a certain embodiment, when 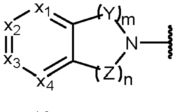 is 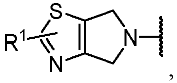 each R^{j} is independently OR¹²⁻³ or SR¹²⁻⁴.

In a certain embodiment, when X₁ or X₄ is -CR¹-, and R¹ is independently 3- to 7-membered heterocycloalkyl or -NR¹⁻¹R¹⁻², then each R^{j} is independently OR¹²⁻³ or SR¹²⁻⁴.

In a certain embodiment, R¹²⁻³ and R¹²⁻⁴ are independently C₁-C₃ alkyl.

In a certain embodiment, R¹²⁻³ and R¹²⁻⁴ are independently C₁-C₃ alkyl; wherein the C₁-C₃ alkyl is optionally and independently substituted by 1, 2, or 3 R^{k-1}.

In a certain embodiment, R¹²⁻¹, R¹²⁻², R¹²⁻³, and R¹²⁻⁴ are independently C₁-C₃ alkyl.

In a certain embodiment, each R^{k} is independently halogen.

In a certain embodiment, R^{k} is independently halogen.

In a certain embodiment, each R^{k-1} is independently halogen.

In a certain embodiment, R^{k-1} is independently halogen.

In a certain embodiment, the heteroatom in the 3- to 7-membered heterocycloalkyl, 4-to 6-membered heterocycloalkyl, and 5- to 12-membered heteroaryl is N, O, or S, and the number of heteroatoms is 1 to 3.

In a certain embodiment, the heteroatom in the 3- to 7-membered heterocycloalkyl, 4-to 6-membered heterocycloalkyl, and 5- to 12-membered heteroaryl is N, O, or S, and the number of heteroatoms is 1 to 5.

In a certain embodiment, the heteroatom in the 3- to 7-membered heterocycloalkyl, 4-to 6-membered heterocycloalkyl, 5- to 6-membered heteroaryl, and 5- to 12-membered heteroaryl is N, O, or S, and the number of heteroatoms is 1 to 3.

In a certain embodiment, the heteroatom in the 3- to 7-membered heterocycloalkyl, 4-to 6-membered heterocycloalkyl, 5- to 6-membered heteroaryl, and 5- to 12-membered heteroaryl is selected from one, two, or three kinds of N, O, or S, and the number of heteroatoms is 1, 2, or 3.

In a certain embodiment, the 3- to 7-membered heterocycloalkyl is 3- to 6-membered heterocycloalkyl, wherein the heteroatom is, for example, one or two kinds of N and O, and the number of heteroatoms is, for example, 1 or 2.

In a certain embodiment, the 4- to 6-membered heterocycloalkyl is 4-membered heterocycloalkyl, wherein the heteroatom is, for example, one or two kinds of N and O, and the number of heteroatoms is, for example, 1.

In a certain embodiment, the 5- to 6-membered heteroaryl is 6-membered heteroaryl, wherein the heteroatom is, for example, one or two kinds of N and O, and the number of heteroatoms is, for example, 1.

In a certain embodiment, the 5- to 12-membered heteroaryl is 5- to 10-membered heterocycloalkyl, wherein the heteroatom is, for example, one or two kinds of N and O, and the number of heteroatoms is, for example, 1.

In a certain embodiment, when A is 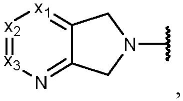 and X₁, X₂, and X₃ are CR¹, then E or F in the compound of formula I is

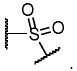

In a certain embodiment, in R^{N-1}, the C₁-C₆ alkyl is preferably C₁-C₃ alkyl, for example, methyl, ethyl, n-propyl, or isopropyl; for another example, methyl, ethyl, or isopropyl.

In a certain embodiment, in R^{N-1}, the C₁-C₆ alkoxy is preferably C₁-C₃ alkoxy, for example, methoxy, ethoxy, n-propoxy, or isopropoxy.

In a certain embodiment, in R^{N-1}, the 3- to 7-membered cycloalkyl is preferably 3- to 6-membered cycloalkyl, for example, cyclopropyl, cyclobutyl, or cyclopentyl.

In a certain embodiment, in R^{N-2}, the halogen is F, Cl, Br, or I, for example, F or Cl.

In a certain embodiment, in R¹, the halogen is F, Cl, Br, or I, for example, F or Cl; for another example, Cl.

In a certain embodiment, in R¹, the C₁-C₆ alkyl is preferably C₁-C₃ alkyl, for example, methyl, ethyl, n-propyl, or isopropyl; for another example, methyl, ethyl, or isopropyl.

In a certain embodiment, in R¹, the C₁-C₆ alkoxy is preferably C₁-C₃ alkoxy, for example, methoxy, ethoxy, n-propoxy, or isopropoxy; for another example, methoxy or ethoxy.

In a certain embodiment, in R¹, the C₁-C₆ alkylthio is preferably C₁-C₃ alkylthio, for example, methylthio, ethylthio, n-propylthio, or isopropylthio; for another example, methylthio.

In a certain embodiment, in R¹, the 3- to 7-membered cycloalkyl is preferably 3- to 6-membered cycloalkyl, for example, cyclopropyl, cyclobutyl, or cyclopentyl; for another example, cyclopropyl or cyclobutyl.

In a certain embodiment, in R¹, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, for example,

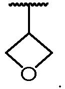

In a certain embodiment, in R¹, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, for example,

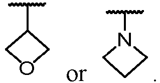

In a certain embodiment, in R^{a}, the halogen is preferably F, Cl, Br, or I, for example, F or Cl; for another example, F.

In a certain embodiment, in R^{a}, the C₁-C₃ alkyl is preferably methyl, ethyl, n-propyl, or isopropyl.

In a certain embodiment, in R^{a}, the C₁-C₆ alkoxy is preferably C₁-C₃ alkoxy, for example, methoxy, ethoxy, n-propoxy, or isopropoxy; for another example, methoxy.

In a certain embodiment, in R^{a}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, for example,

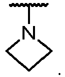

In a certain embodiment, in R^{a}, the 3- to 7-membered cycloalkyl is preferably 3- to 6-membered cycloalkyl, for example, cyclopropyl, cyclobutyl, or cyclopentyl.

In a certain embodiment, in R¹⁻¹ and R¹⁻², the C₁-C₆ alkyl is preferably C₁-C₃ alkyl, for example, methyl, ethyl, n-propyl, or isopropyl; for another example, methyl or ethyl (for example, methyl).

In a certain embodiment, in R¹⁻¹ and R¹⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is, for example, 4-membered heterocycloalkyl; for another example,

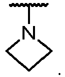

In a certain embodiment, in R^{b}, the halogen is preferably F, Cl, Br, or I, for example, F or Cl.

In a certain embodiment, in R^{b}, the C₁-C₆ alkoxy is preferably C₁-C₃ alkoxy, for example, methoxy, ethoxy, n-propoxy, or isopropoxy.

In a certain embodiment, in R^{b}, the 3- to 7-membered cycloalkyl is preferably 3- to 6-membered cycloalkyl, for example, cyclopropyl, cyclobutyl, or cyclopentyl; for another example, cyclopropyl.

In a certain embodiment, in R^{b}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{b}, the C₁-C₃ alkyl is preferably methyl, ethyl, n-propyl, or isopropyl, for example, methyl.

In a certain embodiment, in R^{b-1}, the halogen is preferably F, Cl, Br, or I, for example, F or Cl.

In a certain embodiment, in R^{b-2}, the halogen is preferably F, Cl, Br, or I, for example, F.

In a certain embodiment, in R^{b-2}, the C₁-C₆ alkyl is preferably C₁-C₃ alkyl, for example, methyl, ethyl, n-propyl, or isopropyl; for another example, methyl (e.g., 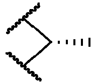 ).

In a certain embodiment, in R¹⁻⁴ and R¹⁻⁵, the C₁-C₃ alkyl is preferably methyl, ethyl, n-propyl, or isopropyl, for example, methyl, ethyl, or n-propyl; for another example, methyl.

In a certain embodiment, in R¹⁻⁴ and R¹⁻⁵, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, for example,

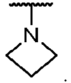

In a certain embodiment, in R¹⁻¹⁻¹ and R¹⁻²⁻¹, the C₁-C₃ alkyl is preferably methyl, ethyl, n-propyl, or isopropyl.

In a certain embodiment, in R¹⁻⁴⁻¹ and R¹⁻¹⁻¹⁻¹, the halogen is preferably F, Cl, Br, or I, for example, F or Cl; for another example, F.

In a certain embodiment, in R² and R³, the halogen is preferably F, Cl, Br, or I, for example, F or Cl.

In a certain embodiment, in R² and R³, the C₁-C₆ alkoxy is preferably C₁-C₃ alkoxy, for example, methoxy, ethoxy, n-propoxy, or isopropoxy.

In a certain embodiment, in R² and R³, the 3- to 7-membered cycloalkyl is preferably 3- to 6-membered cycloalkyl, for example, cyclopropyl, cyclobutyl, or cyclopentyl.

In a certain embodiment, in R² and R³, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R²⁻¹ and R²⁻², the C₁-C₆ alkyl is preferably C₁-C₃ alkyl, for example, methyl, ethyl, n-propyl, or isopropyl; for another example, methyl or ethyl.

In a certain embodiment, in R²⁻¹ and R²⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{d}, the halogen is preferably F, Cl, Br, or I, for example, F or Cl.

In a certain embodiment, in R^{d}, the C₁-C₆ alkoxy is preferably C₁-C₃ alkoxy, for example, methoxy, ethoxy, n-propoxy, or isopropoxy.

In a certain embodiment, in R^{d}, the 3- to 7-membered cycloalkyl is preferably 3- to 6-membered cycloalkyl, for example, cyclopropyl, cyclobutyl, or cyclopentyl.

In a certain embodiment, in R^{d}, the 3- to 7-membered heterocycloalkyl is preferably 3- to 6-membered heterocycloalkyl; the heteroatom in the 3- to 7-membered heterocycloalkyl is preferably N or O; the number of heteroatoms in the 3- to 6-membered heterocycloalkyl is preferably 1 or 2.

In a certain embodiment, in R^{d}, the C₁-C₃ alkyl is preferably methyl, ethyl, n-propyl, or isopropyl.

In a certain embodiment, in R⁴, R⁵, and R⁶, the halogen preferably is F, Cl, Br, or I, for example, F or Cl.

In a certain embodiment, in R⁴, R⁵, and R⁶, the C₁-C₆ alkoxy is preferably C₁-C₃ alkoxy, for example, methoxy, ethoxy, n-propoxy, or isopropoxy.

In a certain embodiment, R⁴, R⁵, and R⁶, the C₁-C₆ alkyl is preferably C₁-C₃ alkyl, for example, methyl, ethyl, n-propyl, or isopropyl.

In a certain embodiment, in R^{e}, the halogen is preferably F, Cl, Br, or I, for example, F or Cl.

In a certain embodiment, in R^{e}, the C₁-C₃ alkyl is preferably methyl, ethyl, n-propyl, or isopropyl.

In a certain embodiment, in R^{e}, the C₁-C₃ alkoxy is preferably methoxy, ethoxy, n-propoxy, or isopropoxy.

In a certain embodiment, in R⁴⁻¹ and R⁴⁻², the C₁-C₆ alkyl is preferably C₁-C₃ alkyl, for example, methyl, ethyl, n-propyl, or isopropyl.

In a certain embodiment, in R⁴⁻¹ and R⁴⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{f}, the halogen is preferably F, Cl, Br, or I, for example, F or Cl.

In a certain embodiment, in R^{f}, the 3- to 7-membered cycloalkyl is preferably 3- to 6-membered cycloalkyl, for example, cyclopropyl, cyclobutyl, or cyclopentyl.

In a certain embodiment, in R^{f}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{f}, the C₁-C₃ alkyl is preferably methyl, ethyl, n-propyl, or isopropyl.

In a certain embodiment, in R^{f}, the C₁-C₃ alkoxy is preferably methoxy, ethoxy, n-propoxy, or isopropoxy.

In a certain embodiment, in R⁴⁻⁴ and R⁴⁻⁵, the C₁-C₃ alkyl is preferably methyl, ethyl, n-propyl, or isopropyl.

In a certain embodiment, t is a natural number of 0 to 3, for example, 0, 1, 2, or 3; for another example, 0, 1, or 2, for example, 0.

In a certain embodiment, u is a natural number of 0 to 3, for example, 0, 1, 2, or 3; for another example, 0, 1, or 2, for example, 1.

In a certain embodiment, in R⁸ and R⁹, the halogen is preferably F, Cl, Br, or I, for example, F or Cl.

In a certain embodiment, in R⁸ and R⁹, the C₁-C₆ alkyl is preferably C₁-C₃ alkyl, for example, methyl, ethyl, n-propyl, or isopropyl; for another example, methyl.

In a certain embodiment, in R⁸ and R⁹, the 3- to 7-membered cycloalkyl is preferably 3- to 6-membered cycloalkyl, for example, cyclopropyl, cyclobutyl, or cyclopentyl; for another example, cyclopropyl.

In a certain embodiment, in R⁸ and R⁹, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is, for example, 4-membered heterocycloalkyl; for another example, oxetanyl.

In a certain embodiment, in R^{g}, the halogen is preferably F, Cl, Br, or I, for example, F or Cl.

In a certain embodiment, in R^{g}, the C₁-C₃ alkyl is preferably methyl, ethyl, n-propyl, or isopropyl, for example, methyl.

In a certain embodiment, in R^{g}, the C₁-C₃ alkoxy is preferably methoxy, ethoxy, n-propoxy, or isopropoxy.

In a certain embodiment, in B, the 3- to 7-membered cycloalkyl is preferably 3- to 6-membered cycloalkyl, for example, cyclopropyl, cyclobutyl, or cyclopentyl.

In a certain embodiment, in B, the 4- to 7-membered cycloalkyl is preferably 3- to 6-membered cycloalkyl, for example, cyclobutyl or cyclopentyl.

In a certain embodiment, in B, the 4- to 6-membered heterocycloalkyl is preferably 4-membered heterocycloalkyl, 5-membered heterocycloalkyl, or 6-membered heterocycloalkyl; the heteroatom in the 4-membered heterocycloalkyl, 5-membered heterocycloalkyl, and 6-membered heterocycloalkyl is preferably N; the number of heteroatoms in the 4- to 6-membered heterocycloalkyl is preferably 1 or 2; for example, the 4- to 6-membered heterocycloalkyl is 4-membered azacycloalkyl (e.g., 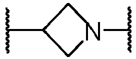 ) or 5-membered azacycloalkyl (e.g., 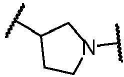 ).

In a certain embodiment, in B, the 6- to 10-membered aryl is preferably phenyl or naphthyl, for example, phenyl.

In a certain embodiment, in Rⁱ, the halogen is preferably F, Cl, Br, or I, for example, F or Cl; for another example, F.

In a certain embodiment, in Rⁱ, the C₁-C₃ alkyl is preferably methyl, ethyl, n-propyl, or isopropyl, for example, methyl.

In a certain embodiment, in Rⁱ, the 3- to 7-membered cycloalkyl is preferably 3- to 6-membered cycloalkyl, for example, cyclopropyl, cyclobutyl, or cyclopentyl; for another example, cyclopropyl or cyclobutyl, for example, cyclopropyl.

In a certain embodiment, in Rⁱ, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in Rⁱ⁻¹, the halogen is preferably F, Cl, Br, or I, for example, F or Cl.

In a certain embodiment, in Rⁱ⁻¹, the C₁-C₃ alkyl is preferably methyl, ethyl, n-propyl, or isopropyl.

In a certain embodiment, in R¹¹⁻¹, R¹¹⁻², R¹¹⁻³, and R¹¹⁻⁴, the C₁-C₃ alkyl is, for example, methyl, ethyl, n-propyl, or isopropyl.

In a certain embodiment, in R¹¹⁻³ and R¹¹⁻⁴, the 3- to 7-membered cycloalkyl may be 3- to 6-membered cycloalkyl, for example, cyclopropyl, cyclobutyl, or cyclopentyl.

In a certain embodiment, in R¹¹⁻³ and R¹¹⁻⁴, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R¹¹⁻¹ and R¹¹⁻², the 3- to 7-membered cycloalkyl is preferably 3- to 6-membered cycloalkyl, for example, cyclopropyl, cyclobutyl, or cyclopentyl.

In a certain embodiment, in R¹¹⁻¹ and R¹¹⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in Rⁱ⁻², the halogen is preferably F, Cl, Br, or I, for example, F or Cl.

In a certain embodiment, in Rⁱ⁻², the C₁-C₃ alkyl is preferably methyl, ethyl, n-propyl, or isopropyl.

In a certain embodiment, in Rⁱ⁻², the 3- to 7-membered cycloalkyl is preferably 3- to 6-membered cycloalkyl, for example, cyclopropyl, cyclobutyl, or cyclopentyl.

In a certain embodiment, in Rⁱ⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in D, the halogen is preferably F, Cl, Br, or I, for example, F or Cl.

In a certain embodiment, in D, the C₁-C₆ alkyl is preferably C₁-C₃ alkyl, for example, methyl, ethyl, n-propyl, or isopropyl; for another example, methyl.

In a certain embodiment, in D, the 3- to 7-membered cycloalkyl is preferably 3- to 6-membered cycloalkyl, for example, cyclopropyl, cyclobutyl, or cyclopentyl, for example, cyclopropyl.

In a certain embodiment, in D, the 6- to 10-membered aryl is phenyl or naphthyl.

In a certain embodiment, in R^{j}, the halogen preferably F, Cl, Br, or I, for example, F or Cl; for another example, F.

In a certain embodiment, in R^{j}, the C₁-C₃ alkyl is preferably methyl, ethyl, n-propyl, or isopropyl, for example, methyl, ethyl, or isopropyl, preferably methyl.

In a certain embodiment, in R^{j}, the 3- to 7-membered cycloalkyl is preferably 3- to 6-membered cycloalkyl, for example, cyclopropyl, cyclobutyl, or cyclopentyl; for another example, cyclopropyl.

In a certain embodiment, in R^{j}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R¹²⁻¹, R¹²⁻², R¹²⁻³, and R¹²⁻⁴, the C₁-C₃ alkyl is preferably methyl, ethyl, n-propyl, or isopropyl, for example, methyl or ethyl; for another example, methyl, ethyl or isopropyl.

In a certain embodiment, in R¹²⁻¹, R¹²⁻², R¹²⁻³, and R¹²⁻⁴, the 3- to 7-membered cycloalkyl is preferably 3- to 6-membered cycloalkyl, for example, cyclopropyl, cyclobutyl, or cyclopentyl.

In a certain embodiment, in R¹²⁻¹, R¹²⁻², R¹²⁻³, and R¹²⁻⁴, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{k}, the halogen preferably F, Cl, Br, or I, for example, F or Cl; for another example, F.

In a certain embodiment, in R^{k}, the C₁-C₃ alkyl is preferably methyl, ethyl, n-propyl, or isopropyl.

In a certain embodiment, in R^{k}, the 3- to 7-membered cycloalkyl is preferably 3- to 6-membered cycloalkyl, for example, cyclopropyl, cyclobutyl, or cyclopentyl.

In a certain embodiment, in R^{k}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{k-1}, the halogen preferably F, Cl, Br, or I, for example, F or Cl; for another example, F.

In a certain embodiment, in R^{k-1}, the C₁-C₃ alkyl is preferably methyl, ethyl, n-propyl, or isopropyl, for example, methyl.

In a certain embodiment, in R^{k-1}, the 3- to 7-membered cycloalkyl is preferably 3- to 6-membered cycloalkyl, for example, cyclopropyl, cyclobutyl, or cyclopentyl.

In a certain embodiment, in R^{k-1}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In some embodiments, the compound of formula I is a compound of formula I-1, a compound of formula I-2, a compound of formula I-3, a compound of formula I-4, or a compound of formula I-5; 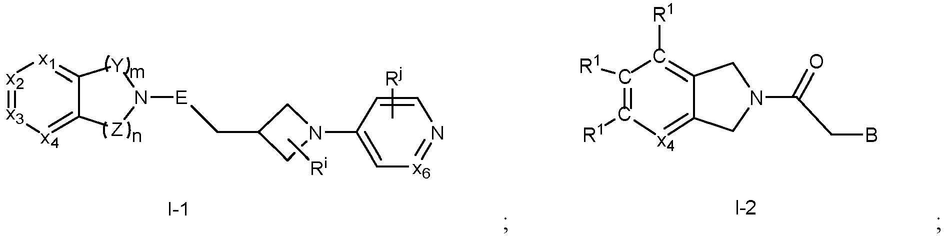 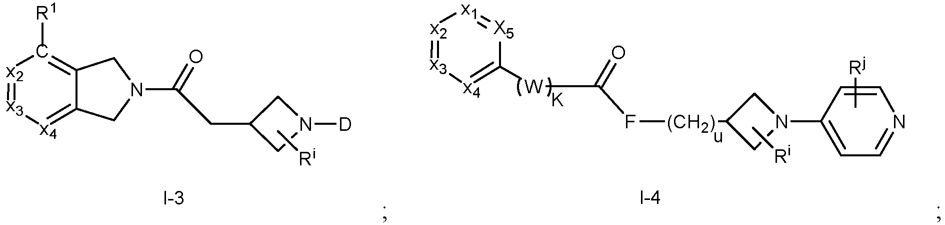 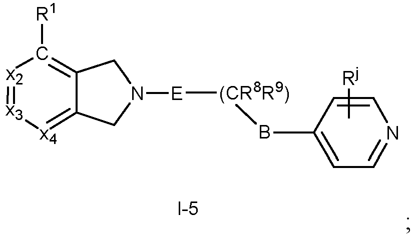 wherein X₆ is N or CH, and X₁, Rⁱ, X₂, X₃, X₄, X₅, Y, Z, m, n, E, R^{j}, R¹, B, D, W, k, F, u, R⁸, and R⁹ are as described in any one of the embodiments of the present disclosure.

In some embodiments, the compound of formula I-1 is a compound of formula I-1-1, a compound of formula 1-1-2, a compound of formula 1-1-3, or a compound of formula 1-1-4; 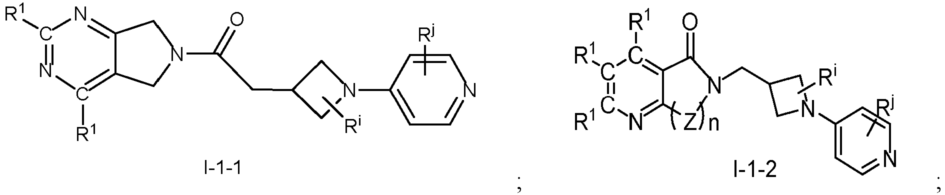 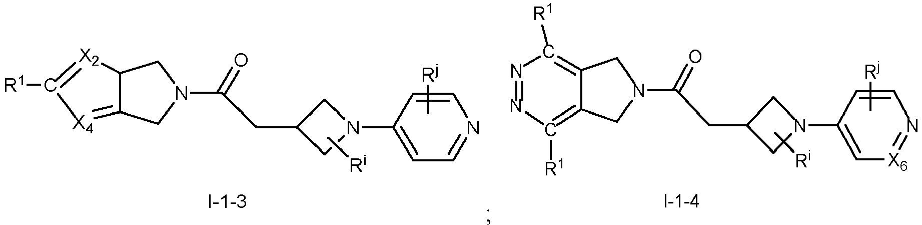 wherein R¹, R^{j}, Z, n, X₆, X₂, and X₄ are as described in any one of the embodiments of the present disclosure.

In some embodiments, the compound of formula I-5 is a compound of formula I-5-1; 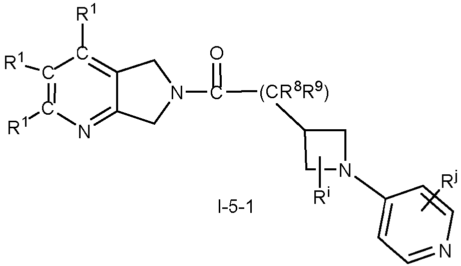 wherein R¹, R⁸, R⁹, and R^{j} as described in any one of the embodiments of the present disclosure.

In some embodiments, the compound of formula I-4 is a compound of formula I-4-1; 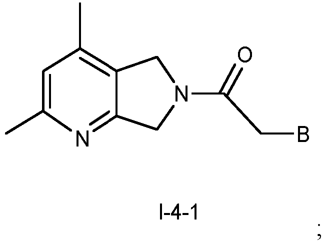 wherein B is as described in any one of the embodiments of the present disclosure.

In a certain embodiment, X₁, X₂, X₃, X₄, and X₅ are independently -CR¹-, N, O, S, 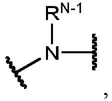 or a chemical bond, and the number of heteroatoms in X₁, X₂, X₃, X₄, and X₅ is 0, 1, 2, or 3.

In a certain embodiment, X₁, X₂, X₃, X₄, and X₅ are independently -CR¹-, N, S, 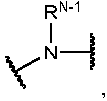 or a chemical bond, and the number of heteroatoms in X₁, X₂, X₃, X₄, and X₅ is 1 or 2.

In a certain embodiment, X₁ and X₃ are independently N; and X₄ and X₂ are independently -CR¹-.

In a certain embodiment, X₂ and X₃ are independently N; and X₄ and X₁ are independently -CR¹-.

In a certain embodiment, X₁ and X₄ are independently N; and X₂ and X₃ are independently -CR¹-.

In some embodiments, in 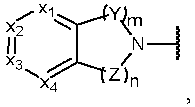 X₁, X₂, X₃, and X₄ are independently - CR¹- or N; the number of heteroatoms is 0, 1, or 2.

In some embodiments, in 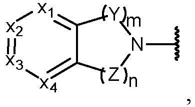 Y and Z are independently carbonyl (CO) or -(CR²R³)ᵣ-; r is 1, R² and R³ are independently H, -(CH₂)-, -(NHCH₂)-, -(NHCH₂CH₂)-, or

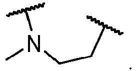

In some embodiments, in 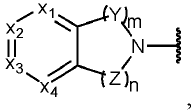 m is 2, n is 2, Y and Z are independently -(CR²R³)ᵣ-; r is 1, R² and R³ are independently H.

In some embodiments, 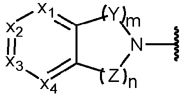 is phenyl-fused 5-membered heterocycloalkyl, wherein the heteroatom in the 5-membered heterocycloalkyl is N, and the number of heteroatoms is 1, for example, 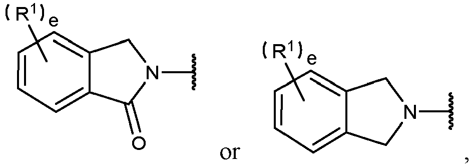 wherein e is independently 1, 2, or 3; for another example, 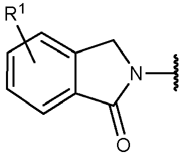 or

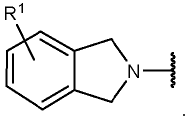

In some embodiments, 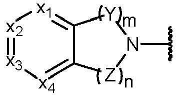 is phenyl-fused 5-membered heterocycloalkyl, wherein the heteroatom in the 5-membered heterocycloalkyl is N, and the number of heteroatoms is 1, for example,

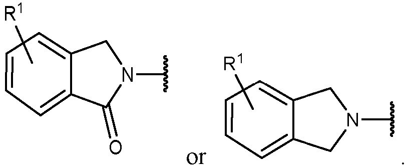

In some embodiments, 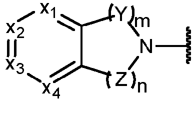 is phenyl-fused 7-membered heterocycloalkyl, wherein the heteroatom in the 7-membered heterocycloalkyl is N, and the number of heteroatoms is 1 or 2, for example, 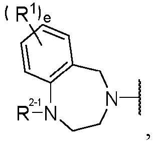 wherein e is independently 0, 1, 2, or 3; for another example,

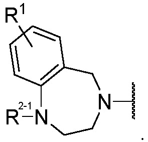

In some embodiments, 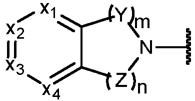 is phenyl-fused 7-membered heterocycloalkyl, wherein the heteroatom in the 7-membered heterocycloalkyl is N, and the number of heteroatoms is 1 or 2, for example,

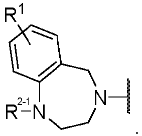

In some embodiments, 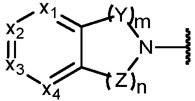 is 6-membered heteroaryl-fused 5-membered heterocycloalkyl, wherein the heteroatom in the 6-membered heteroaryl is N, and the number of heteroatoms is 1 or 2; preferably, the heteroatom in the 5-membered heterocycloalkyl is N, and the number of heteroatoms is 1, for example, 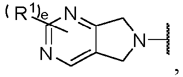 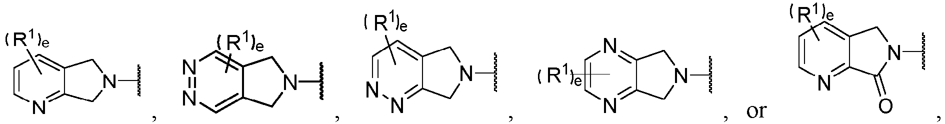 wherein e is independently 0, 1, or 2; for another example,

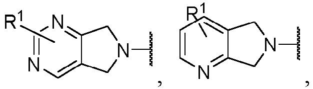

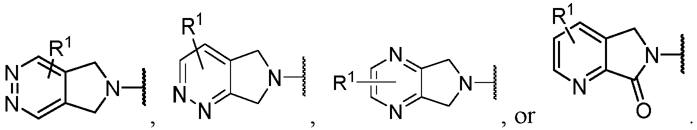

In some embodiments, 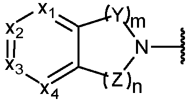 is 6-membered heteroaryl-fused 5-membered heterocycloalkyl, wherein the heteroatom in the 6-membered heteroaryl is N, and the number of heteroatoms is 1 or 2; preferably, the heteroatom in the 5-membered heterocycloalkyl is N, and the number of heteroatoms is 1, for example,

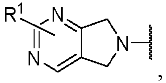

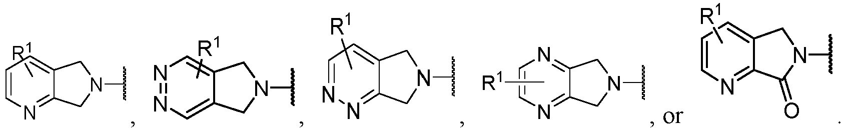

In some embodiments, 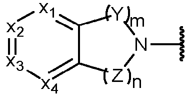 is 6-membered heteroaryl-fused 5-membered heterocycloalkyl, wherein the heteroatom in the 6-membered heteroaryl is N, and the number of heteroatoms is 2; preferably, the heteroatom in the 5-membered heterocycloalkyl is N, and the number of heteroatoms is 1, for example, 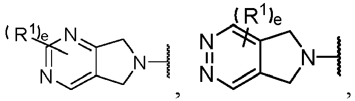 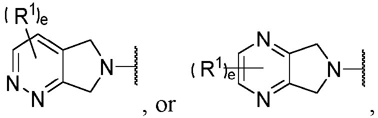 wherein e is independently 0, 1, or 2.

In some embodiments, 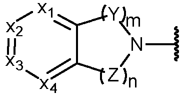 is 6-membered heteroaryl-fused 5-membered heterocycloalkyl, wherein the heteroatom in the 6-membered heteroaryl is N, and the number of heteroatoms is 1, for example, 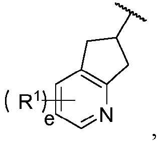 wherein e is independently 0, 1, 2, or 3; for another example,

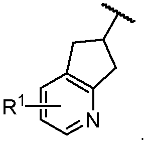

In some embodiments, 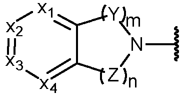 is 6-membered heteroaryl-fused 5-membered heterocycloalkyl, wherein the heteroatom in the 6-membered heteroaryl is N, and the number of heteroatoms is 1, for example,

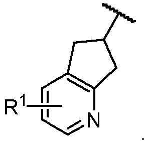

In some embodiments, 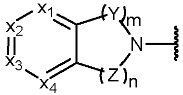 is 6-membered heteroaryl-fused 6-membered heterocycloalkyl, wherein the heteroatom in the 6-membered heteroaryl is N, and the number of heteroatoms is 1; preferably, the heteroatom in the 6-membered heterocycloalkyl is N, and the number of heteroatoms is 1, for example, 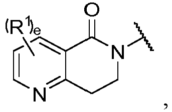 wherein e is independently 0, 1, 2, or 3; for another example,

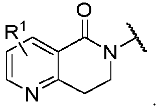

In some embodiments, 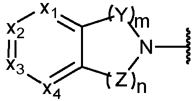 is 6-membered heteroaryl-fused 6-membered heterocycloalkyl, wherein the heteroatom in the 6-membered heteroaryl is N, and the number of heteroatoms is 1; preferably, the heteroatom in the 6-membered heterocycloalkyl is N, and the number of heteroatoms is 1, for example,

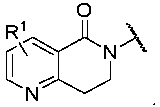

In some embodiments, 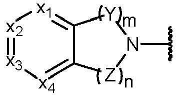 is 6-membered heteroaryl-fused 7-membered heterocycloalkyl, wherein the heteroatom in the 6-membered heteroaryl is N, and the number of heteroatoms is 1 or 2; preferably, the heteroatom in the 7-membered heterocycloalkyl is N, and the number of heteroatoms is 1, for example, 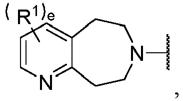 wherein e is independently 0, 1, 2, or 3, 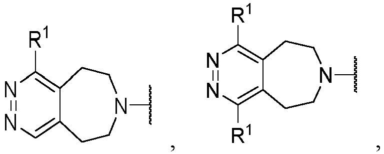 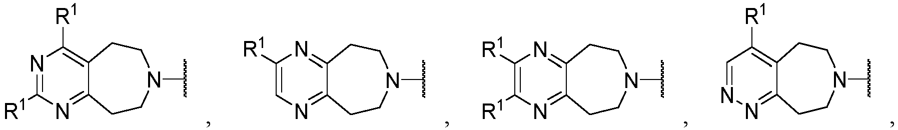 or

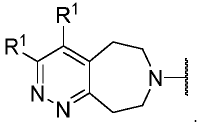

In some embodiments, 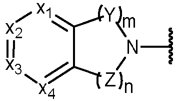 is 6-membered heteroaryl-fused 7-membered heterocycloalkyl, wherein the heteroatom in the 6-membered heteroaryl is N, and the number of heteroatoms is 1 or 2; preferably, the heteroatom in the 7-membered heterocycloalkyl is N, and the number of heteroatoms is 1, for example,

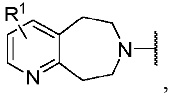

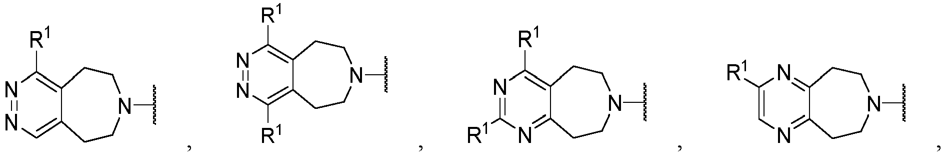

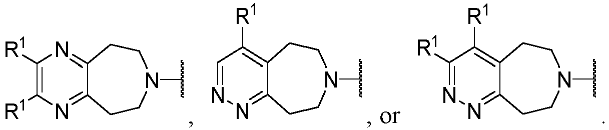

In some embodiments, 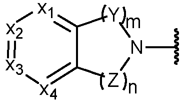 is 5-membered heteroaryl-fused 5-membered heterocycloalkyl, wherein the heteroatom in the 5-membered heteroaryl is N or S, and the number of heteroatoms is 1 or 2; preferably, the heteroatom in the 5-membered heterocycloalkyl is N, and the number of heteroatoms is 1, for example,

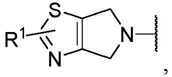

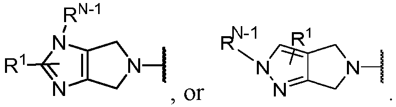

In some embodiments, 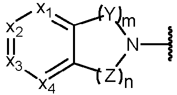 is 5-membered heteroaryl-fused 5-membered heterocycloalkyl, wherein the heteroatom in the 5-membered heteroaryl is N and/or S, and the number of heteroatoms is 1 or 2; preferably, the heteroatom in the 5-membered heterocycloalkyl is N, and the number of heteroatoms is 1, for example,

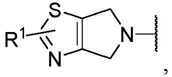

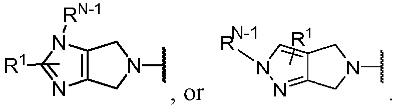

In some embodiments, 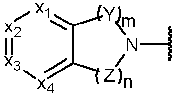 is 5-membered heteroaryl-fused 6-membered heterocycloalkyl, wherein the heteroatom in the 5-membered heteroaryl is N, and the number of heteroatoms is 1 or 2; preferably, the heteroatom in the 6-membered heterocycloalkyl is N, and the number of heteroatoms is 1, for example, 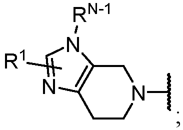 for another example,

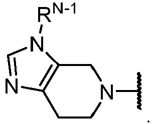

In some embodiments, 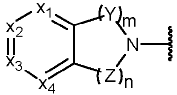 is 5-membered heteroaryl-fused 6-membered heterocycloalkyl, wherein the heteroatom in the 5-membered heteroaryl is N, and the number of heteroatoms is 1; preferably, the heteroatom in the 6-membered heterocycloalkyl is N, and the number of heteroatoms is 1, for example, 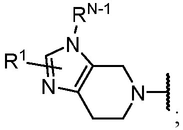 for another example,

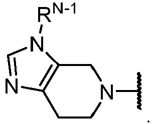

In some embodiments, 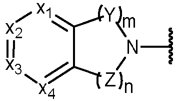 is 5-membered heteroaryl-fused 6-membered heterocycloalkyl, wherein the heteroatom in the 5-membered heteroaryl is N, and the number of heteroatoms is 1; preferably, the heteroatom in the 6-membered heterocycloalkyl is N, and the number of heteroatoms is 1, for example,

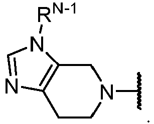

In some embodiments, in 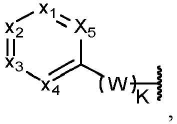 X₁, X₂, X₃, X₄ and X₅ are independently -CR¹-, N, S, 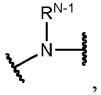 or a chemical bond, and the number of heteroatoms in X₁, X₂, X₃, X₄, and X₅ is 0, 1, or 2.

In some embodiments, 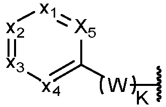 is C-(W)ₖ-,wherein C is 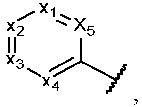 C is 5-membered heteroaryl, 6-membered heteroaryl, or phenyl; preferably, the heteroatom in the 5-membered heteroaryl is N, and the number of heteroatoms is 1 or 2, or the heteroatom in the 6-membered heteroaryl is N, and the number of heteroatoms is 1; for example, the C is pyridyl, phenyl, or

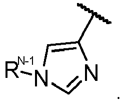

In some embodiments, in 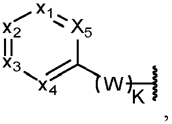 W is -O-, -CH₂-, or -NH-.

In some embodiments, in B, the 5- to 12-membered heteroaryl is preferably 5- to 6-membered heteroaryl, phenyl-fused 5- to 6-membered heteroaryl, 5- to 7-membered cycloalkyl-fused phenyl, 5- to 7-membered cycloalkyl-fused 5- to 6-membered heteroaryl, 5-to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused 5- to 6-membered aryl; further preferably 5- to 6-membered heteroaryl, phenyl-fused 5-to 6-membered heteroaryl, 5- to 6-membered heteroaryl-fused 5- to 6-membered heteroaryl, 5-to 7-membered heterocycloalkyl-fused 5- to 6-membered heteroaryl, or 5- to 7-membered heterocycloalkyl-fused phenyl.

In some embodiments, in B, the heteroatom in the 4- to 6-membered heterocycloalkyl is N, and the number of heteroatoms is 1, for example, 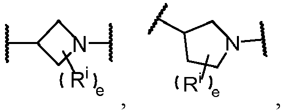 or 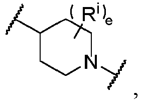 wherein e is independently 0, 1, 2, or 3; for another example,

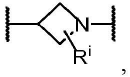

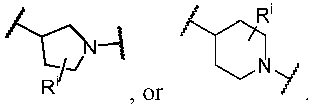

In some embodiments, in B, the heteroatom in the 4- to 6-membered heterocycloalkyl is N, and the number of heteroatoms is 1; for example, 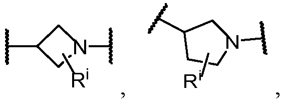 or

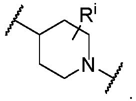

In some embodiments, B is 4- to 6-membered heterocycloalkyl substituted by two Rⁱ; two Rⁱ on the same atom together with the atom to which they are attached form a 3- to 7-membered cycloalkyl group, and the 3- to 7-membered cycloalkyl is preferably 3- to 6-membered cycloalkyl; for example, B is 4-membered heterocycloalkyl or 5-membered heterocycloalkyl, and two Rⁱ form a 3- to 4-membered cycloalkyl group; for another example, B is

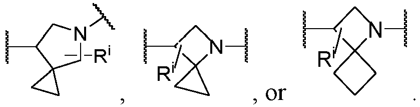

In some embodiments, B is 4- to 6-membered heterocycloalkyl substituted by two Rⁱ; two Rⁱ on the same atom together with the atom to which they are attached form a 3- to 7-membered cycloalkyl group, and the 3- to 7-membered cycloalkyl is preferably 3- to 6-membered cycloalkyl; for example, B is 4-membered heterocycloalkyl, and two Rⁱ form a 3-to 4-membered cycloalkyl group; for another example, B is

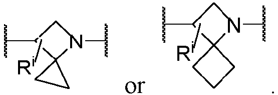

In some embodiments, B is 4- to 6-membered heterocycloalkyl substituted by two Rⁱ; two Rⁱ on the same atom together with the atom to which they are attached form a 3- to 7-membered cycloalkyl group, and the 3- to 7-membered cycloalkyl is preferably 3- to 6-membered cycloalkyl; for example, B is 5-membered heterocycloalkyl, and two Rⁱ form a 3-to 4-membered cycloalkyl group; for another example, B is

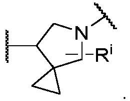

In some embodiments, B is 4- to 6-membered heterocycloalkyl substituted by two Rⁱ at adjacent positions; two adjacent Rⁱ together with the atom to which they are attached form a 3- to 7-membered cycloalkyl group (wherein the 3- to 7-membered cycloalkyl forms a fused ring with B), and the 3- to 7-membered cycloalkyl is preferably 3- to 6-membered cycloalkyl; for example, B is 5-membered heterocycloalkyl, and two Rⁱ together with the atom to which they are attached form a 3-membered cycloalkyl group; for another example, B is

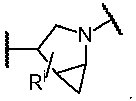

In some embodiments, B is 5-membered heteroaryl, wherein the heteroatom in the 5-membered heteroaryl is N and/or S, and the number of heteroatoms is 1 or 2, for example,

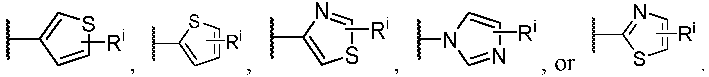

In some embodiments, B is 5-membered heteroaryl, wherein the heteroatom in the 5-membered heteroaryl is N or S, and the number of heteroatoms is 1 or 2, for example,

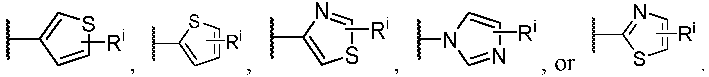

In some embodiments, B is 6-membered heteroaryl, wherein the heteroatom in the 6-membered heteroaryl is N, and the number of heteroatoms is 1 or 2, for example,

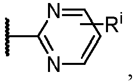

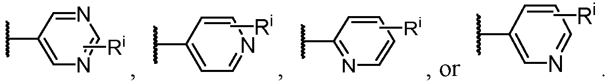

In some embodiments, in B, the 6- to 10-membered aryl, for example, phenyl.

In some embodiments, B is phenyl-fused 5-membered heterocycloalkyl, wherein the heteroatom in the 5-membered heterocycloalkyl is N, and the number of heteroatoms is 1, for example, 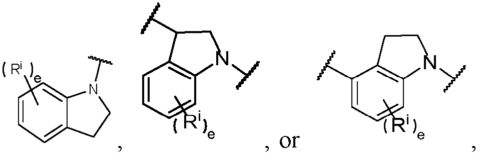 wherein e is independently 0, 1, 2, or 3; for another example,

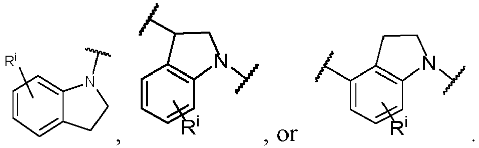

In some embodiments, B is phenyl-fused 5-membered heterocycloalkyl, wherein the heteroatom in the 5-membered heterocycloalkyl is N, and the number of heteroatoms is 1, for example,

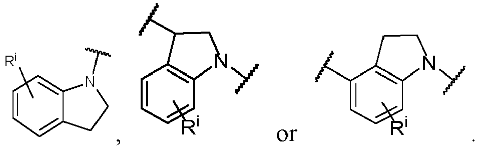

In some embodiments, B is phenyl-fused 6-membered heteroaryl, wherein the heteroatom in the 6-membered heteroaryl is N, and the number of heteroatoms is 1 or 2, for example,

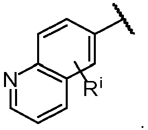

In some embodiments, B is phenyl-fused 6-membered heterocycloalkyl, wherein the heteroatom in the 6-membered heterocycloalkyl is N and/or O, and the number of heteroatoms is 1 or 2, for example, 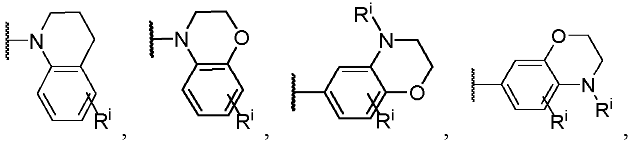 or 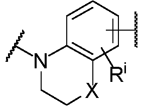 X = O, CH2

In some embodiments, B is phenyl-fused 6-membered heterocycloalkyl, wherein the heteroatom in the 6-membered heterocycloalkyl is N or O, and the number of heteroatoms is 1 or 2, for example, 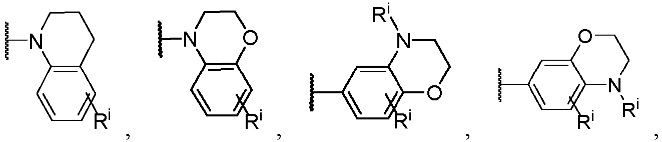 or 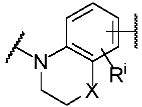 X = O, CH2

In some embodiments, B is 6-membered heteroaryl-fused 5-membered heteroaryl, wherein the heteroatom in the 6-membered heteroaryl is N, and the number of heteroatoms is 1; preferably, the heteroatom in the 5-membered heteroaryl is N or S, and the number of heteroatoms is 1, for example,

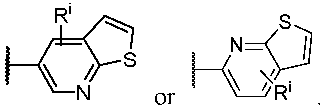

In some embodiments, B is 6-membered heteroaryl-fused 6-membered heterocycloalkyl, wherein the heteroatom in the 6-membered heteroaryl is N, and the number of heteroatoms is 1; preferably, the heteroatom in the 6-membered heterocycloalkyl is O, and the number of heteroatoms is 1, for example,

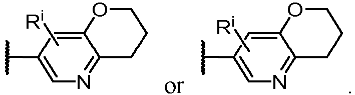

In some embodiments, in D, the 5- to 12-membered heteroaryl is preferably 5- to 6-membered heteroaryl, phenyl-fused 5- to 6-membered heteroaryl, 5- to 7-membered cycloalkyl-fused phenyl, 5- to 7-membered cycloalkyl-fused 5- to 6-membered heteroaryl, 5-to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused 5- to 6-membered aryl; further preferably 5- to 6-membered heteroaryl, phenyl-fused 5-to 6-membered heteroaryl, 5- to 7-membered cycloalkyl-fused 5- to 6-membered heteroaryl, 5- to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused phenyl.

In some embodiments, in D, the 5- to 12-membered heteroaryl is 5- to 6-membered heteroaryl, phenyl-fused 5- to 6-membered heteroaryl, 5- to 7-membered cycloalkyl-fused 5-to 6-membered heteroaryl, 5- to 6-membered heteroaryl-fused 5- to 6-membered heteroaryl, 5-to 7-membered heterocycloalkyl-fused 5- to 6-membered heteroaryl, or 5- to 7-membered heterocycloalkyl-fused phenyl, wherein the heteroatom in the 5- to 6-membered heteroaryl and 5- to 7-membered heterocycloalkyl is preferably selected from one, two, or three kinds of N, O, and S, and the number of heteroatoms is preferably 1, 2, or 3.

In some embodiments, in D, the 5- to 6-membered heteroaryl is preferably 5-membered heteroaryl or 6-membered heteroaryl.

In some embodiments, D is hydrogen, methyl, ethyl, isopropyl, cyclopropyl, or trifluoromethyl.

In some embodiments, D is 6-membered heteroaryl, wherein the heteroatom in the 6-membered heteroaryl is N, and the number of heteroatoms is 1 or 2; for example, 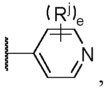 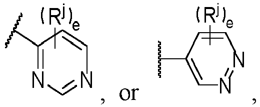 wherein e is independently 0, 1, 2, or 3; for another example,

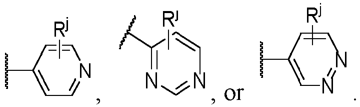

In some embodiments, D is 6-membered heteroaryl, wherein the heteroatom in the 6-membered heteroaryl is N, and the number of heteroatoms is 1 or 2, for example,

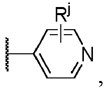

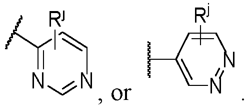

In some embodiments, D is 5-membered heteroaryl, wherein the heteroatom in the 5-membered heteroaryl is one or more kinds of N, O, and S, and the number of heteroatoms is 1, 2, or 3, for example,

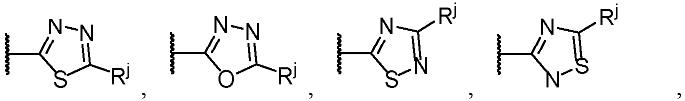

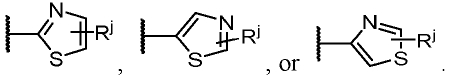

In some embodiments, D is 5-membered heteroaryl, wherein the heteroatom in the 5-membered heteroaryl is N and/or S, and the number of heteroatoms is 1, 2, or 3, for example,

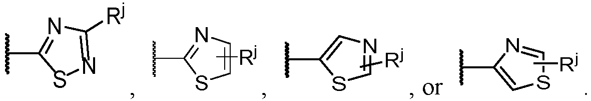

In some embodiments, D is 5-membered heteroaryl, wherein the heteroatom in the 5-membered heteroaryl is N or S, and the number of heteroatoms is 1, 2, or 3, for example,

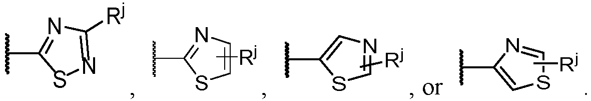

In some embodiments, D is 6-membered heteroaryl-fused 5-membered heterocycloalkyl, wherein the heteroatom in the 6-membered heteroaryl is N, and the number of heteroatoms is 1; preferably, the heteroatom in the 5-membered heterocycloalkyl is N or O, and the number of heteroatoms is 1, for example, 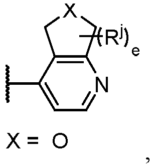 wherein e is independently 0, 1, 2, or 3; for another example,

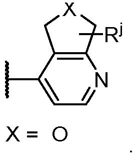

In some embodiments, D is 6-membered heteroaryl-fused 5-membered heterocycloalkyl, wherein the heteroatom in the 6-membered heteroaryl is N, and the number of heteroatoms is 1; preferably, the heteroatom in the 5-membered heterocycloalkyl is N or O, and the number of heteroatoms is 1, for example,

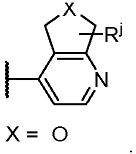

In some embodiments, D is 6-membered heteroaryl-fused 5-membered cycloalkyl, wherein the heteroatom in the 6-membered heteroaryl is N, and the number of heteroatoms is 1, for example, 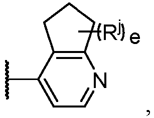 wherein e is independently 0, 1, 2, or 3; for another example,

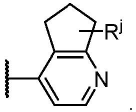

In some embodiments, D is 6-membered heteroaryl-fused 5-membered cycloalkyl, wherein the heteroatom in the 6-membered heteroaryl is N, and the number of heteroatoms is 1, for example,

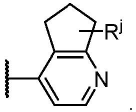

In some embodiments, D is phenyl-fused 5-membered heteroaryl, wherein the heteroatom in the 5-membered heteroaryl is N and/or S, and the number of heteroatoms is 2, for example, 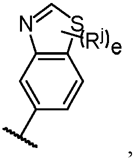 wherein e is independently 0, 1, 2, or 3; for another example,

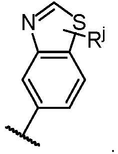

In some embodiments, D is 5- to 6-membered heteroaryl-fused 5- to 6-membered heteroaryl, wherein the heteroatom in the 5- to 6-membered heteroaryl is N and/or S, and the number of heteroatoms is 1, 2, or 3, for example, 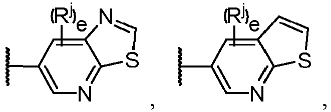 or 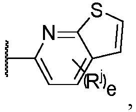 wherein e is independently 0, 1, 2, or 3.

In some embodiments, D is 5- to 6-membered heteroaryl-fused 5- to 6-membered heterocycloalkyl, wherein the heteroatom in the 5- to 6-membered heteroaryl is N, and the number of heteroatoms is 1, 2, or 3; preferably, the heteroatom in the 5- to 6-membered heterocycloalkyl is O, and the number of heteroatoms is 1, 2, or 3, for example, 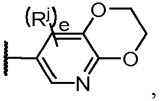 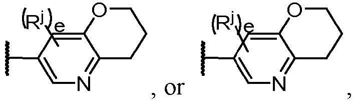 wherein e is independently 0, 1, 2, or 3.

In some embodiments, D is phenyl-fused 5- to 6-membered heterocycloalkyl, wherein the heteroatom in the 5- to 6-membered heterocycloalkyl is one or more kinds of O, N, and O, and the number of heteroatoms is 1, 2, or 3, for example, 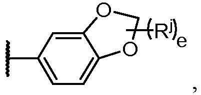 wherein e is independently 0, 1, 2, or 3.

In some embodiments, D is phenyl-fused 5- to 6-membered heteroaryl, wherein the heteroatom in the 5- to 6-membered heteroaryl is N, and the number of heteroatoms is 1, 2, or 3, for example, 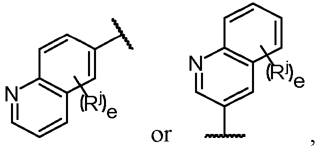 wherein e is independently 0, 1, 2, or 3.

In a certain embodiment, 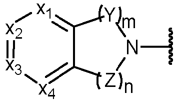 is preferably 6-membered heteroaryl-fused 5-membered heterocycloalkyl, wherein the heteroatom in the 6-membered heteroaryl is N, and the number of heteroatoms is 2, for example, 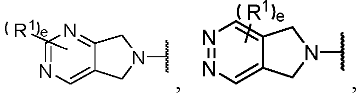 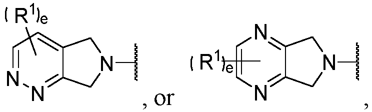 wherein e is independently 0, 1, or 2; for another example,

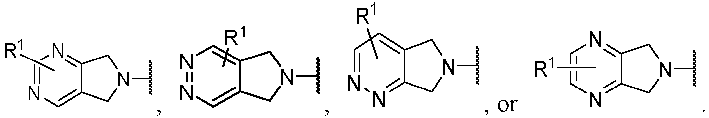

In a certain embodiment, 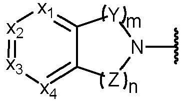 is preferably 6-membered heteroaryl-fused 5-membered heterocycloalkyl, wherein the heteroatom in the 6-membered heteroaryl is N, and the number of heteroatoms is 2, for example,

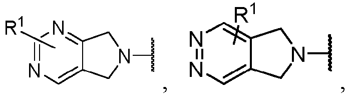

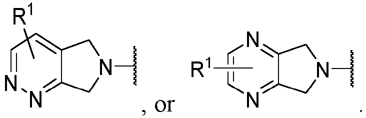

In a certain embodiment, 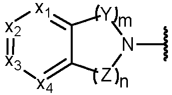 is preferably 6-membered heteroaryl-fused 7-membered heterocycloalkyl, wherein the heteroatom in the 6-membered heteroaryl is N, and the number of heteroatoms is 1 or 2, for example, 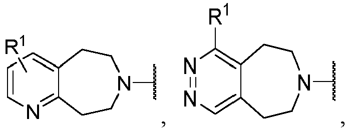 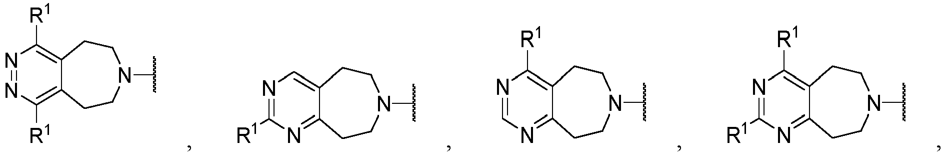 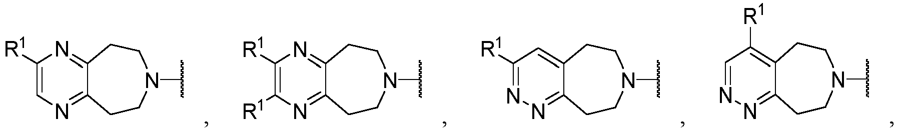 or

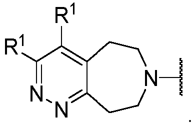

In a certain embodiment, in L, E is preferably carbonyl, F is a chemical bond, t is 0, and u is 1.

In a certain embodiment, B is preferably 4-membered heterocycloalkyl, wherein the heteroatom in the 4-membered heterocycloalkyl is N, and the number of heteroatoms is 1 or 2, for example,

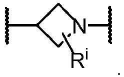

In a certain embodiment, D is preferably 6-membered heteroaryl, wherein the heteroatom in the 6-membered heteroaryl is N, and the number of heteroatoms is 1 or 2, for example,

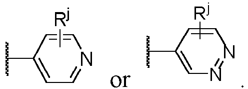

In a certain preferred embodiment, in the compound of formula I or the pharmaceutically acceptable salt thereof, 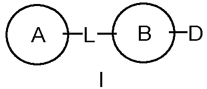 wherein 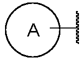 is 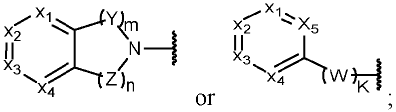
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, S, 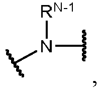 or a chemical bond;
R^{N-1} is hydrogen, C₁-C₆ alkyl, or 3- to 7-membered cycloalkyl;
Y and Z are independently carbonyl (CO) or -(CR²R³)ᵣ-; r is a natural number of 0 to 5;
each W is independently -O-, -(CR⁴R⁵)-*,* -NR⁶-, or a chemical bond;
each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a};
each R^{a} is independently halogen, hydroxyl, C₁-C₆ alkoxy, or - NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently hydroxyl, 3- to 7-membered cycloalkyl, or C₁-C₃ alkyl;
each R^{b-2} is independently halogen or C₁-C₆ alkyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
each R¹⁻⁴⁻¹ is independently halogen;
R² and R³ are independently hydrogen or NR²⁻¹R²⁻²;
R²⁻¹ and R²⁻² are independently hydrogen or C₁-C₆ alkyl;
R⁴, R⁵, and R⁶ are independently hydrogen or NR⁴⁻¹R⁴⁻²;
R⁴⁻¹ and R⁴⁻² are independently hydrogen;
L is 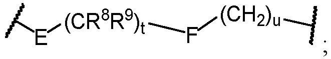
t is a natural number of 0 to 3;
u is a natural number of 0 to 3;
E is carbonyl, 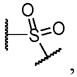 -NHCO-, or a chemical bond;
F is carbonyl, 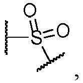 -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl; wherein the 4- to 6-membered heterocycloalkyl, 6- to 10-membered aryl, or 5- to 12-membered heteroaryl are optionally and independently substituted by 1, 2, or 3 Rⁱ;
each Rⁱ is independently hydrogen, halogen, hydroxyl, or C₁-C₃ alkyl; or two Rⁱ on the same atom together with the atom to which they are attached form a 3- to 7-membered cycloalkyl group; or two adjacent Rⁱ together with the atom to which they are attached form a 3- to 7-membered cycloalkyl group;
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently hydrogen, halogen, C₁-C₃ alkyl, 3- to 7-membered cycloalkyl, -OR¹²⁻³, or -SR¹²⁻⁴; the C₁-C₃ alkyl and 3- to 7-membered cycloalkyl are optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen;
each R^{k-1} is independently halogen;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a certain preferred embodiment, in the compound of formula I or the pharmaceutically acceptable salt thereof, 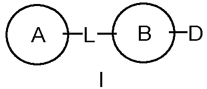 wherein 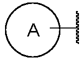 is 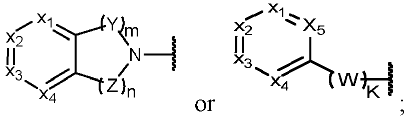
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, S, 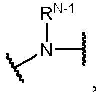 or a chemical bond;
R^{N-1} is C₁-C₆ alkyl;
Y and Z are independently carbonyl (CO) or -(CR²R³)ᵣ-; r is a natural number of 0 to 5;
each W is independently -(CR⁴R⁵)-*,* -NR⁶-, or a chemical bond;
each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a};
each R^{a} is independently hydroxyl, C₁-C₆ alkoxy, or -NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently 3- to 7-membered cycloalkyl;
each R^{b-2} is independently halogen or C₁-C₆ alkyl;
R¹⁻⁴ and R¹⁻⁵ are independently C₁-C₃ alkyl;
R² and R³ are independently hydrogen or NR²⁻¹R²⁻²;
R²⁻¹ and R²⁻² are independently hydrogen;
R⁴, R⁵, and R⁶ are independently hydrogen;
L is 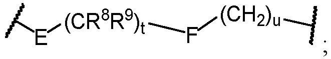
t is a natural number of 0 to 3;
u is a natural number of 0 to 3;
E is carbonyl, 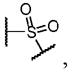 -NHCO-, or a chemical bond;
F is a chemical bond;
R⁸ and R⁹ are independently hydrogen or C₁-C₆ alkyl; or R⁸ and R⁹ together with the atom to which they are attached form a 3- to 7-membered cycloalkyl group;
B is 4- to 6-membered heterocycloalkyl, 6- to 10-membered aryl, or 5- to 12-membered heteroaryl; wherein the 4- to 6-membered heterocycloalkyl, 6- to 10-membered aryl, or 5- to 12-membered heteroaryl are optionally and independently substituted by 1, 2, or 3 Rⁱ;
each Rⁱ is independently hydrogen, halogen, hydroxyl, or C₁-C₃ alkyl; or two Rⁱ on the same atom together with the atom to which they are attached form a 3- to 7-membered cycloalkyl group;
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently hydrogen, halogen, C₁-C₃ alkyl, 3- to 7-membered cycloalkyl, -OR¹²⁻³, or -SR¹²⁻⁴; the C₁-C₃ alkyl and 3- to 7-membered cycloalkyl are optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen;
each R^{k-1} is independently halogen;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a certain preferred embodiment, in the compound of formula I or the pharmaceutically acceptable salt thereof, 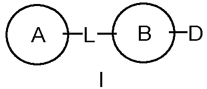 wherein 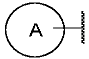 is 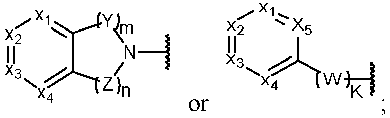
m is a natural number of 1 to 3;
n is a natural number of 1 to 3;
k is a natural number of 1 to 3;
X₁, X₂, X₃, X₄, and X₅ are independently -CR¹-, N, O, S, 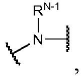 or a chemical bond, and the number of heteroatoms in X₁, X₂, X₃, X₄, and X₅ is 0, 1, 2, or 3;
R^{N-1} is hydrogen or C₁-C₆ alkyl;
Y and Z are independently carbonyl (CO) or -(CR²R³)ᵣ-; r is a natural number of 1 to 2;
each W is independently -O-, -(CR⁴R⁵)-, -NR⁶-, or a chemical bond;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl; wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
each R¹⁻⁴⁻¹ is independently halogen;
R¹⁻¹⁻¹ and R¹⁻²⁻¹ are independently hydrogen or C₁-C₃ alkyl;
R² and R³ are independently hydrogen or NR²⁻¹R²⁻²;
R²⁻¹ and R²⁻² are independently hydrogen or C₁-C₆ alkyl;
R⁴, R⁵, and R⁶ are independently hydrogen;
L is 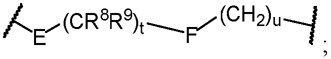
t is a natural number of 0 to 2;
u is a natural number of 0 to 2;
E is carbonyl, -NHCO-, or a chemical bond;
F is -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl; wherein the 5- to 12-membered heteroaryl, 6- to 10-membered aryl, and 4- to 6-membered heterocycloalkyl are optionally and independently substituted by 1, 2, or 3 Rⁱ;
each Rⁱ is independently hydrogen, hydroxyl, halogen, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl or 3- to 7-membered heterocycloalkyl forms a fused ring with B);
D is hydrogen, 3- to 7-membered cycloalkyl, C₁-C₆ alkyl, or 5- to 12-membered heteroaryl; wherein the C₁-C₆ alkyl or 5- to 12-membered heteroaryl are optionally and independently substituted by 1, 2, or 3 R^{j};
each R^{j} is independently halogen, C₁-C₃ alkyl, -OR¹²⁻³, or -SR¹²⁻⁴; the C₁-C₃ alkyl is optionally and independently substituted by 1, 2, or 3 R^{k}; each R^{k} is independently halogen;
R¹²⁻³ and R¹²⁻⁴ are independently C₁-C₃ alkyl; the C₁-C₃ alkyl is optionally and independently substituted by 1, 2, or 3 R^{k}; each R^{k} is independently halogen;
the heteroatom in the 3- to 7-membered heterocycloalkyl, 4- to 6-membered heterocycloalkyl, and 5- to 12-membered heteroaryl is N, O, or S, and the number of heteroatoms is 1 to 3; or, the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a certain preferred embodiment, in the compound of formula I or the pharmaceutically acceptable salt thereof, 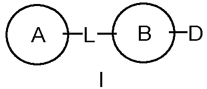 wherein 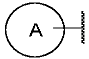 is 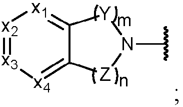
m is a natural number of 1 to 3;
n is a natural number of 1 to 3;
X₁, X₂, X₃, and X₄ are independently -CR¹-, N, S, 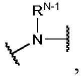 or a chemical bond, and the number of heteroatoms in X₁, X₂, X₃, X₄, and X₅ is 1 or 2;
R^{N-1} is C₁-C₆ alkyl;
Y and Z are independently carbonyl (CO) or -(CR²R³)ᵣ-; r is a natural number of 1 to 2;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl;
R¹⁻⁴⁻¹ is halogen;
R² and R³ are independently hydrogen;
L is 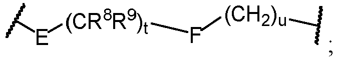
t is a natural number of 0 to 2;
u is a natural number of 0 to 2;
E is carbonyl or a chemical bond;
F is -O-, -NH-, or a chemical bond;
R⁸ and R⁹ are independently C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl or 5- to 12-membered heteroaryl, wherein the 5- to 12-membered heteroaryl and 4- to 6-membered heterocycloalkyl are optionally and independently substituted by 1, 2, or 3 Rⁱ;
each Rⁱ is independently hydrogen, hydroxyl, halogen, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl or 3- to 7-membered heterocycloalkyl forms a fused ring with B);
D is hydrogen, 3- to 7-membered cycloalkyl, C₁-C₆ alkyl, or 5- to 6-membered heteroaryl; wherein the C₁-C₆ alkyl and 5- to 6-membered heteroaryl are optionally and independently substituted by 1, 2, or 3 R^{j};
each R^{j} is independently halogen or C₁-C₃ alkyl; the C₁-C₃ alkyl is optionally and independently substituted by 1, 2, or 3 R^{k}; each R^{k} is independently halogen;
the heteroatom in the 3- to 7-membered heterocycloalkyl, 4- to 6-membered heterocycloalkyl, 5- to 6-membered heteroaryl, and 5- to 12-membered heteroaryl is N, O, or S, and the number of heteroatoms is 1 to 3; or, the heteroatom in the 3- to 7-membered heterocycloalkyl, 4- to 6-membered heterocycloalkyl, 5- to 6-membered heteroaryl, and 5- to 12-membered heteroaryl is one or more kinds of N, O, and S, and the number of heteroatoms is 1 to 3.

In a certain preferred embodiment, in the compound of formula I or the pharmaceutically acceptable salt thereof, 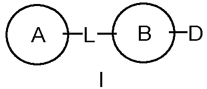 wherein 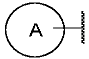 is 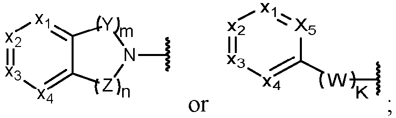
m is a natural number of 1 to 2;
n is a natural number of 1 to 2;
k is a natural number of 1 to 3;
X₁, X₂, X₃, X₄, and X₅ are independently -CR¹-, N, O, S, 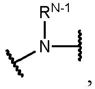 or a chemical bond, and the number of heteroatoms in X₁, X₂, X₃, X₄, and X₅ is 0, 1, 2, or 3;
R^{N-1} is C₁-C₆ alkyl;
each W is independently -(CR⁴R⁵)-, -O-, -NR⁶-, or a chemical bond; Y and Z are independently carbonyl (CO) or -(CR²R³)ᵣ-; r is a natural number of 1 to 2;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl;
each R¹⁻⁴⁻¹ is independently halogen;
R² and R³ are independently hydrogen or NR²⁻¹R²⁻²;
R²⁻¹ and R²⁻² are independently hydrogen or C₁-C₆ alkyl;
R⁴, R⁵, and R⁶ are independently hydrogen; L is 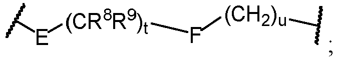
t is a natural number of 0 to 2;
u is a natural number of 0 to 2;
E is carbonyl, -NHCO-, or a chemical bond;
F is -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl; wherein the 5- to 12-membered heteroaryl, 6- to 10-membered aryl, and 4- to 6-membered heterocycloalkyl are optionally and independently substituted by 1, 2, or 3 Rⁱ;
each Rⁱ is independently hydrogen, hydroxyl, halogen, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl or 3- to 7-membered heterocycloalkyl forms a fused ring with B);
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently halogen, C₁-C₃ alkyl, OR¹²⁻³, or SR¹²⁻⁴; the C₁-C₃ alkyl is optionally and independently substituted by 1, 2, or 3 R^{k}; each R^{k} is independently halogen;
R¹²⁻³ and R¹²⁻⁴ are independently C₁-C₃ alkyl, wherein the C₁-C₃ alkyl is optionally and independently substituted by 1, 2, or 3 R^{k-1}; each R^{k-1} is independently halogen; the heteroatom in the 3- to 7-membered heterocycloalkyl, 4- to 6-membered heterocycloalkyl, and 5- to 12-membered heteroaryl is N, O, or S, and the number of heteroatoms is 1 to 3; or, the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a certain preferred embodiment, in the compound of formula I or the pharmaceutically acceptable salt thereof, 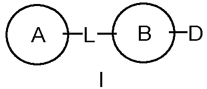 wherein 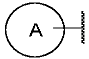 is 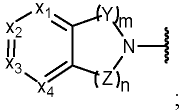
m is 1;
n is 1;
X₁, X₂, X₃, and X₄ are independently -CR¹- or N, and the number of heteroatoms in X₁, X₂, X₃, and X₄ is 2;
Y and Z are independently -(CR²R³)ᵣ-; r is 1;
each R¹ is independently C₁-C₆ alkyl (e.g., methyl) or C₁-C₆ alkoxy (e.g., methoxy);
R² and R³ are independently hydrogen;
L is 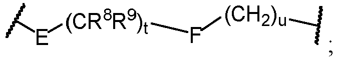
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen;
D is 5- to 12-membered heteroaryl; wherein the 5- to 12-membered heteroaryl is optionally and independently substituted by 1, 2, or 3 R^{j} (e.g., 1);
each R^{j} is independently -OR¹²⁻³;
R¹²⁻³ is C₁-C₃ alkyl (e.g., ethyl or isopropyl); the C₁-C₃ alkyl is optionally and independently substituted by 1, 2, or 3 R^{k}; each R^{k} is independently halogen (e.g., F);
the heteroatom in the 4- to 6-membered heterocycloalkyl and 5- to 12-membered heteroaryl is N, O, or S, and the number of heteroatoms is 1 to 3; or, the heteroatom in the 4-to 6-membered heterocycloalkyl and 5- to 12-membered heteroaryl is one or more kinds of N, O, and S, and the number of heteroatoms is 1 to 3;
when A is 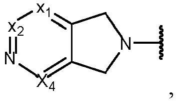 the number of heteroatoms in X₁, X₂, and X₄ is 1 or 2;
when A is 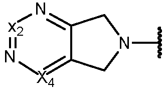 and L is carbonyl, then u is a natural number of 1 to 3, B is 4- to 6-membered heterocycloalkyl, and the 4- to 6-membered heterocycloalkyl is optionally and independently substituted by 1, 2, or 3 Rⁱ.

In a certain preferred embodiment, in the compound of formula I or the pharmaceutically acceptable salt thereof,
wherein 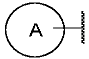 is 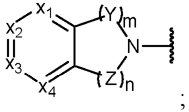
m is 1;
n is 1;
X₂ and X₃ are N;
X₁ and X₄ are independently -CR¹-;
Y and Z are independently -(CR²R³)ᵣ-; r is 1;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
R¹⁻¹ and R¹⁻² are independently hydrogen or C₁-C₆ alkyl; or R¹⁻¹ and R¹⁻² together with the atom to which they are attached form a 3- to 7-membered heterocycloalkyl group;
each R^{a} is independently hydroxyl, C₁-C₆ alkoxy, or NR¹⁻⁴R¹⁻⁵;
R¹⁻⁴ and R¹⁻⁵ are independently hydrogen or C₁-C₃ alkyl;
R² and R³ are independently hydrogen;
L is 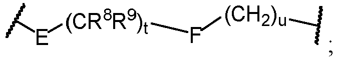
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen, halogen, hydroxyl, or C₁-C₃ alkyl;
D is 5- to 12-membered heteroaryl; wherein the 5- to 12-membered heteroaryl is optionally and independently substituted by 1, 2, or 3 R^{j}; preferably, D is 5- to 6-membered heteroaryl; wherein the 5- to 6-membered heteroaryl is optionally and independently substituted by 1, 2, or 3 R^{j}; the heteroatom in the 5- to 6-membered heteroaryl is selected from one, two, or three kinds of N, S, and O, and the number of heteroatoms is 1, 2, or 3;
each R^{j} is independently H, halogen, C₁-C₃ alkyl, OR¹²⁻³, or SR¹²⁻⁴; wherein the C₁-C₃ alkyl is optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen or C₁-C₃ alkyl;
each R^{k-1} is independently halogen or C₁-C₃ alkyl;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a certain preferred embodiment, in the compound of formula I or the pharmaceutically acceptable salt thereof,
wherein 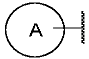 is 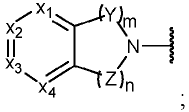
m is 1;
n is 1;
X₁ and X₃ are N;
X₂ and X₄ are independently -CR¹-;
Y and Z are independently -(CR²R³)ᵣ-; r is 1;
each R¹ is independently hydrogen, halogen, cyano, hydroxyl, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkyl, C₁-C₆ alkoxy, C₁-C₆ alkylthio are optionally and independently substituted by 1, 2, 3, or 4 R^{a};
R¹⁻¹ and R¹⁻² are independently hydrogen or C₁-C₆ alkyl; or R¹⁻¹ and R¹⁻² together with the atom to which they are attached form a 3- to 7-membered heterocycloalkyl group;
each R^{a} is independently hydroxyl, C₁-C₆ alkoxy, or NR¹⁻⁴R¹⁻⁵;
R¹⁻⁴ and R¹⁻⁵ are independently hydrogen or C₁-C₃ alkyl;
R² and R³ are independently hydrogen;
L is 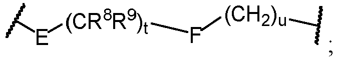
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen, halogen, hydroxyl, or C₁-C₃ alkyl; or two Rⁱ together with the atom to which they are attached form a 3- to 7-membered cycloalkyl group;
D is 5- to 12-membered heteroaryl; wherein the 5- to 12-membered heteroaryl is optionally and independently substituted by 1, 2, or 3 R^{j}; preferably, D is 5- to 6-membered heteroaryl; wherein the 5- to 6-membered heteroaryl is optionally and independently substituted by 1, 2, or 3 R^{j}; the heteroatom in the 5- to 6-membered heteroaryl is selected from one, two, or three kinds of N, S, and O, and the number of heteroatoms is 1, 2, or 3;
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻⁴, and R¹²⁻³ are independently C₁-C₃ alkyl; the C₁-C₃ alkyl is optionally and independently substituted by 1, 2, or 3 R^{k-1};
each R^{k} is independently halogen or C₁-C₃ alkyl;
each R^{k-1} is independently halogen or C₁-C₃ alkyl;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a certain embodiment, each R¹ is independently hydrogen, methyl, 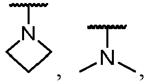 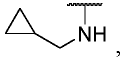 ethyl, chlorine, 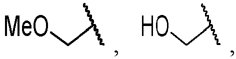 methoxy, 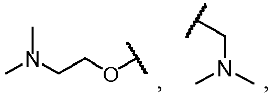 isopropyl, 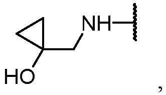 amino, 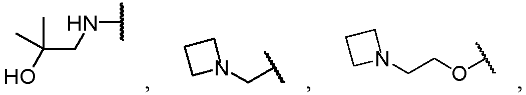 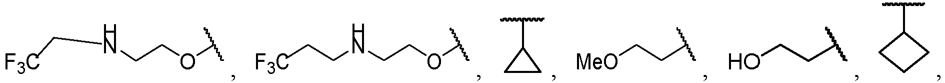 or

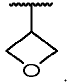

In a certain embodiment, each R¹ is independently hydrogen, methyl, 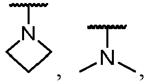 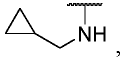 ethyl, chlorine, 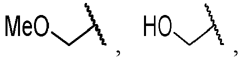 methoxy, 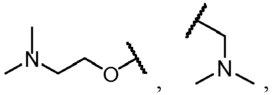 isopropyl, 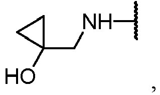 amino(-NH₂), 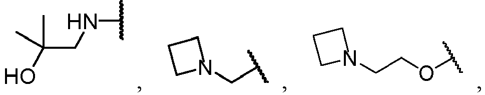 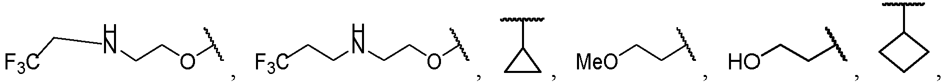 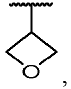 trifluoromethyl, 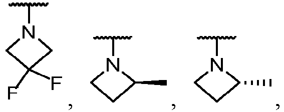 or methylthio.

Preferably, each R¹ is independently methyl,

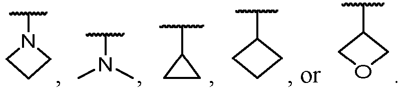

In a certain embodiment, 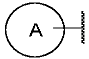 is

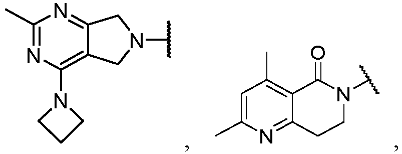

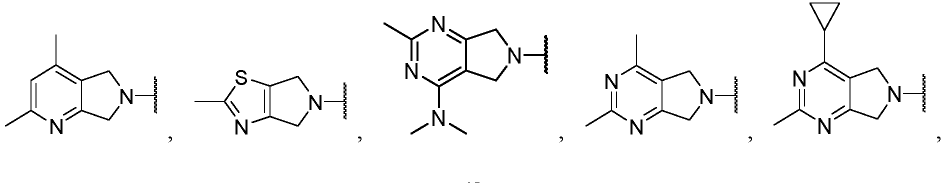

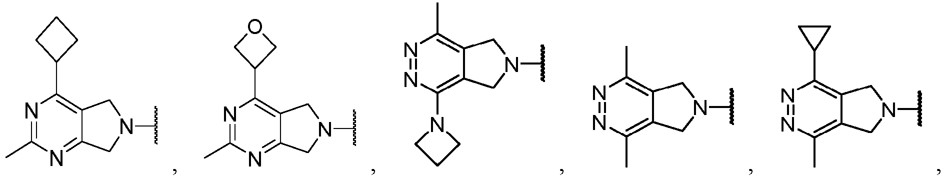

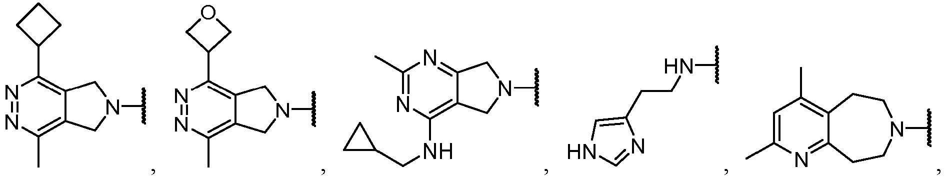

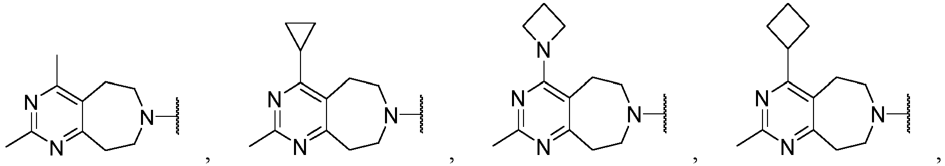

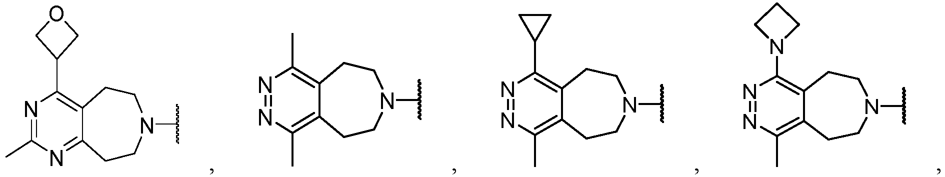

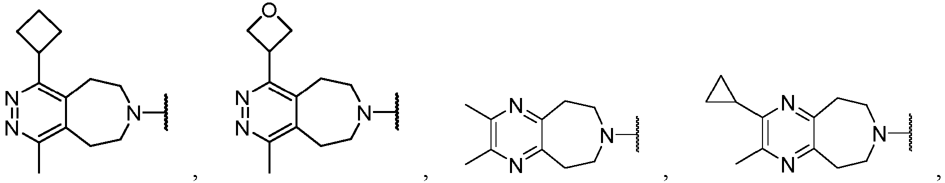

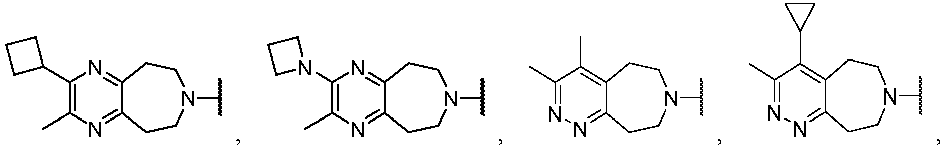

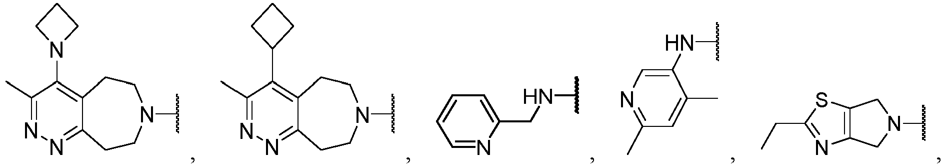

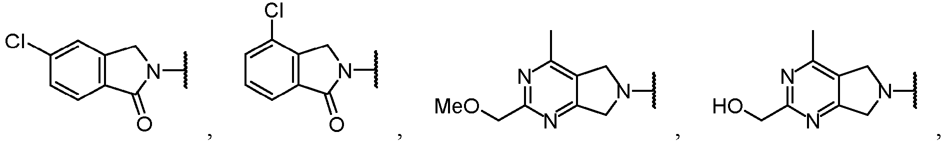

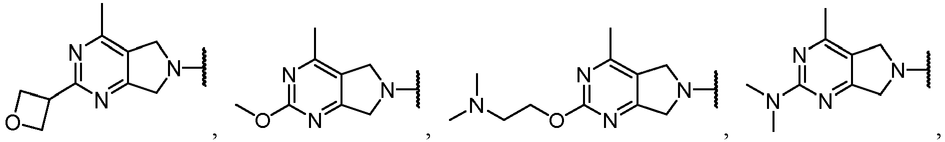

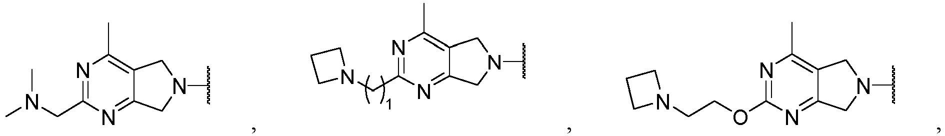

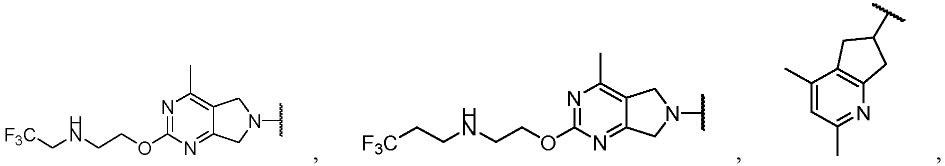

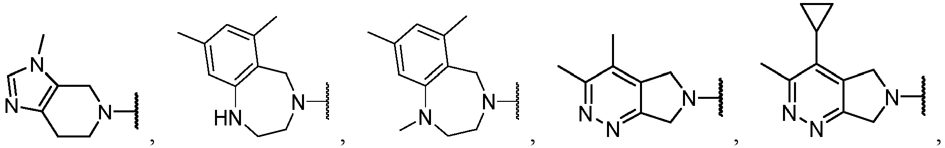

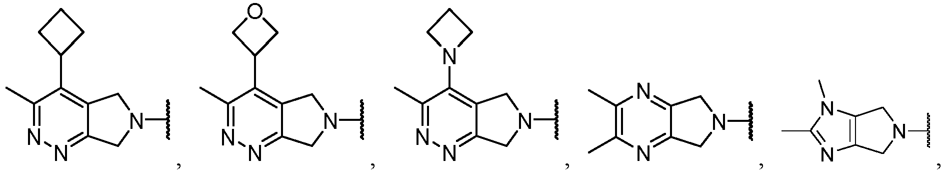

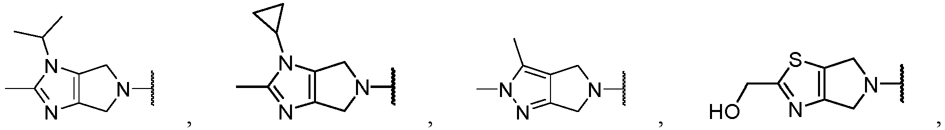

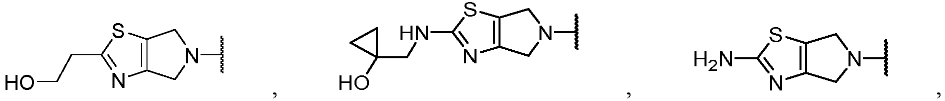

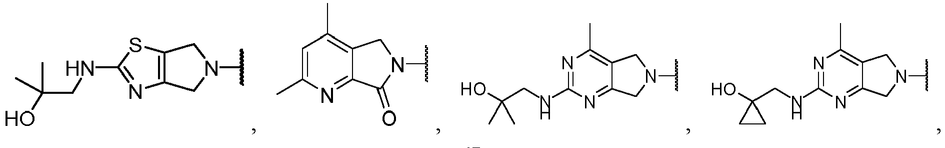

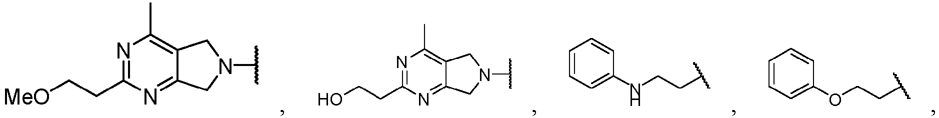

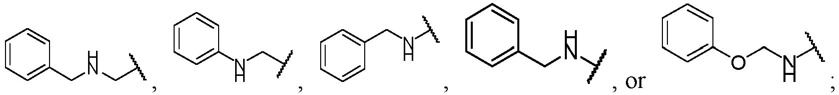

Preferably, 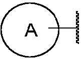 is

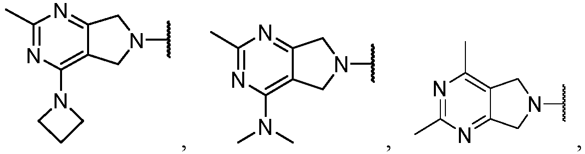

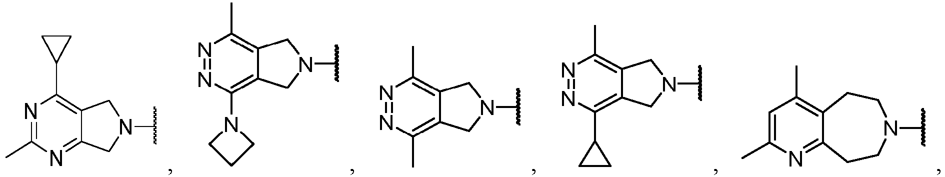

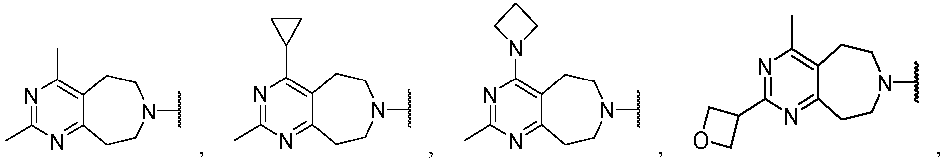

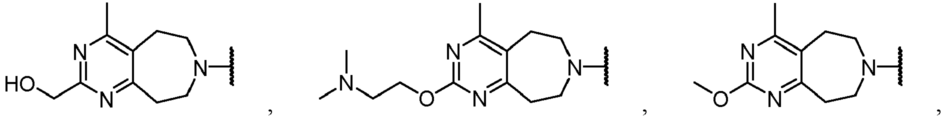

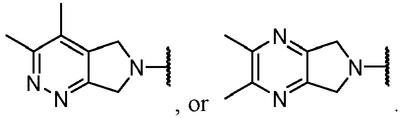

In a certain embodiment, 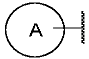 is 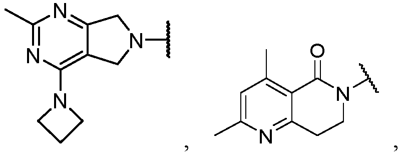 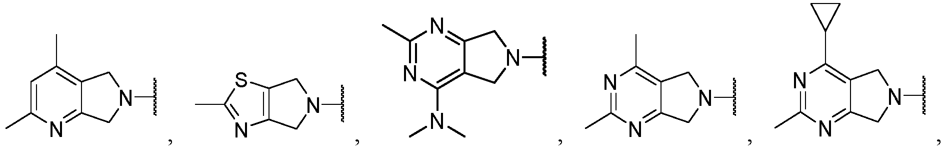 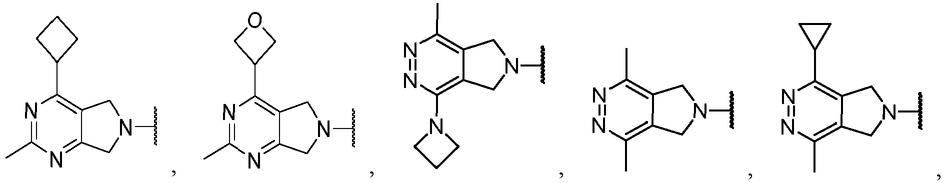 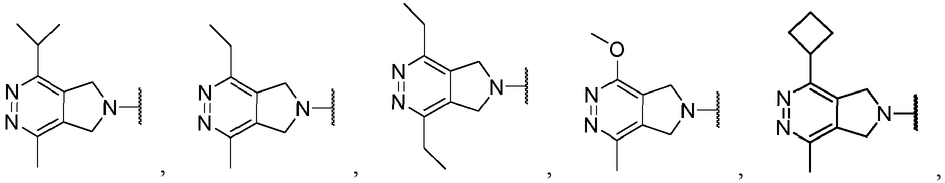 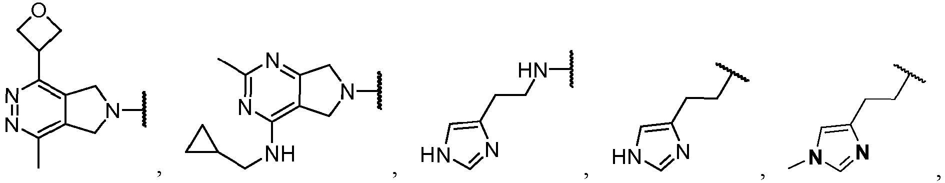 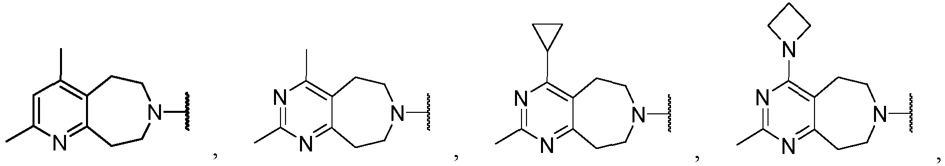 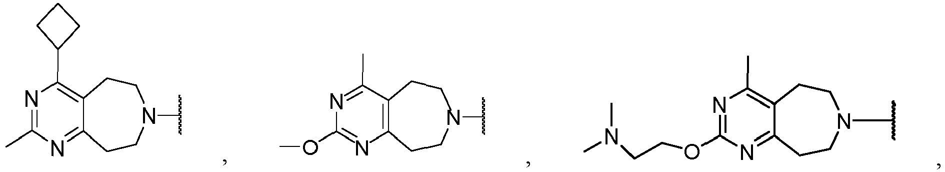 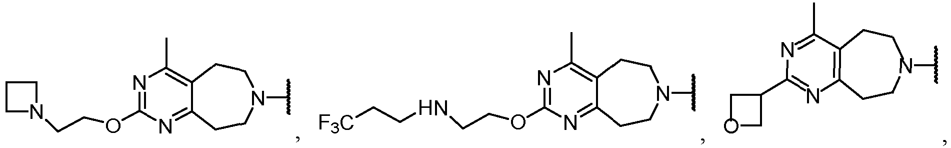 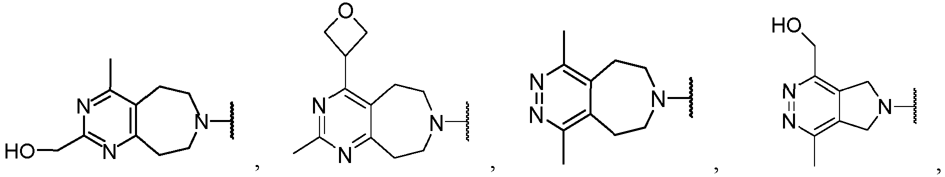 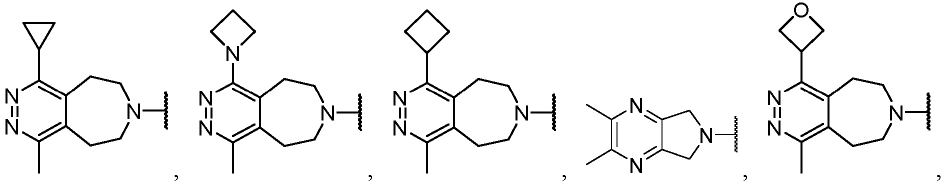 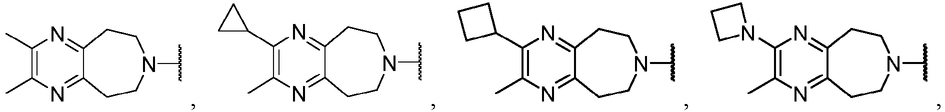 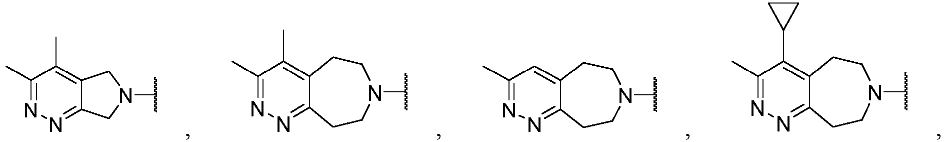 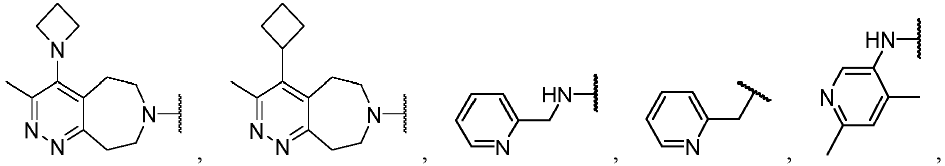 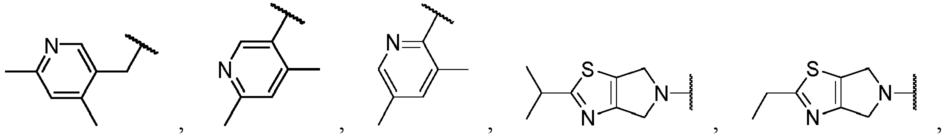 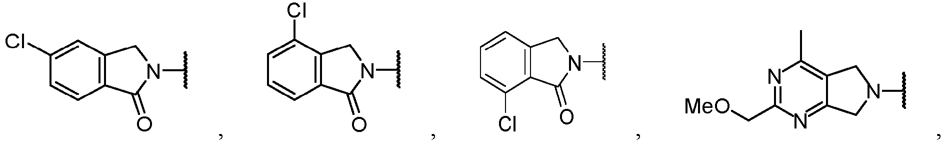 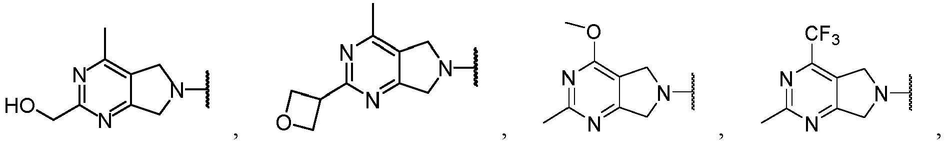 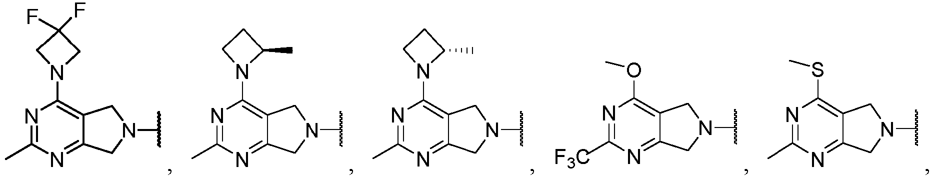 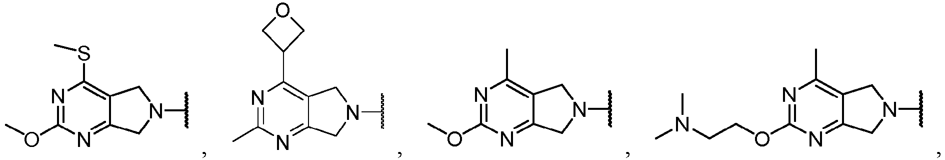 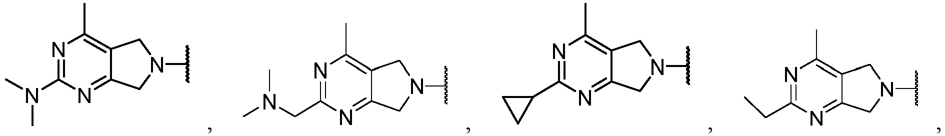 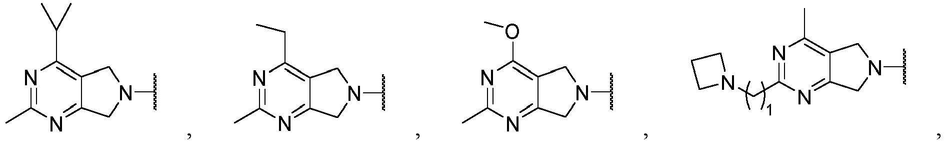 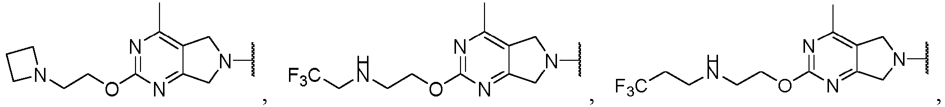 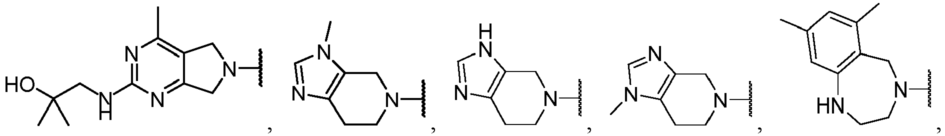 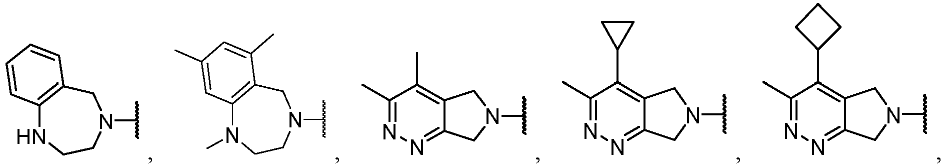 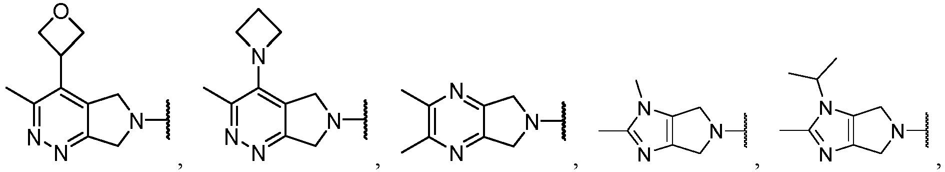 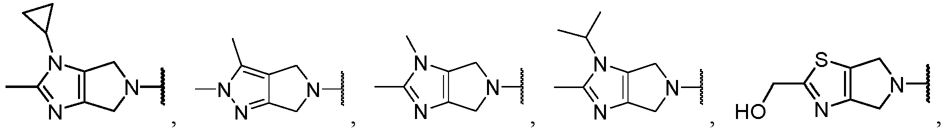 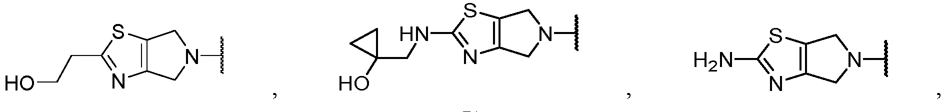 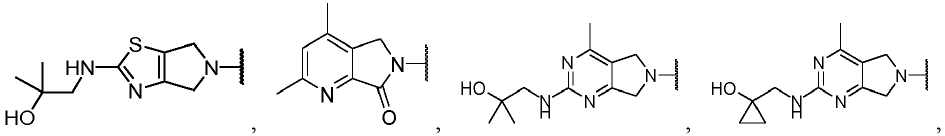 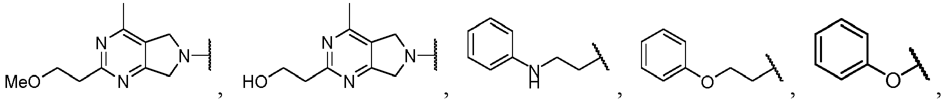 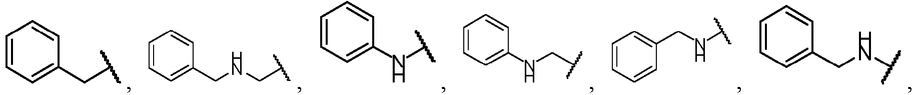 or

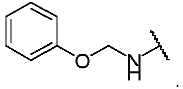

In a certain embodiment, L is -(CH₂)-, -(CH₂)₂-, 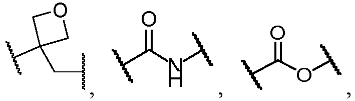 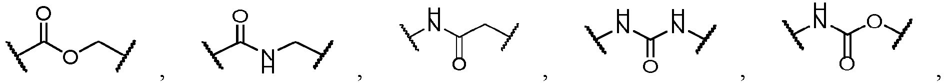 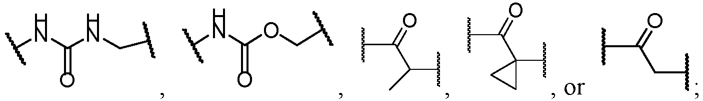 preferably, L is 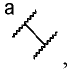 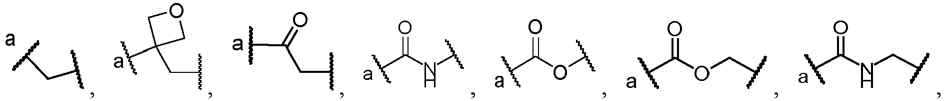 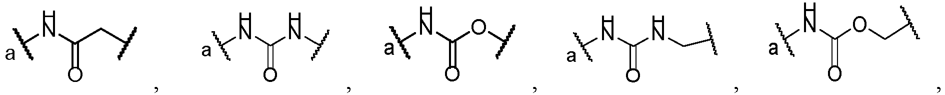 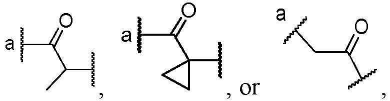 wherein " 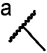 " represents the site connected to A.

Preferably, L is

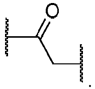

In a certain embodiment, L is -(CH₂)-, -(CH₂)₂-, 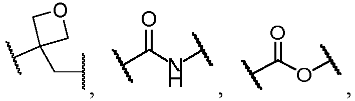 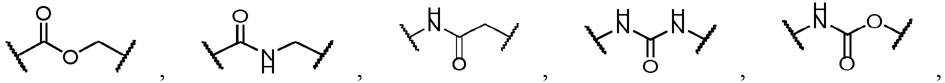 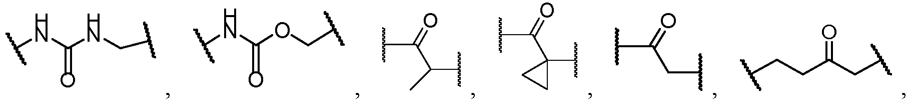 or 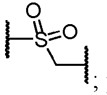 preferably, L is 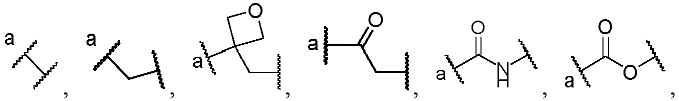 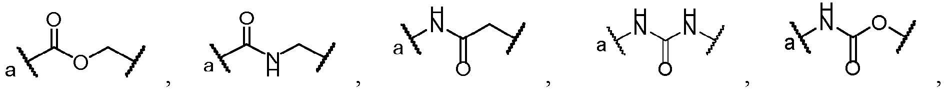 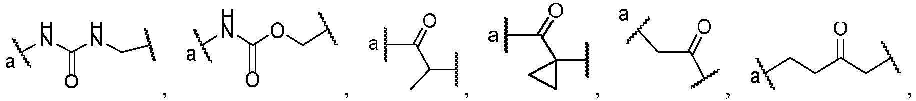 or 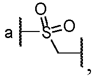 wherein " 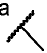 " represents the site connected to A.

In a certain embodiment, B is

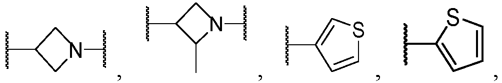

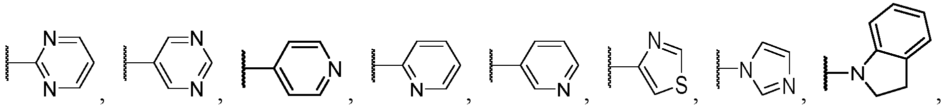

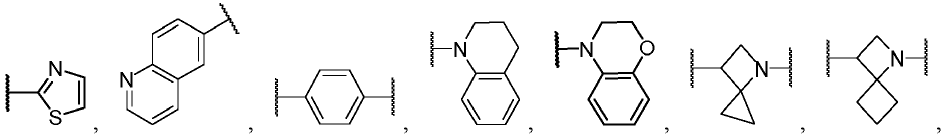

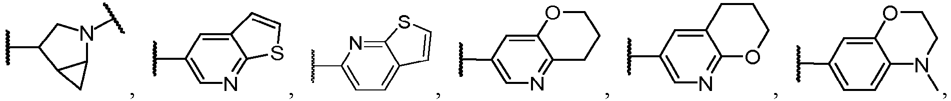

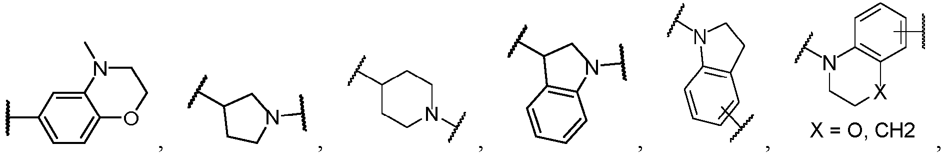

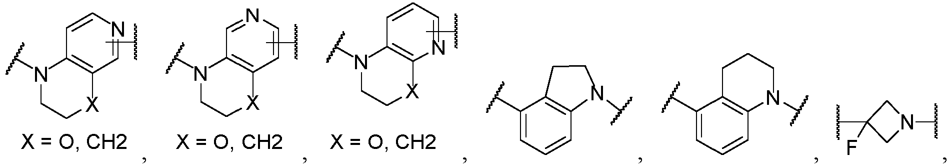

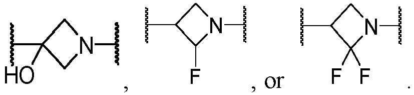

In a certain embodiment, B is

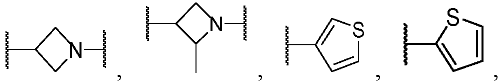

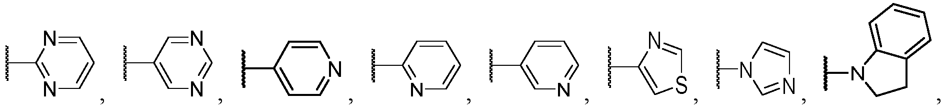

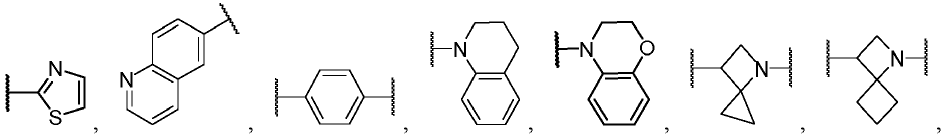

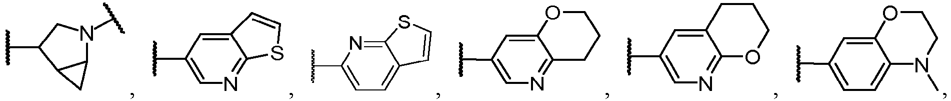

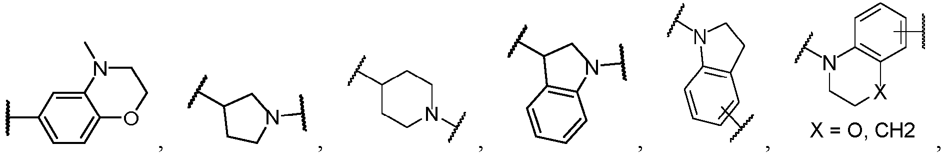

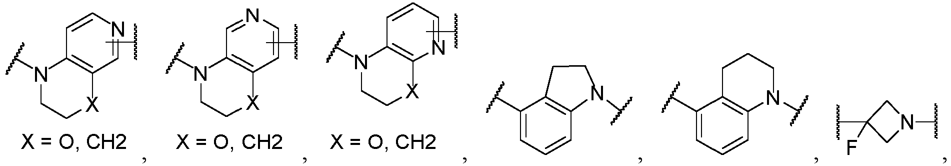

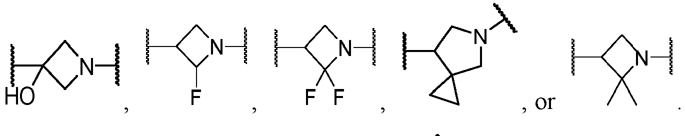

Preferably, B is

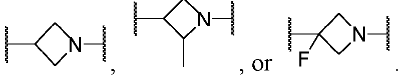

In a certain embodiment, B is 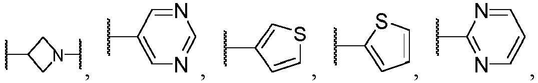 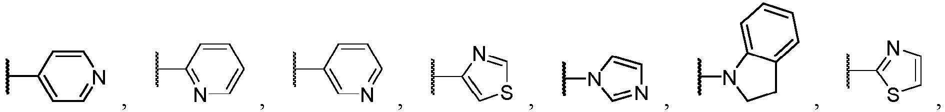 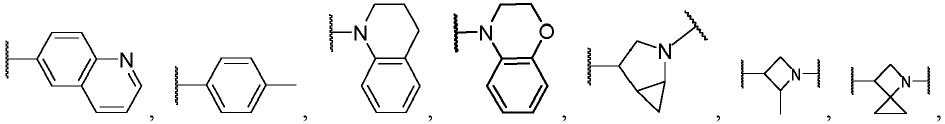 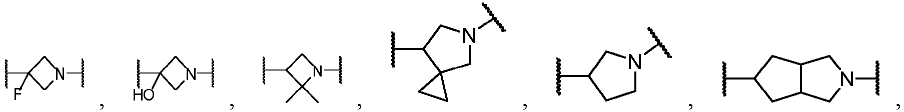 or

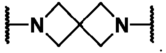

In a certain embodiment, D is hydrogen, methyl, 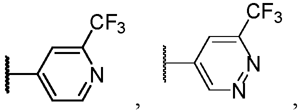 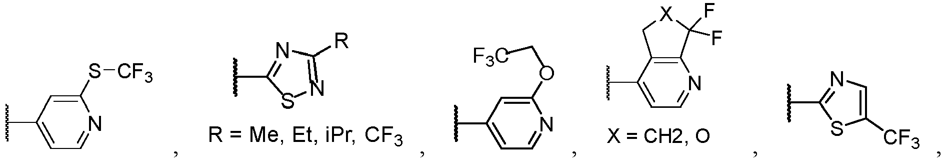 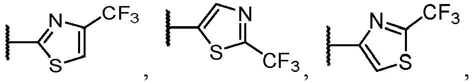 ethyl, isopropyl, cyclopropyl, trifluoromethyl, or

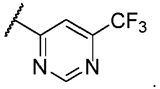

Preferably, D is

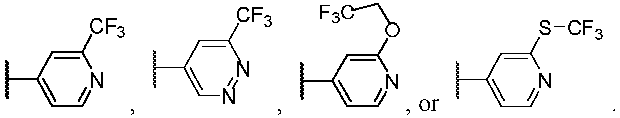

In a certain embodiment, D is hydrogen, methyl, 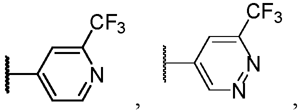 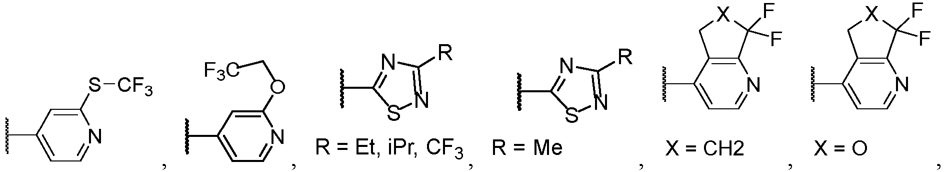 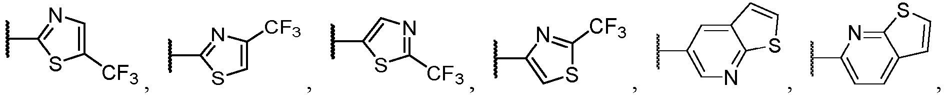 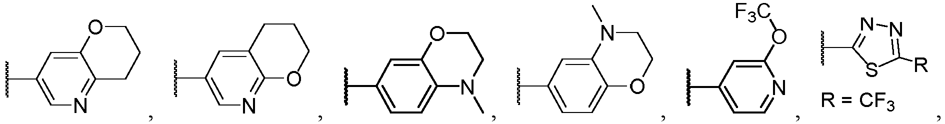 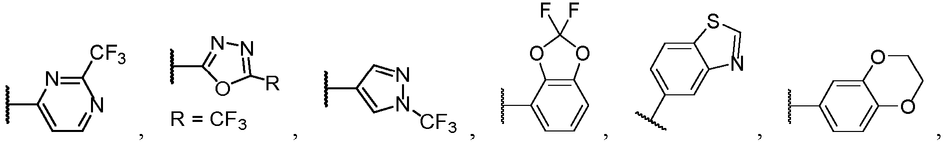 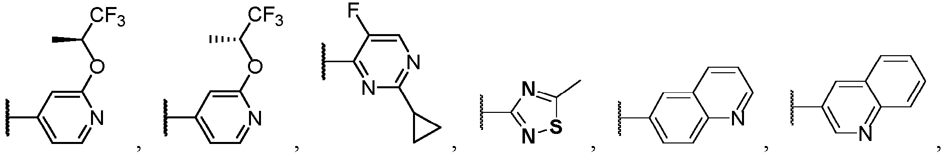 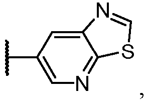 ethyl, isopropyl, cyclopropyl, trifluoromethyl, or

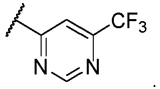

In a certain embodiment, the compound of formula I is any one of the following compounds:

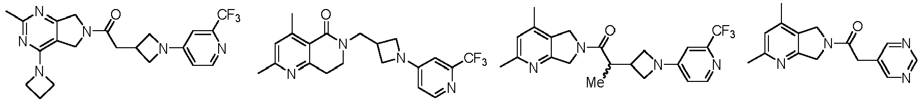

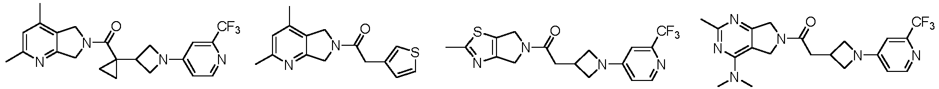

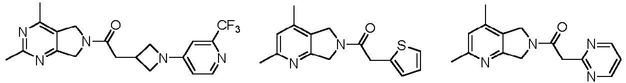

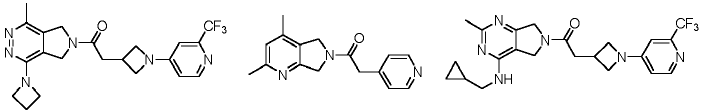

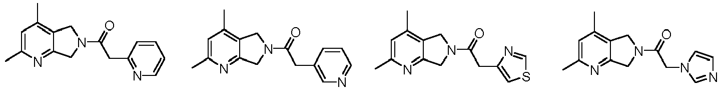

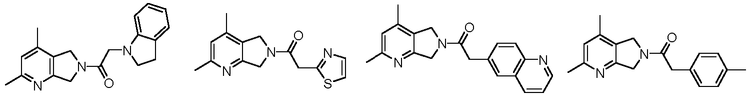

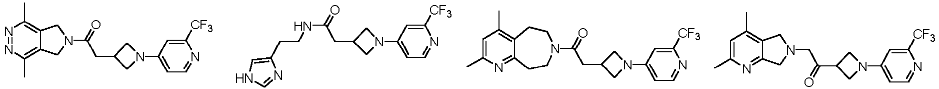

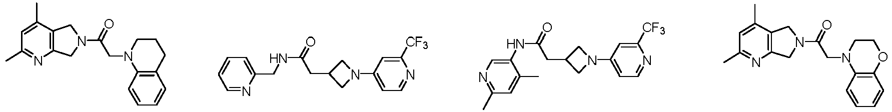

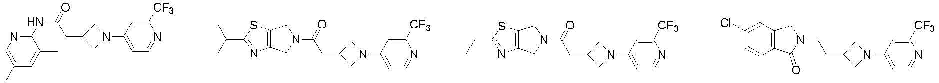

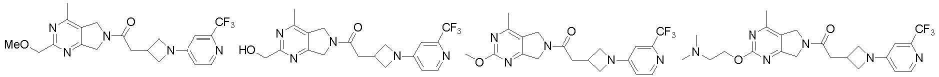

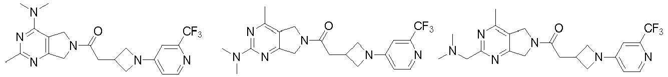

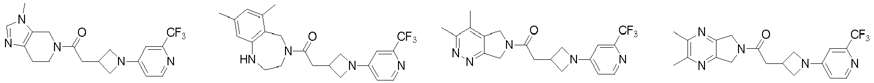

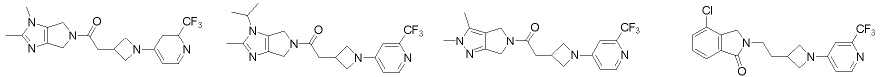

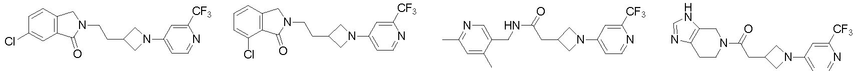

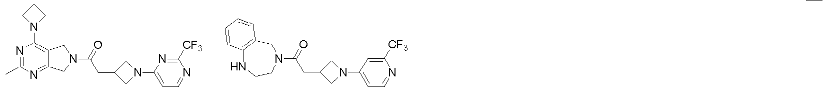

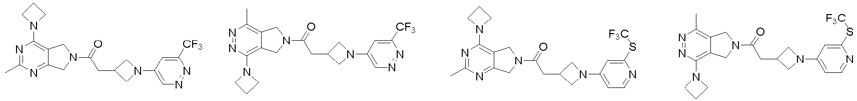

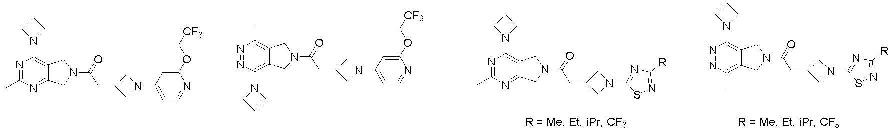

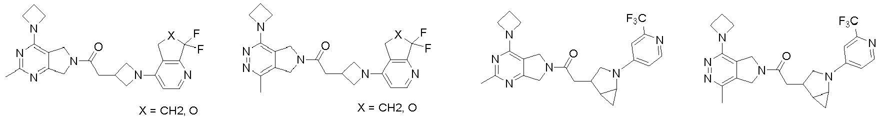

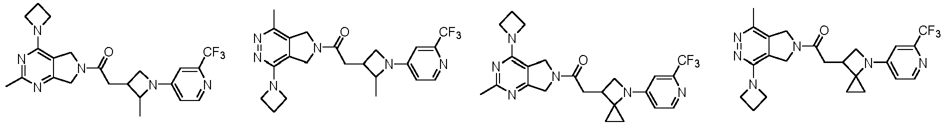

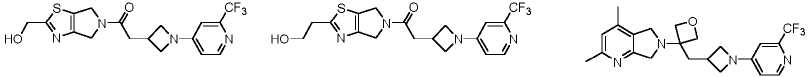

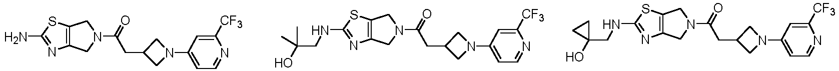

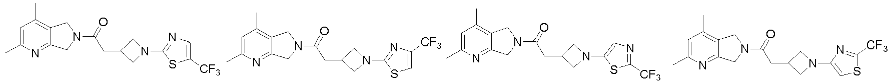

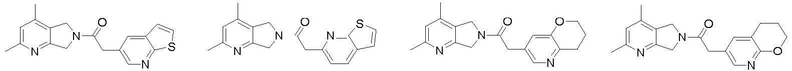

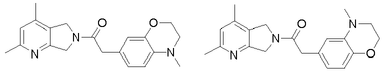

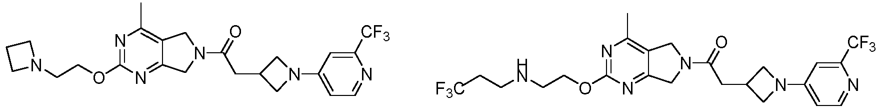

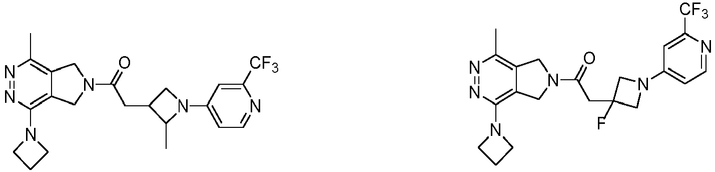

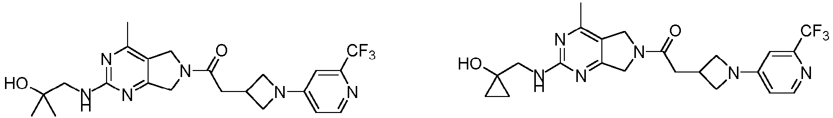

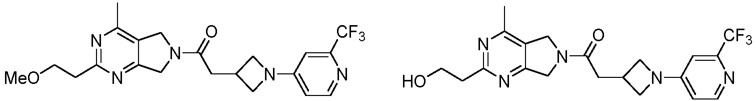

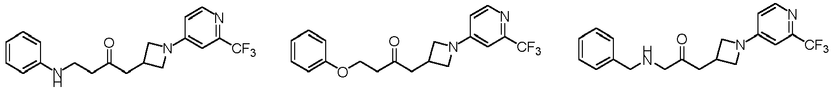

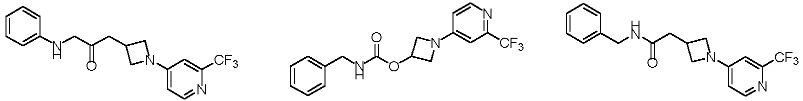

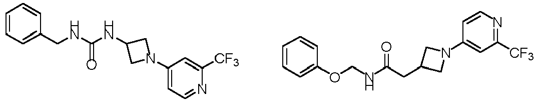

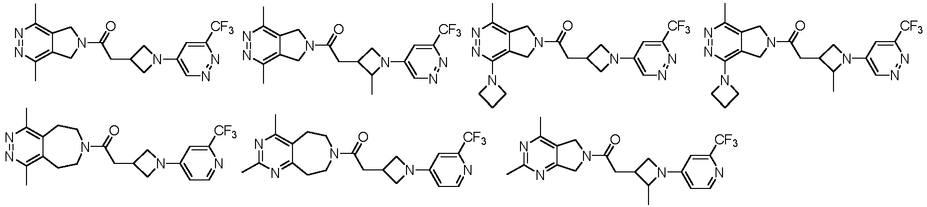

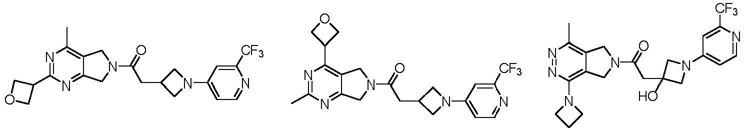

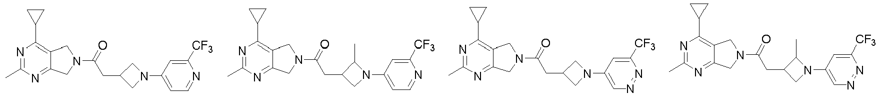

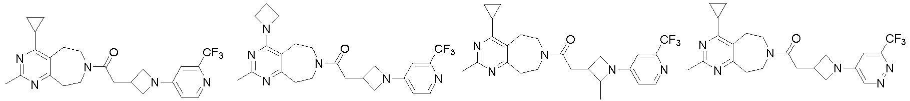

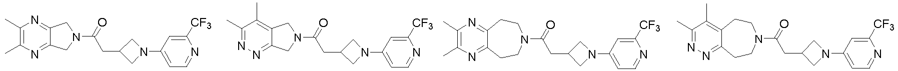

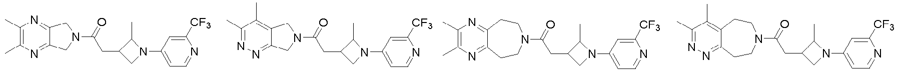

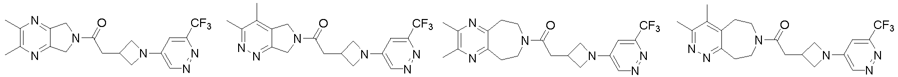

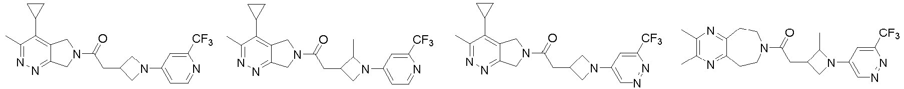

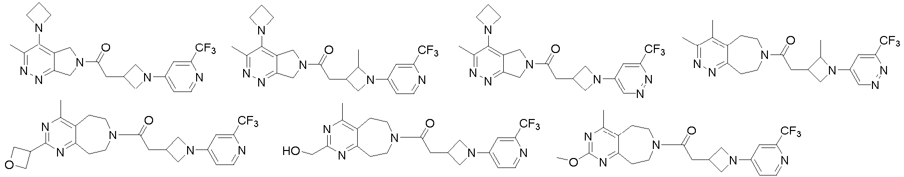

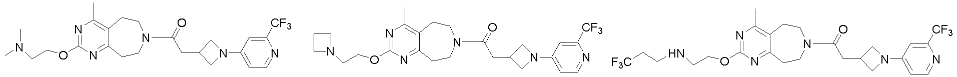

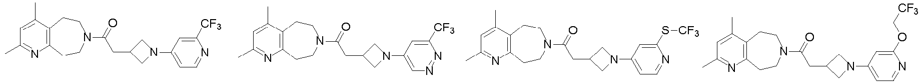

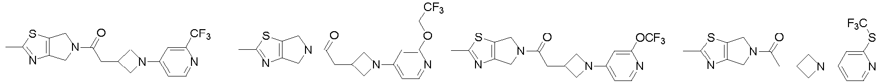

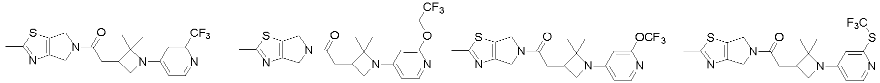

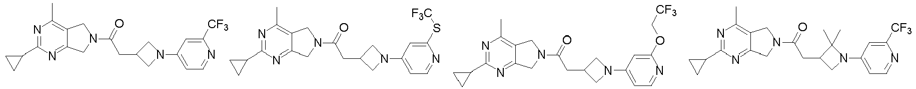

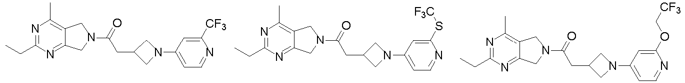

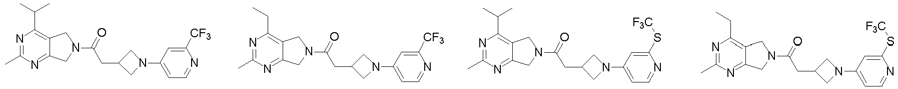

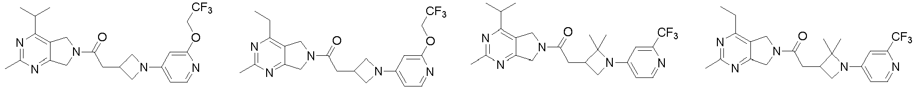

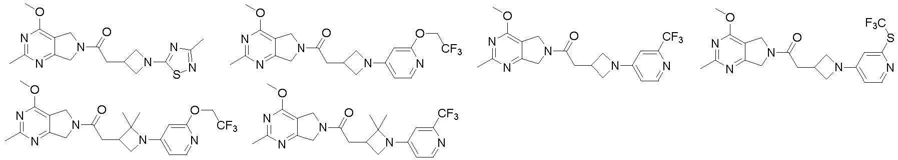

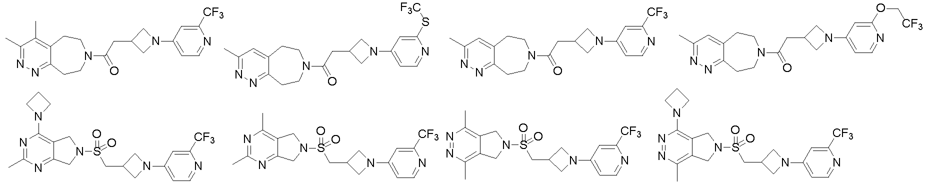

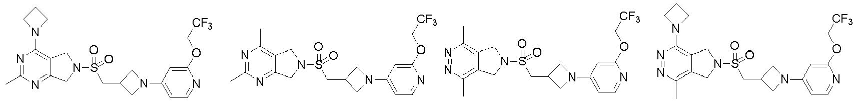

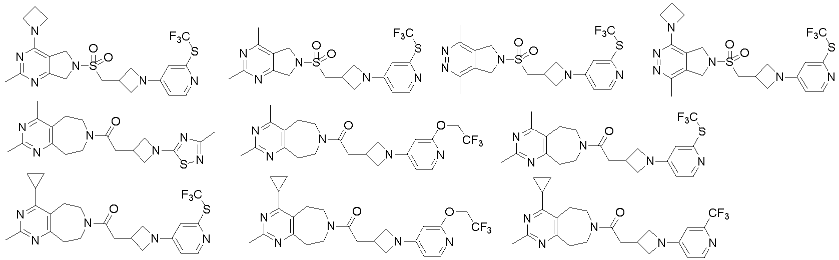

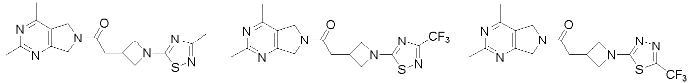

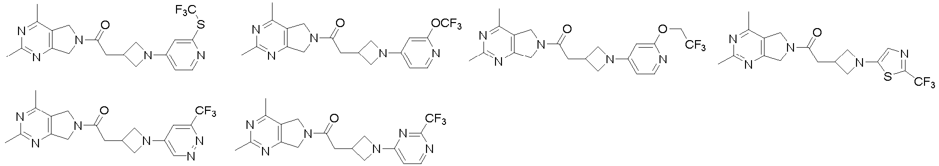

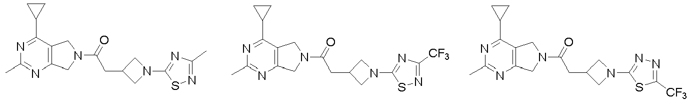

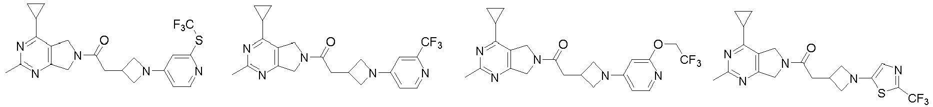

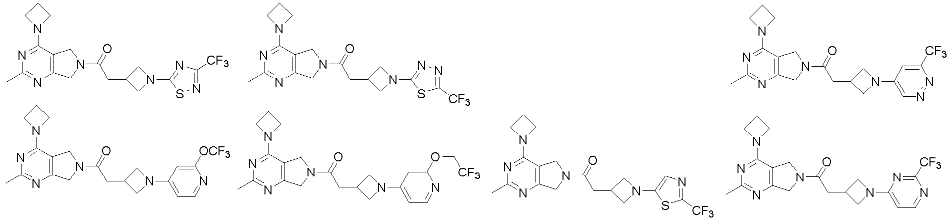

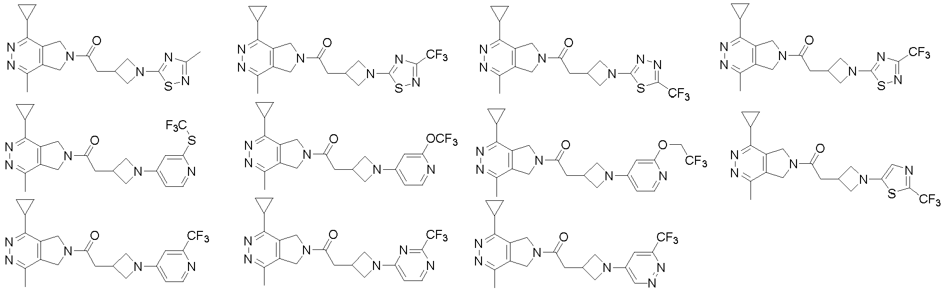

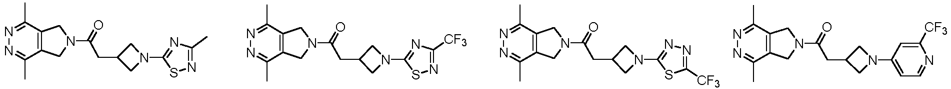

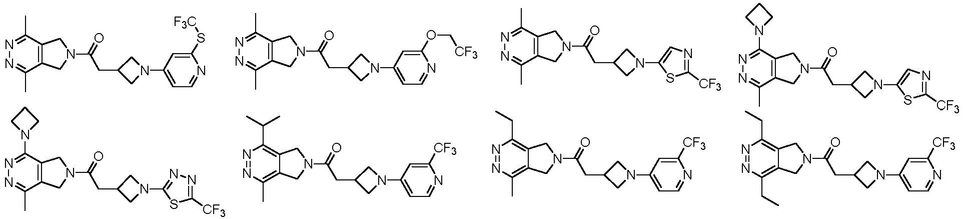

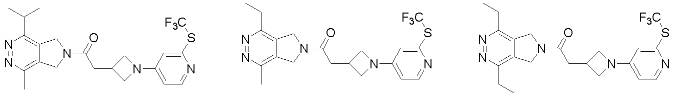

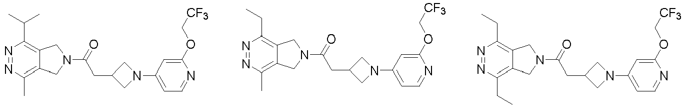

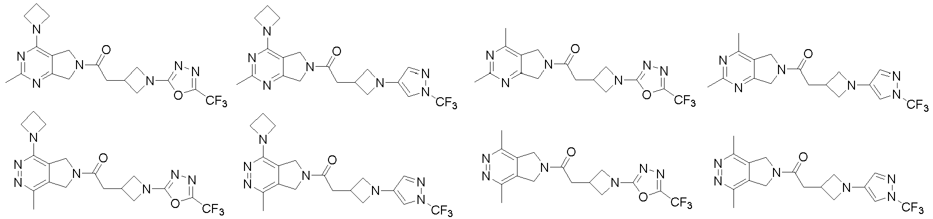

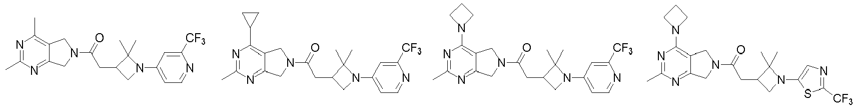

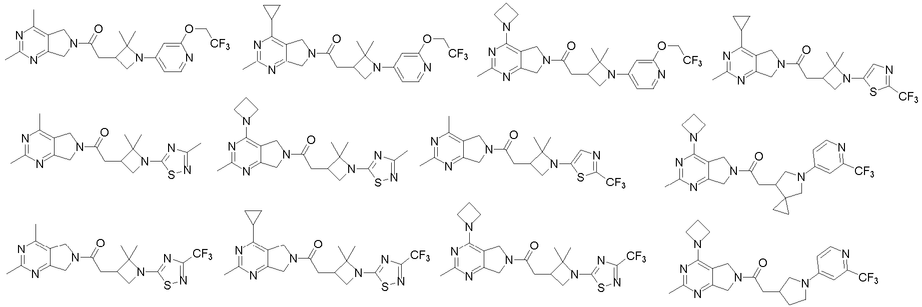

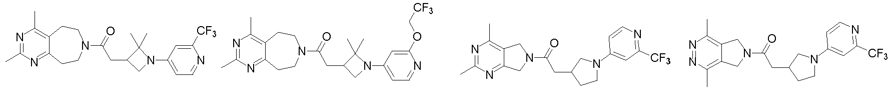

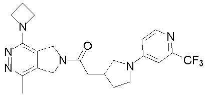

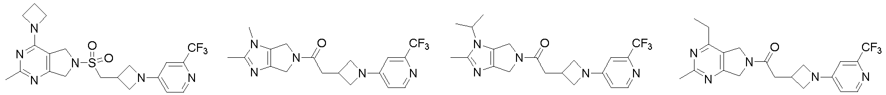

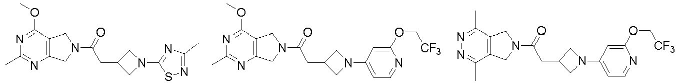

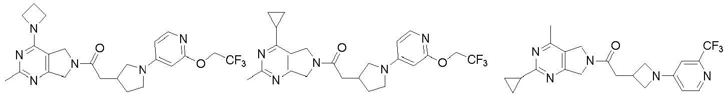

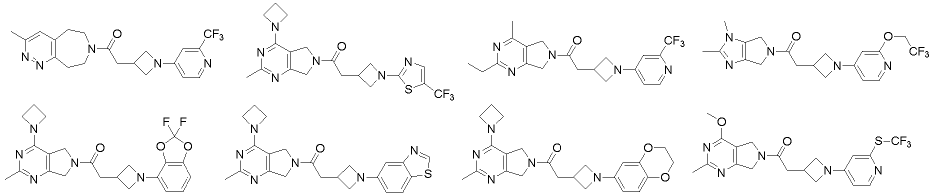

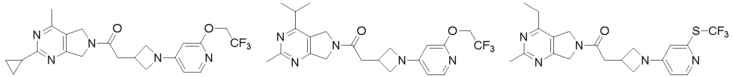

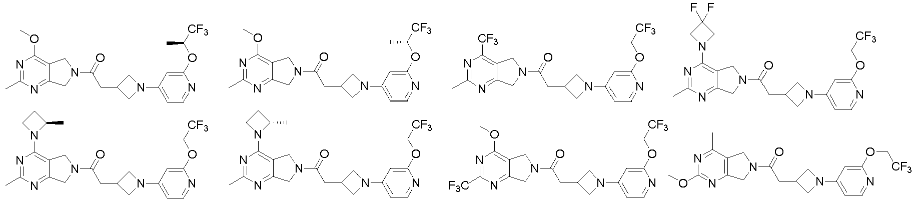

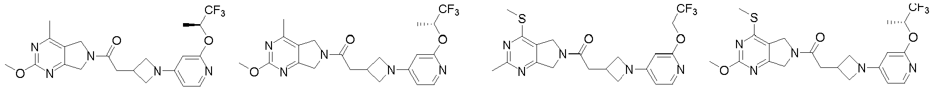

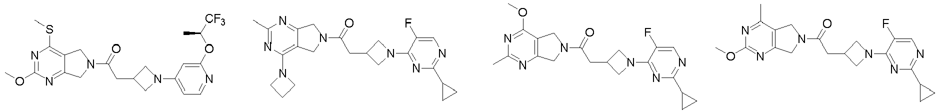

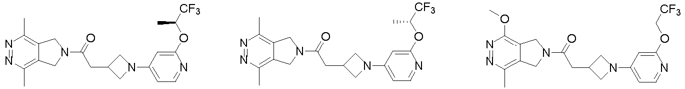

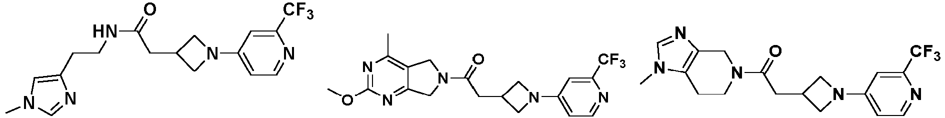

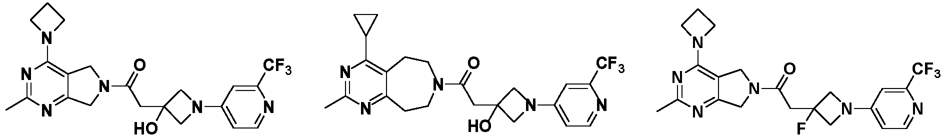

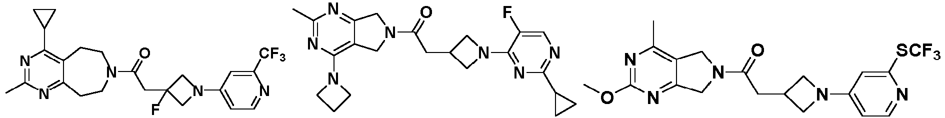

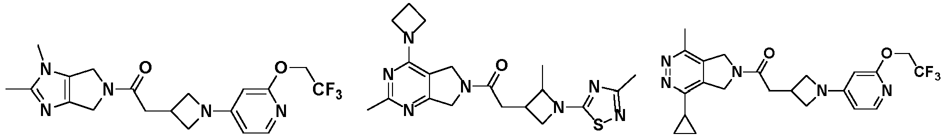

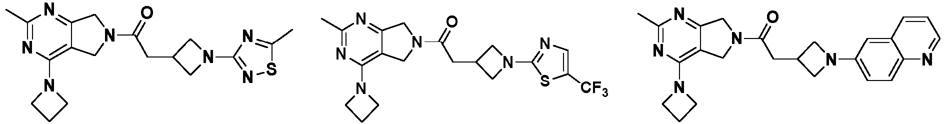

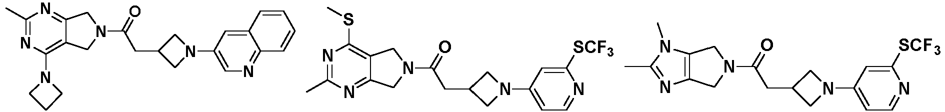

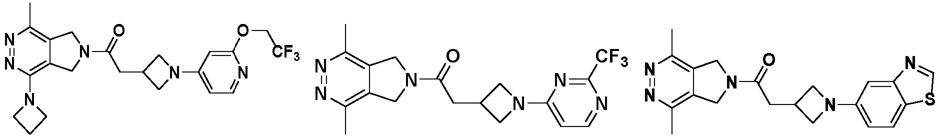

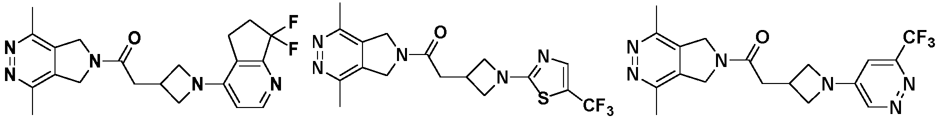

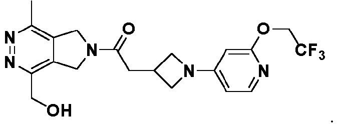

Preferably, the compound of formula I is

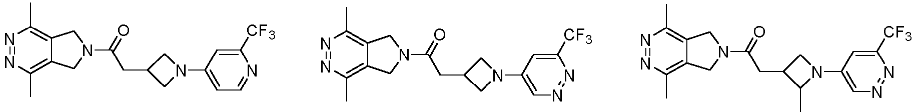

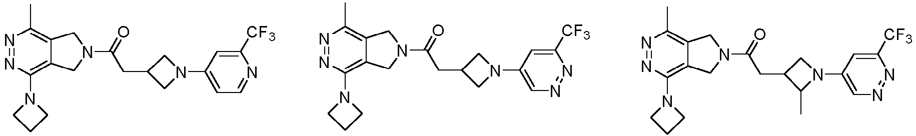

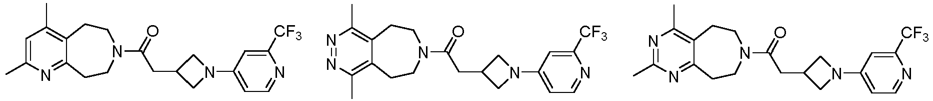

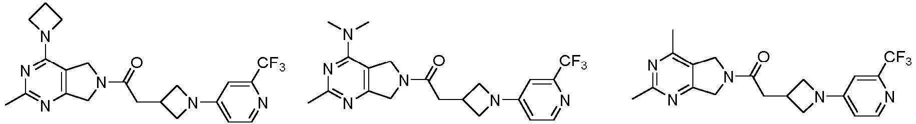

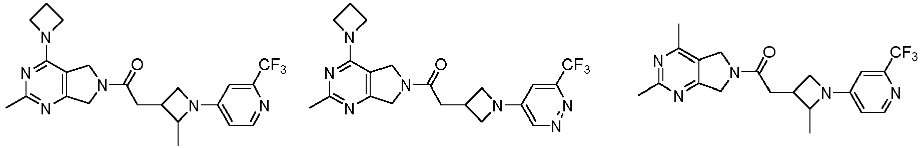

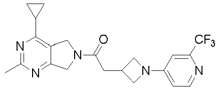

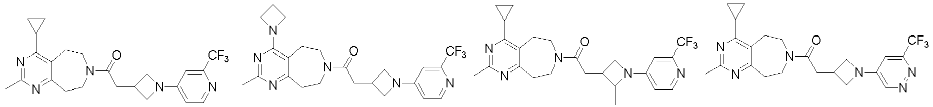

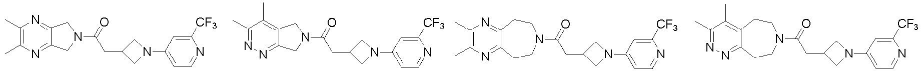

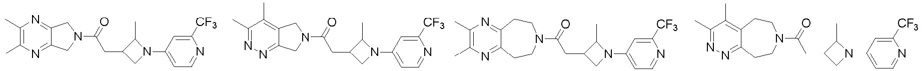

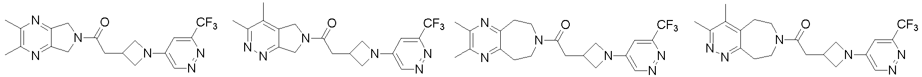

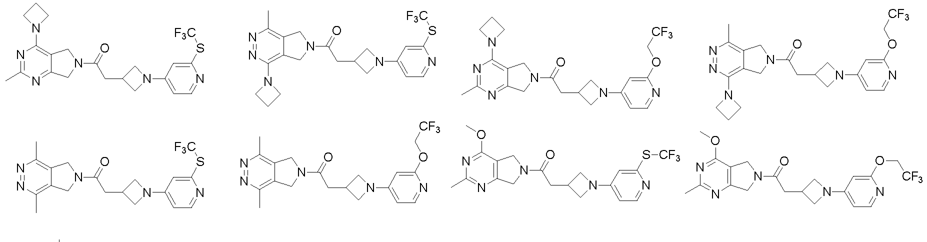

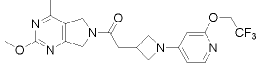

The present disclosure also provides a preparation method for the compound of formula I, which can be prepared by any one of the following schemes:
scheme (a): in an organic solvent, in the presence of a catalyst, performing a condensation reaction between a compound of formula II and a compound of formula III as follows to obtain the compound of formula I; 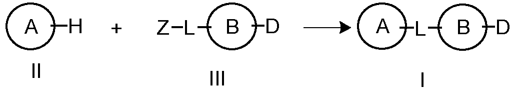
scheme (b): in an organic solvent, in the presence of a catalyst, performing a condensation reaction between a compound of formula IV and a compound of formula V as follows to obtain the compound of formula I; 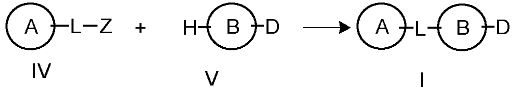
scheme (C): in an organic solvent, in the presence of a catalyst, performing a cyclization reaction between a compound of formula VI and a compound of formula VII as follows to obtain the compound of formula I; 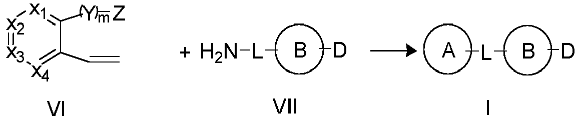
wherein Z is halogen (*e.g*., chlorine or bromine), TsO-, hydroxyl, methoxy, ethoxy, *n-*propoxy, or isopropoxy; preferably, Z is hydroxyl, methoxy, ethoxy, *n*-propoxy, or isopropoxy;
A, L, B, D, X₁, X₂, X₃, X₄, Y, and m are as described in any one of the embodiments of the present disclosure.

The present disclosure also provides a preparation method for the compound of formula I, which can be prepared by the following scheme:
scheme (D): in an organic solvent, in the presence of a catalyst, performing a cyclization reaction between a compound of formula VIII and a compound of formula VIIII as follows to obtain the compound of formula I; 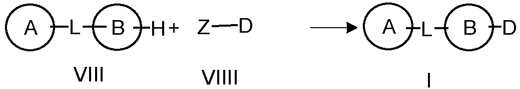 wherein Z, A, L, B, and D are as described in any one of the embodiments of the present disclosure.

The present disclosure also provides a pharmaceutical composition comprising a therapeutically effective amount of substance A and a pharmaceutical excipient; the substance A is the compound of formula I or the pharmaceutically acceptable salt thereof.

The present disclosure also provides a use of substance A in the manufacture of a positive allosteric modulator of a muscarinic receptor; the substance A is the compound of formula I or the pharmaceutically acceptable salt thereof, or the pharmaceutical composition.

The present disclosure also provides a use of substance A in the manufacture of a medicament for treating and/or preventing a muscarinic receptor-mediated disease; the substance A is the compound of formula I or the pharmaceutically acceptable salt thereof, or the pharmaceutical composition; preferably, the disease is Parkinson's disease, Alzheimer's disease, Huntington's disease, schizophrenia, drug addiction, or pain.

The present disclosure also provides a use of substance A in the manufacture of a medicament for treating Parkinson's disease, Alzheimer's disease, Huntington's disease, schizophrenia, drug addiction, or pain.

The positive and progressive effect of the present disclosure is that the compound of the present disclosure can be used as a positive allosteric modulator of the muscarinic receptor; the compound of the present disclosure can be used for the treatment of an M receptor (muscarinic receptor)-mediated (or M receptor-related) disease.

### Explanation of terms:

The term "-" means that the group is attached to the rest of the molecule through the site. For example, "CH₃O-" refers to alkoxy.

The term " 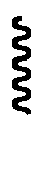 "(" 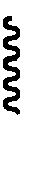 "in 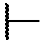 ) means that the structural moiety is attached to the rest of the molecule through the site.

The term "pharmaceutically acceptable" refers to being relatively non-toxic, safe, and suitable for patient use.

The term "pharmaceutically acceptable salt" refers to a salt obtained by reacting a compound with a pharmaceutically acceptable acid or base. When a compound contains a relatively acidic functional group, a base addition salt can be obtained by contacting the compound with a sufficient amount of a pharmaceutically acceptable base in a suitable inert solvent. The pharmaceutically acceptable base addition salt includes, but is not limited to, lithium salts, sodium salts, potassium salts, calcium salts, aluminum salts, magnesium salts, zinc salts, bismuth salts, ammonium salts, and diethanolamine salts. When a compound contains a relatively basic functional group, an acid addition salt can be obtained by contacting the compound with a sufficient amount of a pharmaceutically acceptable acid in a suitable inert solvent. The pharmaceutically acceptable acid includes inorganic acids and organic acids (e.g., trifluoroacetic acid, hydrochloric acid, formic acid). For details, please refer to Handbook of Pharmaceutical Salts: Properties, Selection, and Use (P. Heinrich Stahl, Camille G. Wermuth, 2011, 2nd Revised Edition), e.g., formates.

The term "pharmaceutical excipient" refers to all substances, other than the active pharmaceutical ingredient, that are contained in the pharmaceutical formulation, and is generally divided into two categories: excipients and additives. For details, please refer to the Pharmacopoeia of the People's Republic of China (2020 Edition) and Handbook of Pharmaceutical Excipients (Paul J Sheskey, Bruno C Hancock, Gary P Moss, David J Goldfarb, 2020, 9th Edition).

The term "treating" refers to the elimination of the cause of a disease or the alleviation of symptoms.

The term "preventing" refers to reducing the risk of developing a disease.

The term "patient" refers to any animal, typically a mammal, for example, a human, in need of treatment or prevention of a disease. The mammal includes, but is not limited to, cattle, horses, sheep, pigs, cats, dogs, mice, rats, rabbits, guinea pigs, monkeys, humans, *etc.*

The term "therapeutically effective amount" refers to an amount of a compound administered to a patient that is sufficient to effectively treat a disease. The therapeutically effective amount will vary depending on the type of compound, type of disease, severity of disease, age of patient, *etc.,* but can be adjusted by those skilled in the art as appropriate.

The expression "group B substituted by one or more groups A" means that one or more hydrogen atoms in group B are independently replaced by group A. When a plurality of groups A are present at the same time, their definitions are independent of each other and do not affect each other, unless otherwise specified.

The term "halogen" refers to fluorine, chlorine, bromine, or iodine.

The term "oxo" refers to =O, where an oxygen atom replaces two hydrogen atoms on the same atom, *e.g*., a methylene group (-CH₂-) is oxidized to a carbonyl group (-C(=O)-).

The term "alkyl" refers to a linear or branched, saturated monovalent hydrocarbon group with a specified number of carbon atoms. For example, C₁-C₆ alkyl (C₁₋₆ alkyl) or C₄-C₂₀ alkyl (C₄₋₂₀ alkyl), preferably C₁-C₄ alkyl (C₁₋₄ alkyl) or C₉-C₁₅ alkyl (C₁₋₆ alkyl). Alkyl groups include, but are not limited to, methyl, ethyl, *n*-propyl, isopropyl, *n*-butyl, isobutyl, *sec-*butyl, or *tert*-butyl.

The definition of alkyl in the term "alkyl-O-" is as described above. Examples of alkyl-O- include C₁-C₆ alkyl-O- or C₁-C₄ alkyl-O-, more specifically, CH₃-O-, CH₃CH₂-O-, CH₃CH₂CH₂-O-, or CH₃CH(CH₃)-O-. Similarly, for the rest of the expressions in the form of "Rx-O-" in the present disclosure, the definition of Rx corresponds to its respective definition, e.g., the definition of cycloalkyl in "cycloalkyl-O-" is as described in the following term "cycloalkyl".

The definition of alkyl in the term "alkyl-S-" is as described above. Examples of alkyl-S- include C₁-C₆ alkyl-S- or C₁-C₄ alkyl-S-, more specifically, CH₃-S-, CH₃CH₂-S-, CH₃CH₂CH₂-S-, or CH₃CH(CH₃)-S-. Similarly, for the rest of the expressions in the form of "Rx-S-" in the present disclosure, the definition of Rx corresponds to its respective definition, *e.g*., the definition of cycloalkyl in "cycloalkyl-S-" is as described in the following term "cycloalkyl".

The term "alkoxy" means that an oxygen atom is attached to one end of an alkyl group as a bond to form "alkyl-O-". The definition of "alkyl-O-" is as described above.

The term "alkylthio" means that a sulfur atom is attached to one end of an alkyl group as a bond to form "alkyl-S-". The definition of "alkyl-S-" is as described above.

The term "heteroaryl" refers to a cyclic, unsaturated monovalent group with a specified number of ring atoms *(e.g.,* 5- to 12-membered, 5- to 10-membered, or 5- to 6-membered), a specified number of heteroatoms (e.g., 1, 2, or 3), and a specified type of heteroatoms (one or more kinds of N, O, and S), which is aromatic.

The term "heterocycloalkyl" refers to a saturated heterocycloalkyl group or a partially unsaturated monocyclic or polycyclic *(e.g.,* a bridged, fused (condensed), or spiro system that is bicyclic, tricyclic, or more cyclic) heterocyclic group with a specified number of ring atoms *(e.g.,* 4- to 12-membered, 4- to 10-membered, 3- to 7-membered, 6- to 10-membered, 4- to 7-membered, or 5- to 6-membered), a specified number of heteroatoms (e.g., 1, 2, or 3), and a specified type of heteroatoms or heteroatom group (one or more kinds of N, O, S, S(=O), and S(=O)₂).

The term "cycloalkyl" means a saturated carbocyclic substituent which may be attached to the rest of the molecule by a single bond via any suitable carbon atom; C₃-C₇ cycloalkyl with 3 to 7 carbon atoms, preferably C₃-C₆ cycloalkyl with 3 to 6 carbon atoms, for example, cyclopropyl, cyclobutyl, cyclopentyl, or cyclohexyl.

The term "aryl" refers to a cyclic, unsaturated monovalent hydrocarbon group with a specified number of carbon atoms (e.g., C₆-C₁₀). Examples of aryl groups include, but are not limited to, phenyl or naphthyl.

The term "heteroaryl" refers to a cyclic or unsaturated monovalent group with a specified number of ring atoms *(e.g.,* 5- to 10-membered, 5- to 6-membered), a specified number of heteroatoms (e.g., 1, 2, or 3), and a specified type of heteroatoms (one or more kinds of N, O, and S), which is monocyclic or polycyclic, and (at least one ring/each ring) is aromatic. The heteroaryl group is attached to the rest of the molecule via a carbon atom or a heteroatom; the heteroaryl group is attached to the rest of the molecule via a ring with or without a heteroatom; the heteroaryl group is attached to the rest of the molecule via a ring with or without aromaticity.

Unless otherwise specified, the absolute configuration of a stereogenic center is represented by a wedged solid bond ( 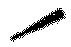 ) and a wedged dashed bond ( 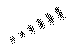 ), and when an atom is connected to its substituent by a wavy line ( 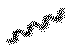 ), it denotes 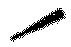 , 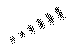 , or a mixture thereof.

On the basis of not violating the common sense in the art, the above-mentioned preferred conditions can be arbitrarily combined to obtain preferred examples of the present disclosure.

The reagents and starting materials used in the present disclosure are commercially available.

### Abbreviations:

PMB: 4-Methoxybenzyl.
Boc: tert-Butyloxycarbonyl.
DBU: 1,8-Diazabicyclo[5.4.0]undec-7-ene.
NBS: N-Bromosuccinimide.
DIEA: N,N-Diisopropylethylamine.
DMF: N,N-Dimethylformamide.
Et: Ethyl.
Me: Methyl.
i-Pr: Isopropyl.
DMSO: Dimethyl sulfoxide.
HATU: 2-(7-Azabenzotriazol-1-yl)-*N*,*N*,*N*',*N*'-tetramethyluronium hexafluorophosphate.
PyBOP: 1*H*-Benzotriazol-1-yloxytripyrrolidinophosphonium hexafluorophosphate.
TEA: Triethylamine.
LiHMDS: Lithium bis(trimethylsilyl)amide.
NCS: N-Chlorosuccinimide.

### DETAILED DESCRIPTION OF THE PREFERRED EMBODIMENT

The present disclosure is further illustrated by way of examples below, but the present disclosure is not limited to the scope of the described examples. The experimental methods for which the specific conditions are not specified in the following examples are selected according to the conventional methods and conditions, or according to the commodity instructions.

### Intermediate A

### Synthetic route:

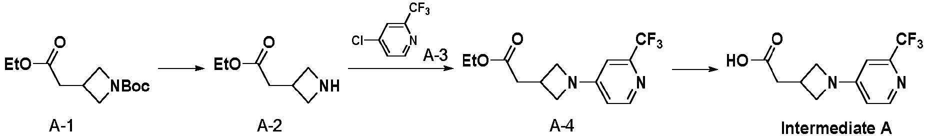

### Step 1

**A-1** (5.0 g, 20.5 mmol) was dissolved in dichloromethane (10 mL) and trifluoroacetic acid (10 mL), and the mixture was stirred at room temperature for 2 hours. The reaction mixture was concentrated under reduced pressure to obtain a trifluoroacetate salt of **A-2.** ¹H NMR (400 MHz, DMSO-*d*₆) δ 4.13-3.91 (m, 4H), 3.83-3.66 (m, 2H), 3.11-3.03 (m, 1H), 2.74-2.63 (m, 2H), 1.19-1.13 (m, 3H). ESI-MS calculated for C₇H₁₄NO₂ [M+H]⁺ = 144.1, found 144.2.

### Step 2

The trifluoroacetate salt of **A-2** (5.0 g, 19.4 mmol) and **A-3** (6.0 g, 33.5 mmol) were dissolved in dimethyl sulfoxide (10 mL), then triethylamine (13.4 g, 132.2 mmol) and cesium fluoride (5.0 g, 33.0 mmol) were sequentially added thereto, and the mixture was heated to 80°C and stirred for 18 hours. The reaction mixture was poured into saturated ammonium chloride solution (20 mL) and extracted with dichloromethane (50 mL × 1). The organic phase was dried over anhydrous sodium sulfate, filtered, and concentrated under reduced pressure to obtain a crude product containing the target compound, which was purified by silica gel column chromatography (petroleum ether/ethyl acetate, 3/2, v/v) to obtain **A-4.** ¹H NMR (400 MHz, DMSO-*d*₆) δ 8.21 (d, *J =* 5.64 Hz, 1H), 6.70 (d, *J =* 2.28 Hz, 1H), 6.53-6.48 (m, 1H), 4.17-4.04 (m, 4H), 3.70-3.67 (m, 2H), 3.10-3.01 (m, 1H), 2.76-2.72 (m, 2H), 1.21-1.17 (m, 3H). ESI-MS calculated for C₁₃H₁₆F₃N₂O₂ [M+H]⁺ = 289.1, found 289.2.

### Step 3

**A-4** (1.0 g, 3.5 mmol) was dissolved in tetrahydrofuran (20 mL) and water (4 mL), then lithium hydroxide (0.22 g, 5.2 mmol) was added thereto, and the mixture was stirred at room temperature for 2 hours. The pH of the reaction mixture was adjusted to 7, then water (50 mL) was added thereto, and the mixture was lyophilized to obtain a crude product containing intermediate **A,** which was directly used in the next reaction step. ¹H NMR (400 MHz, DMSO-*d*₆) δ 8.25 (d, *J =* 5.64 Hz, 1H), 6.72 (d, *J =* 2.26 Hz, 1H), 6.55 (dd, *J =* 5.64, 2.26 Hz, 1H), 4.20-4.12 (m, 2H), 3.74-3.64 (m, 2H), 3.14-3.04 (m, 1H), 2.54-2.50 (m, 2H). ESI-MS calculated for C₁₁H₁₁F₃N₂O₂ [M+H]⁺ = 261.1, found 261.0.

### Intermediate B

### Synthetic route:

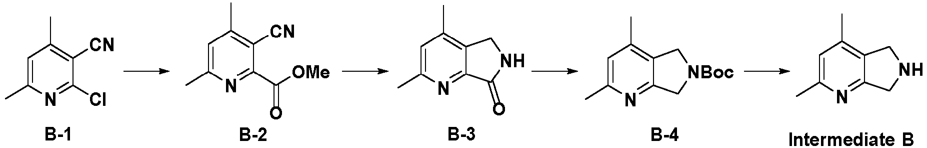

### Step 1

**B-1** (5.0 g, 30.0 mmol) was dissolved in methanol (60 mL), then triethylamine (12.5 mL, 90.0 mmol) and [1,1'-bis(diphenylphosphino)ferrocene]dichloropalladium(II) (2.2 g, 3.0 mmol) were added thereto, and the reaction mixture was heated to 70°C and stirred for 12 hours. The reaction mixture was cooled to room temperature and concentrated under reduced pressure to obtain a crude product containing the target compound, which was purified by silica gel column chromatography (petroleum ether/ethyl acetate, 3/2, v/v) to obtain **B-2.** ¹H NMR (400 MHz, Chloroform-*d*) δ 7.36 (s, 1H), 4.09 (s, 3H), 2.70 (s, 3H), 2.63 (s, 3H). ESI-MS calculated for C₁₀H₁₁N₂O₂ [M+H]⁺ = 191.1, found 191.0.

### Step 2

**B-2** (4.5 g, 7.89 mmol) was dissolved in methanol (20 mL), and Raney nickel (930 mg, 15.8 mmol) was added thereto. The reaction system was replaced with hydrogen three times, then heated to 50°C and stirred for 12 hours. The reaction mixture was cooled to room temperature, filtered through diatomite, and concentrated under reduced pressure to obtain a crude product containing the target compound, which was purified by silica gel column chromatography (petroleum ether/ethyl acetate, 17/3, v/v) to obtain **B-3.** ¹H NMR (400 MHz, DMSO-*d*₆) δ 8.82 (br, s, 1H), 7.25 (s, 1H), 4.31 (s, 2H), 2.52 (s, 3H), 2.32 (s, 3H). ESI-MS calculated for C₉H₁₁N₂O [M+H]⁺ = 163.1, found 163.2.

### Step 3

**B-3** (4.5 g, 27.8 mmol) was dissolved in tetrahydrofuran (50 mL), and borane dimethyl sulfide complex in tetrahydrofuran (10 mol/L, 13.9 mL, 138.7 mmol) was added thereto, and the mixture was heated to 75°C and stirred for 12 hours. The reaction mixture was cooled to 0°C and quenched with methanol (200 mL), and hydrochloric acid (6 mol/L, 69.38 mL, 416.25 mmol) was added thereto. The mixture was heated to 70°C and stirred for 3 hours. The reaction mixture was cooled to room temperature, and the pH was adjusted to 9-10 with sodium hydroxide aqueous solution (2 mol/L), and then di-*tert*-butyl dicarbonate (12.1 g, 55.5 mmol) was added thereto. The mixture was stirred at room temperature for 3 hours. The reaction mixture was concentrated under reduced pressure and extracted with ethyl acetate (300 mL × 3). The organic phases were combined, dried over anhydrous sodium sulfate, and concentrated to obtain a crude product containing the target compound, which was purified by silica gel column chromatography (dichloromethane/methanol, 17/3, v/v) to obtain **B-4.** ¹H NMR (400 MHz, Chloroform-*d*) δ 6.88 (s, 1H), 4.73-4.56 (m, 4H), 2.53 (s, 3H), 2.25 (s, 3H), 1.54 (s, 9H). ESI-MS calculated for C₁₄H₂₁N₂O₂ [M+H]⁺ = 249.2, found 249.2.

### Step 4

**B-4** (5.0 g, 20.1 mmol) was dissolved in dichloromethane (10 mL), then trifluoroacetic acid (10 mL) was added thereto, and the mixture was stirred at room temperature for 2 hours. The reaction mixture was concentrated under reduced pressure to obtain a trifluoroacetate salt of intermediate **B**. ¹H NMR (400 MHz, DMSO-*d*₆) δ 7.11 (s, 1H), 4.57-4.45 (m, 4H), 2.52 (s, 3H), 2.27 (s, 3H). ESI-MS calculated for C₉H₁₃N₂ [M+H]⁺ = 149.1, found 149.1.

### Intermediate C

### Synthetic route:

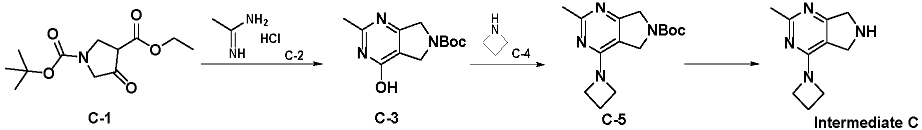

### Step 1

**C-1** (10.0 g, 39.9 mmol) and **C-2** (4.4 g, 46.6 mmol) were dissolved in methanol (32 mL) and water (128 mL), then potassium carbonate (8.1 g, 58.3 mmol) was added thereto, and the mixture was stirred at 60°C for 18 hours. The pH of the reaction mixture was adjusted to 7 with dilute hydrochloric acid (1 mol/L) and the mixture was extracted with dichloromethane (300 mL × 1). The organic phase was dried over anhydrous sodium sulfate, filtered, and concentrated under reduced pressure to obtain a crude product containing the target compound. Petroleum ether (50 mL) was added and the mixture was stirred at room temperature for 30 minutes and then filtered to obtain **C-3.** ¹H NMR (400 MHz, DMSO-*d*₆) δ 4.38-4.26 (m, 4H), 2.37 (s, 3H), 1.44 (s, 9H). ESI-MS calculated for C₁₂H₁₈N₃O₃ [M+H]⁺ = 252.1, found 252.2.

### Step 2

**C-3** (2.0 g, 7.9 mmol) and **C-4** (0.9 g, 15.9 mmol) were dissolved in DMF (10 mL), then DBU (2.4 g, 15.9 mmol) and PyBOP (6.2 g, 11.9 mmol) were sequentially added thereto, and the mixture was heated to 80°C and stirred for 18 hours. The reaction mixture was cooled to room temperature and concentrated under reduced pressure to obtain a crude product containing the target compound, which was purified by silica gel column chromatography (petroleum ether/ethyl acetate, 1/1, v/v) to obtain **C-5.** ¹H NMR (400 MHz, DMSO-*d₆*) δ 4.58-4.50 (m, 2H), 4.34-4.20 (m, 2H), 4.18-4.10 (m, 4H), 2.33 (s, 3H), 2.32-2.26 (m, 2H), 1.45 (s, 9H). ESI-MS calculated for C₁₅H₂₃N₄O₂ [M+H]⁺ = 291.2, found 291.2.

### Step 3

**C-5** (0.6 g, 2.1 mmol) was dissolved in dichloromethane (10 mL) and trifluoroacetic acid (10 mL), and the mixture was stirred at room temperature for 2 hours. The reaction mixture was concentrated under reduced pressure to obtain a trifluoroacetate salt of the intermediate **C,** which was directly used in the next reaction step. ESI-MS calculated for C₁₀H₁₅N₄ [M+H]⁺ = 191.1, found 191.2.

### Intermediate D

### Synthetic route:

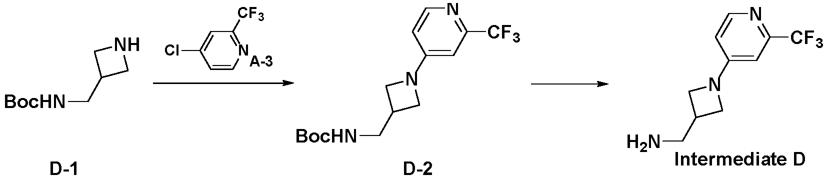

### Step 1

**D-1** (1.0 g, 5.37 mmol), **A-3** (970 mg, 5.37 mmol), cesium fluoride (820 mg, 5.37 mmol), and triethylamine (540 mg, 5.37 mmol) were added to DMSO (10 mL), and the reaction mixture was heated to 100°C and stirred for 10 hours. The reaction mixture was diluted with water (30 mL) and extracted with ethyl acetate (10 mL × 3). The organic phases were combined, washed with saturated brine (30 mL), dried over anhydrous sodium sulfate, and concentrated under reduced pressure to obtain a crude product containing the target compound, which was purified by silica gel column chromatography (dichloromethane/methanol, 23/2, v/v) to obtain **D-2.** ¹H NMR (400 MHz, Chloroform-*d*) δ 8.20 (d, *J* = 5.60 Hz, 1H), 6.50 (d, *J =* 2.30 Hz, 1H), 6.24 (dd, *J =* 5.70, 2.30 Hz, 1H), 4.76-4.70 (m, 1H), 4.02-3.95 (m, 2H), 3.68-3.61 (m, 2H), 3.39-3.32 (m, 2H), 2.97-2.87 (m, 1H), 1.38 (s, 9H). ESI-MS calculated for C₁₅H₂₁F₃N₃O₂ [M+H]⁺ = 332.2, found 332.2.

### Step 2

**D-2** (1.1 g, 3.32 mmol) was dissolved in dichloromethane (10 mL) and trifluoroacetic acid (1.0 mL), and the mixture was stirred at 25°C for 16 hours. The reaction mixture was concentrated under reduced pressure to obtain a crude product containing the target compound, which was purified by preparative high performance liquid chromatography (chromatographic column: Boston ODS C18 120 g Flash, mobile phase: acetonitrile-10 mmol/L ammonium bicarbonate aqueous solution, gradient: 25 to 50%, retention time: 6 minutes) to obtain intermediate **D.** ESI-MS calculated for C₁₀H₁₃F₃N₃ [M+H]⁺ = 232.1, found 232.0.

### Intermediate E

### Synthetic route:

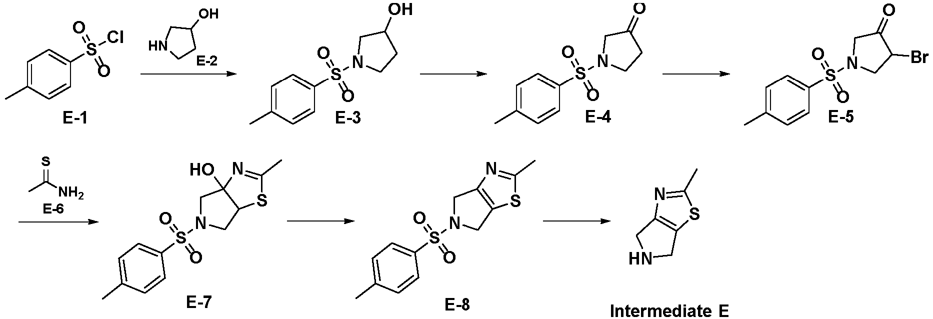

### Step 1

**E-2** (910 mg, 10.5 mmol) and triethylamine (4.36 mL, 31.5 mmol) were dissolved in tetrahydrofuran (20 mL), cooled to 0°C, and **E-1** (2.0 g, 10.5 mmol) was added thereto. The reaction system was slowly warmed to room temperature and stirred for 12 hours. The reaction mixture was diluted with water (50 mL) and extracted with ethyl acetate (20 mL × 3). The organic phases were combined, washed with saturated brine (50 mL), dried over anhydrous sodium sulfate, and concentrated under reduced pressure to obtain a crude product containing the target compound, which was purified by silica gel column chromatography (petroleum ether/ethyl acetate, 7/3, v/v) to obtain **E-3.** ¹HNMR (400 MHz, DMSO-*d*6) δ 7.68 (d, *J =* 8.01 Hz, 2H), 7.42 (d, *J =* 8.10 Hz, 2H), 4.90 (d, *J =* 3.52 Hz, 1H), 4.17-4.12 (m, 1H), 3.27-3.13 (m, 2H), 3.00-2.96 (m, 1H), 2.40 (s, 3H), 1.72-1.67 (m, 1H), 1.66-1.56 (m, 1H). ESI-MS calculated for C₁₁H₁₆NO₃S [M+H]⁺ = 242.1, found 242.0.

### Step 2

**E-3** (1.2 g, 4.97 mmol) was dissolved in dichloromethane (12 mL), then Dess-Martin periodinane (2.5 g, 5.97 mmol) was added thereto, and the mixture was stirred at room temperature for 6 hours. After the reaction mixture was filtered, the filtrate was added with saturated sodium bicarbonate solution (50 mL) and extracted with dichloromethane (40 mL × 3). The organic phases were combined, dried over anhydrous sodium sulfate, and concentrated under reduced pressure to obtain a crude product containing the target compound, which was purified by silica gel column chromatography (petroleum ether/ethyl acetate, 7/3, v/v) to obtain **E-4.** ¹H NMR (400 MHz, DMSO-*d*6) δ 7.68 (d, *J* = 8.01 Hz, 2H), 7.47 (d, *J* = 8.08 Hz, 2H), 3.49-3.43 (m, 4H), 2.45-2.39 (m, 5H). ESI-MS calculated for C₁₁H₁₄NO₃S [M+H]⁺ = 240.1, found 240.0.

### Step 3

**E-4** (700 mg, 2.93 mmol) was dissolved in tetrahydrofuran (10 mL), then pyridinium tribromide (937 mg, 2.93 mmol) was added thereto, and the mixture was stirred at room temperature for 4 hours. The reaction mixture was diluted with water (20 mL) and extracted with ethyl acetate (20 mL × 3). The organic phases were combined, dried over anhydrous sodium sulfate, and concentrated under reduced pressure to obtain a crude product containing the target compound, which was purified by silica gel column chromatography (petroleum ether/ethyl acetate, 4/1, v/v) to obtain **E-5.** ¹H NMR (400 MHz, DMSO-*d*6) δ 7.68 (d, *J =* 8.01 Hz, 2H), 7.48 (d, *J =* 8.08 Hz, 2H), 4.76-4.74 (m, 1H), 3.93-3.84 (m, 1H), 3.61-3.58 (m, 1H), 3.56-3.50 (m, 1H), 3.47-3.38 (m, 1H), 2.42 (s, 3H). ESI-MS calculated for C₁₁H₁₃BrNO₃S [M+H]⁺ = 318.0, found 317.8.

### Step 4

**E-5** (500 mg, 1.57 mmol) and **E-6** (142 mg, 1.89 mmol) were dissolved in DMF (5 mL). The reaction system was heated to 60°C and stirred for 2 hours under nitrogen atmosphere. The reaction mixture was cooled to room temperature, then diluted with water (20 mL), and extracted with ethyl acetate (20 mL × 3). The organic phases were combined, washed with saturated brine (20 mL × 3), dried over anhydrous sodium sulfate, and concentrated under reduced pressure to obtain a crude product containing E-7, which was directly used in the next reaction step. ESI-MS calculated for C₁₃H₁₇N₂O₃S₂ [M+H]⁺=313.1, found 313.0.

### Step 5

**E-7** (450 mg, 1.44 mmol) and triethylamine (729 mg, 7.20 mmol) were dissolved in dichloromethane (5 mL), then methanesulfonyl chloride (330 mg, 2.88 mmol) was added dropwise thereto, and the mixture was stirred at room temperature for 3 hours. The reaction mixture was quenched with water (20 mL) and extracted with ethyl acetate (20 mL × 3). The organic phases were combined, dried over anhydrous sodium sulfate, filtered, and concentrated under reduced pressure to obtain **E-8,** which was directly used in the next reaction step. ¹HNMR (400 MHz, DMSO-*d*6) δ 7.78 (d, *J =* 8.01 Hz, 2H), 7.43 (d, *J =* 8.07 Hz, 2H), 4.56 (dd, *J* = 4.31, 2.70 Hz, 2H), 4.43 (dd, *J* = 4.32, 2.70 Hz, 2H), 2.60 (s, 3H), 2.38 (s, 3H). ESI-MS calculated for C₁₃H₁₅N₂O₂S₂ [M+H]⁺ = 295.1, found 295.0.

### Step 6

**E-8** (300 mg, 1.02 mmol) and phenol (96 mg, 1.02 mmol) were dissolved in aqueous hydrobromic acid (48%, 3 mL), and the mixture was heated to 90°C and stirred for 3 hours. The reaction mixture was cooled to room temperature and then washed with ethyl acetate (10 mL × 3) to remove impurities. The aqueous phase was then concentrated to obtain a crude product containing the hydrobromide salt of intermediate **E,** which was directly used in the next reaction step. ESI-MS calculated for C₆H₉N₂S [M+H]⁺ = 141.0, found 141.0.

### Intermediate F

### Synthetic route:

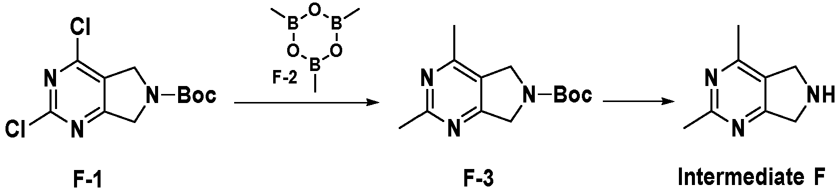

### Step 1

**F-1** (1.0 g, 3.45 mmol), [1,1'-bis(diphenylphosphino)ferrocene]dichloropalladium(II) (250 mg, 0.34 mmol), potassium carbonate (1.43 g, 10.34 mmol), F-2 (1.43 g, 10.34 mmol), and 1,4-dioxane (10 mL) and water (1 mL) were heated to 80°C under nitrogen atmosphere and stirred for 18 hours. The reaction mixture was cooled to room temperature, filtered, and the filtrate was diluted with water (30 mL) and extracted with ethyl acetate (10 mL × 3). The organic phases were combined, washed with saturated brine (30 mL), dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **F-3.** ESI-MS calculated for C₁₃H₂₀N₃O₂ [M+H]⁺ = 250.2, found 250.2.

### Step 2

**F-3** (13.0 g, 37.42 mmol) was dissolved in dichloromethane (10 mL), then trifluoroacetic acid (2 mL) was added thereto, and the mixture was stirred at room temperature for 3 hours. The reaction mixture was concentrated under reduced pressure to obtain a trifluoroacetate salt of intermediate **F.** ESI-MS calculated for C₈H₁₂N₃ [M+H]⁺ = 150.1, found 150.1.

### Intermediate G

### Synthetic route:

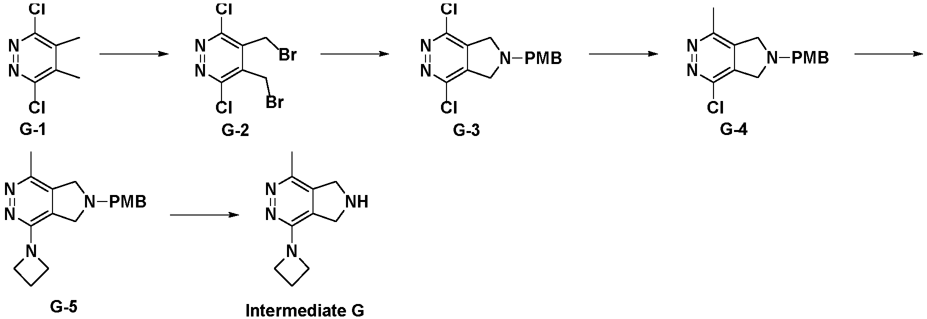

### Step 1

**G-1** (2.0 g, 11.3 mmol), NBS (4.2 g, 23.7 mmol), and azobisisobutyronitrile (370 mg, 2.26 mmol) were sequentially added to carbon tetrachloride (25 mL), and the reaction system was heated to 80°C under nitrogen atmosphere and stirred for 16 hours. The reaction mixture was cooled to room temperature, filtered, and concentrated under reduced pressure to obtain an oil. The oil was diluted with ethyl acetate (20 mL), washed with saturated sodium thiosulfate solution (15 mL × 2), dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **G-2.** ESI-MS calculated for C₆H₅Br₂Cl₂N₂ [M+H]⁺ = 332.8, found 332.0.

### Step 2

**G-2** (4.00 g, 11.9 mmol) and potassium carbonate (4.9 g, 35.8 mmol) were added to tetrahydrofuran (4 mL), then a solution of 4-methoxybenzylamine in tetrahydrofuran (1.64 g, 11.9 mmol) was added dropwise thereto at room temperature, and the mixture was stirred at room temperature for 1 hour. The reaction mixture was filtered and concentrated under reduced pressure to obtain a crude product containing the target compound, which was purified by silica gel column chromatography (petroleum ether/ethyl acetate, 9/1, v/v) to obtain **G-3.** ESI-MS calculated for C₁₄H₁₄Cl₂N₃O [M+H]⁺ = 310.0, found 310.0.

### Step 3

**G-3** (400 mg, 1.29 mmol), methylboronic acid (116 mg, 1.93 mmol), potassium carbonate (535 mg, 3.87 mmol), [1,1'-bis(diphenylphosphino)ferrocene]dichloropalladium(II) (189 mg, 0.26 mmol), and 1,4-dioxane (3 mL) were added to a 10 mL microwave tube, and the mixture was heated to 90°C under nitrogen atmosphere and stirred for 18 hours. The reaction mixture was cooled to room temperature, filtered, diluted with water (15 mL), and extracted with ethyl acetate (5 mL × 3). The organic phases were combined, washed with saturated brine (20 mL), dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **G-4.** ESI-MS calculated for C₁₅H₁₇ClN₃O [M+H]⁺ = 290.1, found 290.0.

### Step 4

**G-4** (130 mg, 0.45 mmol) and azetidine (2 mL) were added to a 10 mL microwave tube, and the mixture was heated to 90°C and stirred for 16 hours. The reaction mixture was cooled to room temperature, diluted with water (15 mL), and extracted with ethyl acetate (5 mL × 3). The organic phases were combined, washed with saturated brine (20 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **G-5.** ESI-MS calculated for C₁₈H₂₃N₄O [M+H]⁺ = 311.2, found 311.2.

### Step 5

**G-5** (120 mg, 0.39 mmol) and trifluoroacetic acid (2 mL) were added to a 5 mL microwave tube, and the mixture was heated to 100°C and stirred for 8 hours. The reaction mixture was cooled to room temperature, diluted with water (15 mL), added with saturated sodium bicarbonate solution to adjust the pH to 8, and extracted with ethyl acetate (5 mL × 3). The organic phases were combined, washed with saturated brine (20 mL), dried over anhydrous sodium sulfate, filtered, and concentrated under reduced pressure to obtain intermediate G, which was directly used in the next reaction step. ESI-MS calculated for C₁₀H₁₅N₄ [M+H]⁺ = 191.1, found 191.1.

### Intermediate H

### Synthetic route:

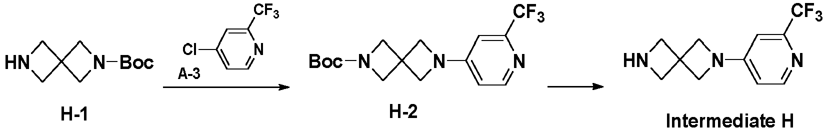

### Step 1

**H-1** (1.0 g, 5.04 mmol), **A-3** (920 mg, 5.04 mmol), cesium fluoride (0.77 g, 5.04 mmol), and triethylamine (1.0 g, 10.1 mmol) were dissolved in DMSO (8 mL), and the reaction mixture was heated to 100°C and stirred for 18 hours. The reaction mixture was diluted with water (30 mL) and extracted with ethyl acetate (10 mL × 3). The organic phases were combined, washed with saturated brine (30 mL), dried over anhydrous sodium sulfate, and concentrated under reduced pressure to obtain a crude product containing the target compound, which was purified by silica gel column chromatography (petroleum ether/ethyl acetate, 3/2, v/v) to obtain **H-2.** ESI-MS calculated for C₁₆H₂₁F₃N₃O₂ [M+H]⁺ = 344.2, found 344.2.

### Step 2

**H-2** (600 mg, 1.75 mmol) was dissolved in trifluoroacetic acid (5 mL), and the mixture was stirred at room temperature for 3 hours. The reaction mixture was concentrated under reduced pressure to obtain a trifluoroacetate salt of the intermediate **H,** which was directly used in the next reaction step. ESI-MS calculated for C₁₁H₁₃F₃N₃ [M+H]⁺ = 244.1, found 244.0.

### Intermediate I

### Synthetic route:

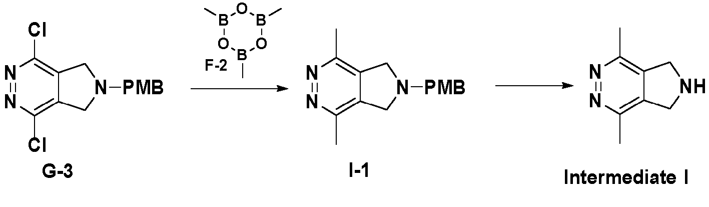

### Step 1

**G-3** (1.5 g, 4.84 mmol), [1,1'-bis(diphenylphosphino)ferrocene]dichloropalladium(II) (710 mg, 0.97 mmol), potassium carbonate (2.0 g, 14.5 mmol), **F-2** (1.21 g, 9.67 mmol), and 1,4-dioxane (10 mL) were heated to 90°C under nitrogen atmosphere and stirred for 18 hours. The reaction mixture was cooled to room temperature and concentrated under reduced pressure to obtain a crude product containing the target compound, which was purified by silica gel column chromatography (petroleum ether/ethyl acetate, 4/1, v/v) to obtain **I-1.** ESI-MS calculated for C₁₆H₂₀N₃O [M+H]⁺ = 270.2, found 270.1.

### Step 2

**I-1** (120 mg, 0.45 mmol) and trifluoroacetic acid (2 mL) were added to a 5 mL microwave tube, and the mixture was heated to 90°C and stirred for 8 hours. The reaction mixture was cooled to room temperature and concentrated under reduced pressure to obtain a trifluoroacetate containing intermediate **I,** which was directly used in the next reaction step. ESI-MS calculated for C₈H₁₂N₃ [M+H]⁺ = 150.1, found 150.1.

### Intermediate J

### Synthetic route:

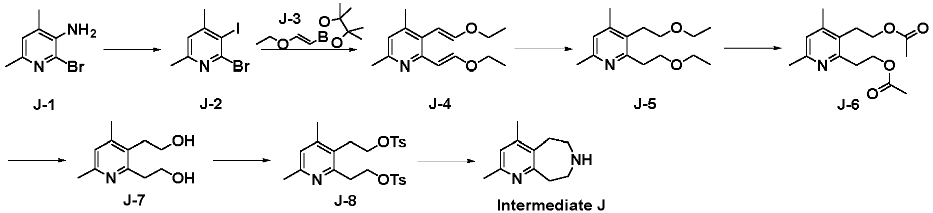

### Step 1

**J-1** (500 mg, 2.50 mmol) was added to water (3 mL), followed by the addition of concentrated hydrochloric acid (12 mol/L, 1 mL). After cooling to 0°C, sodium nitrite (223 mg, 3.23 mmol) was slowly added while controlling the temperature of the reaction mixture below 5°C. The mixture was stirred for 30 minutes, and then potassium iodide (1.04 g, 6.22 mmol) was added thereto. The reaction mixture was stirred at room temperature for 2 hours. The reaction mixture was added with saturated sodium sulfite solution (10 mL), diluted with water (15 mL), and then extracted with ethyl acetate (15 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **J-2.** ¹H NMR (400 MHz, DMSO-*d*₆) δ 7.13 (s, 1H), 2.35 (s, 3H), 2.29 (s, 3H). ESI-MS calculated for C₇H₈BrIN [M+H]⁺ = 311.9, found 311.8.

### Step 2

To **J-2** (500 mg, 1.60 mmol), potassium carbonate (664 mg, 4.81 mmol), **J-3** (790 mg, 4.10 mmol), and 1,4-dioxane (20 mL) and water (4 mL) was added [1,1'-bis(diphenylphosphino)ferrocene]dichloropalladium(II) (120 mg, 0.16 mmol), and the reaction mixture was heated to 90°C under nitrogen atmosphere and stirred for 18 hours. After the reaction was completed, the reaction mixture was diluted with water (15 mL) and extracted with ethyl acetate (15 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 3/1, v/v) to obtain **J-4.** ESI-MS calculated for C₁₅H₂₂NO₂ [M+H]⁺ = 248.2, found 248.2.

### Step 3

**J-4** (100 mg, 0.40 mmol) was dissolved in methanol (10 mL), and wet palladium on carbon (10%, 43 mg) was added thereto. The reaction system was replaced with hydrogen three times and stirred at room temperature for 3 hours. The reaction mixture was filtered, and the filtrate was concentrated under reduced pressure to obtain **J-5.** ¹H NMR (400 MHz, Chloroform-d) δ 6.80 (s, 1H), 3.76 (t, *J =* 7.42 Hz, 2H), 3.57-3.42 (m, 6H), 3.09 (t, *J =* 7.42 Hz, 2H), 2.96 (t, *J =* 6.94 Hz, 2H), 2.43 (s, 3H), 2.30 (s, 3H), 1.25-1.14 (m, 6H). ESI-MS calculated for C₁₅H₂₆NO₂ [M+H]⁺ =252.2, found 252.2.

### Step 4

**J-5** (300 mg, 1.19 mmol) was dissolved in an acetic acid solution of hydrobromic acid (33% wt, 10 mL), and the mixture was stirred at 60°C for 18 hours. The reaction mixture was slowly poured into saturated sodium bicarbonate solution and extracted with ethyl acetate (20 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 3/1, v/v) to obtain **J-6.** ¹H NMR (400 MHz, DMSO-*d*₆) δ 6.90 (s, 1H), 4.30 (t, *J =* 7.06 Hz, 2H), 4.04 (t, *J =* 7.36 Hz, 2H), 2.99 (t, *J =* 7.06 Hz, 2H), 2.88 (t, *J =* 7.36 Hz, 2H), 2.29 (s, 3H), 2.23 (s, 3H), 1.93 (s, 3H), 1.90 (s, 3H). ESI-MS calculated for C₁₅H₂₂NO₄ [M+H]⁺ =280.2, found 280.2.

### Step 5

**J-6** (100 mg, 0.36 mmol) was dissolved in methanol (5 mL), then potassium carbonate (247 mg, 1.8 mmol) was added thereto, and the mixture was stirred at room temperature for 2 hours. The reaction mixture was concentrated under reduced pressure to obtain a crude product containing **J-7,** which was directly used in the next reaction step. ESI-MS calculated for C₁₁H₁₈NO₂ [M+H]⁺ =196.3, found 196.2.

### Step 6

**J-7** (50 mg, 0.26 mmol) was dissolved in dichloromethane (5 mL), then TEA (105 mg, 1.04 mmol) and *p*-toluenesulfonyl chloride (198 mg, 1.04 mmol) were added thereto, and the mixture was stirred at room temperature for 18 hours. The reaction mixture was concentrated under reduced pressure to obtain a crude product containing the target compound, which was purified by silica gel column chromatography (petroleum ether/ethyl acetate, 4/1, v/v) to obtain **J-8.** ¹H NMR (400 MHz, DMSO-*d*₆) δ 7.61-7.52 (m, 4H), 7.38-7.26 (m, 4H), 6.74 (s, 1H), 4.32-4.25 (m, 2H), 4.05-3.97 (m, 2H), 2.85-2.76 (m, 4H), 2.34 (s, 3H), 2.32 (s, 3H), 2.15 (s, 3H), 2.05 (s, 3H). ESI-MS calculated for C₂₅H₃₀NO₆S₂ [M+H]⁺ = 504.2, found 504.2.

### Step 7

**J-8** (80 mg, 0.16 mmol) was dissolved in concentrated aqueous ammonia (5 mL) in a microwave reaction tube and heated to 90°C for 3 hours. The reaction mixture was concentrated under reduced pressure to obtain the intermediate **J**, which was directly used in the next step. ESI-MS calculated for C₁₁H₁₇N₂ [M+H]⁺ =177.1, found 177.2.

### Intermediate K

### Synthetic route:

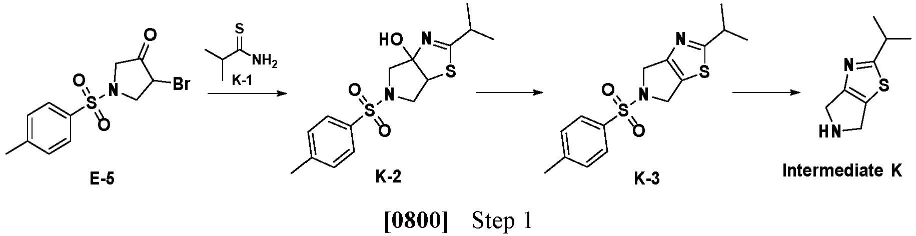

### Step 1

**E-5** (900 mg, 2.83 mmol) and **K-1** (292 mg, 2.83 mmol) were dissolved in DMF (10 mL). The reaction system was heated to 60°C and stirred for 2 hours under nitrogen atmosphere. The reaction mixture was cooled to room temperature, then diluted with water (20 mL), and extracted with ethyl acetate (20 mL × 3). The organic phases were combined, washed with saturated brine (20 mL × 3), dried over anhydrous sodium sulfate, and concentrated under reduced pressure to obtain a crude product containing **K-2,** which was directly used in the next reaction step. ESI-MS calculated for C₁₅H₂₁N₂O₃S₂ [M+H]⁺ = 341.1, found 341.0.

### Step 2

**K-2** (450 mg, 1.26 mmol) and triethylamine (635 mg, 6.28 mmol) were dissolved in dichloromethane (5 mL), then methanesulfonyl chloride (287 mg, 2.51 mmol) was added dropwise thereto, and the mixture was stirred at room temperature for 3 hours. The reaction mixture was quenched with water (20 mL) and extracted with ethyl acetate (20 mL × 3). The organic phases were combined, dried over anhydrous sodium sulfate, filtered, and concentrated under reduced pressure to obtain a crude product containing the target product, which was purified by silica gel column chromatography (dichloromethane/methanol, 9/1, v/v) to obtain **K-3.** ESI-MS calculated for C₁₅H₁₉N₂O₂S₂ [M+H]⁺ = 323.1, found 323.1.

### Step 3

**K-3** (450 mg, 1.12 mmol) and phenol (131 mg, 1.12 mmol) were dissolved in aqueous hydrobromic acid (48%, 5 mL), and the mixture was heated to 90°C and stirred for 3 hours. The reaction mixture was cooled to room temperature and then washed with ethyl acetate (10 mL × 3) to remove impurities. The aqueous phase was then concentrated to obtain a crude product containing the hydrobromide salt of intermediate **K,** which was directly used in the next reaction step. ESI-MS calculated for C₈H₁₃N₂S [M+H]⁺ = 169.1, found 169.1.

### Intermediate L

### Synthetic route:

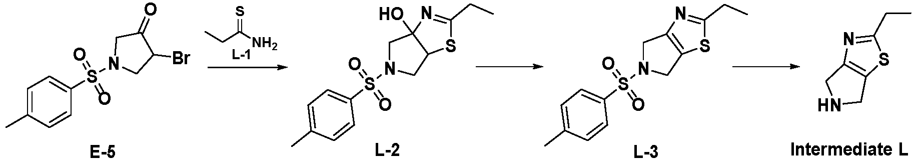

### Step 1

**E-5** (600 mg, 1.89 mmol) and **L-1** (168 mg, 1.89 mmol) were dissolved in DMF (7 mL). The reaction system was heated to 60°C and stirred for 2 hours under nitrogen atmosphere. The reaction mixture was cooled to room temperature, then diluted with water (20 mL), and extracted with ethyl acetate (20 mL × 3). The organic phases were combined, washed with saturated brine (20 mL × 3), dried over anhydrous sodium sulfate, and concentrated under reduced pressure to obtain a crude product containing **L-2,** which was directly used in the next reaction step. ESI-MS calculated for C₁₄H₁₉N₂O₃S₂ [M+H]⁺ = 327.1, found 327.0.

### Step 2

**L-2** (350 mg, 1.07 mmol) and triethylamine (543 mg, 5.36 mmol) were dissolved in dichloromethane (5 mL), then methanesulfonyl chloride (246 mg, 2.14 mmol) was added dropwise thereto, and the mixture was stirred at room temperature for 3 hours. The reaction mixture was quenched with water (20 mL) and extracted with ethyl acetate (20 mL × 3). The organic phases were combined, dried over anhydrous sodium sulfate, filtered, and concentrated under reduced pressure to obtain a crude product containing the target product, which was purified by silica gel column chromatography (dichloromethane/methanol, 9/1, v/v) to obtain **L-3.** ESI-MS calculated for C₁₄H₁₇N₂O₂S₂ [M+H]⁺ = 309.1, found 309.0.

### Step 3

**L-3** (300 mg, 0.88 mmol) and phenol (91.5 mg, 0.88 mmol) were dissolved in aqueous hydrobromic acid (48%, 3 mL), and the mixture was heated to 90°C and stirred for 3 hours. The reaction mixture was cooled to room temperature and then washed with ethyl acetate (10 mL × 3) to remove impurities. The aqueous phase was then concentrated to obtain a crude product containing the hydrobromide salt of intermediate **L,** which was directly used in the next reaction step. ESI-MS calculated for C₇H₁₁N₂S [M+H]⁺ = 155.1, found 155.1.

### Intermediate M

### Synthetic route:

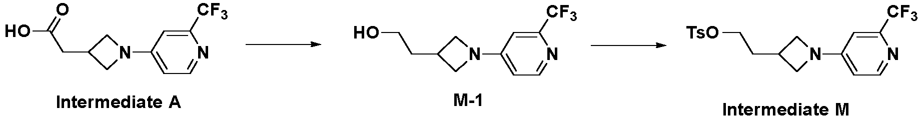

### Step 1

Intermediate **A** (2.0 g, 7.69 mmol) was dissolved in tetrahydrofuran (10 mL), cooled to 0°C, and a solution of borane dimethyl sulfide in tetrahydrofuran (1 mol/L, 10 mL, 10 mmol) was added dropwise thereto. The mixture was stirred at room temperature for 18 hours. The reaction mixture was quenched with methanol (5 mL), then diluted with water (10 mL), and extracted with ethyl acetate (20 mL × 3). The organic phases were combined, dried over anhydrous sodium sulfate, filtered, and concentrated under reduced pressure to obtain a crude product containing the target product, which was purified by silica gel column chromatography (petroleum ether/ethyl acetate, 1/1, v/v) to obtain **M-1.** ¹H NMR (400 MHz, DMSO-*d₆*) δ 8.20 (d, *J =* 5.64 Hz, 1H), 6.68 (d, *J* = 2.24 Hz, 1H), 6.50 (dd, *J =* 5.64, 2.26 Hz, 1H), 4.52-4.46 (m, 1H), 4.09 (t, *J =* 8.12 Hz, 2H), 3.67-3.61 (m, 2H), 3.47-3.41 (m, 2H), 2.90-2.79 (m, 1H), 1.80-1.72 (m, 2H).

### Step 2

Compound **M-1** (1.0 g, 4.06 mmol) was dissolved in dichloromethane (5 mL), then *p-*toluenesulfonyl chloride (1.16 g, 6.09 mmol) and TEA (820 mg, 8.12 mmol) were added thereto, and the mixture was stirred at room temperature for 2 hours. After the reaction was completed, the reaction mixture was diluted with water (15 mL) and extracted with dichloromethane (10 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain intermediate **M.** ESI-MS calculated for C₁₈H₂₀F₃N₂O₃S [M+H]⁺ = 401.1, found 401.1.

### Intermediate N

### Synthetic route:

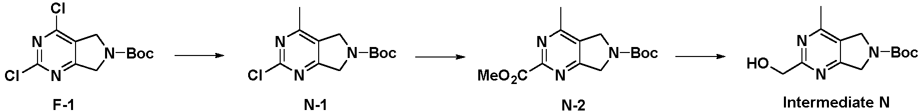

### Step 1

**F-1** (6.0 g, 20.7 mmol) was dissolved in tetrahydrofuran (125 mL), followed by the addition of iron(III) acetylacetonate (2.19 g, 6.20 mmol). The mixture was cooled to 0°C, and then a solution of methylmagnesium bromide in tetrahydrofuran (3 mol/L, 11 mL, 33.1 mmol) was added. The reaction mixture was stirred at room temperature for 18 hours. The reaction mixture was quenched with saturated ammonium chloride solution (50 mL) and extracted with ethyl acetate (50 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **N-1.** ¹H NMR (400 MHz, DMSO-*d₆*) δ 4.66-4.53 (m, 4H), 2.43 (s, 3H), 1.50-1.45 (m, 9H). ESI-MS calculated for C₁₂H₁₇ClN₃O, [M+H]⁺ = 270.1, found 270.1.

### Step 2

**N-1** (2.5 g, 9.27 mmol) was dissolved in methanol (100 mL), then 1,1'-bis(diphenylphosphino)ferrocene (520 mg, 0.93 mmol), TEA (3.75 g, 37.1 mmol), and palladium acetate (210 mg, 0.93 mmol) were added thereto. The reaction system was replaced with carbon monoxide three times, then heated to 70°C, and stirred for 18 hours. The reaction mixture was filtered, and the filtrate was concentrated under reduced pressure and purified by silica gel column chromatography (petroleum ether/ethyl acetate, 2/3, v/v) to obtain **N-2.** ESI-MS calculated for C₁₄H₂₀N₃O₄ [M+H]⁺ =294.1, found 294.1.

### Step 3

**N-2** (1.9 g, 6.48 mmol) was dissolved in methanol (35 mL), then sodium borohydride (490 mg, 12.9 mmol) was added thereto, and the mixture was stirred at room temperature for 1 hour. The reaction mixture was quenched with water (100 mL) and extracted with dichloromethane (50 mL × 3). The organic phases were combined, dried over anhydrous sodium sulfate, filtered, concentrated under reduced pressure, and purified by silica gel column chromatography (petroleum ether/ethyl acetate, 1/4, v/v) to obtain intermediate **N.** ¹H NMR (400 MHz, DMSO-*d₆*) δ 4.63-4.54 (m, 6H), 2.41 (s, 3H), 1.47 (s, 9H). ESI-MS calculated for C₁₃H₂₀N₃O₃ [M+H]⁺ = 266.1, found 266.1.

### Intermediate O

### Synthetic route:

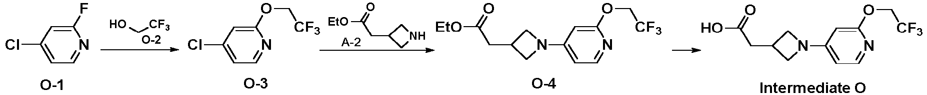

### Step 1

**0-1 (2.0** g, 15.2 mmol) was dissolved in DMF (20 mL), then **O-2** (1.52 g, 15.2 mmol) and sodium hydride (60%, 1.82 g, 45.6 mmol) were sequentially added thereto, and the mixture was heated to 60°C and stirred for 18 hours. The reaction mixture was cooled, then slowly added with saturated ammonium chloride solution (50 mL), and extracted with ethyl acetate (70 mL × 3). The organic phases were combined, washed with saturated brine (150 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **O-3.** ¹H NMR (400 MHz, DMSO-*d*₆) δ 8.21 (d, *J =* 5.50 Hz, 1H), 7.26 (dd, *J =* 5.50, 1.76 Hz, 1H), 7.22 (d, *J =* 1.74 Hz, 1H), 5.09-4.96 (m, 2H).

### Step 2

**O-3** (650 mg, 3.07 mmol) was dissolved in DMSO (6 mL), then intermediate **A-2** (1.1 g, 3.07 mmol), TEA (1.86 g, 18.4 mmol), and cesium fluoride (700 mg, 4.02 mmol) were sequentially added thereto, and the mixture was heated to 100°C and stirred for 18 hours. The reaction mixture was cooled, then added with water (30 mL), and extracted with dichloromethane (30 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **O-4.** ¹H NMR (400 MHz, DMSO-*d₆*) δ 7.76 (d, *J =* 5.76 Hz, 1H), 6.14 (dd, *J* = 5.80, 1.98 Hz, 1H), 5.77 (d, *J* = 1.96 Hz, 1H), 4.93-4.84 (m, 2H), 4.10-4.01 (m, 4H), 3.61-3.56 (m, 2H), 3.05-2.97 (m, 1H), 2.74-2.68 (m, 2H), 1.20-1.16 (m, 3H). ESI-MS calculated for C₁₄H₁₈F₃N₂O₃ [M+H]⁺ = 319.1, found 319.1.

### Step 3

**O-4** (150 mg, 0.47 mmol) was dissolved in tetrahydrofuran (4 mL) and water (1 mL), then lithium hydroxide (29 mg, 0.71 mmol) was added thereto, and the mixture was heated to 60°C and stirred for 1 hour. The reaction mixture was cooled, then added with hydrochloric acid (1 mol/L) to adjust the pH to 7, followed by the addition of water and lyophilization to obtain a crude product containing intermediate **O,** which was directly used in the next reaction step. ¹H NMR (400 MHz, DMSO-*d₆*) δ 7.73 (d, *J =* 5.80 Hz, 1H), 6.10 (dd, *J =* 5.76, 1.98 Hz, 1H), 5.72 (d, *J =* 1.96 Hz, 1H), 4.92-4.84 (m, 2H), 4.01-3.96 (m, 2H), 3.54-3.50 (m, 2H), 2.98-2.88 (m, 1H), 2.35-2.32 (m, 2H). ESI-MS calculated for C₁₂H₁₄F₃N₂O₃ [M+H]⁺ = 291.1, found 291.1.

### Intermediate P

### Synthetic route:

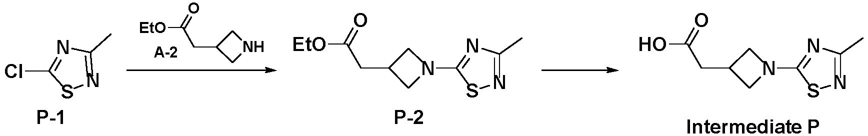

### Step 1

**P-1** (500 mg, 3.72 mmol) and **A-2** (585 mg, 4.09 mmol) were dissolved in DMSO (5 mL), then TEA (1.50 g, 14.90 mmol) and cesium fluoride (152 mg, 3.72 mmol) were added thereto, and the mixture was heated to 100°C and stirred for 18 hours. The reaction mixture was added with saturated ammonium chloride solution (20 mL) and extracted with dichloromethane (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **P-2.** ¹H NMR (400 MHz, Chloroform-*d*) δ 4.35-4.27 (m, 2H), 4.19-4.12 (m, 2H), 3.90-3.82 (m, 2H), 3.30-3.17 (m, 1H), 2.73 (d, *J* = 7.84 Hz, 2H), 2.42 (s, 3H), 1.26 (t, *J* = 7.78 Hz, 3H). ESI-MS calculated for C₁₀H₁₆N₃O₂S [M+H]⁺ = 242.1, found 242.0.

### Step 2

**P-2** (200 mg, 0.83 mmol) was dissolved in methanol (2 mL) and water (1 mL), then lithium hydroxide monohydrate (35 mg, 0.83 mmol) was added thereto, and the mixture was stirred at room temperature for 3 hours. After the reaction was completed, the reaction mixture was added with dilute hydrochloric acid (1 mol/L) to adjust the pH to 5, concentrated under reduced pressure, and then lyophilized to obtain a crude product containing intermediate **P,** which was directly used in the next reaction step. ¹H NMR (400 MHz, Chloroform-*d*) δ 4.39-4.29 (m, 2H), 3.97-3.86 (m, 2H), 3.35-3.22 (m, 1H), 2.78 (d, *J =* 8.00 Hz, 2H), 2.44 (s, 3H). ESI-MS calculated for C₈H₁₂N₃O₂S [M+H]⁺ = 214.1, found 214.0.

### Intermediate Q

### Synthetic route:

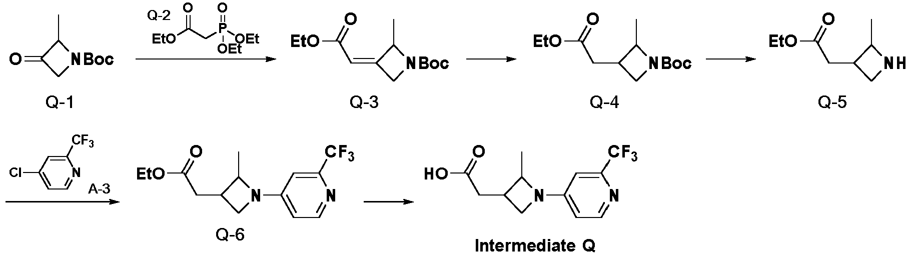

### Step 1

**Q-2** (726 mg, 3.24 mmol) was dissolved in tetrahydrofuran (20 mL), and the mixture was cooled to 0°C. Sodium hydride (60%, 130 mg, 3.24 mmol) was added thereto, and the mixture was stirred for 1 hour. **Q-1** (500 mg, 2.70 mmol) was then added thereto, and the mixture was stirred at room temperature for 18 hours. After the reaction was completed, the reaction mixture was quenched with saturated ammonium chloride solution (5 mL) and extracted with ethyl acetate (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **Q-3.** ¹H NMR (400 MHz, Chloroform-*d*) δ 5.69-5.63 (m, 1H), 5.06-5.01 (m, 0.3H), 4.81-4.76 (m, 0.7H), 4.68-4.50 (m, 2H), 4.15-4.03 (m, 2H), 1.40-1.36 (m, 12H), 1.24-1.15 (m, 3H).

### Step 2

**Q-3** (500 mg, 1.96 mmol) was dissolved in methanol (10 mL), and wet palladium on carbon (10%, 450 mg) was added thereto. The reaction system was replaced with hydrogen three times and stirred at room temperature for 1 hour. The reaction mixture was filtered, and the filtrate was concentrated under reduced pressure to obtain **Q-4.** ¹H NMR (400 MHz, Chloroform-*d*) δ 4.47-4.35 (m, 0.6H), 4.13 (q, *J =* 7.14 Hz, 2H), 4.06-3.87 (m, 1.4H), 3.52-3.48 (m, 1H), 3.02-2.87 (m, 0.6H), 2.61-2.53 (m, 2H), 2.45-2.40 (m, 0.4H) 1.44 (s, 9H), 1.40 (d, *J* = 6.20 Hz, 1.2H), 1.27-1.23 (m, 4.8H).

### Step 3

**Q-4** (250 mg, 0.97 mmol) was dissolved in dichloromethane (5 mL), then trifluoroacetic acid (3 mL) was added thereto, and the mixture was stirred at room temperature for 1 hour. The reaction mixture was concentrated under reduced pressure to obtain a crude product containing **Q-5,** which was directly used in the next reaction step. ESI-MS calculated for C₈H₁₆NO₂ [M+H]⁺ = 158.1, found 158.1.

### Step 4

The trifluoroacetate salt of **Q-5** (140 mg, 0.89 mmol) and **A-3** (173 mg, 0.95 mmol) were dissolved in dimethyl sulfoxide (5 mL), then TEA (385 mg, 3.80 mmol) and cesium fluoride (144 mg, 0.95 mmol) were sequentially added thereto, and the mixture was heated to 100°C and stirred for 18 hours. The reaction mixture was poured into water (20 mL) and extracted with dichloromethane (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 2/3, v/v) to obtain Q-6. ¹H NMR (400 MHz, DMSO-*d₆*) δ 8.23-8.19 (m, 1H), 6.72 (s, 1H), 6.58 (d, *J =* 5.84 Hz, 1H), 4.53 (t, *J =* 7.24 Hz, 0.6H), 4.23-3.88 (m, 2.4H), 3.77-3.73 (m, 0.6H), 3.51-3.47 (m, 0.4H), 3.18-3.09 (m, 1H), 2.74-2.69 (m, 3H), 1.46 (d, *J =* 6.16 Hz, 1H), 1.31 (d, *J =* 6.52 Hz, 2H), 1.21-1.18 (m, 3H). ESI-MS calculated for C₁₄H₁₈F₃N₂O₂ [M+H]⁺ = 303.1, found 303.1.

### Step 5

**Q-6** (180 mg, 0.57 mmol) was dissolved in tetrahydrofuran (4 mL) and water (1 mL), then lithium hydroxide monohydrate (38 mg, 0.89 mmol) was added thereto, and the mixture was stirred at room temperature for 3 hours. After the reaction was completed, the reaction mixture was added with dilute hydrochloric acid (1 mol/L) to adjust the pH to 5, followed by the addition of water and lyophilization to obtain a crude product containing intermediate Q, which was directly used in the next reaction step. ESI-MS calculated for C₁₂H₁₄F₃N₂O₂ [M+H]⁺ = 275.1, found 275.0.

### Intermediate R

### Synthetic route:

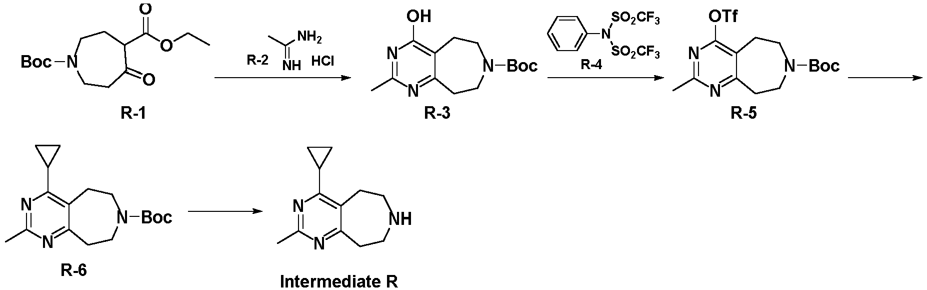

### Step 1

**R**-1 (5.0 g, 17.5 mmol) and **R**-2 (1.82 g, 19.3 mmol) were dissolved in water (32 mL) and methanol (8 mL), then potassium carbonate (3.63 g, 26.3 mmol) was added thereto, and the reaction mixture was heated to 60°C and stirred for 18 hours. The pH of the reaction mixture was adjusted to 7 with dilute hydrochloric acid (1 mol/L), and the mixture was diluted with water (50 mL) and extracted with dichloromethane (100 mL × 3). The organic phases were combined, dried over anhydrous sodium sulfate, filtered, and concentrated under reduced pressure to obtain a crude product containing the target compound. Petroleum ether (20 mL) was added and the mixture was stirred at room temperature for 30 minutes and then filtered to obtain **R-3.** ¹H NMR (400 MHz, DMSO-*d₆*) δ 12.33 (br, 1H), 3.50-3.35 (m, 4H), 2.79-2.65 (m, 4H), 2.21 (s, 3H), 1.41 (s, 9H). ESI-MS calculated for C₁₄H₂₂N₃O₃ [M+H]⁺ = 280.2, found 280.2.

### Step 2

**R-3** (1.0 g, 3.58 mmol) was dissolved in tetrahydrofuran (20 mL), and the mixture was cooled to 0°C. Sodium hydride (60%, 260 mg, 6.50 mmol) was added thereto, and the mixture was stirred for 30 minutes. **R-4** (1.92 g, 5.37 mmol) was then added thereto, and the mixture was warmed to 25°C and stirred for another 2 hours. After the reaction was completed, the pH of the reaction mixture was adjusted to 7 with dilute hydrochloric acid (1 mol/L), and the mixture was extracted with dichloromethane (50 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **R-5.** ESI-MS calculated for C₁₅H₂₁F₃N₃O₅S [M-56+H]⁺ = 356.1, found 356.0.

### Step 3

**R-5** (500 mg, 1.22 mmol), cyclopropylboronic acid (208 mg, 2.43 mmol), potassium carbonate (504 mg, 3.65 mmol), and [1,1'-bis(diphenylphosphino)ferrocene]dichloropalladium(II) (89 mg, 0.12 mmol) were added to 1,4-dioxane (15 mL) and water (5 mL), and the reaction system was heated to 90°C under nitrogen atmosphere and stirred for 18 hours. The reaction mixture was cooled to room temperature, filtered, diluted with water (15 mL), and extracted with dichloromethane (20 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **R-6.** ESI-MS calculated for C₁₇H₂₆N₃O₂ [M+H]⁺ = 304.2, found 304.2.

### Step 4

**R-6** (260 mg, 0.86 mmol) was dissolved in dichloromethane (5 mL), then trifluoroacetic acid (5 mL) was added thereto, and the mixture was stirred at room temperature for 3 hours. The reaction mixture was concentrated under reduced pressure to obtain the trifluoroacetate salt of the intermediate **R,** which was directly used in the next reaction step. ESI-MS calculated for C₁₂H₁₈N₃ [M+H]⁺ = 204.1, found 204.2.

### Intermediate S

### Synthetic route:

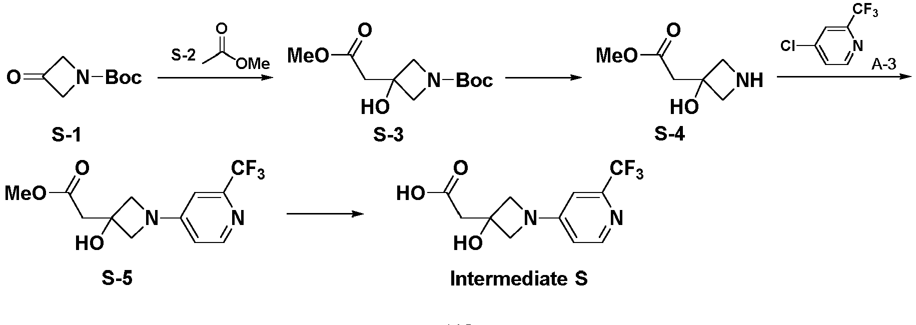

### Step 1

**S-2** (4.54 g, 61.3 mmol) was dissolved in tetrahydrofuran (120 mL), and the mixture was cooled to -78°C. A solution of LiHMDS in tetrahydrofuran (1.0 mol/L, 64.3 mL, 64.3 mmol) was slowly added dropwise thereto, and the mixture was stirred for 10 minutes. **S-1** (10 g, 58.4 mmol) was then added thereto, and the mixture was stirred for 15 minutes. The reaction system was warmed to 0°C, stirred for another 2 hours, added with ice water (100 mL), and extracted with ethyl acetate (100 mL × 2). The organic phases were combined, dried over anhydrous sodium sulfate, filtered, and concentrated under reduced pressure to obtain a crude product containing **S-3,** which was directly used in the next reaction step. ESI-MS calculated for C₁₁H₂₀NO₅ [M-56+H]⁺ = 190.1, found 190.1.

### Step 2

**S-3** (2.0 g, 8.15 mmol) was dissolved in dichloromethane (20 mL), then trifluoroacetic acid (20 mL) was added thereto, and the mixture was stirred at room temperature for 3 hours. The reaction mixture was concentrated under reduced pressure to obtain a trifluoroacetate salt of **S-4,** which was directly used in the next reaction step. ESI-MS calculated for C₆H₁₂NO₃ [M+H]⁺ = 146.1, found 146.2.

### Step 3

The trifluoroacetate salt of **S-4** (2.0 g, 13.8 mmol) and **A-3** (2.49 g, 13.8 mmol) were dissolved in dimethyl sulfoxide (10 mL), then TEA (5.58 g, 55.11 mmol) and cesium fluoride (2.1 g, 13.8 mmol) were sequentially added thereto, and the mixture was heated to 100°C and stirred for 18 hours. The reaction mixture was poured into water (80 mL) and extracted with dichloromethane (50 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 3/7, v/v) to obtain **S-5.** ¹H NMR (400 MHz, DMSO-*d₆*) δ 8.22 (d, *J* = 5.68 Hz, 1H), 6.76 (d, *J* = 2.26 Hz, 1H), 6.62 -6.51 (m, 1H), 6.03 (s, 1H), 4.16-4.10 (m, 2H), 3.90-3.85 (m, 2H), 3.60 (s, 3H), 2.82 (s, 2H). ESI-MS calculated for C₁₂H₁₄F₃N₂O₃ [M+H]⁺ = 291.1, found 291.1.

### Step 4

**S-5b** mg, 0.45 mmol) was dissolved in tetrahydrofuran (2 mL) and water (0.2 mL), then lithium hydroxide monohydrate (28 mg, 0.68 mmol) was added thereto, and the mixture was stirred at room temperature for 2 hours. After the reaction was completed, the reaction mixture was added with dilute hydrochloric acid (1 mol/L) to adjust the pH to 5, followed by the addition of water and lyophilization to obtain a crude product containing intermediate S, which was directly used in the next reaction step. ESI-MS calculated for C₁₁H₁₂F₃N₂O₃ [M+H]⁺ = 277.1, found 277.0.

### Intermediate T

### Synthetic route:

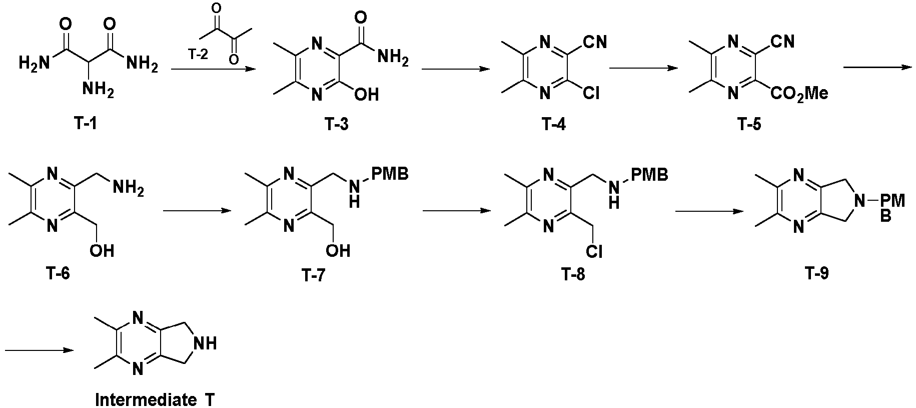

### Step 1

T-1 (20 g, 170.8 mmol) and T-2 (14.7 g, 170.8 mmol) were dissolved in water (250 mL). The mixture was cooled to 0°C, and sodium hydroxide aqueous solution (0.5 g/mL, 17.1 mL, 213 mmol) was added dropwise thereto. The reaction mixture was stirred at 0°C for 2 hours. After the reaction was completed, the reaction mixture was filtered and the solid was washed with water (20 mL × 3) to obtain **T-3.** ESI-MS calculated for C₇H₁₀N₃O₂ [M+H]⁺ = 168.1, found 168.0.

### Step 2

**T-3** (8.0 g, 47.9 mmol) was dissolved in phosphorus oxychloride (80 mL) at 0°C, and the mixture was heated to 105°C and stirred for 12 hours. After the reaction was completed, the reaction mixture was concentrated under reduced pressure, dissolved in ethyl acetate (100 mL), poured into an ice-cold saturated sodium bicarbonate solution (200 mL), and extracted with ethyl acetate (100 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 3/1, v/v) to obtain **T-4.** ¹H NMR (400 MHz, DMSO-*d₆*) δ 2.59 (s, 3H), 2.54 (s, 3H).

### Step 3

**T-4** (3.50 g, 20.9 mmol) was dissolved in methanol (35 mL), then 1,1'-bis(diphenylphosphino)ferrocene (2.36 g, 4.18 mmol), TEA (6.34 g, 62.6 mmol), and palladium acetate (470 mg, 2.09 mmol) were added thereto. The reaction system was replaced with carbon monoxide three times, then heated to 70°C, and stirred for 18 hours. The reaction mixture was filtered, and the filtrate was concentrated under reduced pressure and purified by silica gel column chromatography (petroleum ether/ethyl acetate, 7/3, v/v) to obtain **T-5.** ESI-MS calculated for C₉H₁₀N₃O₂ [M+H]⁺ = 192.1, found 192.0.

### Step 4

**T-5** (1.20 g, 6.28 mmol) was dissolved in tetrahydrofuran (12 mL), cooled to 0°C, and a solution of lithium aluminum hydride in tetrahydrofuran (1 mol/L, 15.7 mL, 15.7 mmol) was added dropwise thereto. The mixture was stirred at 0°C for 2 hours. After the reaction was completed, water (3 mL) was added dropwise to the reaction mixture. The mixture was filtered, and the filtrate was dried over anhydrous sodium sulfate, filtered, and concentrated under reduced pressure to obtain a crude product containing **T-6,** which was directly used in the next reaction step. ESI-MS calculated for C₈H₁₄N₃O [M+H]⁺ = 168.1, found 168.1.

### Step 5

**T-6** (800 mg, 4.78 mmol) and p-methoxybenzaldehyde (780 mg, 5.74 mmol) were dissolved in methanol (10 mL) and stirred at 25°C for 6 hours. Sodium borohydride (900 mg, 23.9 mmol) was then added, and the mixture was stirred for another 2 hours. Dilute hydrochloric acid (1 mol/L) was added dropwise to the reaction mixture until the pH reached 6. The mixture was then diluted with water (20 mL) and extracted with ethyl acetate (50 mL × 3). The organic phases were combined, dried over anhydrous sodium sulfate, filtered, and concentrated under reduced pressure to obtain a crude product containing the target compound, which was purified by silica gel column chromatography (dichloromethane/methanol, 9/1, v/v) to obtain **T-7.** ESI-MS calculated for C₁₆H₂₂N₃O₂ [M+H]⁺ = 288.2, found 288.1.

### Step 6

**T-7** (160 mg, 0.56 mmol) was dissolved in dichloromethane (2 mL), then thionyl chloride (341 mg, 2.88 mmol) was added thereto, and the mixture was stirred at room temperature for 3 hours. The reaction mixture was concentrated under reduced pressure to obtain a crude product containing **T-8.** ESI-MS calculated for C₁₆H₂₁ClN₃O [M+H]⁺ = 306.1, found 306.1.

### Step 7

**T-8** (140 mg, 0.46 mmol) was dissolved in tetrahydrofuran (2 mL), then potassium carbonate (130 mg, 0.92 mmol) was added thereto, and the mixture was heated to 60°C and stirred for 3 hours. The reaction mixture was filtered and concentrated under reduced pressure to obtain a crude product containing the target compound, which was purified by silica gel column chromatography (dichloromethane/methanol, 23/2, v/v) to obtain **T-9.** ESI-MS calculated for C₁₆H₂₀N₃O [M+H]⁺ = 270.2, found 270.1.

### Step 8

**T-9** (25 mg, 0.09 mmol) was dissolved in trifluoroacetic acid (1 mL) in a 10 mL microwave tube, and the mixture was heated to 80°C and stirred for 2 hours. The reaction mixture was concentrated under reduced pressure to obtain a trifluoroacetate salt of the intermediate **T,** which was directly used in the next reaction step. ESI-MS calculated for C₈H₁₂N₃ [M+H]⁺ = 150.1, found 150.1.

### Intermediate U

### Synthetic route:

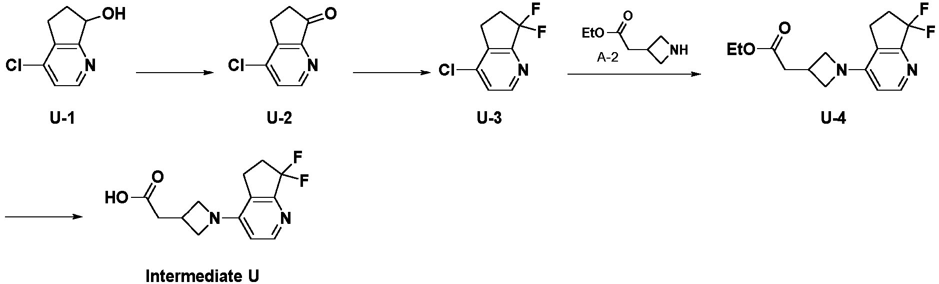

### Step 1

**U-1** (1.0 g, 5.90 mmol) and manganese dioxide (6.66 g, 76.65 mmol) were added to dichloromethane (15 mL), and the mixture was stirred at 25°C for 8 hours. The reaction mixture was filtered through diatomite, and the filtrate was concentrated under reduced pressure to obtain **U-2,** which was directly used in the next reaction step. ¹H NMR (400 MHz, Chloroform-d) δ 8.74-8.66 (m, 1H), 7.51 (d, *J* = 5.06 Hz, 1H), 3.24-3.17 (m, 2H), 2.88-2.80 (m, 2H). ESI-MS calculated for C₈H₇ClNO [M+H]⁺ = 168.0, found 168.0.

### Step 2

**U-2** (1.1 g, 6.56 mmol) was dissolved in dichloromethane (15 mL), then diethylaminosulfur trifluoride (3.7 g, 22.97 mmol) was added thereto at 0°C, and the mixture was warmed to room temperature and stirred for 18 hours. The reaction mixture was added with saturated sodium bicarbonate solution (20 mL) and extracted with dichloromethane (20 mL × 1). The organic phases were combined, dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **U-3.** ¹H NMR (400 MHz, Chloroform-d) δ 8.55 (d, *J* = 5.24 Hz, 1H), 7.39 (d, *J* = 5.22 Hz, 1H), 3.12-3.02 (m, 2H), 2.76-2.61 (m, 2H). ESI-MS calculated for C₈H₇ClF₂N [M+H]⁺ = 190.0, found 190.0.

### Step 3

Compounds **U-3** (300 mg, 1.58 mmol) and **A-2** (249 mg, 1.74 mmol) were dissolved in DMSO (10 mL), then TEA (641 mg, 6.33 mmol) and cesium fluoride (240 mg, 1.58 mmol) were added thereto, and the mixture was heated to 70°C and stirred for 18 hours. The reaction mixture was cooled to room temperature, then added with saturated ammonium chloride solution (50 mL), and extracted with ethyl acetate (40 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 3/7, v/v) to obtain **U-4.** ¹H NMR (400 MHz, Chloroform-d) δ 8.24 (d, *J* = 5.58 Hz, 1H), 6.15 (d, *J* = 5.60 Hz, 1H), 4.36-4.30 (m, 2H), 4.16 (q, *J* = 7.12 Hz, 2H), 3.88-3.81 (m, 2H), 3.20-3.09 (m, 1H), 3.03-2.96 (m, 2H), 2.71 (d, *J* = 7.78 Hz, 2H), 2.62-2.48 (m, 2H), 1.27 (t, *J =* 7.06 Hz, 3H). ESI-MS calculated for C₁₅H₁₉F₂N₂O₂ [M+H]⁺ = 297.1, found 297.1.

### Step 4

**U-4** (70 mg, 0.24 mmol) was dissolved in tetrahydrofuran (2 mL) and water (0.4 mL), then lithium hydroxide monohydrate (10 mg, 0.24 mmol) was added thereto, and the mixture was stirred at room temperature for 3 hours. After the reaction was completed, the reaction mixture was added with dilute hydrochloric acid (1 mol/L) to adjust the pH to 5, followed by the addition of water and lyophilization to obtain a crude product containing intermediate U, which was directly used in the next reaction step. ESI-MS calculated for C₁₃H₁₅F₂N₂O₂ [M+H]⁺ = 269.1, found 269.1.

### Intermediate V

### Synthetic route:

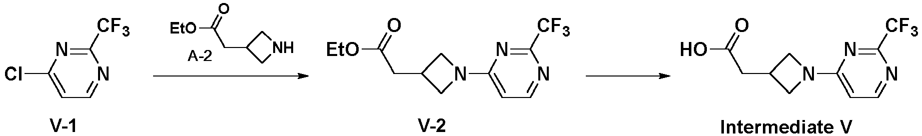

### Step 1

Compounds V-1 (100 mg, 0.55 mmol) and **A-2** (157 mg, 0.55 mmol) were dissolved in DMSO (5 mL), then TEA (221 mg, 2.19 mmol) and cesium fluoride (80 mg, 0.55 mmol) were added thereto, and the mixture was heated to 100°C and stirred for 18 hours. The reaction mixture was cooled to room temperature, then added with saturated ammonium chloride solution (20 mL), and extracted with ethyl acetate (15 mL × 3). The organic phases were combined, washed with saturated brine (30 mL), dried over anhydrous sodium sulfate, filtered, and concentrated under reduced pressure to obtain a crude product containing the target compound, which was purified by silica gel column chromatography (petroleum ether/ethyl acetate, 2/3, v/v) to obtain **V-2.** ¹H NMR (400 MHz, Chloroform-d) δ 8.24 (d, *J* = 5.70 Hz, 1H), 6.23 (d, *J* = 5.86 Hz, 1H), 4.35-4.27 (m, 2H), 4.19-4.12 (m, 2H), 3.97-3.72 (m, 2H), 3.14-3.22 (m, 1H), 2.72 (d, *J* = 7.76 Hz, 2H), 1.28-1.24 (m, 3H). ESI-MS calculated for C₁₂H₁₅F₃N₃O₂ [M+H]⁺ = 290.3, found 290.2.

### Step 2

**V-2** (80 mg, 0.28 mmol) was dissolved in tetrahydrofuran (2 mL) and water (0.4 mL), then lithium hydroxide monohydrate (18 mg, 0.41 mmol) was added thereto, and the mixture was stirred at room temperature for 2 hours. After the reaction was completed, the reaction mixture was added with dilute hydrochloric acid (1 mol/L) to adjust the pH to 5, followed by the addition of water and lyophilization to obtain a crude product containing intermediate **V,** which was directly used in the next reaction step. ESI-MS calculated for C₁₀H₁₁F₃N₃O₂ [M+H]⁺ = 262.1, found 262.0.

### Intermediate W

### Synthetic route:

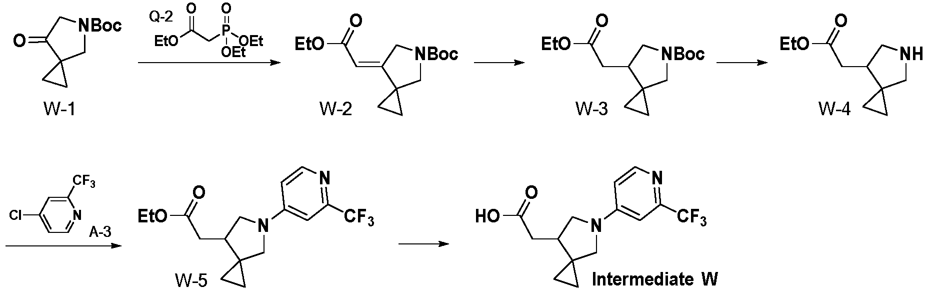

### Step 1

**Q-2** (1.0 mg, 4.54 mmol) was dissolved in tetrahydrofuran (10 mL), and the mixture was cooled to 0°C. Sodium hydride (60%, 182 mg, 4.54 mmol) was added thereto, and the mixture was stirred for 1 hour. **W-1** (800 mg, 3.79 mmol) was then added thereto, and the mixture was stirred at room temperature for 18 hours. After the reaction was completed, the reaction mixture was quenched with saturated ammonium chloride solution (10 mL) and extracted with ethyl acetate (30 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **W-2.** ESI-MS calculated for C₁₅H₂₄NO₄ [M-56+H]⁺ = 226.2, found 226.2.

### Step 2

**W-2** (300 mg, 1.07 mmol) was dissolved in ethanol (10 mL), followed by the addition of cobalt chloride hexahydrate (26 mg, 0.11 mmol) and sodium borohydride (80 mg, 2.13 mmol). The mixture was stirred at room temperature for 18 hours. The reaction mixture was concentrated under reduced pressure to obtain a crude product containing **W-3,** which was directly used in the next reaction step. ESI-MS calculated for C₁₅H₂₆NO₄ [M-56+H]⁺ = 228.2, found 228.2.

### Step 3

**W-3** (300 mg, 1.06 mmol) was dissolved in dichloromethane (5 mL), then trifluoroacetic acid (3 mL) was added thereto, and the mixture was stirred at room temperature for 3 hours. The reaction mixture was concentrated under reduced pressure to obtain a crude product containing **W-4,** which was directly used in the next reaction step. ESI-MS calculated for C₁₀H₁₈NO₂ [M+H]⁺ = 184.1, found 184.1.

### Step 4

The trifluoroacetate salt of **W-4** (180 mg, 0.98 mmol) and A-3 (200 mg, 1.09 mmol) were dissolved in DMSO (10 mL), then TEA (442 mg, 4.37 mmol) and cesium fluoride (166 mg, 1.09 mmol) were added thereto, and the mixture was heated to 100°C and stirred for 10 hours. The reaction mixture was cooled to room temperature, then added with saturated ammonium chloride solution (20 mL), and extracted with dichloromethane (15 mL × 3). The organic phases were combined, washed with saturated brine (30 mL), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **W-5.** ¹H NMR (400 MHz, Chloroform-d) δ 8.24 (d, *J* = 5.70 Hz, 1H), 6.23 (d, *J* = 5.86 Hz, 1H), 4.35-4.27 (m, 2H), 4.19-4.12 (m, 2H), 3.97-3.72 (m, 2H), 3.14-3.22 (m, 1H), 2.72 (d, *J* = 7.76 Hz, 2H), 1.28-1.24 (m, 3H). ESI-MS calculated for C₁₆H₂₀F₃N₂O₂ [M+H]⁺ = 329.1, found 329.2.

### Step 5

**W-5** (50 mg, 0.15 mmol) was dissolved in tetrahydrofuran (2 mL) and water (0.4 mL), then lithium hydroxide monohydrate (10 mg, 0.23 mmol) was added thereto, and the mixture was stirred at room temperature for 2 hours. After the reaction was completed, the reaction mixture was added with dilute hydrochloric acid (1 mol/L) to adjust the pH to 5, followed by the addition of water and lyophilization to obtain a crude product containing intermediate **W,** which was directly used in the next reaction step. ESI-MS calculated for C₁₄H₁₆F₃N₂O₂ [M+H]⁺ = 301.1, found 301.1.

### Intermediate X

### Synthetic route:

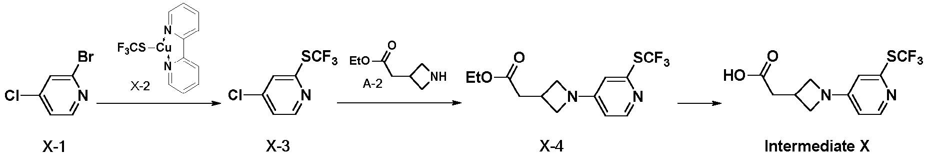

### Step 1

**X-1** (900 mg, 4.68 mmol) was dissolved in 1,4-dioxane (40 mL), then **X-2** (1.8 g, 5.61 mmol) was added thereto, and the mixture was heated to 90°C and stirred for 18 hours. The reaction mixture was cooled to room temperature, diluted with water (50 mL), and extracted with ethyl acetate (50 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/0, v/v) to obtain **X-3.** ¹H NMR (400 MHz, Chloroform-d) δ 8.52 (d, *J =* 5.34 Hz, 1H), 7.58 (d, *J =* 1.82 Hz, 1H), 7.32 (dd, *J* = 5.30, 1.84 Hz, 1H).

### Step 2

**X-3** (100 mg, 0.47 mmol) and **A-2** (201 mg, 0.70 mmol) were dissolved in DMSO (3 mL), then TEA (142 mg, 1.40 mmol) and cesium fluoride (71 mg, 0.47 mmol) were added thereto, and the mixture was heated to 100°C and stirred for 18 hours. The reaction mixture was cooled to room temperature, then added with saturated ammonium chloride solution (10 mL), and extracted with dichloromethane (15 mL × 3). The organic phases were combined, washed with saturated brine (30 mL), dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **X-4.** ESI-MS calculated for C₁₃H₁₄F₃N₂O₂S [M+H]⁺ = 321.1, found 321.1.

### Step 3

**X-4** (150 mg, 2.50 mmol) was dissolved in tetrahydrofuran (3 mL) and water (1 mL), then lithium hydroxide monohydrate (30 mg, 0.70 mmol) was added thereto, and the mixture was heated to 40°C and stirred for 2 hours. After the reaction was completed, the reaction mixture was added with dilute hydrochloric acid (1 mol/L) to adjust the pH to 5, followed by the addition of water and lyophilization to obtain a crude product containing intermediate **X,** which was directly used in the next reaction step. ESI-MS calculated for C₁₁H₁₂F₃N₂O₂S [M+H]⁺ = 293.1, found 293.1.

### Intermediate Y

### Synthetic route:

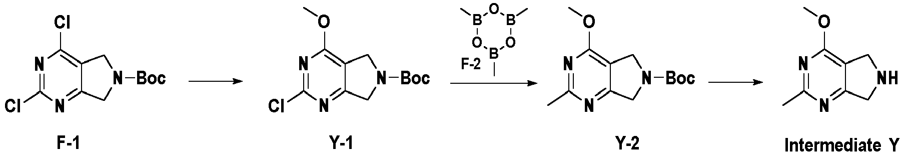

### Step 1

**F-1** (3.0 g, 10.3 mmol) was dissolved in methanol (10 mL) and cooled to 0°C, followed by addition of sodium methoxide (630 mg, 11.4 mmol). The mixture was stirred at room temperature for 18 hours. The reaction mixture was diluted with water (20 mL) and extracted with dichloromethane (20 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 3/2, v/v) to obtain **Y-1.** ¹H NMR (400 MHz, Chloroform-d) δ 4.68-4.52 (m, 4H), 4.06 (s, 3H), 1.52 (s, 9H). ESI-MS calculated for C₁₂H₁₇ClN₃O₃ [M+H]⁺ = 286.1, found 286.0.

### Step 2

**Y-1** (1.6 g, 5.60 mmol), [1,1'-bis(diphenylphosphino)ferrocene]dichloropalladium(II) dichloromethane complex (460 mg, 0.56 mmol), potassium carbonate (2.32 g, 16.8 mmol), **F-2** (1.41 g, 11.20 mmol), and 1,4-dioxane (10 mL) and water (1 mL) were heated to 90°C under nitrogen atmosphere and stirred for 18 hours. The reaction mixture was cooled to room temperature, filtered, and the filtrate was diluted with water (20 mL) and extracted with ethyl acetate (20 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/4, v/v) to obtain **Y-2**. ¹H NMR (400 MHz, Chloroform-d) δ 4.68-4.49 (m, 4H), 4.02 (s, 3H), 2.65 (s, 3H), 1.51 (s, 9H). ESI-MS calculated for C₁₃H₂₀N₃O₃ [M+H]⁺ = 266.1, found 266.1.

### Step 3

**Y-2** (1.1 g, 4.15 mmol) was dissolved in dichloromethane (10 mL), then trifluoroacetic acid (10 mL) was added thereto, and the mixture was stirred at room temperature for 3 hours. The reaction mixture was concentrated under reduced pressure to obtain a trifluoroacetate salt of intermediate Y. ESI-MS calculated for C₈H₁₂N₃O [M+H]⁺ = 166.1, found 166.1.

### Intermediate Z

### Synthetic route:

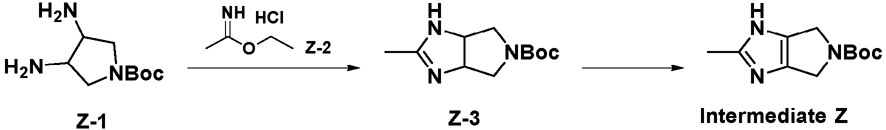

### Step 1

**Z-1** (500 mg, 2.48 mmol) and **Z-2** (340 mg, 2.73 mmol) were dissolved in ethanol (10 mL), and the reaction mixture was heated to 80°C and stirred for 2 hours. The reaction mixture was concentrated under reduced pressure to obtain a crude product containing the target compound, which was purified by silica gel column chromatography (dichloromethane/methanol, 9/1, v/v) to obtain **Z-3.** ESI-MS calculated for C₁₁H₂₀N₃O₂ [M+H]⁺ = 226.1, found 226.1.

### Step 2

Under nitrogen atmosphere, oxalyl chloride (197 mg, 1.55 mmol) was dissolved in dichloromethane (1 mL) and cooled to -78°C. DMSO (303 mg, 3.88 mmol) was slowly added dropwise thereto, and the mixture was stirred at -78°C for 30 minutes. **Z-3** (347 mg, 1.54 mmol) was dissolved in dichloromethane (1 mL) and slowly added to the above reaction mixture. After stirring for 15 minutes, DIEA (502 mg, 3.88 mmol) was added dropwise thereto. The reaction mixture was warmed to room temperature and stirred for 3 hours. The reaction mixture was diluted with dichloromethane (30 mL) and washed with saturated ammonium chloride solution (5 mL). The organic phase was washed with saturated brine (10 mL × 2), dried over anhydrous sodium sulfate, filtered, and concentrated under reduced pressure to obtain intermediate **Z.** ESI-MS calculated for C₁₁H₁₈N₃O₂ [M+H]⁺ = 224.1, found 224.2.

### Intermediate AA

### Synthetic route:

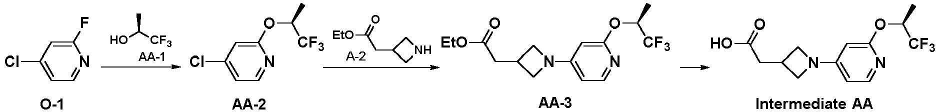

### Step 1

**AA-1** (1.4 g, 12.2 mmol) was dissolved in DMF (30 mL), and the mixture was cooled to 0°C. Sodium hydride (60%, 910 mg, 22.8 mmol) was added thereto, and the mixture was stirred for 30 minutes. **O-1** (2.0 g, 15.2 mmol) was then added thereto, and the mixture was stirred at room temperature for 18 hours. After the reaction was completed, the reaction mixture was quenched with saturated ammonium chloride solution (150 mL) and extracted with ethyl acetate (150 mL × 3). The organic phases were combined, washed with saturated brine (300 mL), dried over anhydrous sodium sulfate, filtered, and concentrated under reduced pressure to obtain a crude product containing the target compound, which was purified by silica gel column chromatography (petroleum ether/ethyl acetate, 19/1, v/v) to obtain **AA-2.** ¹H NMR (400 MHz, DMSO-*d*₆) δ 8.21 (d, *J* = 5.50 Hz, 1H), 7.25 (dd, *J* = 5.54, 1.78 Hz, 1H), 7.16 (d, *J=* 1.80 Hz, 1H), 5.95-5.86 (m, 1H), 1.45 (d, *J=* 6.68 Hz, 3H).

### Step 2

**AA-2** (400 mg, 1.77 mmol) and **A-2** (254 mg, 1.77 mmol) were dissolved in DMSO (15 mL), then TEA (1.08 g, 10.6 mmol) and cesium fluoride (404 mg, 2.66 mmol) were added thereto, and the mixture was heated to 100°C and stirred for 10 hours. The reaction mixture was cooled to room temperature, then added with saturated ammonium chloride solution (50 mL), and extracted with ethyl acetate (50 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 3/17, v/v) to obtain **AA-3.** ¹H NMR (400 MHz, Chloroform-d) δ 7.75 (d, *J* = 5.74 Hz, 1H), 6.12 (dd, *J =* 5.82, 1.98 Hz, 1H), 5.87-5.80 (m, 1H), 5.73 (d, *J* = 1.96 Hz, 1H), 4.10-4.01 (m, 4H), 3.62-3.54 (m, 2H), 3.07-2.97 (m, 1H), 2.70 (d, *J* = 7.70 Hz, 2H), 1.38 (d, *J* = 6.54 Hz, 3H), 1.18 (t, *J* = 7.10 Hz, 3H). ESI-MS calculated for C₁₅H₂₀F₃N₂O₃ [M+H]⁺ = 333.1, found 333.1.

### Step 3

**AA-3** (350 mg, 1.05 mmol) was dissolved in tetrahydrofuran (2 mL) and water (0.4 mL), then lithium hydroxide monohydrate (66 mg, 1.58 mmol) was added thereto, and the mixture was stirred at room temperature for 2 hours. After the reaction was completed, the reaction mixture was added with dilute hydrochloric acid (1 mol/L) to adjust the pH to 5, and filtered. The resulting solid was washed with water (3 mL) and dried to obtain intermediate **AA,** which was directly used in the next reaction step. ESI-MS calculated for C₁₃H₁₆F₃N₂O₃ [M+H]⁺ = 305.1, found 305.1.

### Intermediate AB

### Synthetic route:

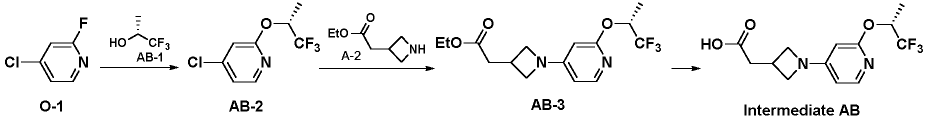

### Step 1

**AB-1** (208 mg, 1.82 mmol) was dissolved in DMF (4 mL), and the mixture was cooled to 0°C. Sodium hydride (60%, 137 mg, 3.43 mmol) was added thereto, and the mixture was stirred for 30 minutes. **O-1** (300 mg, 2.28 mmol) was then added thereto, and the mixture was stirred at room temperature for 18 hours. After the reaction was completed, the reaction mixture was quenched with saturated ammonium chloride solution (30 mL) and extracted with ethyl acetate (30 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 19/1, v/v) to obtain **AB-2**. ¹H NMR (400 MHz, DMSO-*d*₆) δ 8.21 (d, *J* = 5.50 Hz, 1H), 7.25 (dd, *J* = 5.58, 1.76 Hz, 1H), 7.16 (d, *J* = 1.74 Hz, 1H), 5.93-5.85 (m, 1H), 1.45 (d, *J=* 6.68 Hz, 3H).

### Step 2

**AB-2** (200 mg, 0.89 mmol) and **A-2** (317 mg, 2.22 mmol) were dissolved in DMSO (5 mL), then TEA (538 mg, 5.32 mmol) and cesium fluoride (202 mg, 1.33 mmol) were added thereto, and the mixture was heated to 100°C and stirred for 10 hours. The reaction mixture was cooled to room temperature, then added with saturated ammonium chloride solution (20 mL), and extracted with ethyl acetate (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 3/17, v/v) to obtain **AB-3.** ESI-MS calculated for C₁₅H₂₀F₃N₂O₃ [M+H]⁺ = 333.1, found 333.0.

### Step 3

**AB-3** (100 mg, 0.30 mmol) was dissolved in tetrahydrofuran (2 mL) and water (0.4 mL), then lithium hydroxide monohydrate (10 mg, 0.45 mmol) was added thereto, and the mixture was stirred at room temperature for 2 hours. After the reaction was completed, the reaction mixture was added with dilute hydrochloric acid (1 mol/L) to adjust the pH to 5, and filtered. The resulting solid was washed with water (3 mL) and dried to obtain intermediate AB, which was directly used in the next reaction step. ESI-MS calculated for C₁₃H₁₆F₃N₂O₃ [M+H]⁺ = 305.1, found 305.0.

### Intermediate AC

### Synthetic route:

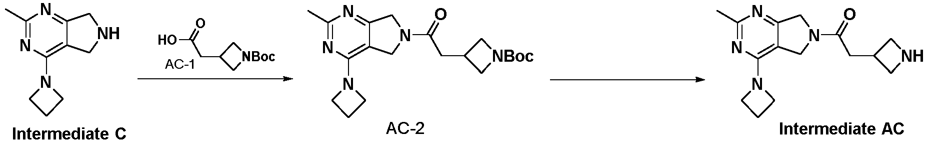

### Step 1

Intermediate **C** (1.06 g, 5.57 mmol) and intermediate AC-1 (1.20 g, 5.57 mmol) were dissolved in DMF (50 mL), then DIEA (4.32 g, 33.5 mmol) was added thereto, and the mixture was stirred at room temperature for 30 minutes. HATU (2.54 g, 6.69 mmol) was then added thereto, and the mixture was stirred at room temperature for another 1 hour. After the reaction was completed, the reaction mixture was diluted with water (100 mL) and extracted with ethyl acetate (80 mL × 3). The organic phases were combined, washed with saturated brine (150 mL), dried over anhydrous sodium sulfate, filtered, and concentrated under reduced pressure to obtain a crude product containing the target compound, which was purified by silica gel column chromatography (dichloromethane/methanol, 5/1, v/v) to obtain **AC-2.** ESI-MS calculated for C₂₀H₃₀N₅O₃ [M+H]⁺ = 388.2, found 388.3.

### Step 2

**AC-2** (2.00 g, 5.16 mmol) was dissolved in dichloromethane (15 mL), then trifluoroacetic acid (15 mL) was added thereto, and the mixture was stirred at room temperature for 3 hours. The reaction mixture was concentrated under reduced pressure to obtain a crude product containing intermediate AC, which was directly used in the next reaction step. ESI-MS calculated for C₁₅H₂₂N₅O [M+H]⁺ = 288.2, found 288.1.

### Intermediate AD

### Synthetic route:

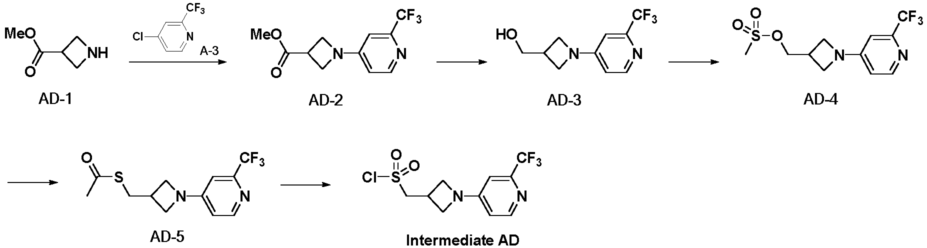

### Step 1

**AD-1** (1.90 g, 16.5 mmol) and **A-3** (2.00 g, 11.0 mmol) were dissolved in DMSO (30 mL), then TEA (4.46 g, 44.1 mmol) and cesium fluoride (1.67 g, 11.0 mmol) were added thereto, and the mixture was heated to 80°C and stirred for 12 hours. The reaction mixture was cooled to room temperature, diluted with water (150 mL), and extracted with ethyl acetate (100 mL × 3). The organic phases were combined, washed with saturated brine (200 mL), dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **AD-2**. ESI-MS calculated for C₁₁H₁₂F₃N₂O₂ [M+H]⁺ = 260.1, found 260.1.

### Step 2

**AD-2** (1.61 g, 6.15 mmol) was dissolved in tetrahydrofuran (20 mL), and a solution of lithium aluminum hydride in tetrahydrofuran (1.0 mol/L, 12.3 mL, 12.3 mmol) was added thereto at 0°C. The mixture was stirred for 2 hours. After the reaction was completed, the reaction mixture was quenched with ice water (40 mL) and extracted with ethyl acetate (40 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (dichloromethane/methanol, 19/1, v/v) to obtain **AD-3**. ¹H NMR (400 MHz, DMSO-*d*₆) δ 8.20 (d, *J =* 5.68 Hz, 1H), 6.68 (d, *J* = 2.28 Hz, 1H), 6.51 (dd, *J* = 5.70, 2.24 Hz, 1H), 4.85-4.81 (m, 1H), 4.00 (t, *J* = 8.22 Hz, 2H), 3.75-3.70 (m, 2H), 3.57 (t, *J* = 5.72 Hz, 2H), 2.91-2.82 (m, 1H). ESI-MS calculated for C₁₀H₁₂F₃N₂O [M+H]⁺ = 233.1, found 233.0.

### Step 3

**AD-3** (200 mg, 0.86 mmol) and TEA (261 mg, 2.58 mmol) were dissolved in dichloromethane (10 mL), then methanesulfonyl chloride (148 mg, 1.29 mmol) was added thereto at 0°C, and the mixture was stirred for 2 hours. After the reaction was completed, the reaction mixture was quenched with water (10 mL) and extracted with dichloromethane (20 mL × 3). The organic phases were combined, dried over anhydrous sodium sulfate, filtered, and concentrated under reduced pressure to obtain **AD-4**, which was directly used in the next reaction step. ESI-MS calculated for C₁₁H₁₄F₃N₂O₃S [M+H]⁺ = 311.1, found 311.0.

### Step 4

**AD-4** (300 mg, 0.97 mmol) was dissolved in DMF (5 mL), then potassium thioacetate (334 mg, 2.90 mmol) and 18-crown-6 ether (767 mg, 2.90 mmol) were added thereto, and the mixture was heated to 40°C and stirred for 4 hours. The reaction mixture was cooled to room temperature, diluted with water (25 mL), and extracted with ethyl acetate (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 17/3, v/v) to obtain **AD-5**. ¹H NMR (400 MHz, Chloroform-*d*) δ 8.28 (d, *J* = 5.66 Hz, 1H), 6.57 (d, *J =* 2.32 Hz, 1H), 6.32 (dd, *J* = 5.66, 2.34 Hz, 1H), 4.12-4.06 (m, 2H), 3.71-3.62 (m, 2H), 3.20 (d, *J* = 7.34 Hz, 2H), 3.10-2.99 (m, 1H), 2.38 (s, 3H). ESI-MS calculated for C₁₂H₁₄F₃N₂OS [M+H]⁺ = 291.1, found 291.0.

### Step 5

**AD-5 (170** mg, 0.59 mmol) was dissolved in acetonitrile (5 mL), followed by addition of NCS (236 mg, 1.77 mmol). Hydrochloric acid (1.0 M, 0.5 mL) was added dropwise thereto at 0°C, and the reaction mixture was stirred for 3 hours. The reaction mixture was dried over anhydrous sodium sulfate, filtered, and concentrated under reduced pressure to obtain a crude product containing intermediate **AD,** which was directly used in the next reaction step. ESI-MS calculated for C₁₀H₁₁ClF₃N₂O₂S [M+H]⁺ = 315.0, found 315.0.

### Intermediate AE

### Synthetic route:

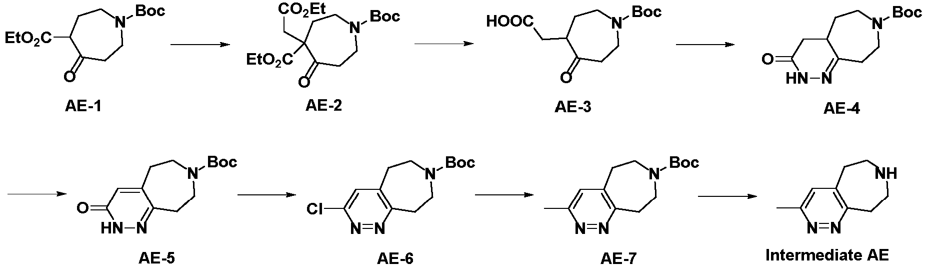

### Step 1

**AE-1** (40 g, 140 mmol) was dissolved in DMF (400 mL), then potassium carbonate (38.7 g, 280 mmol) was added thereto, and the mixture was stirred at room temperature for 30 minutes. Ethyl bromoacetate (35.1 g, 210 mmol) was then added thereto, and the mixture was stirred at room temperature for another 18 hours. The reaction mixture was diluted with water (500 mL) and extracted with ethyl acetate (500 mL × 3). The organic phases were combined, washed with saturated brine (800 mL × 3), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **AE-2.** ESI-MS calculated for C₁₈H₂₉NNaO₇ [M+Na]⁺ = 394.2, found 394.2.

### Step 2

**AE-2** (38 g, 102 mmol) was dissolved in tetrahydrofuran (150 mL) and water (150 mL), then sodium hydroxide (23.3 g, 582 mmol) was slowly added thereto, and the mixture was stirred at room temperature for 18 hours. The reaction mixture was extracted with dichloromethane (50 mL), and the aqueous phase was adjusted to pH 3 with hydrochloric acid (3 mol/L) and extracted with dichloromethane (150 mL × 3). The organic phases were combined, washed with saturated brine (200 mL × 3), dried over anhydrous sodium sulfate, filtered, and concentrated under reduced pressure to obtain a crude product containing **AE-3,** which was directly used in the next reaction step. ESI-MS calculated for C₁₃H₂₁NNaO₅ [M+H]⁺ = 294.1, found 294.1.

### Step 3

**AE-3** (14 g, 51.6 mmol) was dissolved in tetrahydrofuran (100 mL), and the mixture was cooled to 0°C. Acetic acid (55.1 g, 918 mmol) and hydrazine hydrate (98%, 2.58 g, 51.6 mmol) were added thereto, and the mixture was heated to 80°C and stirred for 18 hours. The reaction mixture was cooled to room temperature, added with saturated sodium bicarbonate solution to adjust the pH to 8, and extracted with dichloromethane (150 mL × 3). The organic phases were combined, washed with saturated brine (200 mL × 3), dried over anhydrous sodium sulfate, filtered, and concentrated under reduced pressure to obtain a crude product containing **AE-4,** which was directly used in the next reaction step. ESI-MS calculated for C₁₃H₂₂N₃O₃ [M+H]⁺ = 268.2, found 268.1.

### Step 4

**AE-4** (7.0 g, 26.2 mmol) was dissolved in toluene (70 mL), then manganese dioxide (18.2 g, 209 mmol) was added thereto, and the mixture was heated to 110°C and stirred for 12 hours. The reaction mixture was filtered and the filtrate was concentrated under reduced pressure to obtain a crude product containing the target compound, which was purified by silica gel column chromatography (dichloromethane/methanol, 9/1, v/v) to obtain **AE-5.** ESI-MS calculated for C₁₃H₂₀N₃O₃ [M+H]⁺ = 266.1, found 266.2.

### Step 5

**AE-5** (1.0 g, 3.77 mmol) was dissolved in phosphorus oxychloride (10 mL), and the reaction mixture was heated to 100°C and stirred for 8 hours. The reaction mixture was cooled to room temperature and concentrated under reduced pressure. The resulting oil was dissolved in tetrahydrofuran (10 mL) and water (2 mL). Potassium carbonate (2.6 g, 18.9 mmol) and di-tert-butyl dicarbonate (2.5 g, 11.3 mmol) were added thereto, and the mixture was stirred at room temperature for 12 hours. The reaction mixture was diluted with water (15 mL) and extracted with ethyl acetate (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **AE-6.** ESI-MS calculated for C₁₃H₂₀N₃O₃ [M+H]⁺ = 266.1, found 266.2.

### Step 6

**AE-6** (200 mg, 0.77 mmol), methylboric acid (84 mg, 1.40 mmol), potassium carbonate (292 mg, 2.11 mmol), and [1,1'-bis(diphenylphosphino)ferrocene]dichloropalladium(II) (46 mg, 0.07 mmol) were added to 1,4-dioxane (5 mL) and water (1 mL), and the reaction mixture was heated to 100°C under nitrogen atmosphere and stirred for 18 hours. The reaction mixture was cooled to room temperature, diluted with water (10 mL), and extracted with ethyl acetate (20 mL × 3). The organic phases were combined, washed with saturated brine (40 mL), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **AE-7.** ESI-MS calculated for C₁₄H₂₂N₃O₂ [M+H]⁺ = 264.2, found 264.1.

### Step 7

**AE-7** (150 mg, 0.57 mmol) was dissolved in 1,4-dioxane (5 mL), then a solution of hydrochloric acid in 1,4-dioxane (4 mol/L, 0.43 mL, 1.71 mmol) was added thereto, and the mixture was stirred at room temperature for 3 hours. The reaction mixture was concentrated under reduced pressure to obtain a crude product containing intermediate **AE,** which was directly used in the next reaction step. ESI-MS calculated for C₉H₁₄N₃ [M+H]⁺ = 164.1, found 164.1.

### Intermediate AF

### Synthetic route:

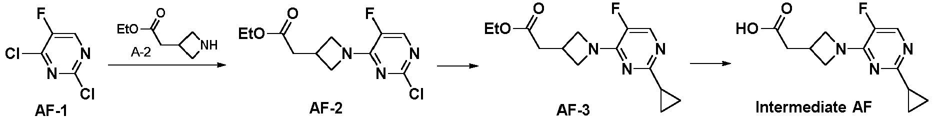

### Step 1

**AF-1** (5.0 g, 30.0 mmol) and **A-2** (4.3 g, 30.0 mmol) were dissolved in DMSO (60 mL), then TEA (18.2 g, 180 mmol) and cesium fluoride (6.8 g, 44.9 mmol) were added thereto, and the mixture was heated to 60°C and stirred for 5 hours. The reaction mixture was cooled to room temperature, diluted with water (300 mL), and extracted with ethyl acetate (250 mL × 3). The organic phases were combined, washed with saturated brine (500 mL), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **AF-2.** ESI-MS calculated for C₁₁H₁₄ClFN₃O₂ [M+H]⁺ = 274.1, found 274.0.

### Step 2

**AF-2** (3.0 g, 11.0 mmol), cyclopropylboronic acid (1.9 g, 21.9 mmol), potassium carbonate (4.5 g, 32.9 mmol), and [1,1'-bis(diphenylphosphino)ferrocene]dichloropalladium(II) (800 mg, 1.10 mmol) were added to 1,4-dioxane (10 mL) and water (2 mL), and the reaction mixture was heated to 90°C under nitrogen atmosphere and stirred for 18 hours. The reaction mixture was cooled and then concentrated under reduced pressure to obtain a crude product containing the target compound, which was purified by silica gel column chromatography (petroleum ether/ethyl acetate, 2/3, v/v) to obtain **AF-3.** ESI-MS calculated for C₁₄H₁₉FN₃O₂ [M+H]⁺ = 280.1, found 280.2.

### Step 3

**AF-3** (500 mg, 1.79 mmol) was dissolved in tetrahydrofuran (10 mL) and water (2 mL), then lithium hydroxide monohydrate (113 mg, 2.69 mmol) was added thereto, and the mixture was stirred at 40°C for 5 hours. After the reaction was completed, the reaction mixture was added with dilute hydrochloric acid (1 mol/L) to adjust the pH to 5, and filtered. The resulting solid was washed with water (3 mL) and dried to obtain intermediate **AF,** which was directly used in the next reaction step. ESI-MS calculated for C₁₂H₁₅FN₃O₂ [M+H]⁺ = 252.1, found 252.2.

### Intermediate AG

### Synthetic route:

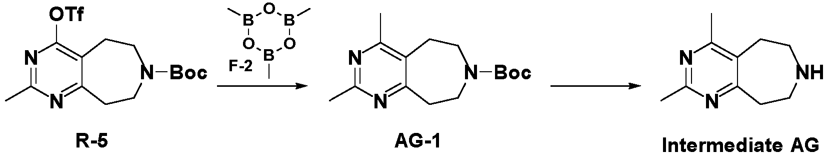

### Step 1

**R-5** (500 mg, 1.22 mmol), [1,1'-bis(diphenylphosphino)ferrocene]dichloropalladium(II) (99 mg, 0.12 mmol), potassium carbonate (504 mg, 3.65 mmol), and **F-2** (305 mg, 2.43 mmol) were added to 1,4-dioxane (10 mL) and water (1 mL), and the reaction mixture was heated to 90°C under nitrogen atmosphere and stirred for 18 hours. The reaction mixture was cooled to room temperature, diluted with water (30 mL), and extracted with ethyl acetate (15 mL × 3). The organic phases were combined, dried over anhydrous sodium sulfate, and concentrated under reduced pressure to obtain a crude product containing the target compound, which was purified by silica gel column chromatography (petroleum ether/ethyl acetate, 2/3, v/v) to obtain **AG-1.** ¹H NMR (400 MHz, DMSO-*d*₆) δ 3.54-3.47 (m, 4H), 2.99-2.95 (m, 2H), 2.88-2.84 (m, 2H), 2.46 (s, 3H), 2.41 (s, 3H), 1.37 (s, 9H). ESI-MS calculated for C₁₅H₂₄N₃O₂ [M+H]⁺ = 278.2, found 278.2.

### Step 2

**AG-1** (230 mg, 0.83 mmol) was dissolved in dichloromethane (4 mL), then trifluoroacetic acid (4 mL) was added thereto, and the mixture was stirred at room temperature for 3 hours. The reaction mixture was concentrated under reduced pressure to obtain the trifluoroacetate salt of the intermediate **AG,** which was directly used in the next reaction step. ESI-MS calculated for C₁₀H₁₆N₃ [M+H]⁺ = 178.1, found 178.1.

### Intermediate AH

### Synthetic route:

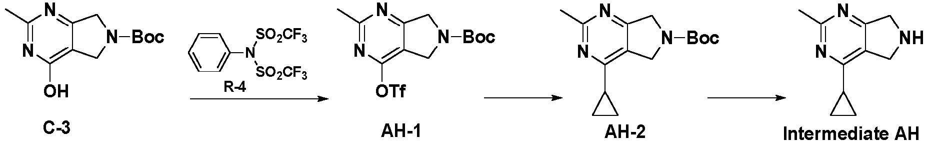

### Step 1

**C-3** (2.0 g, 4.78 mmol) was dissolved in tetrahydrofuran (40 mL), and the mixture was cooled to 0°C. Sodium hydride (60%, 480 mg, 12.0 mmol) was added thereto, and the mixture was stirred for 30 minutes. **R-4** (5.12 g, 14.3 mmol) was then added thereto, and the mixture was warmed to 25°C and stirred for another 18 hours. After the reaction was completed, the reaction mixture was diluted with saturated ammonium chloride solution (100 mL) and extracted with ethyl acetate (80 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **AH-1.** ESI-MS calculated for C₁₃H₁₇F₃N₃O₅S [M+H]⁺ = 384.1, found 384.2.

### Step 2

**AH-1** (2.0 g, 5.22 mmol), cyclopropylboronic acid (900 mg, 10.4 mmol), potassium carbonate (2.16 g, 15.7 mmol), and [1,1'-bis(diphenylphosphino)ferrocene]dichloropalladium(II) (380 mg, 0.52 mmol) were added to 1,4-dioxane (20 mL) and water (5 mL), and the reaction system was heated to 90°C under nitrogen atmosphere and stirred for 18 hours. The reaction mixture was cooled to room temperature, filtered, diluted with water (30 mL) and extracted with ethyl acetate (30 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **AH-2**. ESI-MS calculated for C₁₅H₂₂N₃O₂ [M+H]⁺ = 276.2, found 276.1.

### Step 3

**AH-2** (600 mg, 2.18 mmol) was dissolved in dichloromethane (4 mL), then trifluoroacetic acid (4 mL) was added thereto, and the mixture was stirred at room temperature for 3 hours. The reaction mixture was concentrated under reduced pressure to obtain the trifluoroacetate salt of the intermediate **AH,** which was directly used in the next reaction step. ESI-MS calculated for C₁₀H₁₄N₃ [M+H]⁺ = 176.1, found 176.1.

### Intermediate AI

### Synthetic route:

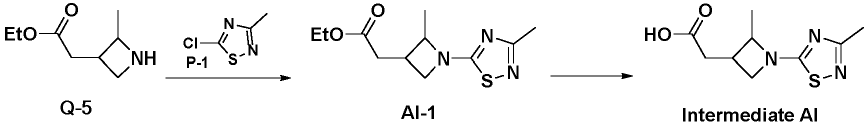

### Step 1

**Q-5** (109 mg, 0.69 mmol) and **P-1** (112 mg, 0.83 mmol) were dissolved in DMSO (5 mL), then TEA (280 mg, 2.77 mmol) and cesium fluoride (105 mg, 0.69 mmol) were added thereto, and the mixture was heated to 70°C and stirred for 18 hours. The reaction mixture was added with saturated ammonium chloride solution (20 mL) and extracted with ethyl acetate (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **AI-1.** ESI-MS calculated for C₁₁H₁₈N₃O₂S [M+H]⁺ = 256.1, found 256.1.

### Step 2

**AI-1** (90 mg, 0.35 mmol) was dissolved in tetrahydrofuran (2 mL) and water (1 mL), then lithium hydroxide monohydrate (18 mg, 0.42 mmol) was added thereto, and the mixture was stirred at room temperature for 3 hours. After the reaction was completed, the reaction mixture was added with dilute hydrochloric acid (1 mol/L) to adjust the pH to 5, concentrated under reduced pressure, and then lyophilized to obtain a crude product containing intermediate **AI,** which was directly used in the next reaction step. ESI-MS calculated for C₉H₁₄N₃O₂S [M+H]⁺ = 228.1, found 228.0.

### Intermediate AJ

### Synthetic route:

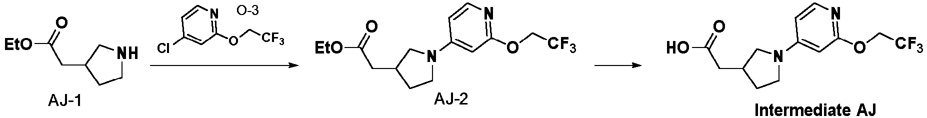

### Step 1

**O-3** (1.0 g, 5.16 mmol) was dissolved in DMSO (20 mL), then intermediate **AJ-1** (1.1 g, 5.16 mmol), TEA (1.86 g, 18.4 mmol), and cesium fluoride (1.18 g, 7.75 mmol) were sequentially added thereto, and the mixture was heated to 100°C and stirred for 18 hours. The reaction mixture was cooled, then added with water (100 mL), and extracted with dichloromethane (100 mL × 3). The organic phases were combined, washed with saturated brine (300 mL), dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **AJ-2.** ESI-MS calculated for C₁₅H₂₀F₃N₂O₃ [M+H]⁺ = 333.1, found 333.2.

### Step 2

**AJ-2** (700 mg, 2.11 mmol) was dissolved in tetrahydrofuran (20 mL) and water (5 mL), then lithium hydroxide (133 mg, 3.16 mmol) was added thereto, and the mixture was stirred at 25°C for 2 hours. After the reaction was completed, the reaction mixture was added with hydrochloric acid (1 mol/L) to adjust the pH to 6, followed by the addition of water and lyophilization to obtain a crude product containing intermediate **AJ,** which was directly used in the next reaction step. ¹H NMR (400 MHz, DMSO-*d*₆) δ 7.74 (d, *J* = 6.02Hz, 1H), 6.26 (d, *J= 6.06* Hz, 1H), 5.84 (s, 1H), 4.93-4.86 (m, 2H), 3.50-3.45 (m, 2H), 3.30-3.20 (m, 2H), 2.95-2.88 (m, 1H), 2.37-2.30 (m, 2H), 2.12 (s, 1H), 1.70-1.60 (m, 1H). ESI-MS calculated for C₁₃H₁₆F₃N₂O₃ [M+H]⁺ = 305.1, found 305.2.

### Intermediate AK

### Synthetic route:

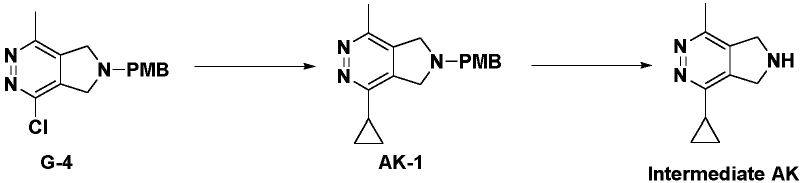

### Step 1

**G-4** (500 mg, 1.73 mmol), cyclopropylboronic acid (296 mg, 3.45 mmol), potassium carbonate (596 mg, 4.31 mmol), and [1,1'-bis(diphenylphosphino)ferrocene]dichloropalladium(II) (253 mg, 0.35 mmol) were added to 1,4-dioxane (6 mL), and the mixture was heated to 100°C under nitrogen atmosphere and stirred for 12 hours. The reaction mixture was cooled to room temperature, filtered, diluted with water (30 mL), and extracted with ethyl acetate (30 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 2/3, v/v) to obtain **AK-1.** ¹H NMR (400 MHz, DMSO-*d*₆) δ 7.30 (d, *J* = 8.60 Hz, 2H), 6.92 (d, *J* = 8.62 Hz, 2H), 4.03-4.00 (m, 2H), 3.91-3.88 (m, 2H), 3.85 (s, 2H), 3.75 (s, 3H), 2.44 (s, 3H), 1.99-1.95 (m, 1H), 1.12-1.08 (m, 2H), 1.00-0.95 (m, 2H). ESI-MS calculated for C₁₈H₂₂N₃O [M+H]⁺ = 296.2, found 296.1.

### Step 2

**AK-1** (300 mg, 1.02 mmol) and trifluoroacetic acid (4 mL) were added to a microwave tube, and the mixture was heated to 90°C and stirred for 10 hours. The reaction mixture was cooled to room temperature and concentrated under reduced pressure to obtain a trifluoroacetate containing intermediate AK, which was directly used in the next reaction step. ESI-MS calculated for C₁₀H₁₄N₃ [M+H]⁺ = 176.1, found 176.0.

### Intermediate AL

### Synthetic route:

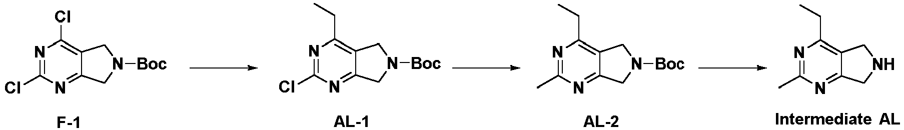

### Step 1

**F-1** (3.0 g, 10.3 mmol) was dissolved in tetrahydrofuran (30 mL), followed by the addition of iron(III) acetylacetonate (1.1 g, 3.10 mmol). The mixture was cooled to 0°C, and then a solution of ethylmagnesium bromide in tetrahydrofuran (1.0 mol/L, 19.4 mL, 19.4 mmol) was added. The reaction mixture was stirred at room temperature for 18 hours. The reaction mixture was quenched with saturated ammonium chloride solution (50 mL) and extracted with ethyl acetate (50 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **AL-1.** ESI-MS calculated for C14H21ClN3O2 [M+H]+ = 298.1, found 298.2.

### Step 2

**AL-1** (1.70 g, 5.99 mmol), methylboric acid (720 mg, 12.0 mmol), potassium carbonate (2.48 g, 18.0 mmol), and [1,1'-bis(diphenylphosphino)ferrocene]dichloropalladium(II) (440 mg, 0.66 mmol) were added to 1,4-dioxane (20 mL) and water (5 mL), and the reaction mixture was heated to 90°C under nitrogen atmosphere and stirred for 18 hours. The reaction mixture was cooled to room temperature, filtered, diluted with water (100 mL), and extracted with ethyl acetate (100 mL × 3). The organic phases were combined, washed with saturated brine (200 mL), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **AL-2.** ESI-MS calculated for C₁₄H₂₂N₃O₂ [M+H]⁺ = 264.2, found 264.3.

### Step 3

**AL-2** (380 mg, 1.44 mmol) was dissolved in dichloromethane (4 mL), then trifluoroacetic acid (2 mL) was added thereto, and the mixture was stirred at room temperature for 1 hour. The reaction mixture was concentrated under reduced pressure to obtain a trifluoroacetate salt of the intermediate **AL,** which was directly used in the next reaction step. ESI-MS calculated for C₉H₁₄N₃ [M+H]⁺ = 164.1, found 164.1.

### Intermediate AM

### Synthetic route:

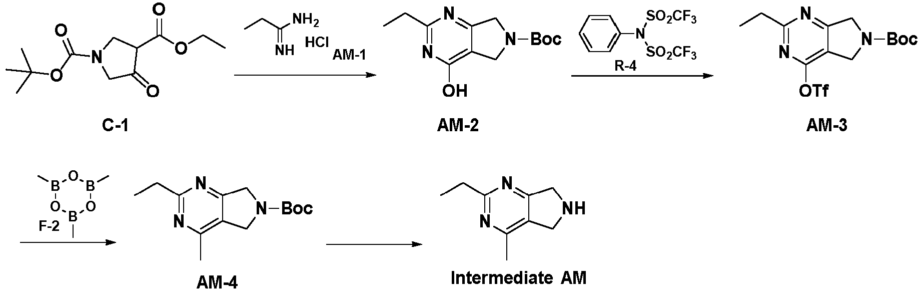

### Step 1

**C-1** (20.0 g, 77.7 mmol) and **AM-1** (10.1 g, 93.3 mmol) were dissolved in water (32 mL) and methanol (128 mL), then potassium carbonate (16.1 g, 117 mmol) was added thereto, and the reaction mixture was heated to 60°C and stirred for 18 hours. The pH of the reaction mixture was adjusted to 5-6 with dilute hydrochloric acid (1 mol/L), and the mixture was concentrated under reduced pressure, then diluted with water (100 mL), and extracted with dichloromethane (100 mL × 3). The organic phases were combined, dried over anhydrous sodium sulfate, filtered, and concentrated under reduced pressure to obtain a crude product containing the target compound. Petroleum ether (20 mL) was added and the mixture was stirred at room temperature for 30 minutes and then filtered to obtain **AM-2.** ESI-MS calculated for C₁₉H₂₀N₃O₃ [M+H]⁺ = 266.1, found 266.2.

### Step 2

**AM-2** (3.0 g, 11.3 mmol) was dissolved in tetrahydrofuran (30 mL), and the mixture was cooled to 0°C. Sodium hydride (60%, 810 mg, 33.9 mmol) was added thereto, and the mixture was stirred for 30 minutes. **R-4** (10.1 g, 28.3 mmol) was then added thereto, and the mixture was warmed to 25°C and stirred for another 18 hours. After the reaction was completed, the reaction mixture was diluted with saturated ammonium chloride solution (100 mL) and extracted with ethyl acetate (80 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **AM-3.** ESI-MS calculated for C₁₄H₁₉F₃N₃O₅S [M+H]⁺ = 398.1, found 398.1.

### Step 3

**AM-3** (1.0 g, 2.52 mmol), [1,1'-bis(diphenylphosphino)ferrocene]dichloropalladium(II) (210 mg, 0.25 mmol), potassium carbonate (1.04 g, 7.55 mmol), and **F-2** (630 mg, 5.03 mmol) were added to 1,4-dioxane (10 mL) and water (1 mL), and the reaction mixture was heated to 90°C under nitrogen atmosphere and stirred for 18 hours. The reaction mixture was cooled to room temperature, diluted with water (50 mL), and extracted with ethyl acetate (50 mL × 3). The organic phases were combined, dried over anhydrous sodium sulfate, and concentrated under reduced pressure to obtain a crude product containing the target compound, which was purified by silica gel column chromatography (petroleum ether/ethyl acetate, 1/4, v/v) to obtain **AM-4.** ESI-MS calculated for C₁₄H₂₂N₃O₂ [M+H]⁺ = 264.2, found 264.2.

### Step 4

**AM-4** (200 mg, 0.76 mmol) was dissolved in dichloromethane (4 mL), then trifluoroacetic acid (2 mL) was added thereto, and the mixture was stirred at room temperature for 1 hour. The reaction mixture was concentrated under reduced pressure to obtain a trifluoroacetate salt of the intermediate **AM,** which was directly used in the next reaction step. ESI-MS calculated for C₉H₁₄N₃ [M+H]⁺ = 164.1, found 164.2.

### Intermediate AN

### Synthetic route:

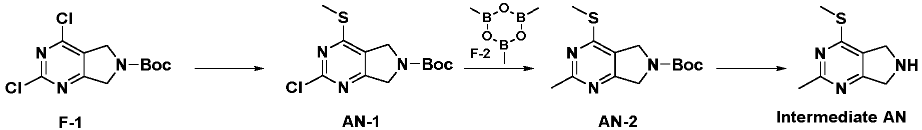

### Step 1

**F-1** (1.0 g, 3.45 mmol) was dissolved in tetrahydrofuran (10 mL) and cooled to 0°C, followed by addition of sodium thiomethoxide (270 mg, 3.79 mmol). The mixture was stirred at room temperature for 2 hours. The reaction mixture was quenched with saturated ammonium chloride solution (50 mL) and extracted with dichloromethane (50 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 3/2, v/v) to obtain **AN-1.** ESI-MS calculated for C₁₂H₁₆³⁵ClN₃O₂S [M+H]⁺ = 302.1, found 302.0.

### Step 2

**AN-1** (900 mg, 2.98 mmol), [1,1'-bis(diphenylphosphino)ferrocene]dichloropalladium(II) (240 mg, 0.30 mmol), potassium carbonate (1.24 g, 8.95 mmol), and **F-2** (748 mg, 5.96 mmol) were added to 1,4-dioxane (10 mL) and water (1 mL), and the reaction mixture was heated to 90°C under nitrogen atmosphere and stirred for 18 hours. The reaction mixture was cooled to room temperature, diluted with water (50 mL), and extracted with ethyl acetate (50 mL × 3). The organic phases were combined, dried over anhydrous sodium sulfate, and concentrated under reduced pressure to obtain a crude product containing the target compound, which was purified by silica gel column chromatography (petroleum ether/ethyl acetate, 1/4, v/v) to obtain **AN-2.** ESI-MS calculated for C₁₃H₂₀N₃O₂S [M+H]⁺ = 282.1, found 282.1.

### Step 3

**AN-2** (500 mg, 1.78 mmol) was dissolved in dichloromethane (4 mL), then trifluoroacetic acid (4 mL) was added thereto, and the mixture was stirred at room temperature for 3 hours. The reaction mixture was concentrated under reduced pressure to obtain a trifluoroacetate salt of the intermediate **AN,** which was directly used in the next reaction step. ESI-MS calculated for C₈H₁₂N₃S [M+H]⁺ = 182.1, found 182.2.

### Intermediate AO

### Synthetic route:

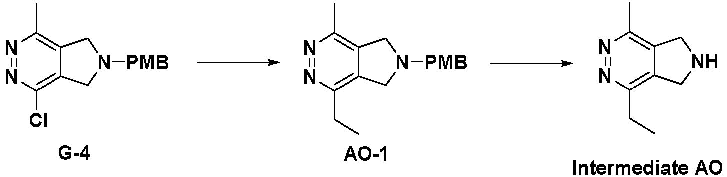

### Step 1

**G-4** (200 mg, 0.69 mmol), methanesulfonato(tricyclohexylphosphine)(2'-amino-1,1'-biphenyl-2-yl)palladium(II) (45 mg, 0.07 mmol), potassium phosphate (513 mg, 2.42 mmol), and ethylboric acid (153 mg, 2.07 mmol) were added to toluene (5 mL) and water (1 mL), and the mixture was heated to 100°C under nitrogen atmosphere and stirred for 12 hours. The reaction mixture was cooled to room temperature, diluted with water (20 mL), and extracted with ethyl acetate (20 mL × 3). The organic phases were combined, dried over anhydrous sodium sulfate, and concentrated under reduced pressure to obtain a crude product containing the target compound, which was purified by silica gel column chromatography (petroleum ether/ethyl acetate, 2/3, v/v) to obtain **AO-1.** ¹H NMR (400 MHz, DMSO-*d*₆) δ 7.29 (d, *J* = 8.54 Hz, 2H), 6.92 (d, *J* = 8.52 Hz, 2H), 3.96-3.85 (m, 6H), 3.75 (s, 3H), 2.80 (q, *J* = 7.58 Hz, 2H), 2.47 (s, 3H), 1.20 (t, *J =* 7.58 Hz, 3H). ESI-MS calculated for C₁₇H₂₂N₃O [M+H]⁺ = 284.2, found 284.1.

### Step 2

**AO-1** (150 mg, 0.53 mmol) and trifluoroacetic acid (4 mL) were added to a microwave tube, and the mixture was heated to 90°C and stirred for 10 hours. The reaction mixture was cooled to room temperature and concentrated under reduced pressure to obtain a trifluoroacetate containing intermediate **AO,** which was directly used in the next reaction step. ESI-MS calculated for C₉H₁₄N₃ [M+H]⁺ = 164.1, found 164.0.

### Intermediate AP

### Synthetic route:

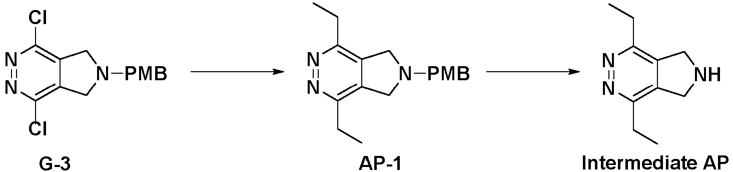

### Step 1

**G-3** (200 mg, 0.64 mmol), methanesulfonato(tricyclohexylphosphine)(2'-amino-1,1'-biphenyl-2-yl)palladium(II) (42 mg, 0.06 mmol), potassium phosphate (479 mg, 2.26 mmol), and ethylboric acid (285 mg, 3.87 mmol) were added to toluene (5 mL) and water (1 mL), and the mixture was heated to 100°C under nitrogen atmosphere and stirred for 18 hours. The reaction mixture was cooled to room temperature, diluted with water (20 mL), and extracted with ethyl acetate (20 mL × 3). The organic phases were combined, dried over anhydrous sodium sulfate, and concentrated under reduced pressure to obtain a crude product containing the target compound, which was purified by silica gel column chromatography (dichloromethane/methanol, 9/1, v/v) to obtain **AP-1.** ¹H NMR (400 MHz, DMSO-*d*₆) δ 7.31 (d, *J =* 8.60 Hz, 2H), 6.91 (d, *J* = 8.62 Hz, 2H), 3.95 (s, 3H), 3.85 (s, 2H), 3.75 (s, 4H), 2.80 (q, *J* = 7.58 Hz, 4H), 1.21 (t, *J* = 7.60 Hz, 6H). ESI-MS calculated for C₁₈H₂₄N₃O [M+H]⁺ = 298.2, found 298.1.

### Step 2

**AP-1** (150 mg, 0.50 mmol) and trifluoroacetic acid (4 mL) were added to a microwave tube, and the mixture was heated to 90°C and stirred for 10 hours. The reaction mixture was cooled to room temperature and concentrated under reduced pressure to obtain a trifluoroacetate containing intermediate **AP,** which was directly used in the next reaction step. ESI-MS calculated for C₁₀H₁₆N₃ [M+H]⁺ = 178.1, found 178.0.

### Intermediate AQ

### Synthetic route:

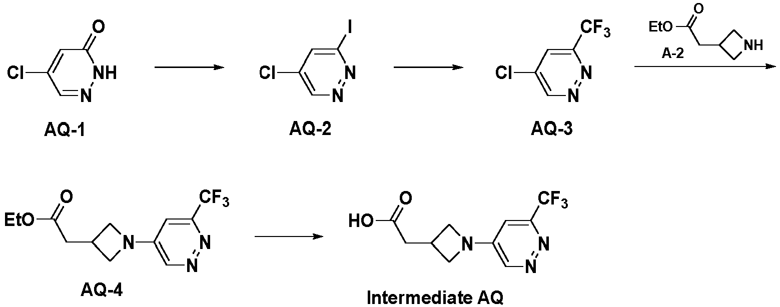

### Step 1

**AQ-1** (5.0 g, 38.3 mmol) and pyridine (3.51 g, 44.4 mmol) were dissolved in acetonitrile (100 mL). Trifluoromethanesulfonic anhydride (12.1 g, 42.9 mmol) was slowly added dropwise thereto at 0°C. The mixture was stirred at 25°C for 30 minutes, then sodium iodide (28.7 g, 191.5 mmol) was added, followed by slow dropwise addition of trifluoromethanesulfonic acid (6.32 g, 42.1 mmol). The reaction mixture was stirred at 25°C for another 3 hours. Water (50 mL) was added to the reaction mixture, and the pH was adjusted to 10 with sodium hydroxide aqueous solution (1 mol/L). Subsequently, 10% sodium carbonate solution (50 mL) and saturated sodium thiosulfate solution (100 mL) were added sequentially. The mixture was extracted with ethyl acetate (200 mL × 3). The organic phases were combined, dried over anhydrous sodium sulfate, and concentrated under reduced pressure to obtain a crude product containing the target compound, which was purified by silica gel column chromatography (petroleum ether/ethyl acetate, 9/1, v/v) to obtain **AQ-2.** ¹H NMR (400 MHz, DMSO-*d*₆) δ 9.41 (d, *J* = 2.24 Hz, 1H), 8.54 (d, *J* = 2.26 Hz, 1H). ESI-MS calculated for C₄H₃ClIN₂ [M+H]⁺ = 240.9, found 241.0.

### Step 2

Copper(I) iodide (1.48 g, 7.78 mmol) and potassium fluoride (450 mg, 7.78 mmol) were mixed and evacuated under vacuum using an oil pump. The mixture was heated with a heat gun while shaking until it turned deep yellow-green, then immediately sealed with a rubber stopper. **AQ-2** (1.70 g, 7.07 mmol) was dissolved in DMF (20 mL), followed by addition of *N*-methylpyrrolidone (7.96 g, 80.3 mmol) and (trifluoromethyl)trimethylsilane (1.11 g, 7.78 mmol). The compounds were quickly injected into a flask with a syringe, and the mixture was stirred at 25°C for 12 hours. After the reaction was completed, the reaction mixture was quenched with saturated ammonium chloride solution (100 mL), extracted with ethyl acetate (100 mL × 3). washed with saturated sodium bicarbonate (250 mL), dried over anhydrous sodium sulfate, and concentrated under reduced pressure to obtain a crude product containing the target compound, which was purified by silica gel column chromatography (petroleum ether/ethyl acetate, 4/1, v/v) to obtain **AQ-3.** ¹H NMR (400 MHz, Chloroform-*d*) δ 9.40 (d, *J* = 2.26 Hz, 1H), 7.89 (d, *J* = 2.30 Hz, 1H).

### Step 3

**A-2** (86 mg, 0.62 mmol) and **AQ-3** (100 mg, 0.55 mmol) were dissolved in dimethyl sulfoxide (5 mL), then triethylamine (222 mg, 2.19 mmol) and cesium fluoride (83 mg, 0.55 mmol) were sequentially added thereto, and the mixture was heated to 70°C and stirred for 18 hours. The reaction mixture was poured into saturated ammonium chloride solution (50 mL) and extracted with ethyl acetate (50 mL × 1). The organic phase was dried over anhydrous sodium sulfate, filtered, and concentrated under reduced pressure to obtain a crude product containing the target compound, which was purified by silica gel column chromatography (petroleum ether/ethyl acetate, 3/7, v/v) to obtain **AQ-4.** ESI-MS calculated for C₁₂H₁₅F₃N₃O₂ [M+H]⁺ = 290.1, found 290.1.

### Step 4

**AQ-4** (87 mg, 0.30 mmol) was dissolved in tetrahydrofuran (4 mL) and water (1 mL), then lithium hydroxide monohydrate (15 mg, 0.36 mmol) was added thereto, and the mixture was then stirred at room temperature for 2 hours. The pH of the reaction mixture was adjusted to 6, then water (50 mL) was added thereto, and the mixture was lyophilized to obtain a crude product containing intermediate **AQ,** which was directly used in the next reaction step. ESI-MS calculated for C₁₀H₁₁F₃N₃O₂ [M+H]⁺ = 262.1, found 262.0.

### Intermediate AR

### Synthetic route:

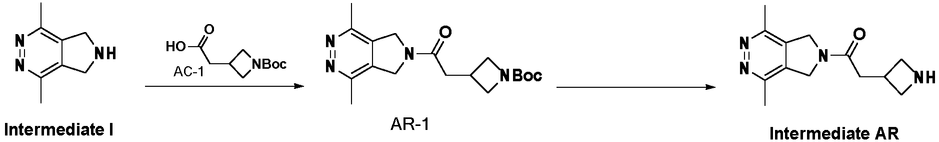

### Step 1

Intermediate **I** (800 mg, 5.36 mmol) and intermediate **AC-1** (1.15 g, 5.36 mmol) were dissolved in DMF (10 mL), then DIEA (2.08 g, 16.1 mmol) was added thereto, and the mixture was stirred at room temperature for 30 minutes. HATU (3.06 g, 8.04 mmol) was then added thereto, and the mixture was stirred at room temperature for another 1 hour. After the reaction was completed, the reaction mixture was diluted with water (100 mL) and extracted with ethyl acetate (80 mL × 3). The organic phases were combined, washed with saturated brine (150 mL), dried over anhydrous sodium sulfate, filtered, and concentrated under reduced pressure to obtain a crude product containing the target compound, which was purified by silica gel column chromatography (dichloromethane/methanol, 4/1, v/v) to obtain **AR-1.** ESI-MS calculated for C₁₈H₂₇N₄O₃ [M+H]⁺ = 347.3, found 347.2.

### Step 2

**AR-1** (1.00 g, 2.89 mmol) was dissolved in dichloromethane (6 mL), then trifluoroacetic acid (2 mL) was added thereto, and the mixture was stirred at room temperature for 3 hours. The reaction mixture was concentrated under reduced pressure to obtain a crude product containing intermediate **AR,** which was directly used in the next reaction step. ESI-MS calculated for C₁₃H₁₉N₄O [M+H]⁺ = 247.2, found 247.1.

### Preparation and synthesis of products

### Example 1

### Synthetic route:

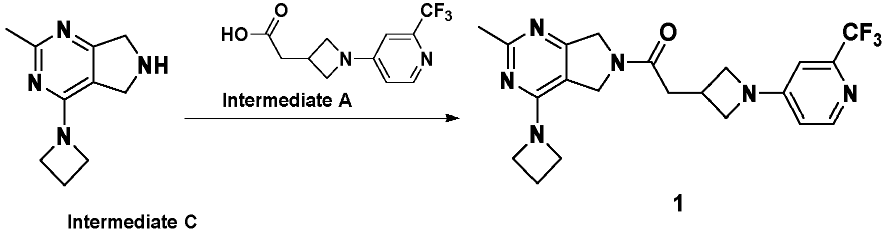

### Step 1

The trifluoroacetate salt of intermediate **Y** (200 mg, 1.1 mmol) and intermediate **A** (274 mg, 1.1 mmol) were dissolved in DMF (5 mL), then DIEA(0.41 g, 3.2 mmol) was added thereto, and the mixture was stirred at room temperature for 30 minutes. HATU (0.6 g, 1.6 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: XBridge^{®} Prep C18 OBD^{™} 10 µm, 19 * 250 mm, mobile phase: acetonitrile-water, gradient: 0 to 48%, retention time: 9 minutes) to obtain compound 1. ¹H NMR (400 MHz, DMSO-*d*₆) δ 8.20 (d, *J =* 5.64 Hz, 1H), 6.75-6.70 (m, 1H), 6.52 (d, *J* = 5.64 Hz, 1H), 4.87-4.79 (m, 1H), 4.62-4.55 (m, 2H), 4.37-4.30 (m, 1H), 4.24-4.10 (m, 6H), 3.80-3.61 (m, 2H), 3.13-3.08 (m, 1H), 2.86-2.77 (m, 2H), 2.40-2.25 (m, 5H). ESI-MS calculated for C₂₁H₂₄F₃N₆O [M+H]⁺ = 433.2, found 433.2.

### Example 2

### Synthetic route:

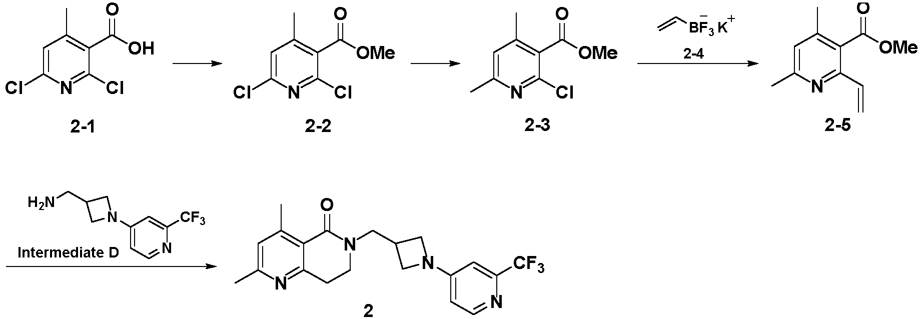

### Step 1

2-1 (500 mg, 2.43 mmol) and potassium carbonate (672 mg, 4.86 mmol) were added to DMF (5 mL), then iodomethane (1.2 g, 7.28 mmol) was added dropwise thereto, and the mixture was stirred at room temperature for 16 hours. The reaction mixture was added with water (15 mL) and extracted with ethyl acetate (5 mL × 3). The organic phases were combined, washed with saturated brine (20 mL), dried over anhydrous sodium sulfate, and concentrated under reduced pressure to obtain 2-2. ESI-MS calculated for C₈H₈Cl₂NO₂ [M+H]⁺ = 220.0, found 219.8.

### Step 2

**2-2** (1.0 g, 4.54 mmol), anhydrous potassium carbonate (3.7 g, 27.24 mmol), methylboronic acid (410 mg, 6.81 mmol), tri(o-methylphenyl)phosphine (140 mg, 0.45 mmol), and bis(triphenylphosphine)palladium(II) dichloride (640 mg, 0.91 mmol) were added DMF (5 mL). The reaction system was heated to 80°C and stirred for 18 hours under nitrogen atmosphere. The reaction mixture was cooled to room temperature and filtered. The filtrate was diluted with water (15 mL) and extracted with ethyl acetate (5 mL × 3). The organic phases were combined, washed with saturated brine (20 mL), dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **2-3.** ESI-MS calculated for C₉H₁₁ClNO₂ [M+H]⁺ = 200.0, found 200.0.

### Step 3

**2-3** (100 mg, 0.50 mmol), **2-4** (134.20 mg, 1.00 mmol), cesium fluoride (228 mg, 1.50 mmol), bis(triphenylphosphine)palladium(II) dichloride (35 mg, 0.05 mmol), and 1,4-dioxane (1.5 mL) were added to a 5 mL microwave tube. The reaction system was heated to 85°C and stirred for 18 hours under nitrogen atmosphere. The reaction mixture was cooled to room temperature and filtered. The filtrate was diluted with water (15 mL), extracted with ethyl acetate (5 mL × 3), washed with saturated brine (20 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **2-5.** ESI-MS calculated for C₁₁H₁₄NO₂ [M+H]⁺ = 192.1, found 192.0.

### Step 4

Compound **2-5** (140 mg, 0.73 mmol), intermediate **D** (220 mg, 0.95 mmol), *N,N-*diisopropylethylamine (284 mg, 2.20 mmol), and n-butanol (2 mL) were added to a 5 mL microwave tube, and the mixture was heated to 130°C and stirred for 18 hours. The reaction mixture was cooled to room temperature, filtered, and the filtrate was concentrated under reduced pressure to obtain a crude product containing the target compound, which was purified by preparative high performance liquid chromatography (chromatographic column: Bonnasil-BR C18 21.2 * 250 mm 10 µm, mobile phase: acetonitrile-0.1% formic acid aqueous solution, gradient: 7 to 37%, retention time: 9.5 minutes) to obtain compound **2.** ¹H NMR (400 MHz, DMSO-*d*₆) δ 8.22 (d, *J* = 5.64 Hz, 1H), 7.03 (s, 1H), 6.70 (d, *J* = 2.27 Hz, 1H), 6.55-6.50 (m, 1H), 4.11 (t, *J* = 8.20 Hz, 2H), 3.85-3.76 (m, 4H), 3.62-3.55 (m, 2H), 3.21-3.10 (m, 1H), 3.00 (t, *J* = 6.50 Hz, 2H), 2.58 (s, 3H), 2.43 (s, 3H). ESI-MS calculated for C₂₀H₂₂F₃N₄O [M+H]⁺ = 391.2, found 391.1.

### Example 3

### Synthetic route:

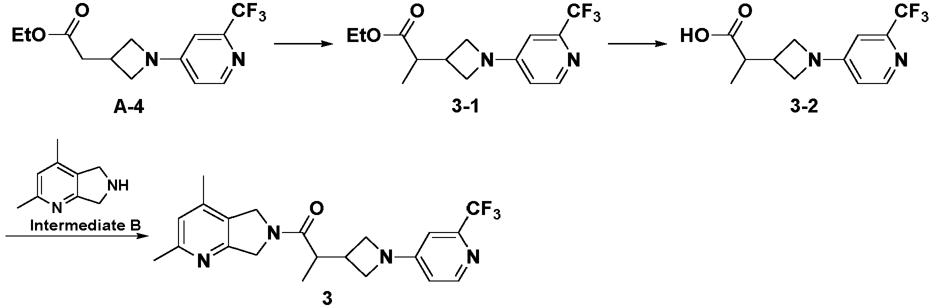

### Step 1

**A-4** (500 mg, 1.73 mmol) was dissolved in tetrahydrofuran (5 mL). The reaction mixture was cooled to -78°C under nitrogen atmosphere, and a solution of lithium bis(trimethylsilyl)amide in tetrahydrofuran (1 mol/L, 2.08 mL, 2.08 mmol) was added thereto. After stirring for 2 hours, iodomethane (295 mg, 2.08 mmol) was added dropwise thereto. The reaction mixture was stirred for another 1 hour at -78°C, then gradually warmed to room temperature. The reaction mixture was added with saturated ammonium chloride solution (10 mL) and extracted with ethyl acetate (10 mL × 3). The organic phases were combined, washed with saturated brine (20 mL), dried over anhydrous sodium sulfate, and concentrated under reduced pressure to obtain a crude product containing the target compound, which was purified by silica gel column chromatography (petroleum ether/ethyl acetate, 13/7, v/v) to obtain **3-1.** ¹H NMR (400 MHz, DMSO-*d*₆) δ 8.21 (d, *J* = 5.66 Hz, 1H), 6.72 (d, *J* = 2.20 Hz, 1H), 6.53 (dd, *J=* 5.66, 2.20 Hz, 1H), 4.15-3.95 (m, 4H), 3.78-3.72 (m, 2H), 2.95-2.83 (m, 1H), 2.81-2.75 (m, 1H), 1.20-1.13 (m, 3H), 1.09 (d, *J* = 6.92 Hz, 3H). ESI-MS calculated for C₁₄H₁₈F₃N₂O₂ [M+H]⁺ = 303.1, found 303.2.

### Step 2

**3-1** (200 mg, 0.66 mmol) was dissolved in tetrahydrofuran (5 mL) and water (1 mL), then lithium hydroxide (42 mg, 0.99 mmol) was added thereto, and the mixture was stirred at 40°C for 12 hours. The reaction mixture was cooled to room temperature, concentrated under reduced pressure, added with water and lyophilized to obtain a crude product containing **3-2,** which was directly used in the next reaction step. ESI-MS calculated for C₁₂H₁₄F₃N₂O₂ [M+H]⁺ = 275.1, found 275.2.

### Step 3

The crude product containing **3-2** (180 mg, 0.66 mmol) and intermediate **B** (200 mg, 1.35 mmol) were dissolved in DMF (5 mL), then DIEA (255 mg, 1.97 mmol) and HATU (300 mg, 0.97 mmol) were added thereto, and the mixture was stirred at room temperature for 3 hours. The reaction mixture was concentrated under reduced pressure to obtain a crude product containing the target compound, which was purified by preparative high performance liquid chromatography (chromatographic column: XBndge prep 19 * 250 mm, mobile phase: acetonitrile-10 mmol/L ammonium bicarbonate aqueous solution, gradient: 34 to 64%, retention time: 9 minutes) to obtain compound 3. ¹H NMR (400 MHz, DMSO-*d*₆) δ 8.21 (d, *J= 5.68* Hz, 1H), 7.01 (s, 1H), 6.70 (s, 1H), 6.52 (d, *J* = 5.68 Hz, 1H), 5.10-4.95 (m, 1H), 4.90-4.78 (m, 1H), 4.64-4.50 (m, 2H), 4.15-4.08 (m, 1H), 4.05-3.98 (m, 1H), 3.83-3.76 (m, 1H), 3.72-3.60 (m, 1H), 3.12-2.98 (m, 2H), 2.43 (s, 3H), 2.23 (s, 3H), 1.15-1.09 (m, 3H). ESI-MS calculated for C₂₁H₂₄F₃N₄O [M+H]⁺ = 405.2, found 405.2.

### Example 4

### Synthetic route:

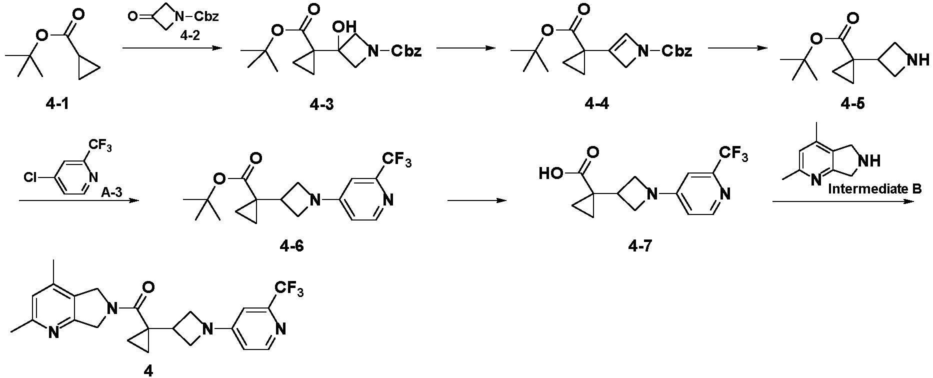

### Step 1

Tetrahydrofuran (5 mL) was added to a reaction flask, cooled to -78°C under nitrogen atmosphere, followed by addition of a solution of lithium diisopropylamide in hexane (2 mol/L, 4.22 mL, 8.44 mmol). A solution of **4-1** (1.0 g, 7.03 mmol) in tetrahydrofuran (5 mL) was then added dropwise thereto. The mixture was stirred at -78°C for 2 hours. A solution of compound **4-2** (1.7 g, 8.44 mmol) in tetrahydrofuran (5 mL) was added dropwise to the reaction. The reaction mixture was stirred at -78°C for another 1 hour. The reaction mixture was added with water (50 mL) and extracted with ethyl acetate (20 mL × 3). The organic phases were combined, washed with saturated brine (50 mL), dried over anhydrous sodium sulfate, and concentrated under reduced pressure to obtain a crude product containing the target compound, which was purified by silica gel column chromatography (petroleum ether/ethyl acetate, 7/3, v/v) to obtain **4-3.** ¹H NMR (400 MHz, DMSO-*d*₆) δ 7.17-7.03 (m, 5H), 5.55 (s, 1H), 4.80 (s, 2H), 3.71-3.57 (m, 2H), 3.55-3.39 (m, 2H), 1.13 (s, 9H), 0.77-0.69 (m, 2H), 0.63-0.56 (m, 2H). ESI-MS calculated for C₁₉H₂₆NO₅ [M+H]⁺ = 348.2, found 348.2.

### Step 2

**4-3** (13.0 g, 37.4 mmol) was dissolved in toluene (10 mL), then Burgess reagent (10.7 g, 44.9 mmol) was added thereto, and the mixture was heated to 90°C and stirred for 1 hour. The reaction mixture was cooled to room temperature and concentrated under reduced pressure to obtain a crude product containing the target compound, which was purified by silica gel column chromatography (petroleum ether/ethyl acetate, 7/3, v/v) to obtain **4-4.** ESI-MS calculated for C₁₉H₂₃NNaO₄ [M+Na]⁺ = 352.2, found 352.0.

### Step 3

**4-4** (1.6 g, 4.86 mmol) was dissolved in methanol (10 mL), and wet palladium on carbon (10%, 100 mg) was added thereto. The reaction system was replaced with hydrogen three times and stirred at room temperature for 18 hours. The reaction mixture was filtered, and the filtrate was concentrated under reduced pressure to obtain **4-5.** ESI-MS calculated for C₁₁H₂₀NO₂ [M+H]⁺ = 198.1, found 198.1.

### Step 4

**4-5** (700 mg, 3.55 mmol), **A-3** (644 mg, 3.55 mmol), cesium fluoride (538.98 mg, 3.55 mmol), triethylamine (1.08 g, 10.64 mmol), and DMSO (10 mL) were added to a 20 mL microwave tube, and the reaction mixture was heated to 100°C and stirred for 18 hours. The reaction mixture was cooled, then added with water (30 mL), and extracted with ethyl acetate (10 mL × 3). The organic phases were combined, washed with saturated brine (30 mL × 3), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **4-6.** ESI-MS calculated for C₁₇H₂₂F₃N₂O₂ [M+H]⁺ = 343.2, found 343.0.

### Step 5

**5-5** (150 mg, 0.44 mmol) was dissolved in hydrochloric acid (3 mol/L, 2 mL), and the reaction mixture was heated to 40°C and stirred for 3 hours. The reaction mixture was cooled to room temperature and concentrated under reduced pressure to obtain **5-6.** ESI-MS calculated for C₁₃H₁₄F₃N₂O₂ [M+H]⁺ = 287.1, found 287.0.

### Step 6

**5-6** (125 mg, 0.44 mmol), HATU (249 mg, 0.66 mmol), DIEA (564 mg, 4.37 mmol), and intermediate **B** (84 mg, 0.57 mmol) were added to tetrahydrofuran (3 mL), and the mixture was stirred at room temperature for 3 hours. The reaction mixture was concentrated under reduced pressure to obtain a crude product containing the target compound, which was purified by preparative high performance liquid chromatography (chromatographic column: XBndge C18 19 * 250 mm 10 µm, mobile phase: acetonitrile-10 mmol/L ammonium bicarbonate aqueous solution, gradient: 34 to 44%, retention time: 9.5 minutes) to obtain compound **4.** ¹H NMR (400 MHz, DMSO-*d*₆) δ 8.21 (d, *J* = 5.66 Hz, 1H), 7.01 (s, 1H), 6.73 (d, *J* = 2.26 Hz, 1H), 6.53 (dd, *J=* 5.66, 2.26 Hz, 1H), 4.98-4.75 (m, 2H), 4.73-4.48 (m, 2H), 4.08-4.01 (m, 2H), 3.69-3.61 (m, 2H), 3.50-3.40 (m, 1H), 2.43 (s, 3H), 2.24 (s, 3H), 1.02-0.95 (m, 2H), 0.89-0.82 (m, 2H). ESI-MS calculated for C₂₂H₂₄F₃N₄O [M+H]⁺ = 417.2, found 417.1.

### Example 5

### Synthetic route:

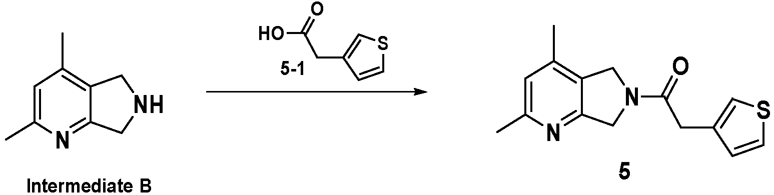

### Step 1

Intermediate **B** (100 mg, 0.70 mmol), HATU (267 mg, 0.70 mmol), and DIEA (91 mg, 2.02 mmol) were dissolved in tetrahydrofuran (2 mL), and the mixture was stirred at room temperature for 3 hours. The reaction mixture was concentrated under reduced pressure to obtain a crude product containing the target compound, which was purified by preparative high performance liquid chromatography (chromatographic column: XBndge C18 19 * 250 mm 10 µm, mobile phase: acetonitrile-10 mmol/L ammonium bicarbonate aqueous solution, gradient: 26 to 56%, retention time: 7.5 minutes) to obtain compound 5. ¹H NMR (400 MHz, DMSO-*d*₆) δ 7.48 (dd, *J* = 4.92, 2.96 Hz, 1H), 7.37-7.31 (m, 1H), 7.10-7.05 (m, 1H), 7.00 (d, *J* = 4.90 Hz, 1H), 4.90-4.82 (m, 2H), 4.63-4.55 (m, 2H), 3.78 (s, 2H), 2.42 (s, 3H), 2.23 (s, 3H). ESI-MS calculated for C₁₅H₁₇N₂OS [M+H]⁺ = 273.1, found 273.2.

### Example 6

### Synthetic route:

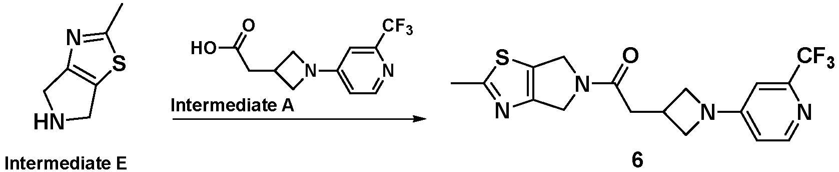

### Step 1

Intermediate **E** (200 mg, 1.00 mmol), triethylamine (505 mg, 4.99 mmol), and intermediate **A** (371 mg, 1.00 mmol) were dissolved in DMF (2 mL), then HATU (456 mg, 1.20 mmol) was added thereto, and the mixture was stirred at room temperature for 3 hours. The reaction mixture was diluted with water (20 mL) and extracted with ethyl acetate (20 mL × 3). The organic phases were combined, washed with saturated brine (20 mL × 3), dried over anhydrous sodium sulfate, and concentrated under reduced pressure to obtain a crude product containing the target compound, which was purified by preparative high performance liquid chromatography (chromatographic column: XBndge C18 19 * 250 mm 10 µm, mobile phase: acetonitrile-10 mmol/L ammonium bicarbonate aqueous solution, gradient: 30 to 52%, retention time: 9.5 minutes) to obtain compound 6. ¹HNMR (400 MHz, DMSO-*d*₆) δ 8.21 (d, *J =* 5.72 Hz, 1H), 6.71 (d, *J =* 2.23 Hz, 1H), 6.53 (dd, *J* = 5.82, 2.3 Hz, 1H), 4.83-4.68 (m, 2H), 4.59-4.44 (m, 2H), 4.21-4.14 (m, 2H), 3.73-3.66 (m, 2H), 3.18-3.06 (m, 1H), 2.85-2.78 (m, 2H), 2.68 (s, 3H). ESI-MS calculated for C₁₇H₁₈F₃N₄OS [M+H]⁺ = 383.1, found 383.0.

### Example 7

### Synthetic route:

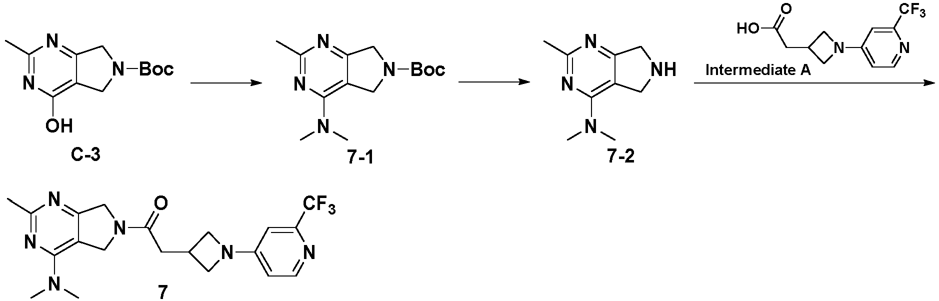

### Step 1

Dimethylamine hydrochloride (230 mg, 2.87 mmol) and **C-3** (600 mg, 2.40 mmol) were dissolved in DMF (10 mL), then DBU (1.1 g, 7.20 mmol) and PyBOP (1.5 g, 2.90 mmol) were sequentially added thereto, and the mixture was heated to 80°C and stirred for 18 hours. The reaction mixture was concentrated under reduced pressure to obtain a crude product containing the target compound, which was purified by silica gel column chromatography (petroleum ether/ethyl acetate, 1/9, v/v) to obtain 7-1. ¹HNMR (400 MHz, DMSO-*d*₆) δ 4.87-4.72 (m, 2H), 4.34-4.26 (m, 2H), 3.13-3.10 (m, 6H), 2.35 (s, 3H), 1.45 (s, 9H). ESI-MS calculated for C₁₄H₂₃N₄O₂ [M+H]⁺ = 279.2, found 279.2.

### Step 2

**7-1** (570 mg, 1.80 mmol) was dissolved in dichloromethane (10 mL) and trifluoroacetic acid (10 mL), and the mixture was stirred at room temperature for 2 hours. The reaction mixture was concentrated under reduced pressure to obtain a trifluoroacetate salt of **7-2.** ¹H NMR (400 MHz, DMSO-*d*₆) δ 4.86 (s, 2H), 4.53 (s, 2H), 3.30 (s, 6H), 2.53 (s, 3H). ESI-MS calculated for C₉H₁₅N₄ [M+H]⁺ = 179.1, found 179.2.

### Step 3

The trifluoroacetate salt of 7-2 (320 mg, 1.80 mmol) and intermediate **A** (600 mg, 1.61 mmol) were dissolved in DMF (10 mL), then DIEA (630 mg, 4.80 mmol) was added thereto, and the mixture was stirred at room temperature for 30 minutes. HATU (920 mg, 2.40 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Pntulips BP-C18, 5 µm, 21.2 * 150 mm, mobile phase: acetonitrile-10 mmol/L ammonium bicarbonate aqueous solution, gradient: 40 to 70%, retention time: 9 minutes) to obtain compound 7. ¹H NMR (400 MHz, DMSO-*d*₆) δ 8.20 (d, *J* = 5.66 Hz, 1H), 6.77-6.66 (m, 1H), 6.52 (dd, *J =* 5.38, 1.98 Hz, 1H), 5.10-5.03 (m, 1H), 4.87-4.81 (m, 1H), 4.61-4.55 (m, 1H), 4.37-4.30 (m, 1H), 4.20-4.12 (m, 2H), 3.71-3.67 (m, 2H), 3.12 (s, 6H), 2.83 (dd, *J* = 15.74, 7.66 Hz, 2H), 2.35 (s, 3H). ESI-MS calculated for C₂₀H₂₄F₃N₆O [M+H]⁺ = 421.2, found 421.2.

### Example 8

### Synthetic route:

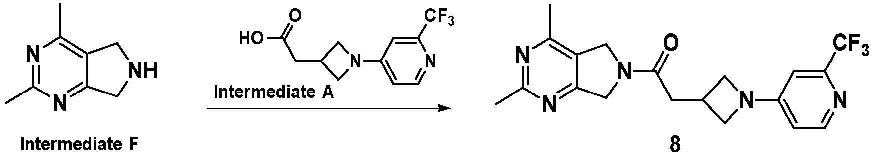

### Step 1

Intermediate **A** (100 mg, 0.38 mmol), HATU (219 mg, 0.58 mmol), DIEA (248 mg, 1.92 mmol), and the trifluoroacetate salt of intermediate **F** (114 mg, 0.77 mmol) were added to tetrahydrofuran (3 mL), and the mixture was stirred at room temperature for 3 hours. The reaction mixture was concentrated under reduced pressure to obtain a crude product containing the target compound, which was purified by preparative high performance liquid chromatography (chromatographic column: XBndge C18 19 * 250 mm 10 µm, mobile phase: acetonitrile -10 mmol/L ammonium bicarbonate aqueous solution, gradient: 25 to 37%, retention time: 8 minutes) to obtain compound **8.** ¹H NMR (400 MHz, DMSO-*d*₆) δ 8.21 (d, *J =* 5.67 Hz, 1H), 6.72 (d, *J* = 2.25 Hz, 1H), 6.53 (dd, *J* = 5.68, 2.24 Hz, 1H), 4.88-4.78 (m, 2H), 4.65-4.55 (m, 2H), 4.18 (t, *J* = 8.26 Hz, 2H), 3.74-3.66 (m, 2H), 3.20-3.06 (m, 1H), 2.89-2.83 (m, 2H), 2.58 (s, 3H), 2.42-2.37 (m, 3H). ESI-MS calculated for C₁₉H₂₁F₃N₅O [M+H]⁺ = 392.2, found 392.1.

### Example 9

### Synthetic route:

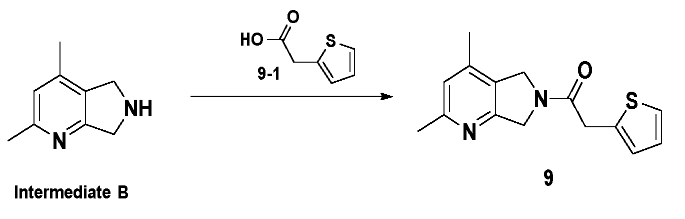

### Step 1

The trifluoroacetate salt of intermediate **B** (100 mg, 0.67 mmol), HATU (308 mg, 0.81 mmol), triethylamine (341 mg, 3.37 mmol), and **9-1** (96 mg, 0.67 mmol) were dissolved in DMF (3 mL), and the mixture was stirred at room temperature for 3 hours. The reaction mixture was poured into water (10 mL) and extracted with ethyl acetate (10 mL × 3). The organic phases were combined, dried over anhydrous sodium sulfate, and concentrated under reduced pressure to obtain a crude product containing the target compound, which was purified by preparative high performance liquid chromatography (chromatographic column: XBndge C18 19 * 250 mm 10 µm, mobile phase: acetonitrile -10 mmol/L ammonium bicarbonate aqueous solution, gradient: 24 to 50%, retention time: 8.5 minutes) to obtain compound **9.** ¹H NMR (400 MHz, DMSO-*d*₆) δ 7.40 (dd, *J =* 4.92, 1.6 Hz, 1H), 7.03 - 6.97 (m, 3H), 4.93-4.82 (m, 2H), 4.65-4.57 (m, 2H), 4.03 (s, 2H), 2.43 (s, 3H), 2.24 (s, 3H). ESI-MS calculated for C₁₅H₁₇N₂OS [M+H]⁺ = 273.1, found 273.0.

### Example 10

### Synthetic route:

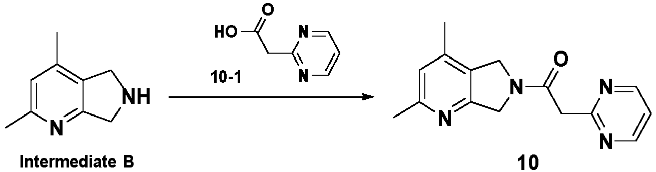

### Step 1

**10-1** (300 mg, 2.17 mmol) and the trifluoroacetate salt of intermediate **B** (644 mg, 4.34 mmol) were dissolved in DMF (5 mL), then DIEA (842 mg, 6.52 mmol) and HATU (991 mg, 2.61 mmol) were added thereto, and the mixture was stirred at room temperature for 2 hours. The reaction mixture was concentrated under reduced pressure to obtain a crude product containing the compound, which was purified by preparative high performance liquid chromatography (chromatographic column: XBndge prep 21.2 * 150 mm, mobile phase: acetonitrile -10 mmol/L ammonium bicarbonate aqueous solution, gradient: 10 to 40%, retention time: 9 minutes) to obtain compound 10. ¹H NMR (400 MHz, DMSO-*d*₆) δ 8.80-8.73 (m, 2H), 7.44-7.38 (m, 1H), 7.04-6.98 (m, 1H), 4.98-4.86 (m, 2H), 4.66-4.56 (m, 2H), 4.10 (s, 2H), 2.46-2.40 (m, 3H), 2.27-2.19 (m, 3H). ESI-MS calculated for C₁₅H₁₇N₄O [M+H]⁺ = 269.1, found 269.1.

### Example 11

### Synthetic route:

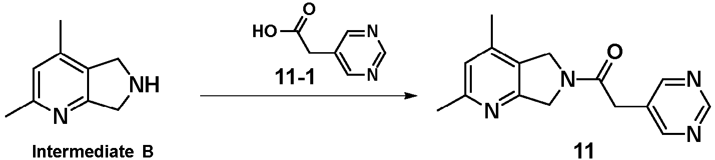

### Step 1

**11-1** (250 mg, 1.81 mmol) and the trifluoroacetate salt of intermediate **B** (536 mg, 3.62 mmol) were dissolved in DMF (5 mL), then DIEA (702 mg, 5.43 mmol) and HATU (826 mg, 2.17 mmol) were added thereto, and the mixture was stirred at room temperature for 2 hours. The reaction mixture was concentrated under reduced pressure to obtain a crude product containing the target compound, which was purified by preparative high performance liquid chromatography (chromatographic column: XBndge prep 21.2 * 150 mm, mobile phase: acetonitrile-10 mmol/L ammonium bicarbonate aqueous solution, gradient: 10 to 40%, retention time: 9 minutes) to obtain compound 11. ¹H NMR (400 MHz, DMSO-*d*₆) δ 9.08 (s, 1H), 8.73-8.69 (m, 2H), 7.06-7.01 (m, 1H), 5.00-4.92 (m, 2H), 4.66-4.57 (m, 2H), 3.89 (s, 2H), 2.44 (s, 3H), 2.29-2.23 (m, 3H). ESI-MS calculated for C₁₅H₁₇N₄O [M+H]⁺ = 269.1, found 269.1.

### Example 12

### Synthetic route:

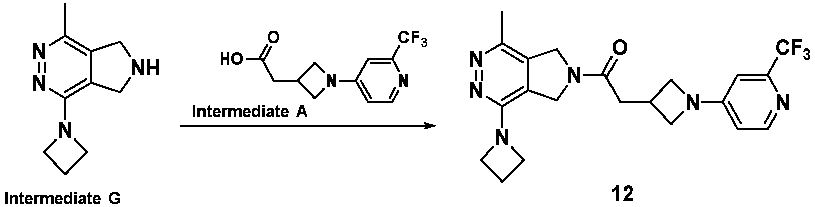

### Step 1

Intermediate **G** (50 mg, 0.26 mmol), HATU (149.9 mg, 0.39 mmol), DIEA (0.13 mL, 0.79 mmol), and intermediate **A** (136.8 mg, 0.53 mmol) were added to tetrahydrofuran (2 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 20 to 50%, retention time: 9 minutes) to obtain compound **12.** ¹H NMR (400 MHz, DMSO-*d*₆) δ 8.21 (d, *J* = 5.66 Hz, 1H), 6.74-6.70 (m, 1H), 6.56-6.51 (m, 1H), 4.88-4.84 (m, 1H), 4.81-4.77 (m, 1H), 4.66-4.61 (m, 1H), 4.58-4.55 (m, 1H), 4.19-4.14 (m, 2H), 4.13-4.08 (m, 4H), 3.73-3.65 (m, 2H), 3.17-3.06 (m, 1H), 2.89-2.80 (m, 2H), 2.40 (s, 3H), 2.38-2.29 (m, 2H). ESI-MS calculated for C₂₁H₂₄F₃N₆O [M+H]⁺ = 433.2, found 433.2.

### Example 13

### Synthetic route:

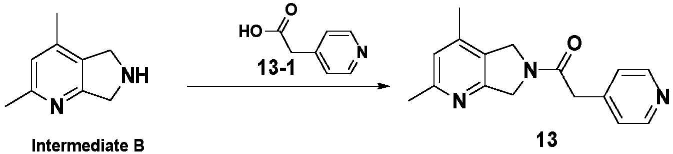

### Step 1

The trifluoroacetate salt of intermediate **B** (650 mg, 4.39 mmol), 13-1 (300 mg, 2.19 mmol), HATU (1.24 g, 3.26 mmol), and DIEA (848 mg, 6.19 mmol) were dissolved in DMF (3 mL), and the mixture was stirred at room temperature for 3 hours. The reaction mixture was diluted with water (10 mL) and extracted with ethyl acetate (10 mL × 3). The organic phases were combined, dried over anhydrous sodium sulfate, and concentrated under reduced pressure to obtain a crude product containing the target compound, which was purified by preparative high performance liquid chromatography (chromatographic column: Pntulips BP-C18, 5 µm, 21.2 * 150 mm, mobile phase: acetonitrile -10 mmol/L ammonium bicarbonate aqueous solution, gradient: 53 to 83%, retention time: 9 minutes) to obtain compound 13. ¹H NMR (400 MHz, DMSO-*d*₆) δ 8.64-8.37 (m, 2H), 7.31 (dd, *J* = 5.60, 3.66 Hz, 2H), 7.00 (d, *J* = 5.08 Hz, 1H), 4.93-4.81 (m, 2H), 4.63-4.54 (m, 2H), 3.84 (s, 2H), 2.42 (s, 3H), 2.25-2.21 (m, 3H). ESI-MS calculated for C₁₆H₁₈N₃O [M+H]⁺ = 268.1, found 268.1.

### Example 14

### Synthetic route:

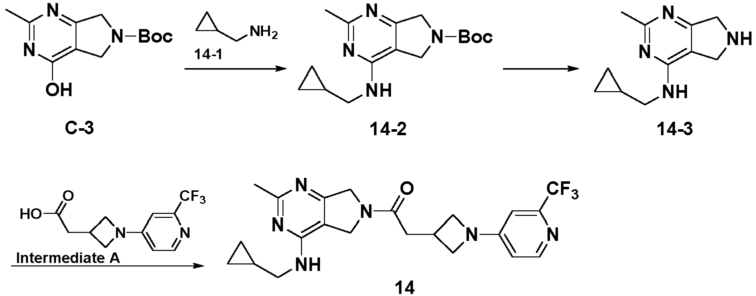

### Step 1

**14-1** (200 mg, 2.82 mmol) and C-3 (600 mg, 2.39 mmol) were dissolved in DMF (10 mL), then DBU (730 mg, 4.80 mmol) and PyBOP (1.5 g, 2.90 mmol) were added thereto, and the mixture was heated to 80°C and stirred for 18 hours. The reaction mixture was concentrated to obtain a crude product containing the target compound, which was purified by silica gel column chromatography (petroleum ether/ethyl acetate, 1/9, v/v) to obtain **14-2.** ¹H NMR (400 MHz, DMSO-*d*₆) δ 7.50-7.47 (m, 1H), 4.62-4.40 (m, 4H), 3.43-3.36 (m 2H), 2.51 (s, 3H), 1.53 (s, 9H), 1.26-1.15 (m, 1H), 0.71-0.53 (m, 2H), 0.52-0.34 (m, 2H). ESI-MS calculated for C₁₆H₂₅N₄O₂ [M+H]⁺ = 305.2, found 305.2.

### Step 2

**14-2** (520 mg, 1.71 mmol) was dissolved in dichloromethane (10 mL) and trifluoroacetic acid (10 mL), and the mixture was stirred at room temperature for 2 hours. The reaction mixture was concentrated under reduced pressure to obtain a trifluoroacetate salt of **14-3.** ¹H NMR (400 MHz, DMSO-*d*₆) δ 4.57-4.50 (m, 2H), 4.43-4.34 (m, 2H), 3.44-3.32 (m, 2H), 2.51 (s, 3H), 1.06-0.99 (m, 1H), 0.55-0.38 (m, 2H), 0.30-0.18 (m, 2H). ESI-MS calculated for C₁₁H₁₇N₄ [M+H]⁺ = 205.1, found 205.2.

### Step 3

The trifluoroacetate salt of **14-3** (330 mg, 1.59 mmol) and intermediate **A** (500 mg, 1.92 mmol) were dissolved in DMF (10 mL), then DIEA (750 mg, 5.81 mmol) was added thereto, and the mixture was stirred at room temperature for 30 minutes. HATU (880 mg, 2.32 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Pntulips BP-C18, 5 µm, 21.2 * 150 mm, mobile phase: acetonitrile-10 mmol/L ammonium bicarbonate aqueous solution, gradient: 35 to 65%, retention time: 9 minutes) to obtain compound **14.** ¹H NMR (400 MHz, DMSO-*d*₆) δ 8.20 (dd, *J* = 5.70, 1.70 Hz, 1H), 7.36-7.29 (m, 1H), 6.71 (d, *J* = 2.24 Hz, 1H), 6.52 (dd, *J* = 5.74, 2.22 Hz, 1H), 4.60-4.55 (m, 2H), 4.37 (d, *J* = 10.94 Hz, 2H), 4.17 (t, *J* = 8.28 Hz, 2H), 3.71-3.67 (m, 2H), 3.28-3.18 (m, 2H), 3.14-3.08 (m, 1H), 2.84-2.79 (m, 2H), 2.35 (s, 3H), 1.14-0.96 (m, 1H), 0.45-0.40 (m, 2H), 0.24-0.20 (m, 2H). ESI-MS calculated for C₂₂H₂₆F₃N₆O [M+H]⁺ = 447.2, found 447.2.

### Example 15

### Synthetic route:

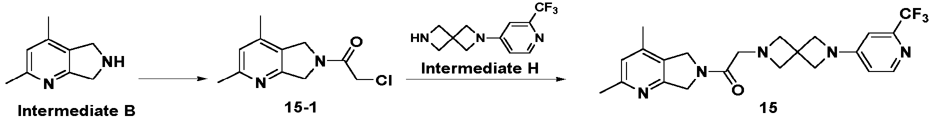

### Step 1

Intermediate **B** (500 mg, 1.69 mmol) and potassium carbonate (1.2 g, 8.43 mmol) were added to dichloromethane (10 mL). At 0°C, chloroacetyl chloride (228 mg, 2.02 mmol) was added dropwise. The reaction mixture was slowly warmed to room temperature and stirred for 3 hours, quenched with water (10 mL) and extracted with dichloromethane (10 mL × 3). The organic phases were combined, dried over anhydrous sodium sulfate, and concentrated under reduced pressure to obtain a crude product containing the target compound **15-1,** which was directly used in the next reaction step. ESI-MS calculated for C₁₁H₁₄ClN₂O [M+H]⁺ = 225.1, found 225.0.

### Step 2

The trifluoroacetate salt of intermediate **H** (216 mg, 0.89 mmol), DIEA (575 mg, 4.45 mmol), and **15-1** (200 mg, 0.89 mmol) were added to acetonitrile (4 mL), and the mixture was stirred at room temperature for 3 hours. The reaction mixture was concentrated under reduced pressure to obtain a crude product containing the compound, which was purified by preparative high performance liquid chromatography (chromatographic column: XBndge C18 19 * 250 mm , 10 µm, mobile phase: acetonitrile -10 mmol/L ammonium bicarbonate aqueous solution, gradient: 25 to 50%, retention time: 10.5 minutes) to obtain compound **15.** ¹HNMR (400 MHz, DMSO-*d*₆) δ 8.22 (d, *J =* 5.60 Hz, 1H), 6.98 (s, 1H), 6.69 (d, *J =* 2.24 Hz, 1H), 6.52 (dd, *J =* 5.62, 2.28 Hz, 1H), 4.80-4.73 (m, 2H), 4.56-4.52 (m, 2H), 4.10 (s, 4H), 3.50 (s, 4H), 3.35-3.34 (m, 2H), 2.44 (s, 3H), 2.24 (s, 3H). ESI-MS calculated for C₂₂H₂₅F₃N₅O [M+H]⁺ = 432.2, found 432.2.

### Example 16

### Synthetic route:

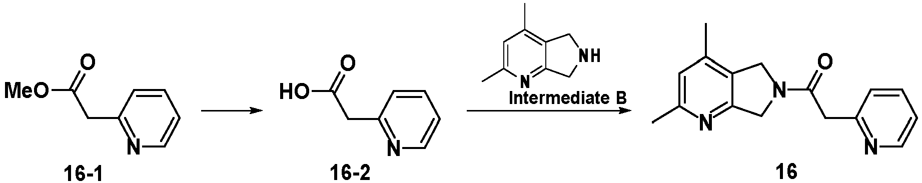

### Step 1

**16-1** (400 mg, 2.65 mmol) was dissolved in tetrahydrofuran (5 mL) and water (1 mL), then lithium hydroxide (167 mg, 3.98 mmol) was added thereto, and the mixture was stirred at 40°C for 12 hours. The reaction mixture was cooled to room temperature, concentrated under reduced pressure, added with water and lyophilized to obtain a crude product containing **16-2,** which was directly used in the next reaction step. ESI-MS calculated for C₇H₈NO₂ [M+H]⁺ = 138.1, found 138.0.

### Step 2

**16-2** (360 mg, 2.63 mmol) and the trifluoroacetate salt of intermediate **B** (778 mg, 5.25 mmol) were dissolved in DMF (5 mL), then DIEA (1.02 g, 7.88 mmol) and HATU (1.20 g, 3.15 mmol) were added thereto, and the mixture was stirred at room temperature for 2 hours. The reaction mixture was concentrated under reduced pressure to obtain a crude product containing the target compound, which was purified by preparative high performance liquid chromatography (chromatographic column: XBndge prep 21.2 * 150 mm, mobile phase: acetonitrile-10 mmol/L ammonium bicarbonate aqueous solution, gradient: 20 to 50%, retention time: 9 minutes) to obtain compound 16. ¹H NMR (400 MHz, DMSO-*d*₆) δ 8.53-8.47 (m, 1H), 7.80-7.72 (m, 1H), 7.40-7.33 (m, 1H), 7.31-7.25 (m, 1H), 7.04-6.99 (m, 1H), 5.00-4.89 (m, 2H), 4.64-4.56 (m, 2H), 3.96 (s, 2H), 2.43 (s, 3H), 2.24 (s, 3H). ESI-MS calculated for C₁₆H₁₈N₃O [M+H]⁺ = 268.1, found 268.1.

### Example 17

### Synthetic route:

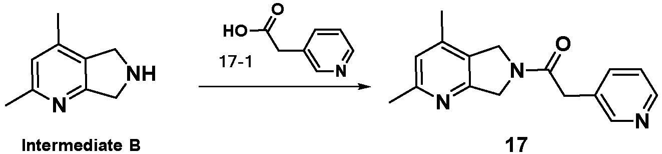

### Step 1

The trifluoroacetate salt of intermediate **B** (100 mg, 0.68 mmol), **17-1** (100 mg, 0.73 mmol), HATU (267 mg, 0.70 mmol), and DIEA (349 mg, 2.03 mmol) were dissolved in tetrahydrofuran (2 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 6 to 36%, retention time: 12 minutes) to obtain compound 17. ¹H NMR (400 MHz, DMSO-*d*₆) δ 8.50-8.45 (m, 2H), 7.72-7.66 (m, 1H), 7.45-7.38 (m, 1H), 7.02 (d, *J =* 5.79 Hz, 1H), 4.97-4.90 (m, 2H), 4.64-4.56 (m, 2H), 3.84 (s, 2H), 2.44 (s, 3H), 2.26-2.24 (m, 3H). ESI-MS calculated for C₁₆H₁₈N₃O [M+H]⁺ = 268.1, found 268.1.

### Example 18

### Synthetic route:

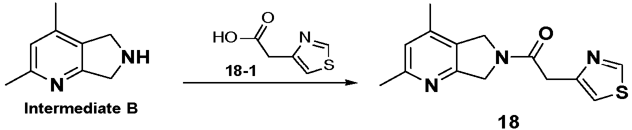

### Step 1

The trifluoroacetate salt of intermediate **B** (200 mg, 1.35 mmol), **18-1** (150 mg, 1.05 mmol), HATU (513 mg, 1.35 mmol), and DIEA (349 mg, 2.70 mmol) were dissolved in tetrahydrofuran (2 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 5 to 35%, retention time: 12 minutes) to obtain compound **18.** ¹H NMR (400 MHz, DMSO-*d*₆) δ 9.05 (s, 1H), 7.55 (s, 1H), 7.03-7.01 (m, 1H), 4.99-4.90 (m, 2H), 4.64-4.56 (m, 2H), 3.97 (s, 2H), 2.43 (s, 3H), 2.23 (s, 3H). ESI-MS calculated for C₁₄H₁₆N₃OS [M+H]⁺ = 274.1, found 274.2.

### Example 19

### Synthetic route:

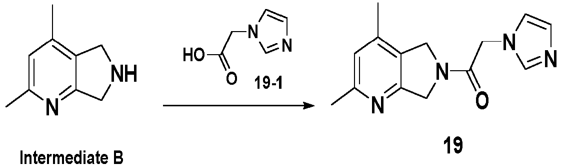

### Step 1

The trifluoroacetate salt of intermediate **B** (150 mg, 1.02 mmol), **19-1** (350 mg, 2.57 mmol), HATU (675 mg, 1.78 mmol), and DIEA (922 mg, 3.33 mmol) were dissolved in DMF (5 mL), and the mixture was stirred at room temperature for 3 hours. The reaction mixture was poured into water (10 mL) and extracted with ethyl acetate (10 mL × 3). The organic phases were combined, dried over anhydrous sodium sulfate, and concentrated under reduced pressure to obtain a crude product containing the target compound, which was purified by preparative high performance liquid chromatography (chromatographic column: Pntulips BP-C18, 5 µm, 21.2 * 150 mm, mobile phase: acetonitrile -10 mmol/L ammonium bicarbonate aqueous solution, gradient: 58 to 88%, retention time: 9 minutes) to obtain compound **19.** ¹H NMR (400 MHz, DMSO-*d*₆) δ 7.59-7.57 (m, 1H), 7.13-7.10 (m, 1H), 7.03 (d, *J =* 7.45 Hz, 1H), 6.89 (s, 1H), 5.06 (s, 2H), 4.90-4.85 (m, 2H), 4.65-4.58 (m, 2H), 2.44 (s, 3H), 2.26-2.23 (m, 3H). ESI-MS calculated for C₁₄H₁₇N₄O [M+H]⁺ = 257.1, found 257.1.

### Example 20

### Synthetic route:

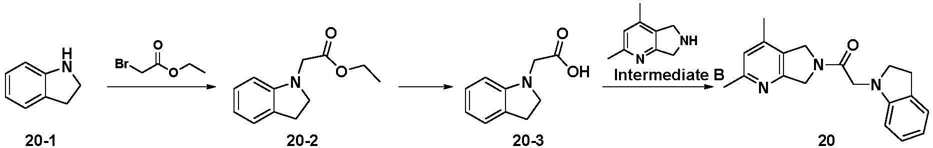

### Step 1

**20-1** (1.0 g, 8.4 mmol) was dissolved in tetrahydrofuran (10 mL), and the mixture was cooled to 0°C. Sodium hydride (60%, 670 mg, 16.8 mmol) was added thereto, and the mixture was stirred for 30 minutes. Ethyl bromoacetate (1.54 g, 9.2 mmol) was added dropwise thereto, and the mixture was stirred at room temperature for 18 hours. The reaction mixture was slowly poured into water (10 mL) and extracted with ethyl acetate (20 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **20-2.** ESI-MS calculated for C₁₂H₁₆NO₂ [M+H]⁺ = 206.1, found 206.1.

### Step 2

**20-2** (1.1 g, 5.36 mmol) was dissolved in methanol (10 mL) and water (3 mL), then lithium hydroxide monohydrate (670 mg, 16.1 mmol) was added thereto, and the mixture was stirred at room temperature for 3 hours. The pH of the reaction mixture was adjusted to 3 with dilute hydrochloric acid (1 mol/L), solids precipitated, and **20-3** was obtained by filtration. ESI-MS calculated for C₁₀H₁₂NO₂ [M+H]⁺ = 178.1, found 178.0.

### Step 3

Intermediate **B** (251 mg, 0.85 mmol) and **20-3** (150 mg, 0.85 mmol) were dissolved in DMF (3 mL), then TEA (429 mg, 4.2 mmol) and HATU (386 mg, 1.02 mmol) were added thereto, and the mixture was stirred at room temperature for 3 hours. The reaction mixture was poured into water (10 mL) and extracted with ethyl acetate (10 mL × 3). The organic phases were combined, dried over anhydrous sodium sulfate, filtered, and concentrated under reduced pressure to obtain a crude product containing the target compound, which was purified by preparative high performance liquid chromatography (chromatographic column: XBridge C18 19 * 250 mm , 10 µm, mobile phase: acetonitrile -10 mmol/L ammonium bicarbonate aqueous solution, gradient: 20 to 55%, retention time: 8.5 minutes) to obtain compound **20.** ¹H NMR (400 MHz, DMSO-*d*₆) δ 7.05-6.99 (m, 2H), 6.95 (t, *J =* 7.62 Hz, 1H), 6.56 (t, *J =* 7.42 Hz, 1H), 6.52 (dd, *J =* 7.80, 2.7 Hz, 1H), 4.93-4.85 (m, 2H), 4.63-4.56 (m, 2H), 4.10-4.05 (m, 2H), 3.55-3.48 (m, 2H), 2.98-2.90 (m, 2H), 2.43 (s, 3H), 2.24 (s, 3H). ESI-MS calculated for C₁₉H₂₂N₃O [M+H]⁺ = 308.2, found 308.1.

### Example 21

### Synthetic route:

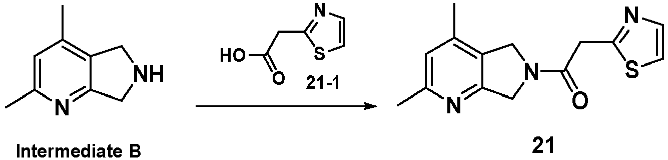

### Step 1

Intermediate **B** (208 mg, 0.70 mmol) and **21-1** (100 mg, 0.70 mmol) were dissolved in DMF (3 mL), then TEA (353 mg, 3.5 mmol) and HATU (319 mg, 0.84 mmol) were added thereto, and the mixture was stirred at room temperature for 3 hours. The reaction mixture was poured into water (10 mL) and extracted with ethyl acetate (10 mL × 3). The organic phases were combined, dried over anhydrous sodium sulfate, filtered, and concentrated under reduced pressure to obtain a crude product containing the target compound, which was purified by preparative high performance liquid chromatography (chromatographic column: XBridge C18 19 * 250 mm , 10 µm, mobile phase: acetonitrile -10 mmol/L ammonium bicarbonate aqueous solution, gradient: 25 to 45%, retention time: 7.5 minutes) to obtain compound **21.** ¹H NMR (400 MHz, DMSO-*d*₆) δ 7.76 (d, *J =* 3.30 Hz, 1H), 7.67 (d, *J =* 3.30 Hz, 1H), 7.02 (d, *J =* 5.32 Hz, 1H), 4.99-4.90 (m, 2H), 4.67-4.58 (m, 2H), 4.29 (s, 2H), 2.43 (s, 3H), 2.24 (s, 3H). ESI-MS calculated for C₁₄H₁₆N₃OS [M+H]⁺ = 274.1, found 274.1.

### Example 22

### Synthetic route:

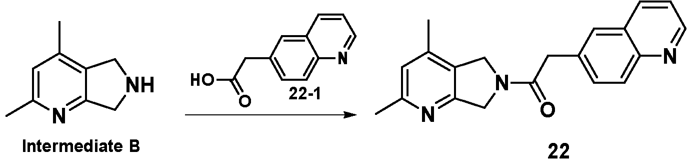

### Step 1

Intermediate **B** (158 mg, 0.53 mmol) and **22-1** (100 mg, 0.53 mmol) were dissolved in DMF (4 mL), then TEA (270 mg, 2.67 mmol) and HATU (244 mg, 0.64 mmol) were added thereto, and the mixture was stirred at room temperature for 3 hours. The reaction mixture was poured into water (10 mL) and extracted with ethyl acetate (10 mL × 3). The organic phases were combined, dried over anhydrous sodium sulfate, filtered, and concentrated under reduced pressure to obtain a crude product containing the target compound, which was purified by preparative high performance liquid chromatography (chromatographic column: XBridge C18 19 * 250 mm , 10 µm, mobile phase: acetonitrile -10 mmol/L ammonium bicarbonate aqueous solution, gradient: 28 to 42%, retention time: 8.3 minutes) to obtain compound 22. ¹H NMR (400 MHz, DMSO-*d*₆) δ 8.87 (dd, *J =* 4.20, 1.72 Hz, 1H), 8.34 (dd, *J =* 8.32, 2.02 Hz, 1H), 7.98 (d, *J =* 8.60 Hz, 1H), 7.87 (d, *J =* 2.22 Hz, 1H), 7.71-7.69 (m, 1H), 7.52 (dd, *J =* 8.32, 4.20 Hz, 1H), 7.01 (d, *J =* 6.60 Hz, 1H), 4.99-4.90 (m, 2H), 4.68-4.60 (m, 2H), 4.01 (s, 2H), 2.43 (s, 3H), 2.24 (s, 3H). ESI-MS calculated for C₂₀H₂₀N₃O [M+H]⁺ = 318.2, found 318.1.

### Example 23

### Synthetic route:

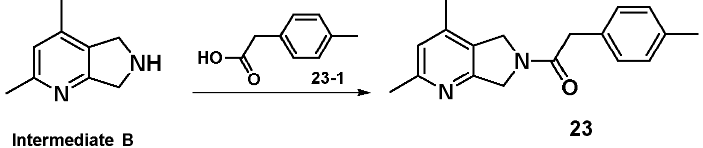

### Step 1

Intermediate **B** (197 mg, 0.67 mmol) and 23-1 (100 mg, 0.67 mmol) were dissolved in DMF (4 mL), then TEA (337 mg, 3.33 mmol) and HATU (309 mg, 0.80 mmol) were added thereto, and the mixture was stirred at room temperature for 3 hours. The reaction mixture was poured into water (10 mL) and extracted with ethyl acetate (10 mL × 3). The organic phases were combined, dried over anhydrous sodium sulfate, filtered, and concentrated under reduced pressure to obtain a crude product containing the target compound, which was purified by preparative high performance liquid chromatography (chromatographic column: XBridge C18 19 * 250 mm , 10 µm, mobile phase: acetonitrile -10 mmol/L ammonium bicarbonate aqueous solution, gradient: 30 to 55%, retention time: 9.5 minutes) to obtain compound 23. ¹H NMR (400 MHz, DMSO-*d*₆) δ 7.20-7.10 (m, 4H), 7.00 (d, *J =* 5.24 Hz, 1H), 4.88-4.79 (m, 2H), 4.63-4.55 (m, 2H), 3.72 (s, 2H), 2.42 (s, 3H), 2.28 (s, 3H), 2.23 (s, 3H). ESI-MS calculated for C₁₈H₂₁N₂O [M+H]⁺ = 281.2, found 281.1.

### Example 24

### Synthetic route:

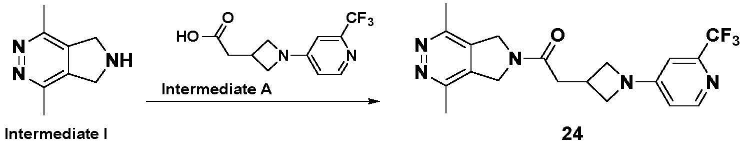

### Step 1

The trifluoroacetate salt of intermediate **I** (100 mg, 0.67 mmol) and intermediate **A** (262 mg, 1.01 mmol) were dissolved in tetrahydrofuran (4 mL), then DIEA (260 mg, 2.01 mmol) and HATU (382 mg, 1.01 mmol) were added thereto, and the mixture was stirred at room temperature for 3 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 17 to 47%, retention time: 9 minutes) to obtain compound **24.** ¹H NMR (400 MHz, DMSO-*d*₆) δ 8.21 (d, *J =* 5.66 Hz, 1H), 6.72 (d, *J =* 2.24 Hz, 1H), 6.54 (dd, *J =* 5.66, 2.24 Hz, 1H), 4.95-4.90 (m, 2H), 4.76-4.69 (m, 2H), 4.22-4.15 (m, 2H), 3.75-3.68 (m, 2H), 3.17-3.10 (m, 1H), 2.90-2.82 (m, 2H), 2.57-2.52 (m, 6H). ESI-MS calculated for C₁₉H₂₁F₃N₅O [M+H]⁺ = 392.2, found 392.2.

### Example 25

### Synthetic route:

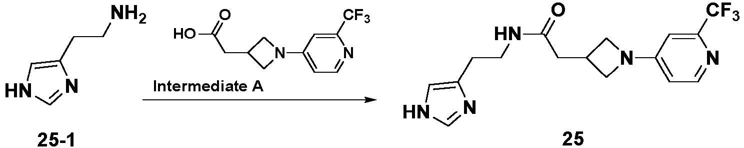

### Step 1

Intermediate **A** (500 mg, 1.35 mmol) and **25-1** (160 mg, 1.48 mmol) were dissolved in DMF (10 mL), then DIEA (350 mg, 2.69 mmol) and HATU (770 mg, 2.02 mmol) were added thereto, and the mixture was stirred at room temperature for 2 hours. The reaction mixture was poured into water (10 mL) and extracted with ethyl acetate (10 mL × 3). The organic phases were combined, dried over anhydrous sodium sulfate, filtered, and concentrated under reduced pressure to obtain a crude product containing the target compound, which was purified by preparative high performance liquid chromatography (chromatographic column: Pntulips BP-C18 21.2 * 250 mm , 5 µm, mobile phase: acetonitrile -10 mmol/L ammonium bicarbonate aqueous solution, gradient: 18 to 28%, retention time: 9 minutes) to obtain compound 25. ¹H NMR (400 MHz, DMSO-*d*₆) δ 11.80 (br, 1H), 8.21 (d, *J =* 5.64 Hz, 1H), 7.99 (t, *J =* 5.68 Hz, 1H), 7.53 (s, 1H), 6.82-6.78 (m, 1H), 6.70 (d, *J =* 2.26 Hz, 1H), 6.51 (dd, *J* = 5.70, 2.26 Hz, 1H), 4.09 (t, *J =* 8.24 Hz, 2H), 3.68-3.63 (m, 2H), 3.30-3.24 (m, 2H), 3.05-2.97 (m, 1H), 2.52-2.46 (m, 4H). ESI-MS calculated for C₁₆H₁₉F₃N₅O [M+H]⁺ = 354.2, found 354.1.

### Example 26

### Synthetic route:

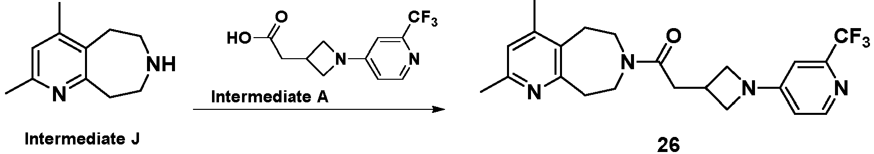

### Step 1

Intermediate **J** (20 mg, 0.10 mmol) and intermediate **A** (64 mg, 0.17 mmol) were dissolved in DMF (10 mL), then DIEA (66 mg, 0.51 mmol) was added thereto, and the mixture was stirred at room temperature for 30 minutes. HATU (97 mg, 0.26 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Pntulips BP-C18, 5 µm, 21.2 * 150 mm, mobile phase: acetonitrile-10 mmol/L ammonium bicarbonate aqueous solution, gradient: 35 to 52%, retention time: 9 minutes) to obtain compound **26.** ¹H NMR (400 MHz, DMSO-*d*₆) δ 8.20 (d, *J =* 5.64 Hz, 1H), 6.89 (d, *J =* 6.30 Hz, 1H), 6.67 (s, 1H), 6.50 (d, *J =* 5.22 Hz, 1H), 4.15-4.06 (m, 2H), 3.68-3.52 (m, 6H), 3.12-3.07 (m, 1H), 3.06-2.95 (m, 2H), 2.92-2.85 (m, 1H), 2.83-2.77 (m, 3H), 2.34-2.27 (m, 3H), 2.22 (s, 3H). ESI-MS calculated for C₂₂H₂₆F₃N₄O [M+H]⁺ = 419.2, found 419.2.

### Example 27

### Synthetic route:

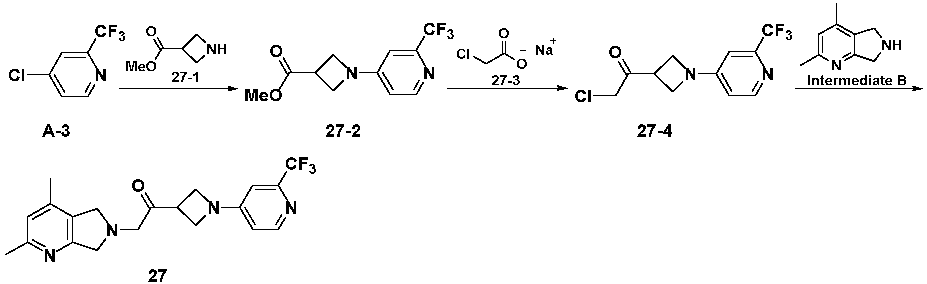

### Step 1

**A-3** (1.0 g, 5.51 mmol), the hydrochloride salt **of 27-1** (840 mg, 5.51 mmol), cesium fluoride (840 mg, 5.51 mmol), and TEA (2.23 g, 22.0 mmol) were added to DMSO (10 mL), and the reaction mixture was heated to 80°C and stirred for 12 hours. The reaction mixture was diluted with water (80 mL) and extracted with ethyl acetate (40 mL × 3). The organic phases were combined, washed with saturated brine (40 mL × 3), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain 27-2. ¹H NMR (400 MHz, DMSO-*d*₆) δ 8.25-8.22 (m, 1H), 6.79-6.75 (m, 1H), 6.58-6.54 (m ,1H), 4.25-4.18 (m, 2H), 4.15-4.08 (m, 2H), 3.75-3.67 (m, 1H), 3.65 (s, 3H). ESI-MS calculated for C₁₁H₁₂F₃N₂O₂ [M+H]⁺ = 261.0, found 261.0.

### Step 2

**27-2** (300 mg, 1.15 mmol), TEA (117 mg, 1.15 mmol), and **27-3** (210 mg, 1.72 mmol) were added to tetrahydrofuran (5 mL), cooled to 0°C, and a solution of *tert*-butylmagnesium chloride in tetrahydrofuran (1 mol/L, 3.46 mL, 3.46 mmol) was added dropwise thereto. The mixture was stirred at room temperature for 12 hours. The reaction mixture was diluted with saturated sodium bicarbonate solution (30 mL) and extracted with ethyl acetate (40 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **27-4.** ¹H NMR (400 MHz, DMSO-*d*₆) δ 8.27-8.24 (m, 1H), 6.79-6.76 (m, 1H), 6.60-6.56 (m, 1H), 4.64 (s, 2H), 4.20-4.13 (m, 2H), 4.10-4.02 (m, 2H), 3.97-3.93 (m, 1H). ESI-MS calculated for C₁₁H₁₁ClF₃N₂O [M+H]⁺ = 279.0, found 279.0.

### Step 3

Intermediate **B** (76.2 mg, 0.36 mmol), potassium carbonate (100 mg, 0.72 mmol), TEA (72 mg, 0.72 mmol), tetrabutylammonium bromide (23 mg, 0.07 mmol), and **27-4** (100 mg, 0.36 mmol) were added to tetrahydrofuran (10 mL), and the mixture was stirred at room temperature for 3 hours. The reaction mixture was diluted with water (20 mL) and extracted with ethyl acetate (20 mL × 3). The organic phases were combined, dried over anhydrous sodium sulfate, filtered, and concentrated under reduced pressure to obtain a crude product containing the target compound, which was purified by preparative high performance liquid chromatography (chromatographic column: XBndge C18 19 * 250 mm 10 µm, mobile phase: acetonitrile-10 mmol/L ammonium bicarbonate aqueous solution, gradient: 22 to 50%, retention time: 9.5 minutes) to obtain compound **27.** ¹H NMR (400 MHz, DMSO-*d*₆) δ 8.24 (d, *J* = 5.62 Hz, 1H), 6.89 (s, 1H), 6.78 (d, *J =* 2.30 Hz, 1H), 6.58 (dd, *J =* 5.62, 2.30 Hz, 1H), 4.19-4.15 (m, 2H), 4.11-4.08 (m, 2H), 3.99-3.93 (m, 5H), 3.79 (s, 2H), 2.39 (s, 3H), 2.17 (s, 3H). ESI-MS calculated for C₂₀H₂₂F₃N₄O [M+H]⁺ = 391.2, found 391.2.

### Example 28

### Synthetic route:

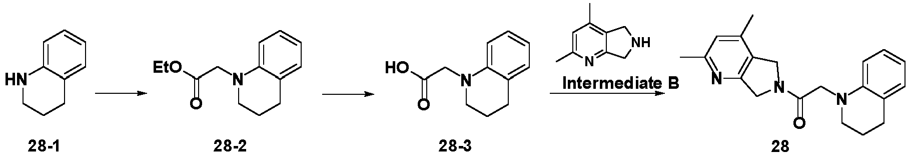

### Step 1

**28-1** (2.0 g, 15.0 mmol) was dissolved in tetrahydrofuran (20 mL), and the mixture was cooled to 0°C. Sodium hydride (60%, 670 mg, 16.8 mmol) was added thereto, and then ethyl bromoacetate (3.0 g, 18.0 mmol) was added dropwise thereto, and the mixture was stirred at room temperature for 18 hours. The reaction mixture was slowly poured into saturated sodium bicarbonate solution, extracted with ethyl acetate (30 mL × 1), dried over anhydrous sodium sulfate, filtered, and concentrated under reduced pressure to obtain a crude product containing the target compound, which was purified by silica gel column chromatography (petroleum ether/ethyl acetate, 3/2, v/v) to obtain **28-2.** ESI-MS calculated for C₁₃H₁₈NO₂ [M+H]⁺ = 220.1, found 220.1.

### Step 2

**28-2** (800 mg, 3.65 mmol) was dissolved in methanol (5 mL) and water (1 mL), then lithium hydroxide monohydrate (153 mg, 3.65 mmol) was added thereto, and the mixture was stirred at room temperature for 3 hours. After the reaction was completed, the reaction mixture was added with dilute hydrochloric acid (1 mol/L) to adjust the pH to 5, concentrated under reduced pressure, and then lyophilized to obtain a crude product containing **28-3,** which was directly used in the next reaction step. ¹H NMR (400 MHz, DMSO-*d*₆) δ 6.96-6.85 (m, 2H), 6.51-6.47 (m, 1H), 6.38-6.36 (m, 1H), 3.98 (s, 2H), 3.35-3.30 (m, 2H), 2.70-2.67 (m, 2H), 1.90-1.82 (m, 2H). ESI-MS calculated for C₁₁H₁₄NO₂ [M+H]⁺ = 192.1, found 192.1.

### Step 3

Intermediate **B** (279 mg, 1.88 mmol) and **28-3** (300 mg, 1.57 mmol) were dissolved in DMF (5 mL), then DIEA (608 mg, 4.71 mmol) and HATU (894 mg, 2.35 mmol) were added thereto, and the mixture was stirred at room temperature for 3 hours. The reaction mixture was concentrated under reduced pressure to obtain a crude product containing the target compound, which was purified by preparative high performance liquid chromatography (chromatographic column: XBndge prep 21.2 * 150 mm, mobile phase: acetonitrile-10 mmol/L ammonium bicarbonate aqueous solution, gradient: 52 to 95%, retention time: 10 minutes) to obtain compound **28.** ¹H NMR (400 MHz, DMSO-*d*₆) δ 7.04-6.84 (m, 1H), 6.92-6.84 (m, 2H), 6.50-6.43 (m, 2H), 4.95-4.86 (m, 2H), 4.63-4.55 (m, 2H), 4.23-4.18 (m, 2H), 3.37-3.31 (m, 2H), 2.75-2.69 (m, 2H), 2.43 (s, 3H), 2.27-2.21 (m, 3H), 1.93-1.86 (m, 2H). ESI-MS calculated for C₂₀H₂₄N₃O [M+H]⁺ = 322.2, found 322.1.

### Example 29

### Synthetic route:

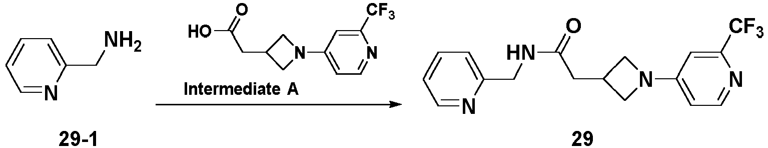

### Step 1

**29-1** (100 mg, 0.92 mmol) and intermediate **A** (361 mg, 1.39 mmol) were dissolved in tetrahydrofuran (5 mL), then DIEA (359 mg, 2.77 mmol) was added thereto, and the mixture was stirred at room temperature for 30 minutes. HATU (527 mg, 1.39 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 24 to 54%, retention time: 9 minutes) to obtain compound **29.** ¹H NMR (400 MHz, DMSO-*d*₆) δ 8.62-8.41 (m, 2H), 8.21 (d, *J =* 5.66 Hz, 1H), 7.79-7.72 (m, 1H), 7.26 (dd, *J =* 7.72, 4.86 Hz, 2H), 6.71 (d, *J* = 2.28 Hz, 1H), 6.53 (dd, *J =* 5.68, 2.28 Hz, 1H), 4.36 (d, *J =* 5.92 Hz, 2H), 4.13 (t, *J* = 8.25 Hz, 2H), 3.75-3.70 (m, 2H), 3.11-3.05 (m, 1H), 2.64-2.59 (m, 2H). ESI-MS calculated for C₁₇H₁₈F₃N₄O [M+H]⁺ = 351.1, found 351.2.

### Example 30

### Synthetic route:

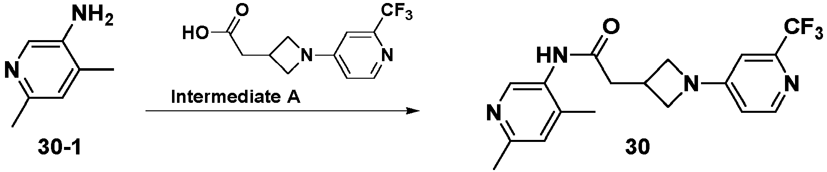

### Step 1

**30-1** (100 mg, 0.82 mmol) and intermediate A (320 mg, 1.23 mmol) were dissolved in tetrahydrofuran (5 mL), then DIEA (317 mg, 2.46 mmol) was added thereto, and the mixture was stirred at room temperature for 30 minutes. HATU (467 mg, 1.23 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 21 to 51%, retention time: 9.5 minutes) to obtain compound **30.** ¹H NMR (400 MHz, DMSO-*d*₆) δ 9.52 (s, 1H), 8.33 (s, 1H), 8.22 (d, *J =* 5.66 Hz, 1H), 7.12 (s, 1H), 6.74 (d, *J =* 2.28 Hz, 1H), 6.55 (dd, *J =* 5.68, 2.28 Hz, 1H), 4.21-4.13 (m, 2H), 3.80-3.73 (m, 2H), 3.18-3.10 (m, 1H), 2.81-2.75 (m, 2H), 2.40 (s, 3H), 2.16 (s, 3H). ESI-MS calculated for C₁₈H₂₀F₃N₄O [M+H]⁺ = 365.2, found 365.1.

### Example 31

### Synthetic route:

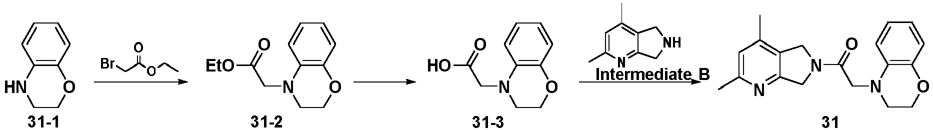

### Step 1

**31-1** (300 mg, 2.22 mmol) was dissolved in tetrahydrofuran (5 mL), then sodium hydride (60%, 54 mg, 2.22 mmol) was slowly added thereto under nitrogen atmosphere, and the mixture was stirred at room temperature for 1 hour. Ethyl bromoacetate (445 mg, 2.66 mmol) was added dropwise to the reaction mixture, and the mixture was stirred at room temperature for another 1 hour. The reaction mixture was diluted with water (15 mL) and extracted with ethyl acetate (20 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain 31-2. ESI-MS calculated for C₁₂H₁₆NO₃ [M+H]⁺ = 222.1, found 222.0.

### Step 2

**31-2** (280 mg, 1.27 mmol) was dissolved in tetrahydrofuran (5 mL) and water (1 mL), then lithium hydroxide monohydrate (159 mg, 3.80 mmol) was added thereto, and the mixture was stirred at room temperature for 16 hours. The pH of the reaction mixture was adjusted to 3 with dilute hydrochloric acid (1 mol/L), solids precipitated, and **31-3** was obtained by filtration.

### Step 3

**31-3** (100 mg, 0.52 mmol) and intermediate **B** (77 mg, 0.52 mmol) were dissolved in tetrahydrofuran (3 mL), then DIEA (67 mg, 1.56 mmol) was added thereto, and the mixture was stirred at room temperature for 30 minutes. HATU (197 mg, 0.52 mmol) was then added thereto, and the mixture was stirred at room temperature for another 3 hours. The reaction mixture was concentrated under reduced pressure to obtain a crude product containing the target compound, which was purified by preparative high performance liquid chromatography (chromatographic column: Waters-SunFire-C18, 10 µm, 19 * 250 mm, mobile phase: acetonitrile-0.1% formic acid aqueous solution, gradient: 20 to 50%, retention time: 8 minutes) to obtain compound **31.** ¹H NMR (400 MHz, DMSO-*d*₆) δ 7.05-7.01 (m, 1H), 6.72-6.59 (m, 3H), 6.54-6.47 (m, 1H), 4.94-4.88 (m, 2H), 4.62-4.56 (m, 2H), 4.30-4.16 (m, 4H), 3.47-3.42 (m, 2H), 2.44 (s, 3H), 2.24 (s, 3H). ESI-MS calculated for C₁₉H₂₂N₃O₂ [M+H]⁺ = 324.2, found 324.2.

### Example 32

### Synthetic route:

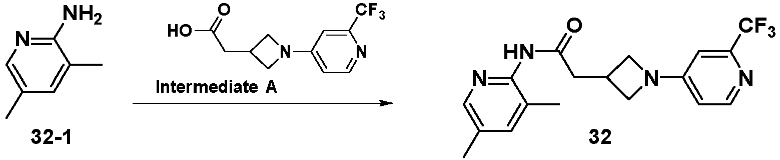

### Step 1

**32-1** (100 mg, 0.82 mmol) and intermediate A (320 mg, 1.23 mmol) were dissolved in tetrahydrofuran (5 mL), then DIEA (317 mg, 2.46 mmol) was added thereto, and the mixture was stirred at room temperature for 30 minutes. HATU (467 mg, 1.23 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Welch-Xtimate-C18, 7 µm, 21.2 * 150 mm, mobile phase: acetonitrile-10 mmol/L ammonium bicarbonate aqueous solution, gradient: 27 to 57%, retention time: 8 minutes) to obtain compound **32.** ¹H NMR (400 MHz, DMSO-*d*₆) δ 9.97 (s, 1H), 8.21 (d, *J =* 5.64 Hz, 1H), 8.06 (d, *J =* 2.24 Hz, 1H), 7.48 (d, *J* = 2.22 Hz, 1H), 6.73 (d, *J* = 2.28 Hz, 1H), 6.56-6.52 (m, 1H), 4.18-4.11 (m, 2H), 3.78-3.72 (m, 2H), 3.16-3.07 (m, 1H), 2.74 (d, *J* = 7.74 Hz, 2H), 2.25 (s, 3H), 2.09 (s, 3H). ESI-MS calculated for C₁₈H₂₀F₃N₄O [M+H]⁺ = 365.2, found 365.1.

### Example 33

### Synthetic route:

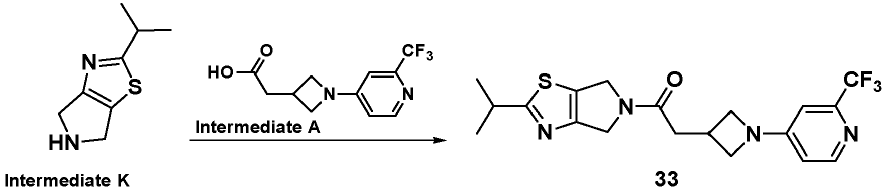

### Step 1

Intermediate **K** (150 mg, 0.89 mmol) and intermediate **A** (331 mg, 0.89 mmol) were dissolved in DMF (2 mL), then TEA (451 mg, 4.46 mmol) was added thereto, and the mixture was stirred at room temperature for 30 minutes. HATU (407 mg, 1.07 mmol) was then added thereto, and the mixture was stirred at room temperature for another 2 hours. After the reaction was completed, the reaction mixture was diluted with water (20 mL) and extracted with ethyl acetate (20 mL × 3). The organic phases were combined, dried over anhydrous sodium sulfate, filtered, and concentrated under reduced pressure to obtain a crude product containing the target compound the reaction mixture was concentrated under reduced pressure to obtain a crude product containing the target compound, which was purified by preparative high performance liquid chromatography (chromatographic column: XBndge C18, 10 µm, 19 * 250mm, mobile phase: acetonitrile-10 mmol/L ammonium bicarbonate aqueous solution, gradient: 25 to 45%, retention time: 8.5 minutes) to obtain compound **33.** ¹H NMR (400 MHz, DMSO-*d*₆) δ 8.21 (d, *J =* 5.72 Hz, 1H), 6.71 (d, *J =* 2.24 Hz, 1H), 6.53 (dd, *J =* 5.72, 2.22 Hz, 1H), 4.86-4.67 (m, 2H), 4.62-4.46 (m, 2H), 4.21-4.14 (m, 2H), 3.72-3.68 (m, 2H), 3.33-3.25 (m, 1H), 3.19-3.06 (m, 1H), 2.84-2.80 (m, 2H), 1.35-1.31 (m, 6H). ESI-MS calculated for C₁₉H₂₂F₃N₄OS [M+H]⁺ = 411.1, found 411.1.

### Example 34

### Synthetic route:

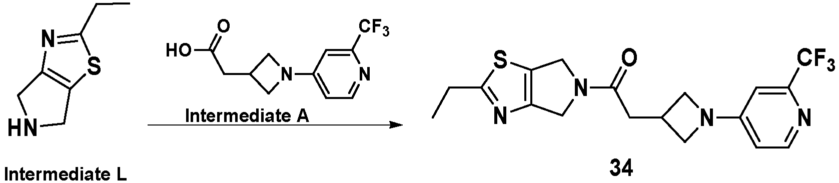

### Step 1

Intermediate **L** (120 mg, 0.78 mmol) and intermediate **A** (289 mg, 0.78 mmol) were dissolved in DMF (2 mL), then TEA (276 mg, 2.72 mmol) was added thereto, and the mixture was stirred at room temperature for 30 minutes. HATU (355 mg, 0.93 mmol) was then added thereto, and the mixture was stirred at room temperature for another 2 hours. After the reaction was completed, the reaction mixture was diluted with water (20 mL) and extracted with ethyl acetate (20 mL × 3). The organic phases were combined, dried over anhydrous sodium sulfate, filtered, and concentrated under reduced pressure to obtain a crude product containing the target compound, which was purified by preparative high performance liquid chromatography (chromatographic column: XBndge C18, 10 µm, 19 * 250mm, mobile phase: acetonitrile-10 mmol/L ammonium bicarbonate aqueous solution, gradient: 30 to 45%, retention time: 9.5 minutes) to obtain compound **34.** ¹H NMR (400 MHz, DMSO-*d*₆) δ 8.21 (d, *J =* 5.66 Hz, 1H), 6.71 (d, *J* = 2.32 Hz, 1H), 6.53 (dd, *J* = 5.72, 2.28 Hz, 1H), 4.83-4.67 (m, 2H), 4.59-4.43 (m, 2H), 4.21-4.15 (m, 2H), 3.74-3.64 (m, 2H), 3.15-3.08 (m, 1H), 3.04-2.97 (m, 2H), 2.85-2.79 (m, 2H), 1.30 (t, J=7.52 Hz, 3H). ESI-MS calculated for C₁₈H₂₀F₃N₄OS [M+H]⁺ = 397.1, found 397.1.

### Example 35

### Synthetic route:

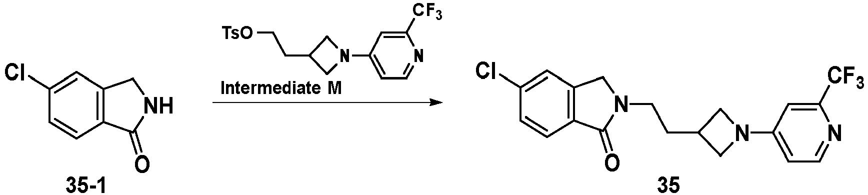

### Step 1

**35-1** (100 mg, 0.60 mmol) was dissolved in dioxane (5 mL), then sodium hydride (60%, 24.0 mg, 0.60 mmol) was added thereto, and the mixture was stirred at room temperature for 10 minutes. Intermediate **M** (240 mg, 0.60 mmol) was then added thereto, and the mixture was stirred at room temperature for another 4 hours. After the reaction was completed, the reaction mixture was diluted with water (3 mL) and concentrated under reduced pressure to obtain a crude product containing the target compound, which was purified by preparative high performance liquid chromatography (chromatographic column: Pntulips BP-C18, 5 µm, 21.2 * 150 mm, mobile phase: acetonitrile-10 mmol/L ammonium bicarbonate aqueous solution, gradient: 47 to 57%, retention time: 9 minutes) to obtain compound **35.** ¹H NMR (400 MHz, DMSO-*d*₆) δ 8.23-8.20 (m, 1H), 7.74-7.64 (m, 2H), 7.58-7.52 (m, 1H), 6.69 (d, *J =* 2.30 Hz, 1H), 6.54-6.50 (m, 1H), 4.51 (s, 2H), 4.12-4.04 (m, 2H), 3.70-3.63 (m, 2H), 3.56-3.51 (m, 2H), 2.78-2.71 (m, 1H), 2.02-1.92 (m, 2H). ESI-MS calculated for C₁₉H₁₈ClF₃N₃O [M+H]⁺ = 396.1, found 396.1.

### Example 36

### Synthetic route:

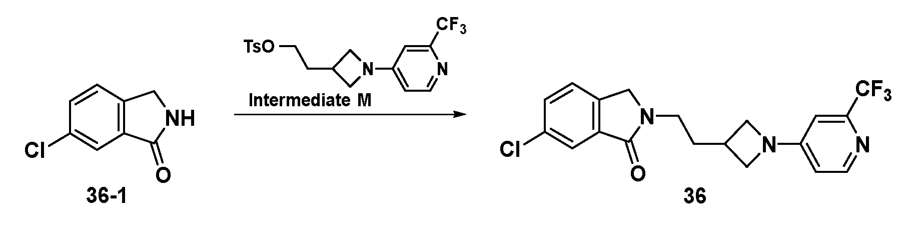

### Step 1

**36-1** (42 mg, 0.25 mmol) was dissolved in dioxane (5 mL), then sodium hydride (60%, 10 mg, 0.25 mmol) was added thereto, and the mixture was stirred at room temperature for 30 minutes. Intermediate **M** (100 mg, 0.25 mmol) was then added thereto, and the mixture was heated to 100°C and stirred for another 18 hours. After the reaction was completed, the reaction mixture was diluted with water (3 mL) and concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 43 to 73%, retention time: 9 minutes) to obtain compound **36.** ¹H NMR (400 MHz, DMSO-*d*₆) δ 8.21 (d, *J =* 5.66 Hz, 1H), 7.68 (d, *J =* 1.34 Hz, 1H), 7.65 (t, *J =* 1.75 Hz, 2H), 6.70 (d, *J =* 2.24 Hz, 1H), 6.51 (dd, *J =* 5.64, 2.24 Hz, 1H), 4.51 (s, 2H), 4.12-4.03 (m, 2H), 3.71-3.64 (m, 2H), 3.57-3.50 (m, 2H), 2.78-2.72 (m, 1H), 1.98-1.92 (m, 2H). ESI-MS calculated for C₁₉H₁₈ClF₃N₃O [M+H]⁺ = 396.1, found 396.1.

### Example 37

### Synthetic route:

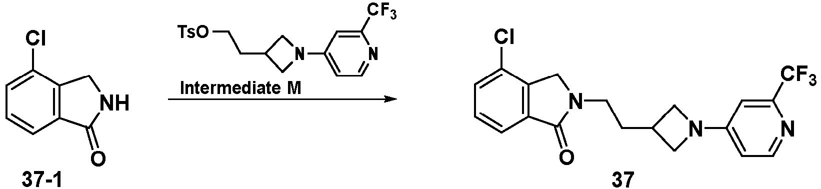

### Step 1

**37-1** (100 mg, 0.60 mmol) was dissolved in dioxane (5 mL), then sodium hydride (60%, 24.0 mg, 0.60 mmol) was added thereto, and the mixture was stirred at room temperature for 30 minutes. Intermediate **M** (240 mg, 0.60 mmol) was then added thereto, and the mixture was heated to 100°C and stirred for another 18 hours. After the reaction was completed, the reaction mixture was diluted with water (3 mL) and concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 43 to 73%, retention time: 9 minutes) to obtain compound 37. ¹H NMR (400 MHz, DMSO-*d*₆) δ 8.20 (d, *J =* 5.68 Hz, 1H), 7.71-7.65 (m, 2H), 7.55 (t, *J =* 7.68 Hz, 1H), 6.69 (d, *J* = 2.24 Hz, 1H), 6.51 (dd, *J* = 5.70, 2.24 Hz, 1H), 4.53 (s, 2H), 4.12-4.04 (m, 2H), 3.72-3.64 (m, 2H), 3.60-3.52 (m, 2H), 2.79-2.72 (m, 1H), 2.04-1.95 (m, 2H). ESI-MS calculated for C₁₉H₁₈ClF₃N₃O [M+H]⁺ = 396.1, found 396.1.

### Example 38

### Synthetic route:

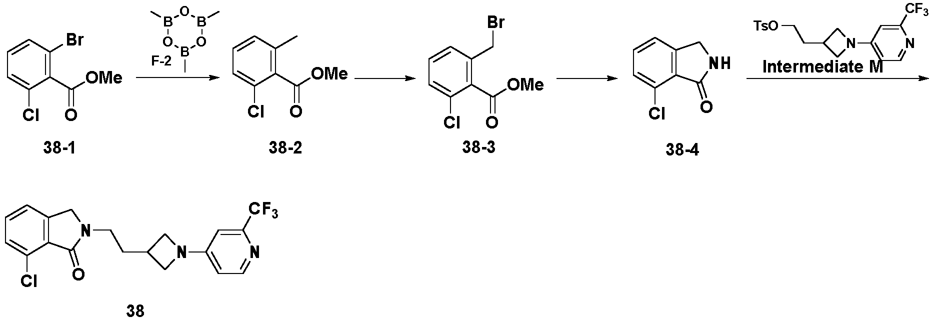

### Step 1

**38-1** (3.80 g, 15.2 mmol) was dissolved in 1,4-dioxane (17.5 mL) and water (2.5 mL), then **F-2** (2.29 g, 18.3 mmol), potassium carbonate (9.33 g, 30.5 mmol), and [1,1'-bis(diphenylphosphino)ferrocene]dichloropalladium(II) (170 mg, 0.23 mmol) were sequentially added thereto. The reaction mixture was heated to 100°C under nitrogen atmosphere and stirred for 18 hours. After the reaction was completed, the reaction mixture was diluted with water (30 mL) and extracted with ethyl acetate (30 mL × 3). The organic phases were combined, dried over anhydrous sodium sulfate, filtered, and concentrated under reduced pressure to obtain a crude product containing the target product, which was purified by silica gel column chromatography (petroleum ether/ethyl acetate, 4/1, v/v) to obtain **38-2.** ¹H NMR (400 MHz, Chloroform-*d*) δ 7.44-7.33 (m, 2H), 7.31-7.25 (m, 1H), 3.85 (s, 3H), 2.26 (s, 3H).

### Step 2

Compound **38-2** (2.0 g, 10.8 mmol) was dissolved in carbon tetrachloride (20 mL), then NBS (1.9 g, 10.8 mmol) and benzoyl peroxide (260 mg, 1.30 mmol) were sequentially added thereto, and the mixture was heated to 75°C and stirred for 3 hours. The reaction mixture was added with water (30 mL) and extracted with dichloromethane (20 mL × 3). The organic phases were combined, washed with saturated brine (50 mL), dried over anhydrous sodium sulfate, filtered, and concentrated under reduced pressure to obtain a crude product containing the target product, which was purified by silica gel column chromatography (dichloromethane/methanol, 9/1, v/v) to obtain **38-3.** ¹H NMR (400 MHz, DMSO-*d*₆) δ 7.60-7.48 (m, 3H), 4.66 (s, 2H), 3.92 (s, 3H).

### Step 3

**38-3** (1.0 g, 3.79 mmol) was dissolved in a methanol solution of ammonia (7 mol/L, 5 mL), and the mixture was stirred at 60°C for 12 hours. The reaction mixture was concentrated and purified by silica gel column chromatography (petroleum ether/ethyl acetate, 9/1, v/v) to obtain **38-4.** ¹H NMR (400 MHz, DMSO-*d*₆) δ 8.69 (s, 1H), 7.61-7.42 (m, 3H), 4.35 (s, 2H). ESI-MS calculated for C₈H₇ClNO [M+H]⁺ = 168.0, found 168.0.

### Step 4

**38-4** (100 mg, 0.60 mmol) was dissolved in dioxane (5 mL), then sodium hydride (60%, 24.0 mg, 0.60 mmol) was added thereto, and the mixture was stirred at room temperature for 30 minutes. Intermediate **M** (240 mg, 0.60 mmol) was then added thereto, and the mixture was heated to 100°C and stirred for another 18 hours. After the reaction was completed, the reaction mixture was diluted with water (3 mL) and concentrated under reduced pressure to obtain a crude product containing the target compound, which was purified by preparative high performance liquid chromatography (chromatographic column: Pntulips BP-C18, 5 µm, 21.2 * 150 mm, mobile phase: acetonitrile-10 mmol/L ammonium bicarbonate aqueous solution, gradient: 45 to 55%, retention time: 9 minutes) to obtain compound **38.** ¹H NMR (400 MHz, DMSO-*d*₆) δ 8.21 (d, *J =* 5.62 Hz, 1H), 7.61-7.54 (m, 2H), 7.51-7.45 (m, 1H), 6.70 (d, *J* = 2.26 Hz, 1H), 6.52 (dd, *J =* 5.68, 2.26 Hz, 1H), 4.49 (s, 2H), 4.13-4.06 (m, 2H), 3.72-3.66 (m, 2H), 3.56-3.50 (m, 2H), 2.79-2.72 (m, 1H), 2.01-1.92 (m, 2H). ESI-MS calculated for C₁₉H₁₈ClF₃N₃O [M+H]⁺ = 396.1, found 396.1.

### Example 39

### Synthetic route:

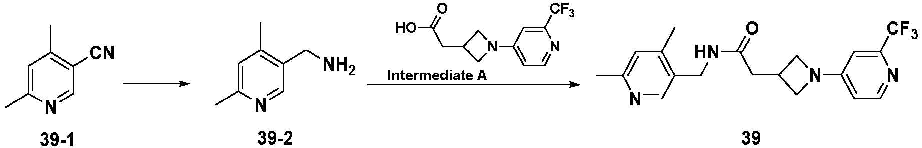

### Step 1

**39-1** (500 mg, 3.78 mmol) and hydrazine hydrate (98%, 3.78 mmol) were dissolved in ethanol (5 mL), cooled to 0°C, and Raney nickel (8.65 g, 39.6 mmol) was added thereto. The reaction system was replaced with hydrogen three times and then stirred at room temperature for 18 hours. The reaction system was filtered through diatomite, and the filtrate was extracted with ethyl acetate (20 mL × 3). The organic phases were combined, dried over anhydrous sodium sulfate, filtered, and concentrated under reduced pressure to obtain a crude product containing **39-2,** which was directly used in the next reaction step. ESI-MS calculated for C₈H₁₃N₂ [M+H]⁺ = 137.1, found 137.1.

### Step 2

**39-2** (100 mg, 0.51 mmol) and intermediate **A** (191 mg, 0.51 mmol) were dissolved in DMF (2 mL), then TEA (156 mg, 2.72 mmol) was added thereto, and the mixture was stirred at room temperature for 30 minutes. HATU (235 mg, 0.62 mmol) was then added thereto, and the mixture was stirred at room temperature for another 3 hours. After the reaction was completed, the reaction mixture was diluted with water (10 mL) and extracted with ethyl acetate (10 mL × 3). The organic phases were combined, dried over anhydrous sodium sulfate, filtered, and concentrated under reduced pressure to obtain a crude product containing the target compound, which was purified by preparative high performance liquid chromatography (chromatographic column: XBndge C18, 10 µm, 19 * 250mm, mobile phase: acetonitrile-10 mmol/L ammonium bicarbonate aqueous solution, gradient: 25 to 45%, retention time: 9.5 minutes) to obtain compound 39. ¹H NMR (400 MHz, DMSO-*d*₆) δ 8.30-8.26 (m, 1H), 8.23-8.20 (m, 2H), 7.05 (s, 1H), 6.70 (d, *J =* 2.20 Hz, 1H), 6.55-6.50 (m, 1H), 4.26-4.21 (m, 2H), 4.15-4.08 (m, 2H), 3.72-3.65 (m, 2H), 3.09-3.01 (m, 1H), 2.56-2.48 (m, 2H), 2.39 (s, 3H), 2.24 (s, 3H). ESI-MS calculated for C₁₉H₂₂F₃N₄O [M+H]⁺ = 379.2, found 397.1.

### Example 40

### Synthetic route:

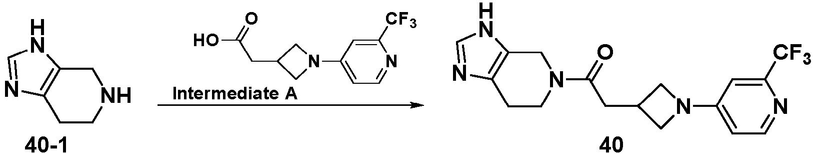

### Step 1

**40-1** (300 mg, 1.88 mmol) and intermediate **A** (586 mg, 2.26 mmol) were dissolved in DMF (5 mL), then DIEA (728 mg, 6.52 mmol) was added thereto, and the mixture was stirred at room temperature for 30 minutes. HATU (1.07 g, 2.82 mmol) was then added thereto, and the mixture was stirred at room temperature for another 3 hours. The reaction mixture was concentrated under reduced pressure to obtain a crude product containing the target compound, which was purified by preparative high performance liquid chromatography (chromatographic column: XBndge C18, 10 µm, 21.5 * 150 mm, mobile phase: acetonitrile-10 mmol/L ammonium bicarbonate aqueous solution, gradient: 20 to 50%, retention time: 8 minutes) to obtain compound **40.** ¹H NMR (400 MHz, DMSO-*d*₆) δ 11.82 (br, 1H), 8.19 (d, *J* = 5.62 Hz, 1H), 7.49 (d, *J =* 9.50 Hz, 1H), 6.69 (s, 1H), 6.61-6.43 (m, 1H), 4.59-4.34 (m, 2H), 4.17-4.10 (m, 2H), 3.79-3.60 (m, 4H), 3.09-3.01 (m, 1H), 2.92-2.80 (m, 2H), 2.65-2.56 (m, 2H). ESI-MS calculated for C₁₇H₁₉F₃N₅O [M+H]⁺ = 366.2, found 366.1.

### Example 41

### Synthetic route:

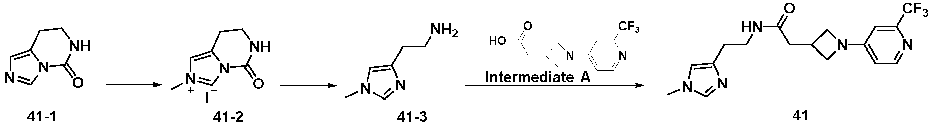

### Step 1

**41-1** (700 mg, 5.12 mmol) was dissolved in DMF (10 mL), then iodomethane (2.17 g, 15.3 mmol) was added thereto, and the mixture was heated to 50°C and stirred for 18 hours. The reaction mixture was concentrated to obtain a crude product containing **41-2,** which was directly used in the next reaction step. ESI-MS calculated for C₇H₁₀N₃O⁺ [M]⁺ = 152.1, found 152.2.

### Step 2

**41-2** (600 mg, 2.15 mmol) was dissolved in concentrated hydrochloric acid (12 mol/L, 5 mL), and the reaction mixture was heated to 100°C and stirred for 48 hours. The pH of the reaction mixture was adjusted to 7 with sodium bicarbonate. The mixture was concentrated under reduced pressure, then methanol was added and the insoluble matter was removed by filtration. The filtrate was concentrated again under reduced pressure to obtain a crude product containing **41-3,** which was directly used in the next reaction step. ESI-MS calculated for C₆H₁₂N₃ [M+H]⁺ = 126.1, found 126.1.

### Step 3

**41-3** (170 mg, 1.35 mmol) and intermediate **A** (500 mg, 1.35 mmol) were dissolved in DMF (10 mL), then DIEA (350 mg, 2.69 mmol) was added thereto, and the mixture was stirred at room temperature for 30 minutes. HATU (770 mg, 2.02 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 32%, retention time: 9 minutes) to obtain compound **41.** ¹H NMR (400 MHz, DMSO-*d*₆) δ 8.21 (d, *J* = 5.70 Hz, 1H), 7.98-7.94 (m, 1H), 7.45 (s, 1H), 6.85 (s, 1H), 6.71-6.68 (m, 1H), 6.53-6.48 (m, 1H), 4.10 (t, *J =* 8.22 Hz, 2H), 3.71-3.62 (m, 2H), 3.58 (s, 3H), 3.29-3.22 (m, 2H), 3.06-3.01 (m, 1H), 2.59-2.45 (m, 4H). ESI-MS calculated for C₁₇H₂₁F₃N₅O [M+H]⁺ = 368.2, found 368.1.

### Example 42

### Synthetic route:

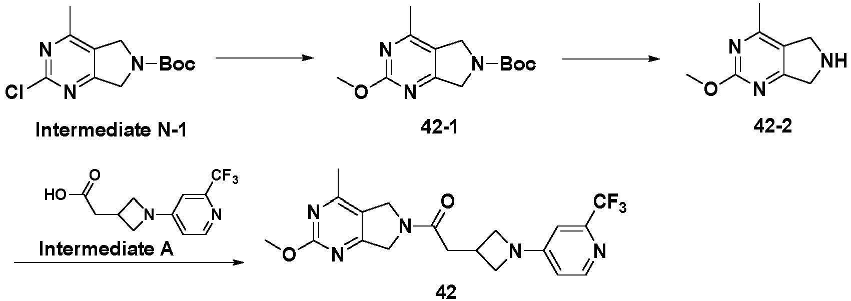

### Step 1

Intermediate **N-1** (300 mg, 1.11 mmol) was dissolved in methanol (5 mL), then sodium methoxide (72 mg, 1.33 mmol) was added thereto, and the mixture was heated to 60°C and stirred for 18 hours. The reaction mixture was diluted with water (30 mL) and extracted with dichloromethane (30 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 9/3, v/v) to obtain **42-1.** ¹H NMR (400 MHz, DMSO-*d*₆) δ 4.58-4.41 (m, 4H), 3.88 (s, 3H), 2.35 (s, 3H), 1.46 (s, 9H). ESI-MS calculated for C₁₃H₂₀N₃O₃ [M+H]⁺ = 266.1, found 266.1.

### Step 2

**42-1** (150 mg, 0.94 mmol) was dissolved in dichloromethane (2.5 mL), then trifluoroacetic acid (2.5 mL) was added thereto, and the mixture was stirred at room temperature for 1 hour. The reaction mixture was concentrated under reduced pressure to obtain a crude product containing **42-2,** which was directly used in the next reaction step. ESI-MS calculated for C₈H₁₂N₃O [M+H]⁺ = 166.1, found 166.1.

### Step 3

**42-2** (150 mg, 0.91 mmol) and intermediate **A** (236 mg, 0.91 mmol) were dissolved in DMF (2 mL), then DIEA (588 mg, 4.55 mmol) was added thereto, and the mixture was stirred at room temperature for 30 minutes. HATU (414 mg, 1.09 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 30 to 40%, retention time: 9 minutes) to obtain compound **42.** ¹H NMR (400 MHz, DMSO-*d*₆) δ 8.21 (d, *J* = 5.68 Hz, 1H), 6.72 (d, *J =* 2.28 Hz, 1H), 6.53 (dd, *J =* 5.74, 2.32 Hz, 1H), 4.82-4.77 (m, 2H), 4.58-4.52 (m, 2H), 4.22-4.14 (m, 2H), 3.90 (s, 3H), 3.73-3.66 (m, 2H), 3.15-3.09 (m, 1H), 2.88-2.81 (m, 2H), 2.37 (s, 3H). ESI-MS calculated for C₁₉H₂₁F₃N₅O₂ [M+H]⁺ = 408.2, found 408.1.

### Example 43

### Synthetic route:

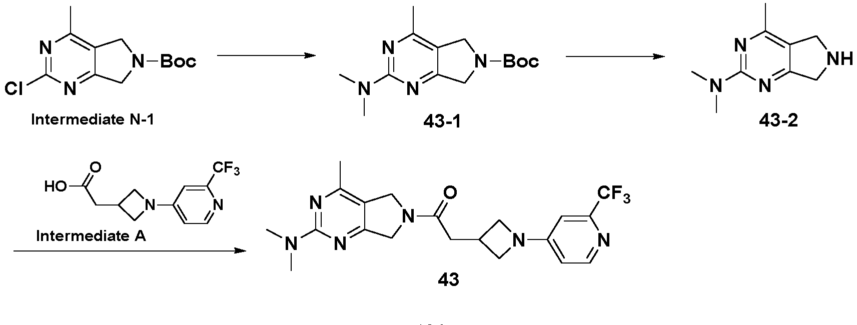

### Step 1

Intermediate **N-1** (300 mg, 1.11 mmol) was dissolved in DMF (5 mL), then potassium carbonate (461 mg, 3.34 mmol) and dimethylamine hydrochloride (109 mg, 1.33 mmol) were added thereto, and the mixture was heated to 70°C and stirred for 2 hours. The reaction mixture was diluted with water (30 mL) and extracted with dichloromethane (30 mL × 3). The organic phases were combined, washed with saturated brine (50 mL × 3), dried over anhydrous sodium sulfate, filtered, and concentrated under reduced pressure to obtain a crude product containing the target compound, which was purified by silica gel column chromatography (petroleum ether/ethyl acetate, 2/3, v/v) to obtain 43-1. ¹H NMR (400 MHz, DMSO-*d*₆) δ 4.50-4.41 (m, 2H), 4.39-4.33 (m, 2H), 3.10 (s, 6H), 2.24 (s, 3H), 1.46 (s, 9H). ESI-MS calculated for C₁₄H₂₃N₄O₂ [M+H]⁺ = 279.2, found 279.2.

### Step 2

**43-1** (260 mg, 0.93 mmol) was dissolved in dichloromethane (3 mL), then trifluoroacetic acid (3 mL) was added thereto, and the mixture was stirred at room temperature for 1 hour. The reaction mixture was concentrated under reduced pressure to obtain a crude product containing **43-2,** which was directly used in the next reaction step. ESI-MS calculated for C₉H₁₅N₄ [M+H]⁺ = 179.1, found 179.1.

### Step 3

**43-2** (100 mg, 0.56 mmol) and intermediate **A** (146 mg, 0.56 mmol) were dissolved in DMF (2 mL), then DIEA (434 mg, 3.36 mmol) was added thereto, and the mixture was stirred at room temperature for 30 minutes. HATU (256 mg, 0.67 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 33 to 63%, retention time: 9 minutes) to obtain compound **43.** ¹H NMR (400 MHz, DMSO-*d*₆) δ 8.20 (d, *J* = 5.70 Hz, 1H), 6.71 (d, *J =* 2.38 Hz, 1H), 6.55-6.51 (m, 1H), 4.71-4.65 (m, 2H), 4.48-4.38 (m, 2H), 4.19-4.13 (m, 2H), 3.72-3.66 (3, 2H), 3.15-3.10 (m, 7H), 2.86-2.81 (m, 2H), 2.25 (s, 3H). ESI-MS calculated for C₂₀H₂₄F₃N₆O [M+H]⁺ = 421.2, found 421.2.

### Example 44

### Synthetic route:

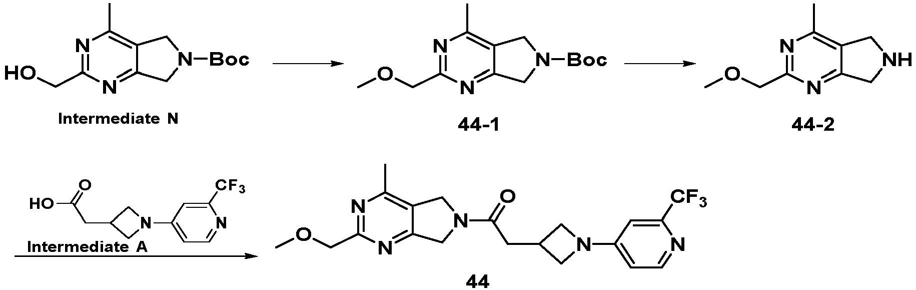

### Step 1

Intermediate N (150 mg, 0.57 mmol) was dissolved in tetrahydrofuran (5 mL), and the mixture was cooled to 0°C. Sodium hydride (60%, 45 mg, 1.13 mmol) was added thereto, and the mixture was stirred for 30 minutes. Iodomethane (80.0 mg, 0.57 mmol) was then added thereto, and the mixture was stirred at room temperature for another 2 hours. The reaction mixture was added with saturated ammonium chloride solution (10 mL) and extracted with ethyl acetate (10 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **44-1.** ESI-MS calculated for C₁₄H₂₂N₃O₃ [M+H]⁺ = 280.2, found 280.1.

### Step 2

**44-1** (100 mg, 0.36 mmol) was dissolved in dichloromethane (5 mL), then trifluoroacetic acid (5 mL) was added thereto, and the mixture was stirred at room temperature for 2 hours. The reaction mixture was concentrated under reduced pressure to obtain a crude product containing **44-2,** which was directly used in the next reaction step. ESI-MS calculated for C₉H₁₄N₃O [M+H]⁺ = 180.1, found 180.1.

### Step 3

**44-2** (50 mg, 0.28 mmol) and intermediate **A** (100 mg, 0.27 mmol) were dissolved in DMF (10 mL), then DIEA (70 mg, 0.54 mmol) was added thereto, and the mixture was stirred at room temperature for 30 minutes. HATU (153 mg, 0.40 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 21 to 51%, retention time: 9 minutes) to obtain compound **44.** ¹H NMR (400 MHz, DMSO-*d*₆) δ 8.27 (d, *J =* 5.60 Hz, 1H), 6.78 (d, *J =* 2.24 Hz, 1H), 6.60 (dd, *J =* 5.62, 2.28 Hz, 1H), 5.06-4.85 (m, 2H), 4.75-4.63 (m, 2H), 4.59 (s, 2H), 4.24 (t, *J* = 8.28 Hz, 2H), 3.79-3.73 (m, 2H), 3.42 (s, 3H), 3.27-3.12 (m, 1H), 2.97-2.89 (m, 2H), 2.50 (s, 3H). ESI-MS calculated for C₂₀H₂₄F₃N₆O [M+H]⁺ = 421.2, found 421.2.

### Example 45

### Synthetic route:

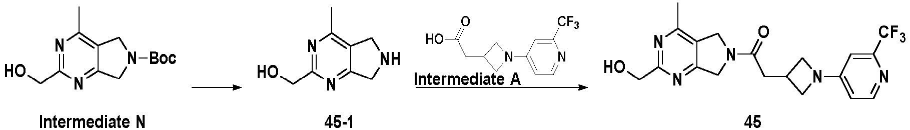

### Step 1

Intermediate **N** (100 mg, 0.38 mmol) was dissolved in dichloromethane (5 mL), then trifluoroacetic acid (5 mL) was added thereto, and the mixture was stirred at room temperature for 2 hours. The reaction mixture was concentrated under reduced pressure to obtain a crude product containing **45-1,** which was directly used in the next reaction step. ESI-MS calculated for C₈H₁₂N₃O [M+H]⁺ = 166.1, found 166.2.

### Step 2

**45-1** (60 mg, 0.36 mmol) and intermediate **A** (135 mg, 0.36 mmol) were dissolved in DMF (10 mL), then DIEA(94 mg, 0.73 mmol) was added thereto, and the mixture was stirred at room temperature for 30 minutes. HATU (207 mg, 0.54 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 17 to 50%, retention time: 9 minutes) to obtain compound **45.** ¹H NMR (400 MHz, DMSO-*d*₆) δ 8.21 (d, *J =* 5.66 Hz, 1H), 6.72 (d, *J =* 2.22 Hz, 1H), 6.54 (dd, *J =* 5.70, 2.28 Hz, 1H), 5.33-5.25 (m, 1H), 4.97-4.80 (m, 2H), 4.67-4.59 (m, 2H), 4.58-4.54 (m, 2H), 4.22-4.15 (m, 2H), 3.74-3.68 (m, 2H), 3.19-3.08 (m, 1H), 2.89-2.85 (m, 2H), 2.44 (s, 3H). ESI-MS calculated for C₁₉H₂₁F₃N₅O₂ [M+H]⁺ = 408.2, found 408.2.

### Example 46

### Synthetic route:

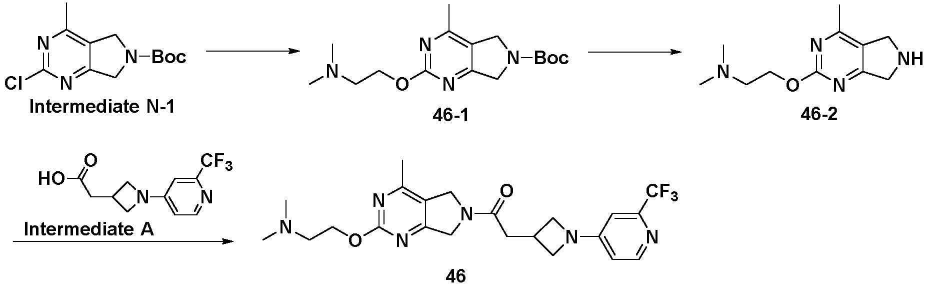

### Step 1

Intermediate **N-1** (300 mg, 1.11 mmol) was dissolved in N,N-dimethylethanolamine (9 mL), then sodium hydride (60%, 177 mg, 4.45 mmol) was added thereto, and the mixture was heated to 60°C and stirred for 3 hours. The reaction mixture was cooled, then slowly added with saturated ammonium chloride solution (15 mL), and extracted with ethyl acetate (20 mL × 3). The organic phases were combined, washed with saturated brine (20 mL), dried over anhydrous sodium sulfate, filtered, and concentrated under reduced pressure to obtain a crude product containing the target compound, which was purified by silica gel column chromatography (petroleum ether/ethyl acetate, 2/3, v/v) to obtain **46-1.** ¹H NMR (400 MHz, DMSO-*d*₆) δ 4.53-4.45 (m, 4H), 4.38-4.32 (m, 2H), 2.61 (t, *J =* 5.80 Hz, 2H), 2.34 (s, 3H), 2.21 (s, 6H), 1.46 (s, 9H). ESI-MS calculated for C₁₆H₂₇N₄O₃ [M+H]⁺ = 323.2, found 323.2.

### Step 2

**46-1** (380 mg, 1.18 mmol) was dissolved in dichloromethane (4 mL), then trifluoroacetic acid (4 mL) was added thereto, and the mixture was stirred at room temperature for 2 hours. The reaction mixture was concentrated under reduced pressure to obtain a crude product containing **46-2,** which was directly used in the next reaction step. ESI-MS calculated for C₁₁H₁₉N₄O [M+H]⁺ = 223.2, found 223.1.

### Step 3

**46-2** (260 mg, 1.17 mmol) and intermediate A (304 mg, 1.17 mmol) were dissolved in DMF (3 mL), then DIEA (755 mg, 5.85 mmol) was added thereto, and the mixture was stirred at room temperature for 30 minutes. HATU (533 mg, 1.40 mmol) was then added thereto, and the mixture was stirred at room temperature for another 3 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 18 to 48%, retention time: 9 minutes) to obtain compound **46.** ¹H NMR (400 MHz, DMSO-*d*₆) δ 8.21 (d, *J =* 5.66 Hz, 1H), 6.71 (d, *J =* 2.24 Hz, 1H), 6.53 (dd, *J =* 5.68, 2.26 Hz, 1H), 4.81-4.74 (m, 2H), 4.58-4.50 (m, 2H), 4.52-4.19 (m, 2H), 4.18-4.15 (m, 2H), 3.73-3.65 (m, 2H), 3.14-3.08 (m, 1H), 2.87-2.82 (m, 2H), 2.65-2.59 (m, 2H), 2.33 (s, 3H), 2.21 (s, 6H). ESI-MS calculated for C₂₂H₂₈F₃N₆O₂ [M+H]⁺ = 465.2, found 465.2.

### Example 47

### Synthetic route:

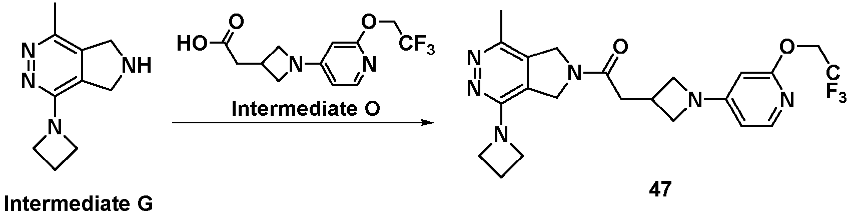

### Step 1

Intermediate **G** (30 mg, 0.16 mmol) and intermediate O (45 mg, 0.16 mmol) were dissolved in DMF (5 mL), then DIEA (122 mg, 0.95 mmol) was added thereto, and the mixture was stirred at room temperature for 30 minutes. HATU (72 mg, 0.19 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 32 to 62%, retention time: 9 minutes) to obtain compound **47.** ¹H NMR (400 MHz, DMSO-*d*₆) δ 7.76 (d, *J* = 5.78 Hz, 1H), 6.17-6.13 (m, 1H), 5.79 (t, *J =* 1.86 Hz, 1H), 4.94-4.77 (m, 4H), 4.64-4.56 (m, 2H), 4.14-4.06 (m, 6H), 3.65-3.56 (m, 2H), 3.13-3.02 (m, 1H), 2.85-2.82 (m, 2H), 2.40 (s, 3H), 2.41-2.33 (m, 2H). ESI-MS calculated for C₂₂H₂₆F₃N₆O₂ [M+H]⁺ = 463.2, found 463.2.

### Example 48

### Synthetic route:

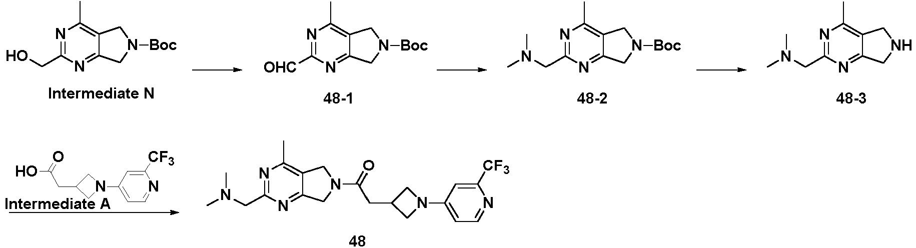

### Step 1

Intermediate **N** (150 mg, 0.57 mmol) was dissolved in dichloromethane (5 mL), then active manganese dioxide (490 mg, 5.70 mmol) was added thereto, and the mixture was stirred at room temperature for 1 hour. The reaction mixture was filtered and concentrated under reduced pressure to obtain **48-1.** ESI-MS calculated for C₁₃H₁₈N₃O₃ [M+H]⁺ = 264.1, found 264.2.

### Step 2

**48-1** (140 mg, 0.53 mmol) was dissolved in dichloromethane (5 mL), then dimethylamine hydrochloride (43 mg, 0.53 mmol), TEA (54 mg, 0.53 mmol), acetic acid (32 mg, 0.53 mmol), and sodium triacetoxyborohydride (225 mg, 1.06 mmol) were added thereto, and the mixture was stirred at room temperature for 3 hours. After the reaction was completed, the reaction mixture was added with saturated sodium bicarbonate solution (15 mL) and extracted with dichloromethane (50 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate/ethanol, 1/7/2, v/v/v) to obtain **48-2.** ¹H NMR (400 MHz, DMSO-*d*₆) δ 4.73-4.52 (m, 4H), 4.25-4.18 (s, 2H), 3.17 (s, 6H), 2.52 (s, 3H), 1.47 (s, 9H). ESI-MS calculated for C₁₅H₂₅N₄O₂ [M+H]⁺ = 293.2, found 293.2.

### Step 3

**48-2** (256 mg, 0.88 mmol) was dissolved in dichloromethane (3 mL), then trifluoroacetic acid (3 mL) was added thereto, and the mixture was stirred at room temperature for 1 hour. The reaction mixture was concentrated under reduced pressure to obtain a crude product containing **48-3,** which was directly used in the next reaction step. ESI-MS calculated for C₁₀H₁₇N₄ [M+H]⁺ = 193.1, found 193.1.

### Step 4

**48-3** (150 mg, 0.78 mmol) and intermediate **A** (203 mg, 0.78 mmol) were dissolved in DMF (3 mL), then DIEA (606 mg, 4.68 mmol) was added thereto, and the mixture was stirred at room temperature for 30 minutes. HATU (359 mg, 0.94 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 32%, retention time: 9 minutes) to obtain compound **48.** ¹H NMR (400 MHz, DMSO-*d*₆) δ 8.21 (d, *J =* 5.66 Hz, 1H), 6.71 (d, *J =* 2.24 Hz, 1H), 6.53 (dd, *J =* 5.68, 2.26 Hz, 1H), 4.89-4.84 (m, 2H), 4.66-4.60 (m, 2H), 4.18-4.15 (m, 2H), 3.73-3.67 (m, 2H), 3.59 (s, 2H), 3.19-3.13 (m, 1H), 2.87-2.82 (m, 2H), 2.41 (s, 3H), 2.21 (s, 6H). ESI-MS calculated for C₂₁H₂₆F₃N₆O [M+H]⁺ = 435.2, found 435.2.

### Example 49

### Synthetic route:

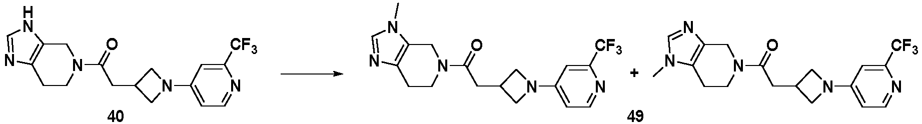

### Step 1

**40** (60 mg, 0.16 mmol) was dissolved in tetrahydrofuran (5 mL), and the mixture was cooled to 0°C. Sodium hydride (60%, 4 mg, 0.16 mmol) was added thereto, and the mixture was stirred for 30 minutes. Iodomethane (24 mg, 0.16 mmol) was then added thereto, and the mixture was stirred at room temperature for 1 hour. After the reaction was completed, the reaction mixture was quenched with water (1 mL) and then purified by preparative high performance liquid chromatography (chromatographic column: Puningtech-Pntulips-C18, 5 µm, 21.2 * 150 mm, mobile phase: acetonitrile-10 mmol/L ammonium bicarbonate aqueous solution, gradient: 28 to 38%, retention time: 9 minutes) to obtain compound **49.** ¹H NMR (400 MHz, DMSO-*d*₆) δ 8.26-8.17 (m, 1H), 7.51-7.43 (m, 1H), 6.71-6.68 (m, 1H), 6.52-6.49 (m, 1H), 4.56-4.53 (m, 1H), 4.40-4.34 (m, 1H), 4.18-4.11 (m, 2H), 3.79-3.70 (m, 2H), 3.68-3.63 (m, 2H), 3.56-3.48 (m, 3H), 3.10-3.01 (m, 1H), 2.94-2.81 (m, 2H), 2.69-2.65 (m, 1H), 2.59-2.54 (m, 1H). ESI-MS calculated for C₁₈H₂₁F₃N₅O [M+H]⁺ = 380.2, found 380.1.

### Example 50

### Synthetic route:

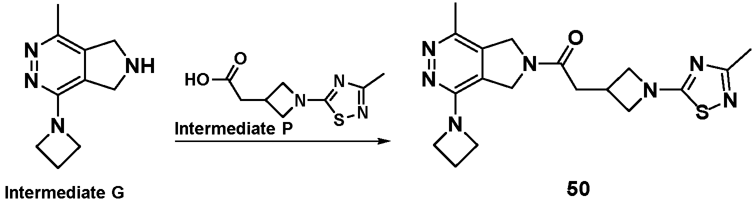

### Step 1

Intermediate **G** (30 mg, 0.16 mmol) and intermediate **P** (41 mg, 0.19 mmol) were dissolved in DMF (5 mL), then DIEA (62 mg, 0.47 mmol) was added thereto, and the mixture was stirred at room temperature for 30 minutes. HATU (90 mg, 0.24 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 9 to 39%, retention time: 9 minutes) to obtain compound **50.** ¹H NMR (400 MHz, DMSO-*d*₆) δ 4.85-4.74 (m, 2H), 4.65-4.51 (m, 2H), 4.25-4.20 (m, 2H), 4.15-4.07 (m, 4H), 3.83-3.76 (m, 2H), 3.25-3.15 (m, 1H), 2.92-2.84 (m, 2H), 2.40 (s, 3H), 2.38-2.31 (m, 2H), 2.28 (s, 3H). ESI-MS calculated for C₁₈H₂₄N₇OS [M+H]⁺ = 386.2, found 386.2.

### Example 51

### Synthetic route:

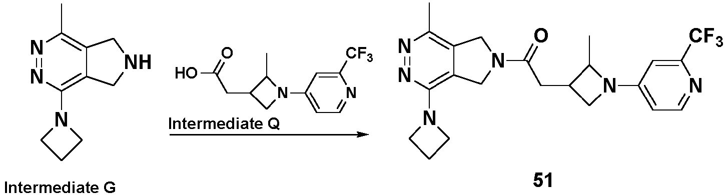

### Step 1

Intermediate **G** (30 mg, 0.16 mmol) and intermediate **Q** (43 mg, 0.11 mmol) were dissolved in DMF (3 mL), then DIEA (122 mg, 0.95 mmol) was added thereto, and the mixture was stirred at room temperature for 30 minutes. HATU (72 mg, 0.19 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 52%, retention time: 9 minutes) to obtain compound **51.** ¹H NMR (400 MHz, DMSO-*d*₆) δ 8.20 (d, *J* = 5.70 Hz, 1H), 6.70 (s, 1H), 6.58 (d, *J =* 5.64 Hz, 1H), 4.92-4.84 (m, 2H), 4.66-4.51 (m, 3H), 4.15-4.02 (m, 4H), 4.01-3.96 (m, 1H), 3.76-3.71 (m, 1H), 3.24-3.20 (m, 1H), 2.89-2.77 (m, 2H), 2.42 (s, 3H), 2.39-2.34 (m, 2H), 1.37-1.34 (m, 3H). ESI-MS calculated for C₂₂H₂₆F₃N₆O [M+H]⁺ = 447.2, found 447.1.

### Example 52

### Synthetic route:

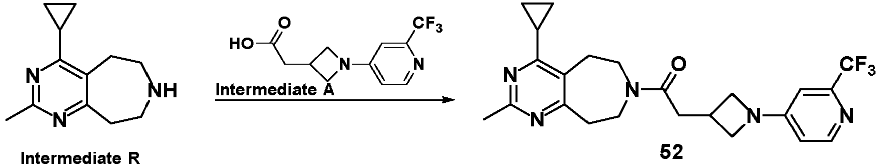

### Step 1

Intermediate **R** (170 mg, 0.84 mmol) and intermediate **A** (220 mg, 0.84 mmol) were dissolved in DMF (5 mL), then DIEA (210 mg, 1.67 mmol) was added thereto, and the mixture was stirred at room temperature for 30 minutes. HATU (480 mg, 1.25 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Agilent-Poroshell HPH-C18, 2.7 µm, 4.6 * 50 mm, mobile phase: acetonitrile-10 mmol/L ammonium bicarbonate aqueous solution, gradient: 30 to 60%, retention time: 9 minutes) to obtain compound **52.** ¹H NMR (400 MHz, DMSO-*d*₆) δ 8.21 (d, *J* = 5.66 Hz, 1H), 6.70-6.67 (m, 1H), 6.51 (dd, *J =* 5.68, 2.18 Hz, 1H), 4.15-4.09 (m, 2H), 3.70-3.55 (m, 6H), 3.14-2.95 (m, 5H), 2.89-2.81 (m, 2H), 2.44-2.41 (m, 3H), 2.31-2.25 (m, 1H), 1.06-0.90 (m, 4H). ESI-MS calculated for C₂₃H₂₇F₃N₅O [M+H]⁺ = 446.2, found 446.2.

### Example 53

### Synthetic route:

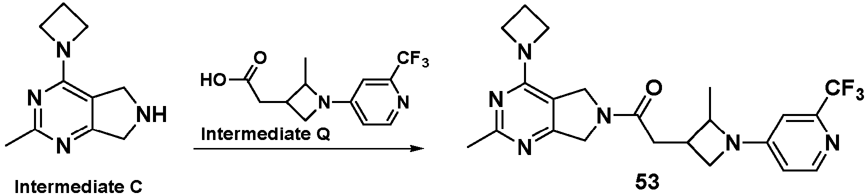

### Step 1

Intermediate **C** (83 mg, 0.22 mmol) and intermediate **Q** (60 mg, 0.22 mmol) were dissolved in DMF (3 mL), then DIEA (113 mg, 0.88 mmol) was added thereto, and the mixture was stirred at room temperature for 30 minutes. HATU (99 mg, 0.26 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 23 to 53%, retention time: 9 minutes) to obtain compound **53.** ¹H NMR (400 MHz, DMSO-*d*₆) δ 8.19 (d, *J* = 5.34 Hz, 1H), 6.70-6.67 (m, 1H), 6.58-6.55 (m, 1H), 4.89-4.81 (m, 1H), 4.63-4.52 (m, 3H), 4.36-4.31 (m, 1H), 4.26-4.10 (m, 4H), 4.02-3.95 (m, 1H), 3.74-3.68 (m, 1H), 3.51-3.46 (m, 1H), 3.22-3.17 (m, 1H), 3.05-2.97 (m, 1H), 2.88-2.64 (m, 3H), 2.42-2.30 (m, 4H), 1.48 (d, *J* = 6.14 Hz, 1.2H), 1.34 (d, *J* = 6.14 Hz, 1.8H). ESI-MS calculated for C₂₂H₂₆F₃N₆O [M+H]⁺ = 447.2, found 447.2.

### Example 54

### Synthetic route:

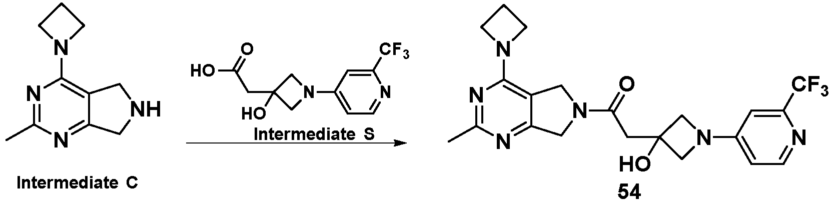

### Step 1

Intermediate **C** (55 mg, 0.29 mmol) and intermediate S (100 mg, 0.29 mmol) were dissolved in DMF (3 mL), then DIEA (150 mg, 1.16 mmol) was added thereto, and the mixture was stirred at room temperature for 30 minutes. HATU (132 mg, 0.35 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 17 to 47%, retention time: 9 minutes) to obtain compound **54.** ¹H NMR (400 MHz, DMSO-*d*₆) δ 8.21 (d, *J* = 5.66 Hz, 1H), 6.75 (d, *J =* 2.11 Hz, 1H), 6.56 (d, *J =* 5.70 Hz, 1H), 5.87 (s, 1H), 4.87 (s, 1H), 4.62 (s, 2H), 4.35 (s, 1H), 4.19-4.15 (m, 6H), 3.90-3.84 (m, 2H), 2.88 (d, *J =* 6.10 Hz, 2H), 2.35-2.27 (m, 5H). ESI-MS calculated for C₂₁H₂₄F₃N₆O₂ [M+H]⁺ = 449.2, found 449.2.

### Example 55

### Synthetic route:

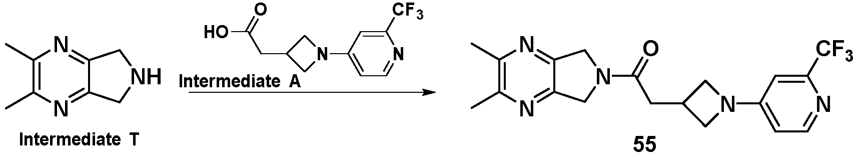

### Step 1

Intermediate **T** (15 mg, 0.10 mmol) and intermediate S (26 mg, 0.10 mmol) were dissolved in DMF (1 mL), then TEA (31 mg, 0.30 mmol) was added thereto, and the mixture was stirred at room temperature for 30 minutes. HATU (46 mg, 0.12 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: XBndge-C18, 10 µm, 19 * 250 mm, mobile phase: acetonitrile-10 mmol/L ammonium bicarbonate aqueous solution, gradient: 25 to 40%, retention time: 9 minutes) to obtain compound 55. ¹H NMR (400 MHz, DMSO-*d*₆) δ 8.29 (d, *J=* 5.70 Hz, 1H), 6.80 (d, *J =* 2.20 Hz, 1H), 6.62 (dd, *J =* 5.72, 2.22 Hz, 1H), 4.92 (s, 2H), 4.67 (s, 2H), 4.28-4.24 (m, 2H), 3.81-3.77 (m, 2H), 3.24-3.17 (m, 1H), 2.97-2.93 (m, 2H), 2.59 (s, 6H). ESI-MS calculated for C₁₉H₂₁F₃N₅O [M+H]⁺ = 392.2, found 392.2.

### Example 56

### Synthetic route:

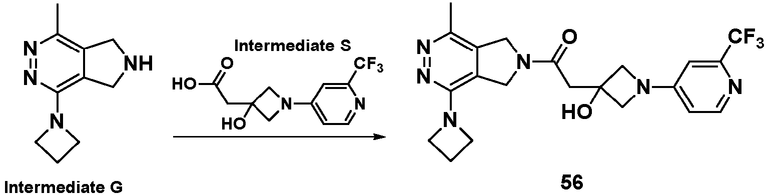

### Step 1

Intermediate **T** (20 mg, 0.11 mmol) and intermediate **S** (29 mg, 0.11 mmol) were dissolved in DMF (3 mL), then DIEA (55 mg, 0.42 mmol) was added thereto, and the mixture was stirred at room temperature for 30 minutes. HATU (48 mg, 0.13 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 17 to 47%, retention time: 9 minutes) to obtain compound **56.** ¹H NMR (400 MHz, DMSO-*d*₆) δ 8.22 (d, *J* = 5.68 Hz, 1H), 6.76 (t, *J =* 2.52 Hz, 1H), 6.58-6.56 (m, 1H), 5.93-5.90 (m, 1H), 4.91-4.82 (m, 2H), 4.65-4.57 (m, 2H), 4.19-4.15 (m, 2H), 4.13-4.09 (m, 4H), 3.90-3.87 (m, 2H), 2.92-2.88 (m, 2H), 2.41-2.39 (m, 3H), 2.36-2.32 (m, 2H). ESI-MS calculated for C₂₁H₂₄F₃N₆O₂ [M+H]⁺ = 449.2, found 449.1.

### Example 57

### Synthetic route:

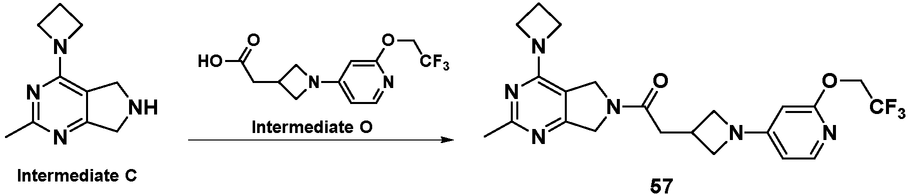

### Step 1

Intermediate **C** (131 mg, 0.69 mmol) and intermediate **O** (100 mg, 0.34 mmol) were dissolved in DMF (5 mL), then DIEA (178 mg, 1.38 mmol) was added thereto, and the mixture was stirred at room temperature for 30 minutes. HATU (157 mg, 0.41 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 37 to 47%, retention time: 9 minutes) to obtain compound **57.** ¹H NMR (400 MHz, DMSO-*d*₆) δ 7.75 (d, *J* = 5.78 Hz, 1H), 6.16-6.12 (m, 1H), 5.78-5.77 (m, 1H), 4.93-4.81 (m, 3H), 4.62-4.57 (m, 2H), 4.36-3.34 (m, 1H), 4.17-4.10 (m, 4H), 4.08-4.04 (m, 2H), 3.61-3.58 (m, 2H), 3.08-3.02 (m, 1H), 2.81-2.78 (m, 2H), 2.36-2.29 (m, 5H). ESI-MS calculated for C₂₂H₂₆F₃N₆O₂ [M+H]⁺ = 463.2, found 463.2.

### Example 58

### Synthetic route:

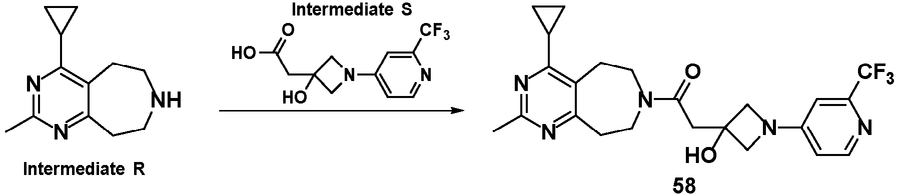

### Step 1

Intermediate **R** (59 mg, 0.29 mmol) and intermediate **S** (100 mg, 0.29 mmol) were dissolved in DMF (3 mL), then DIEA (225 mg, 1.74 mmol) was added thereto, and the mixture was stirred at room temperature for 30 minutes. HATU (132 mg, 0.35 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 31 to 41%, retention time: 9 minutes) to obtain compound 58. ¹H NMR (400 MHz, DMSO-*d*₆) δ 8.21 (d, *J* = 5.64 Hz, 1H), 6.72 (d, *J =* 1.88 Hz, 1H), 6.62-6.49 (m, 1H), 6.09-6.03 (br, 1H), 4.09-4.04 (m, 2H), 3.88-3.84 (m, 2H), 3.69-3.64 (m, 4H), 3.12-2.96 (m, 4H), 2.92-2.89 (s, 2H), 2.42-2.39 (m, 3H), 2.33-2.22 (m, 1H), 1.01-0.91 (m, 4H). ESI-MS calculated for C₂₃H₂₇F₃N₅O₂ [M+H]⁺ = 462.2, found 462.2.

### Example 59

### Synthetic route:

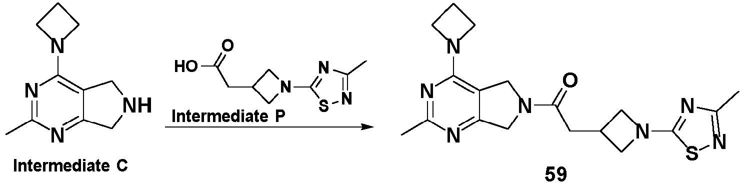

### Step 1

Intermediate **C** (91 mg, 0.48 mmol) and intermediate **P** (122 mg, 0.57 mmol) were dissolved in DMF (5 mL), then DIEA (185 mg, 1.43 mmol) was added thereto, and the mixture was stirred at room temperature for 30 minutes. HATU (272 mg, 0.72 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 13 to 43%, retention time: 8 minutes) to obtain compound **59.** ¹H NMR (400 MHz, DMSO-*d*₆) δ 4.85-4.82 (m, 1H), 4.62-4.55 (m, 2H), 4.35-4.32 (m, 1H), 4.27-4.10 (m, 6H), 3.83-3.76 (m, 2H), 3.22-3.13 (m, 1H), 2.88-2.81 (m, 2H), 2.35 (s, 3H), 2.34-2.28 (m, 2H), 2.27 (s, 3H). ESI-MS calculated for C₁₈H₂₄N₇OS [M+H]⁺ = 386.2, found 386.2.

### Example 60

### Synthetic route:

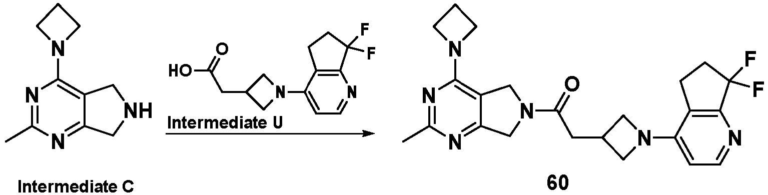

### Step 1

Intermediate **C** (83 mg, 0.43 mmol) and intermediate **U** (140 mg, 0.52 mmol) were dissolved in DMF (5 mL), then DIEA (168 mg, 1.30 mmol) was added thereto, and the mixture was stirred at room temperature for 30 minutes. HATU (248 mg, 0.65 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 20 to 50%, retention time: 9 minutes) to obtain compound **60.** ¹H NMR (400 MHz, DMSO-*d*₆) δ 8.11 (d, *J* = 5.46 Hz, 1H), 6.29-6.25 (m, 1H), 4.85-4.84 (m, 1H), 4.62-4.57 (m, 2H), 4.34-4.25 (m, 3H), 4.22-4.11 (m, 4H), 3.89-3.76 (m, 2H), 3.11-2.94 (m, 3H), 2.85-2.77 (m, 2H), 2.35 (s, 3H), 2.34-2.30 (m, 2H). ESI-MS calculated for C₂₃H₂₇F₂N₆O [M+H]⁺ = 441.2, found 441.2.

### Example 61

### Synthetic route:

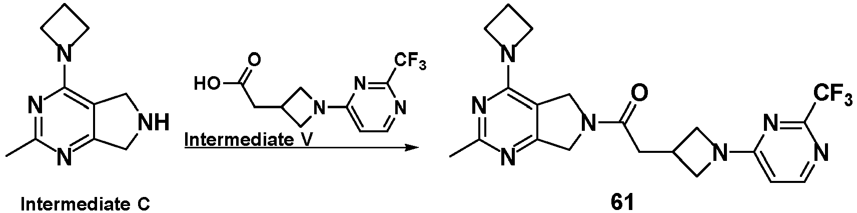

### Step 1

Intermediate **C** (116 mg, 0.31 mmol) and intermediate **V** (80 mg, 0.56 mmol) were dissolved in DMF (5 mL), then DIEA (118.76 mg, 0.92 mmol) was added thereto, and the mixture was stirred at room temperature for 30 minutes. HATU (175 mg, 0.46 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 33 to 63%, retention time: 9 minutes) to obtain compound **61.** ¹H NMR (400 MHz, DMSO-*d*₆) δ 8.27 (d, *J* = 6.06 Hz, 1H), 6.59 (d, *J =* 6.02 Hz, 1H), 4.84-4.82 (m, 1H), 4.61-4.57 (m, 2H), 4.36-4.34 (m, 1H), 4.28-4.13 (m, 6H), 3.84-3.77 (m, 2H), 3.17-3.13 (m, 1H), 2.87-2.82 (m, 2H), 2.39 (s, 3H), 2.38-2.30 (m, 2H). ESI-MS calculated for C₂₀H₂₃F₃N₇O [M+H]⁺ = 434.2, found 434.2.

### Example 62

### Synthetic route:

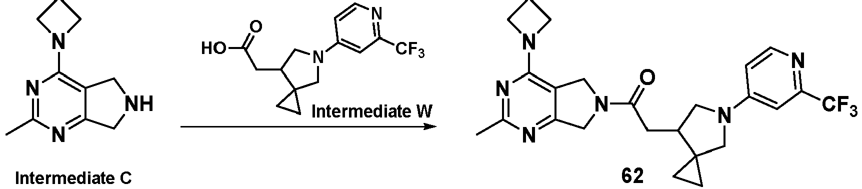

### Step 1

Intermediate **C** (51 mg, 0.13 mmol) and intermediate **W** (40 mg, 0.13 mmol) were dissolved in DMF (5 mL), then DIEA(51 mg, 0.40 mmol) was added thereto, and the mixture was stirred at room temperature for 30 minutes. HATU (76 mg, 0.20 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 33 to 63%, retention time: 9 minutes) to obtain compound **62.** ¹H NMR (400 MHz, DMSO-*d*₆) δ 8.20 (d, *J* = 5.80 Hz, 1H), 6.76 (s, 1H), 6.62 (s, 1H), 4.81-4.78 (m, 1H), 4.62-4.56 (m, 2H), 4.36-4.34 (m, 1H), 4.20-4.08 (m, 4H), 3.74-3.67 (m, 1H), 3.52-3.47 (m, 1H), 3.24-3.19 (m, 2H), 2.42 (s, 3H), 2.35-2.28 (m, 5H), 0.81-0.55 (m, 4H). ESI-MS calculated for C₂₄H₂₈F₃N₆O [M+H]⁺ = 473.2, found 473.2.

### Example 63

### Synthetic route:

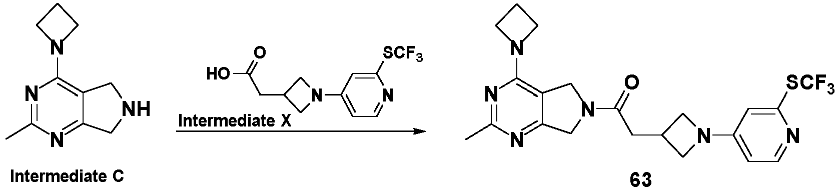

### Step 1

Intermediate **C** (130 mg, 0.34 mmol) and intermediate **X** (100 mg, 0.34 mmol) were dissolved in DMF (5 mL), then DIEA (177 mg, 1.37 mmol) was added thereto, and the mixture was stirred at room temperature for 30 minutes. HATU (156 mg, 0.41 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 26 to 56%, retention time: 9 minutes) to obtain compound **63.** ¹H NMR (400 MHz, DMSO-*d*₆) δ 8.10 (d, *J* = 5.72 Hz, 1H), 6.58 (d, *J =* 2.20 Hz, 1H), 6.39 (dd, *J =* 5.80, 2.12 Hz, 1H), 4.84-4.82 (m, 1H), 4.61-4.57 (m, 2H), 4.35-4.33 (m, 1H), 4.26-4.04 (m, 6H), 3.69-3.60 (m, 2H), 3.10-3.06 (m, 1H), 2.84-2.79 (m, 2H), 2.34-2.30 (m, 5H). ESI-MS calculated for C₂₁H₂₄F₃N₆OS [M+H]⁺ = 465.2, found 465.2.

### Example 64

### Synthetic route:

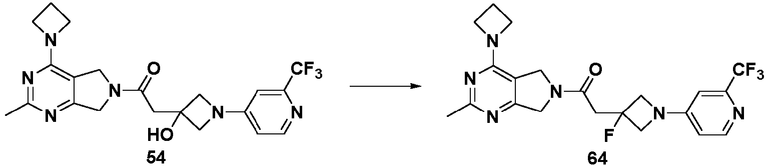

### Step 1

**54** (20 mg, 0.04 mmol) was dissolved in dichloromethane (10 mL) and cooled to - 78°C, followed by addition of diethylaminosulfur trifluoride (15 mg, 0.09 mmol). The mixture was stirred at room temperature for 1 hour. The reaction mixture was concentrated under reduced pressure and purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 24 to 54%, retention time: 9 minutes) to obtain compound 64. ¹H NMR (400 MHz, DMSO-*d*₆) δ 8.26 (d, *J =* 5.52 Hz, 1H), 6.84 (d, *J =* 2.20 Hz, 1H), 6.64 (dd, *J =* 5.72, 2.30 Hz, 1H), 4.92-4.85 (m, 2H), 4.63-4.57 (m, 2H), 4.29-4.15 (m, 7H), 3.25-3.19 (m, 2H), 2.34- 2.30 (m, 6H). ESI-MS calculated for C₂₁H₂₃F₄N₆O [M+H]⁺ = 451.2, found 451.2.

### Example 65

### Synthetic route:

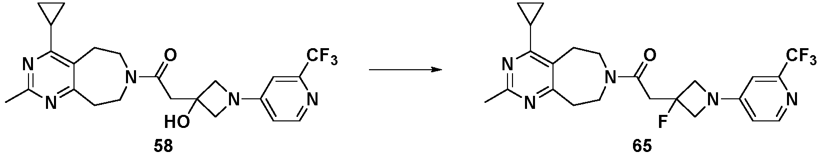

### Step 1

**58** (30 mg, 0.07 mmol) was dissolved in dichloromethane (20 mL) and cooled to - 78°C, followed by addition of diethylaminosulfur trifluoride (31 mg, 0.20 mmol). The mixture was stirred at room temperature for 1 hour. The reaction mixture was concentrated under reduced pressure and purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 36 to 46%, retention time: 13 minutes) to obtain compound **65.** ¹H NMR (400 MHz, DMSO-*d*₆) δ 8.25 (d, *J =* 5.60 Hz, 1H), 6.82 (d, *J =* 2.16 Hz, 1H), 6.65-6.56 (m, 1H), 4.23-4.18 (m, 4H), 3.69-3.64 (m, 4H), 3.22-3.17 (m, 2H), 3.08-2.96 (m, 4H), 2.42-2.40 (s, 3H), 2.33-2.24 (m, 1H), 0.99-0.95 (m, 4H). ESI-MS calculated for C₂₃H₂₆F₄N₅O [M+H]⁺ = 464.2, found 464.2.

### Example 66

### Synthetic route:

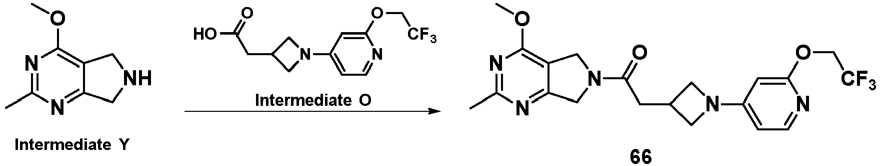

### Step 1

The trifluoroacetate salt of intermediate **Y** (170 mg, 0.31 mmol) and intermediate O (100 mg, 0.31 mmol) were dissolved in DMF (5 mL), then DIEA (240 mg, 1.86 mmol) was added thereto, and the mixture was stirred at room temperature for 30 minutes. HATU (141 mg, 0.37 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 38 to 68%, retention time: 9 minutes) to obtain compound 66. ¹H NMR (400 MHz, DMSO-*d*₆) δ 7.76 (d, *J =* 5.80 Hz, 1H), 6.15 (dd, *J =* 5.80, 1.92 Hz, 1H), 5.78 (d, *J =* 1.94 Hz, 1H), 4.93-4.86 (m, 2H), 4.80-4.70 (m, 2H), 4.53-4.49 (m, 2H), 4.09-3.98 (m, 2H), 3.97 (s, 3H), 3.62-3.59 (m, 2H), 3.10-3.03 (m, 1H), 2.84-2.81 (m, 2H), 2.55 (s, 3H). ESI-MS calculated for C₂₀H₂₃F₃N₅O₃ [M+H]⁺ = 438.2, found 438.2.

### Example 67

### Synthetic route:

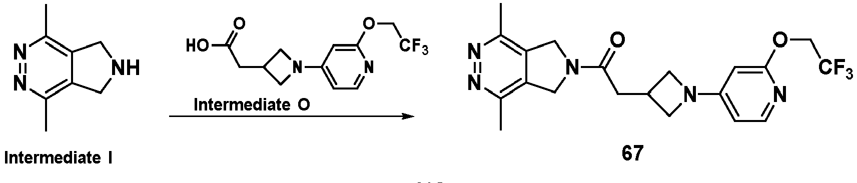

### Step 1

The trifluoroacetate salt of intermediate **I** (80 mg, 0.54 mmol) and intermediate **O** (156 mg, 0.54 mmol) were dissolved in DMF (5 mL), then DIEA (209 mg, 1.62 mmol) was added thereto, and the mixture was stirred at room temperature for 30 minutes. HATU (265 mg, 0.70 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 26 to 56%, retention time: 9 minutes) to obtain compound **67.** ¹H NMR (400 MHz, DMSO-*d*₆) δ 7.76 (d, *J =* 5.78 Hz, 1H), 6.15 (dd, *J =* 5.82, 1.94 Hz, 1H), 5.78 (d, *J =* 1.94 Hz, 1H), 5.01-4.77 (m, 4H), 4.71-4.68 (m, 2H), 4.11-4.06 (m, 2H), 3.63-3.58 (m, 2H), 3.13-3.05 (m, 1H), 2.84 (d, *J =* 7.62 Hz, 2H), 2.55-2.53 (m, 6H). ESI-MS calculated for C₂₀H₂₃F₃N₅O₂ [M+H]⁺ = 422.2, found 422.2.

### Example 68

### Synthetic route:

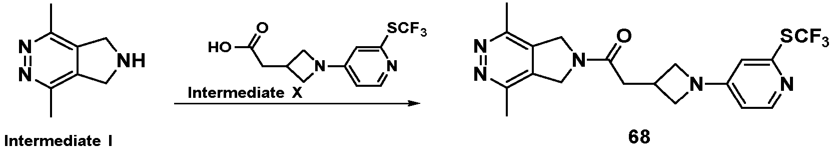

### Step 1

The trifluoroacetate salt of intermediate **I** (54 mg, 0.36 mmol) and intermediate **X** (70 mg, 0.24 mmol) were dissolved in DMF (5 mL), then DIEA (93 mg, 0.72 mmol) was added thereto, and the mixture was stirred at room temperature for 30 minutes. HATU (128 mg, 0.34 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 25 to 55%, retention time: 9 minutes) to obtain compound **68.** ¹H NMR (400 MHz, DMSO-*d*₆) δ 8.10 (d, *J =* 5.72 Hz, 1H), 6.59 (d, *J =* 2.18 Hz, 1H), 6.40 (dd, *J =* 5.76, 2.18 Hz, 1H), 4.93-4.91 (m, 2H), 4.70-4.67 (m, 2H), 4.17-4.11 (m, 2H), 3.69-3.65 (m, 2H), 3.15-3.09 (m, 1H), 2.86 (d, *J* = 7.68 Hz, 2H), 2.55-2.53 (m, 6H). ESI-MS calculated for C₁₉H₂₁F₃N₅OS [M+H]⁺ = 424.1, found 424.1.

### Example 69

### Synthetic route:

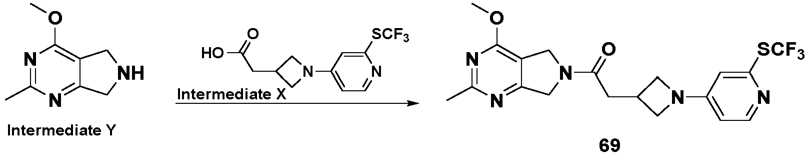

### Step 1

The trifluoroacetate salt of intermediate **Y** (30 mg, 0.10 mmol) and intermediate **X** (17 mg, 0.10 mmol) were dissolved in DMF (5 mL), then DIEA (80 mg, 0.62 mmol) was added thereto, and the mixture was stirred at room temperature for 30 minutes. HATU (47 mg, 0.12 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 34 to 64%, retention time: 9 minutes) to obtain compound **69.** ¹H NMR (400 MHz, DMSO-*d*₆) δ 8.10 (d, *J =* 5.70 Hz, 1H), 6.58 (d, *J =* 2.20 Hz, 1H), 6.39 (dd, *J =* 5.74, 2.20 Hz, 1H), 4.78-4.74 (m, 2H), 4.52-4.48 (m, 2H), 4.15-4.10 (m, 2H), 3.96 (s, 3H), 3.71-3.60 (m, 2H), 3.15-3.02 (m, 1H), 2.85-2.83 (m, 2H), 2.55 (s, 3H). ESI-MS calculated for C₁₉H₂₁F₃N₅O₂S [M+H]⁺ = 440.1, found 440.1.

### Example 70

### Synthetic route:

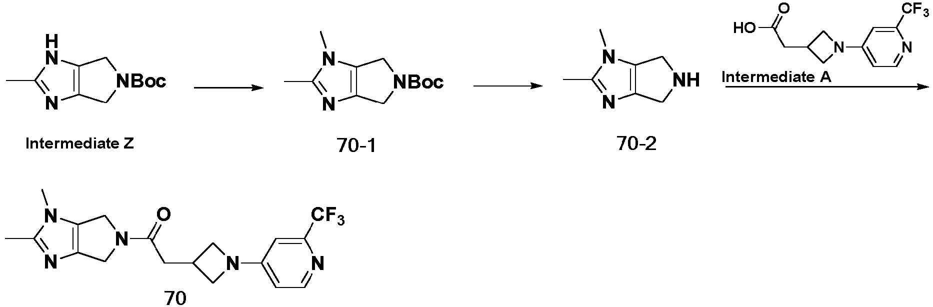

### Step 1

Intermediate **Z** (60 mg, 0.27 mmol) was dissolved in DMF (10 mL), then sodium hydride (60%, 22 mg, 0.54 mmol) was added thereto at 0°C, and the mixture was stirred for 30 minutes. Iodomethane (57 mg, 0.40 mmol) was then added thereto, and the mixture was stirred at room temperature for 1 hour. The reaction mixture was quenched with saturated ammonium chloride solution (10 mL) and extracted with dichloromethane (30 mL × 3). The organic phases were combined, dried over anhydrous sodium sulfate, filtered, concentrated under reduced pressure to obtain **70-1.** ESI-MS calculated for C₁₂H₂₀N₃O₂ [M+H]⁺ = 238.2, found 238.1.

### Step 2

**70-1** (1.1 g, 4.15 mmol) was dissolved in dichloromethane (5 mL), then trifluoroacetic acid (5 mL) was added thereto, and the mixture was stirred at room temperature for 3 hours. The reaction mixture was concentrated under reduced pressure to obtain a trifluoroacetate salt of **70-2.** ESI-MS calculated for C₇H₁₂N₃ [M+H]⁺ = 138.1, found 138.1.

### Step 3

The trifluoroacetate salt of **70-2** (18 mg, 0.16 mmol) and intermediate **A** (42 mg, 0.16 mmol) were dissolved in DMF (3 mL), then DIEA (41 mg, 0.32 mmol) was added thereto, and the mixture was stirred at room temperature for 30 minutes. HATU (91 mg, 0.24 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 19 to 49%, retention time: 10 minutes) to obtain compound **70.** ¹H NMR (400 MHz, DMSO-*d*₆) δ 8.10 (d, *J* = 5.70 Hz, 1H), 6.58 (d, *J =* 2.20 Hz, 1H), 6.39 (dd, *J =* 5.74, 2.20 Hz, 1H), 4.78-4.74 (m, 2H), 4.52-4.48 (m, 2H), 4.15-4.10 (m, 2H), 3.96 (s, 3H), 3.71-3.60 (m, 2H), 3.15-3.02 (m, 1H), 2.85-2.83 (m, 2H), 2.55 (s, 3H). ESI-MS calculated for C₁₈H₂₁F₃N₅O [M+H]⁺ = 380.2, found 380.1.

### Example 71

### Synthetic route:

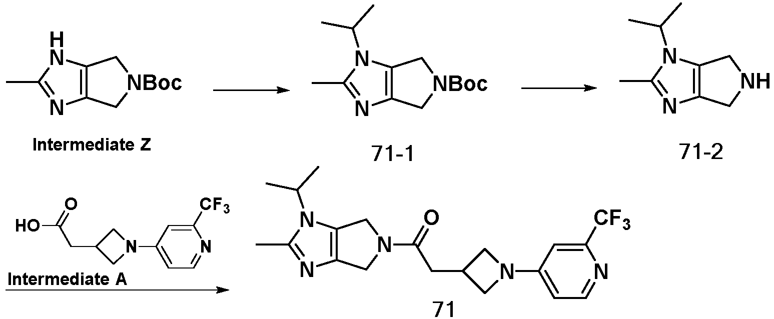

### Step 1

Intermediate **Z** (100 mg, 0.45 mmol) was dissolved in DMF (5 mL), then cesium carbonate (292 mg, 0.90 mmol) and isopropyl iodide (152 mg, 0.90 mmol) were added thereto, and the mixture was stirred at room temperature for 1 hour. The reaction mixture was diluted with water (30 mL) and extracted with dichloromethane (30 mL × 3). The organic phases were combined, dried over anhydrous sodium sulfate, filtered, concentrated under reduced pressure to obtain **71-1.** ESI-MS calculated for C₁₄H₂₄N₃O₂ [M+H]⁺ = 266.2, found 266.3.

### Step 2

**71-1** (80 mg, 0.30 mmol) was dissolved in dichloromethane (5 mL), then trifluoroacetic acid (5 mL) was added thereto, and the mixture was stirred at room temperature for 3 hours. The reaction mixture was concentrated under reduced pressure to obtain a trifluoroacetate salt of **71-2.** ESI-MS calculated for C₉H₁₆N₃ [M+H]⁺ = 166.1, found 166.1.

### Step 3

The trifluoroacetate salt of **71-2** (50.0 mg, 0.30 mmol) and intermediate **A** (123 mg, 0.33 mmol) were dissolved in DMF (3 mL), then DIEA (78 mg, 0.61 mmol) was added thereto, and the mixture was stirred at room temperature for 30 minutes. HATU (172 mg, 0.45 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 29 to 59%, retention time: 9 minutes) to obtain compound **71.** ¹H NMR (400 MHz, DMSO-*d*₆) δ 8.20 (d, *J* = 5.66 Hz, 1H), 6.70 (d, *J* = 2.31 Hz, 1H), 6.53-6.51 (m, 1H), 4.76-4.73 (m, 1H), 4.53-4.50 (m, 1H), 4.44-4.37 (m, 2H), 4.21-4.10 (m, 3H), 3.71-3.66 (m, 2H), 3.14-3.09 (m, 1H), 2.84-2.77 (m, 2H), 2.32 (s, 3H), 1.30 (d, *J =* 6.64 Hz, 6H). ESI-MS calculated for C₂₀H₂₅F₃N₅O [M+H]⁺ = 408.2, found 408.2.

### Example 72

### Synthetic route:

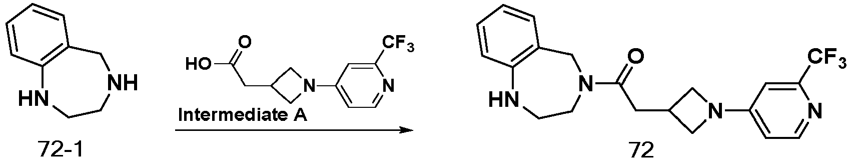

### Step 1

**72-1** (100 mg, 0.67 mmol) and intermediate **A** (176 mg, 0.67 mmol) were dissolved in DMF (5 mL), then DIEA (173 mg, 1.34 mmol) was added thereto, and the mixture was stirred at room temperature for 30 minutes. HATU (305 mg, 0.80 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 36 to 66%, retention time: 13 minutes) to obtain compound **72.** ¹H NMR (400 MHz, DMSO-*d*₆) δ 8.19 (dd, *J =* 5.78, 3.00 Hz, 1H), 7.22-7.06 (m, 1H), 7.04-6.98 (m, 1H), 6.81-6.78 (m, 1H), 6.72-6.60 (m, 2H), 6.50-6.47 (m, 1H), 5.68-5.60 (m, 1H), 4.48-4.42 (m, 2H), 4.10-4.03 (m, 2H), 3.66-3.56 (m, 4H), 3.14-3.06 (m, 2H), 3.04-2.95 (m, 1H), 2.80-2.74 (m, 2H). ESI-MS calculated for C₂₀H₂₂F₃N₄O [M+H]⁺ = 391.2, found 391.2.

### Example 73

### Synthetic route:

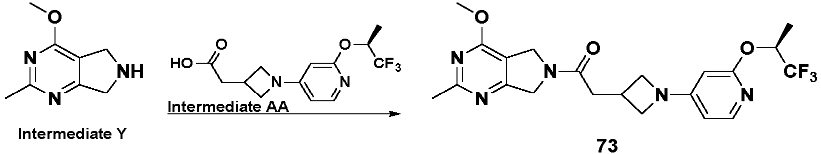

### Step 1

The trifluoroacetate salt of intermediate **Y** (109 mg, 0.66 mmol) and intermediate **AA** (100 mg, 0.33 mmol) were dissolved in DMF (5 mL), then DIEA (127 mg, 0.99 mmol) was added thereto, and the mixture was stirred at room temperature for 30 minutes. HATU (188 mg, 0.49 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 45 to 75%, retention time: 9 minutes) to obtain compound **73.** ¹H NMR (400 MHz, DMSO-*d*₆) δ 7.75 (d, *J =* 5.80 Hz, 1H), 6.12 (dd, *J =* 5.84, 1.98 Hz, 1H), 5.86-5.79 (m, 1H), 5.73 (d, *J =* 1.96 Hz, 1H), 4.78-4.72 (m, 2H), 4.53-4.48 (m, 2H), 4.05-3.96 (m, 2H), 3.96 (s, 3H), 3.62-3.56 (m, 2H), 3.09-3.02 (m, 1H), 2.84-2.78 (m, 2H), 2.54 (s, 3H), 1.38 (d, *J =* 6.52 Hz, 3H). ESI-MS calculated for C₂₁H₂₅F₃N₅O₃ [M+H]⁺ = 452.2, found 452.2.

### Example 74

### Synthetic route:

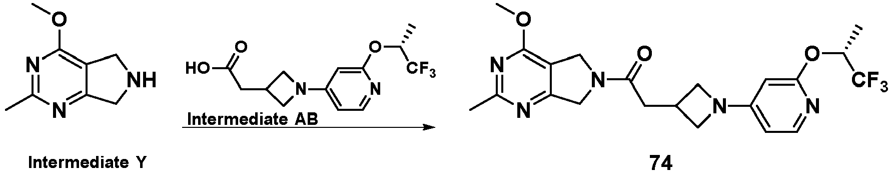

### Step 1

The trifluoroacetate salt of intermediate **Y** (87 mg, 0.53 mmol) and intermediate **AB** (80 mg, 0.26 mmol) were dissolved in DMF (5 mL), then DIEA (102 mg, 0.79 mmol) was added thereto, and the mixture was stirred at room temperature for 30 minutes. HATU (150 mg, 0.39 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 40 to 70%, retention time: 10 minutes) to obtain compound **74.** ¹H NMR (400 MHz, DMSO-*d*₆) δ 7.75 (d, *J =* 5.80 Hz, 1H), 6.12 (dd, *J =* 5.84, 1.98 Hz, 1H), 5.85-5.77 (m, 1H), 5.73 (d, *J =* 1.94 Hz, 1H), 4.79-4.70 (m, 2H), 4.54-4.46 (m, 2H), 4.08-3.97 (m, 2H), 3.96 (s, 3H), 3.62-3.55 (m, 2H), 3.10-3.02 (m, 1H), 2.83-2.78 (m, 2H), 2.54 (s, 3H), 1.38 (d, *J =* 6.52 Hz, 3H). ESI-MS calculated for C₂₁H₂₅F₃N₅O₃ [M+H]⁺ = 452.2, found 452.2.

### Example 75

### Synthetic route:

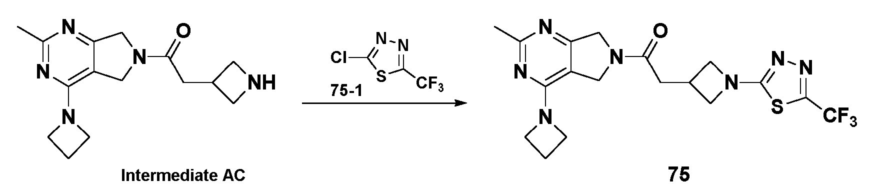

### Step 1

Intermediate **AC** (229 mg, 0.80 mmol) and **75-1** (100 mg, 0.53 mmol) were dissolved in DMSO (5 mL), then TEA (215 mg, 2.12 mmol) and cesium fluoride (81 mg, 0.53 mmol) were added thereto, and the mixture was heated to 90°C and stirred for 18 hours. After the reaction mixture was cooled to room temperature, the insoluble matter was removed by filtration, and the filtrate was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 24 to 54%, retention time: 9 minutes) to obtain compound 75. ¹H NMR (400 MHz, DMSO-*d*₆) δ 4.86-4.80 (m, 1H), 4.62-4.57 (m, 2H), 4.34-4.28 (m, 3H), 4.21-4.13 (m, 4H), 3.94-3.87 (m, 2H), 3.25-3.20 (m, 1H), 2.89-2.82 (m, 2H), 2.33 (s, 3H), 2.32-2.28 (m, 2H). ESI-MS calculated for C₁₈H₂₁F₃N₇OS [M+H]⁺ = 440.2, found 440.2.

### Example 76

### Synthetic route:

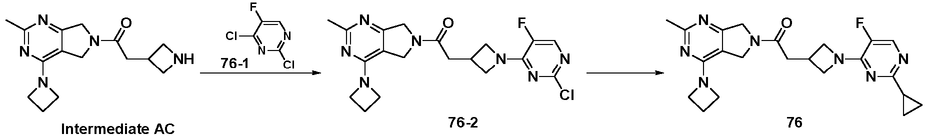

### Step 1

Intermediate **AC** (229 mg, 0.80 mmol) was dissolved in acetonitrile (2 mL), then 76-1 (244 mg, 1.46 mmol) and TEA (148 mg, 1.46 mmol) were added thereto, and the mixture was stirred at 0°C for 3 hours. The reaction mixture was concentrated under reduced pressure and then purified by silica gel column chromatography (dichloromethane/triethylamine, 9/1, v/v) to obtain 76-2. ESI-MS calculated for C₁₉H₂₂ClFN₇O [M+H]⁺ = 418.2, found 418.2.

### Step 2

**76-2** (50 mg, 0.12 mmol), cyclopropylboronic acid (21 mg, 0.24 mmol), potassium carbonate (50 mg, 0.36 mmol), and [1,1'-bis(diphenylphosphino)ferrocene]dichloropalladium(II) (9 mg, 0.01 mmol) were added to 1,4-dioxane (5 mL) and water (1 mL), and the reaction mixture was heated to 90°C under nitrogen atmosphere and stirred for 18 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 25 to 55%, retention time: 9 minutes) to obtain compound **76.** ¹H NMR (400 MHz, DMSO-*d*₆) δ 7.98-7.96 (m, 1H), 4.85-4.83 (m, 1H), 4.64-4.56 (m, 2H), 4.35-4.28 (m, 3H), 4.21-4.14 (m, 4H), 3.90-3.84 (m, 2H), 3.11-3.03 (m, 1H), 2.84-2.79 (m, 2H), 2.35 (s, 3H), 2.34-2.29 (m, 2H), 1.95-1.90 (m, 1H), 0.89-0.80 (m, 4H). ESI-MS calculated for C₂₂H₂₇FN₇O [M+H]⁺ = 424.2, found 424.2.

### Example 77

### Synthetic route:

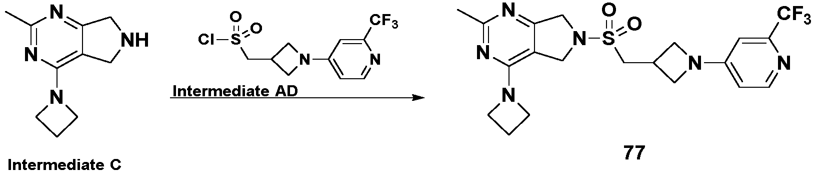

### Step 1

Intermediate **C** (50 mg, 0.26 mmol), intermediate **AD** (165 mg, 0.53 mmol), and TEA (80 mg, 0.78 mmol) were dissolved in dichloromethane (3 mL), and the mixture was stirred at room temperature for 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 30 to 60%, retention time: 9 minutes) to obtain compound 77. ¹H NMR (400 MHz, DMSO-*d*₆) δ 8.22 (d, *J =* 5.62 Hz, 1H), 6.74 (d, *J=* 2.20 Hz, 1H), 6.56 (dd, *J =* 5.66, 2.24 Hz, 1H), 4.72-4.67 (m, 2H), 4.43-4.39 (m, 2H), 4.25-4.17(m, 6H), 3.85-3.80 (m, 2H), 3.69-3.65 (m, 2H), 3.26-3.20 (m, 1H), 2.34 (s, 3H), 2.33-2.29 (m, 2H). ESI-MS calculated for C₂₀H₂₄F₃N₆O₂S [M+H]⁺ = 469.2, found 469.2.

### Example 78

### Synthetic route:

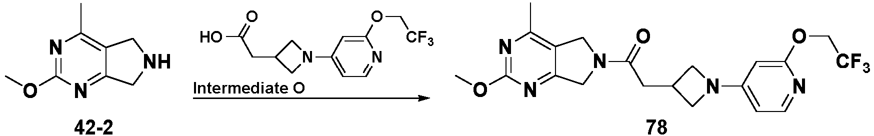

### Step 1

**42-2** (80 mg, 0.48 mmol) and intermediate **O** (100 mg, 0.34 mmol) were dissolved in DMF (5 mL), then DIEA (133 mg, 1.03 mmol) was added thereto, and the mixture was stirred at room temperature for 30 minutes. HATU (170 mg, 0.45 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 37 to 67%, retention time: 9 minutes) to obtain compound 78. ¹H NMR (400 MHz, DMSO-*d*₆) δ 7.76 (d, *J =* 5.76 Hz, 1H), 6.15 (dd, *J* = 5.82, 1.96 Hz, 1H), 5.78 (d, *J =* 1.96 Hz, 1H), 4.92-4.85 (m, 2H), 4.83-4.79 (m, 2H), 4.58-4.52 (m, 2H), 4.08-4.02 (m, 2H), 3.89 (s, 3H), 3.64-3.58 (m, 2H), 3.09-3.05 (m, 1H), 2.85-2.80 (m, 2H), 2.39-2.37 (m, 3H). ESI-MS calculated for C₂₀H₂₃F₃N₅O₃ [M+H]⁺ = 438.2, found 438.2.

### Example 79

### Synthetic route:

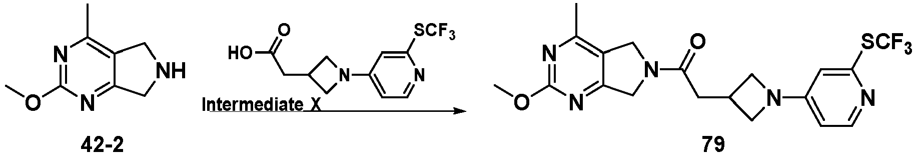

### Step 1

The **42-2** (17 mg, 0.10 mmol) and intermediate **X** (30 mg, 0.10 mmol) were dissolved in DMF (5 mL), then DIEA (40 mg, 0.31 mmol) was added thereto, and the mixture was stirred at room temperature for 30 minutes. HATU (51 mg, 0.13 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 33 to 63%, retention time: 9 minutes) to obtain compound **79.** ¹H NMR (400 MHz, DMSO-*d*₆) δ 8.10 (d, *J =* 5.70 Hz, 1H), 6.59 (d, *J* = 2.18 Hz, 1H), 6.40 (dd, *J =* 5.76, 2.20 Hz, 1H), 4.82-4.76 (m, 2H), 4.57-4.51 (m, 2H), 4.17-4.11 (m, 2H), 3.90 (s, 3H), 3.70-3.64 (m, 2H), 3.14-3.09 (m,1H), 2.87-2.82 (m, 2H), 2.37-2.35 (m, 3H). ESI-MS calculated for C₁₉H₂₁F₃N₅O₂S [M+H]⁺ = 440.1, found 440.1.

### Example 80

### Synthetic route:

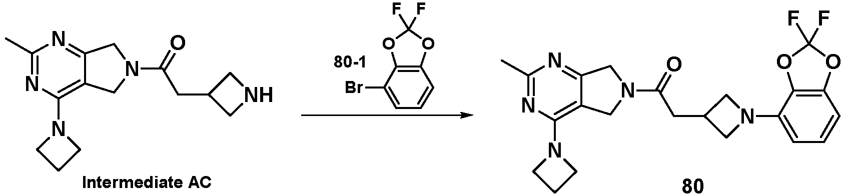

### Step 1

Intermediate **AC** (242 mg, 0.42 mmol), **80-1** (100 mg, 0.42 mmol), and cesium carbonate (412 mg, 1.27 mmol) were dissolved in 1,4-dioxane (5 mL), then methanesulfonato(2-dicyclohexylphosphino-2',4',6'-tri-i-propyl-1,1'-biphenyl)(2'-amino-1,1'-biphenyl-2-yl)palladium(II) (36 mg, 0.04 mmol) was added thereto, and the mixture was heated to 100°C under nitrogen atmosphere and stirred for 18 hours. After the reaction mixture was cooled to room temperature, the insoluble matter was removed by filtration, and the filtrate was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 20 to 37%, retention time: 9 minutes) to obtain compound 80. ¹H NMR (400 MHz, DMSO-*d*₆) δ 7.03-6.97 (m, 1H), 6.68 (d, *J =* 7.98 Hz, 1H), 6.32 (d, *J =* 8.20 Hz, 1H), 4.83-4.81(m, 1H), 4.62-4.57 (m, 2H), 4.35-4.32 (m, 1H), 4.22-4.08 (m, 6H), 3.69-3.65 (m, 2H), 3.10-3.01 (m, 1H), 2.83-2.79 (m, 2H), 2.35 (s, 3H), 2.34-2.30 (m, 2H). ESI-MS calculated for C₂₂H₂₄F₂N₅O₃ [M+H]⁺ = 444.2, found 444.2.

### Example 81

### Synthetic route:

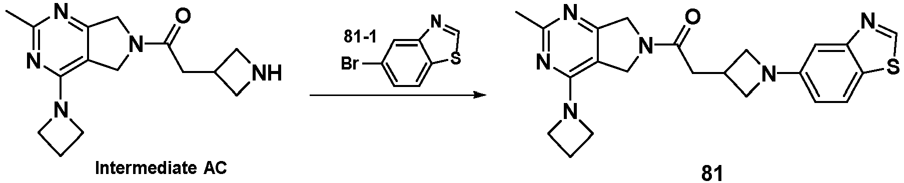

### Step 1

Intermediate **AC** (80 mg, 0.28 mmol), **81-1** (60 mg, 0.28 mmol), and cesium carbonate (181 mg, 0.56 mmol) were dissolved in 1,4-dioxane (5 mL), then methanesulfonato(2-dicyclohexylphosphino-2',4',6'-tri-i-propyl-1,1'-biphenyl)(2'-amino-1,1'-biphenyl-2-yl)palladium(II) (24 mg, 0.03 mmol) was added thereto, and the mixture was heated to 100°C under nitrogen atmosphere and stirred for 18 hours. After the reaction mixture was cooled to room temperature, the insoluble matter was removed by filtration, and the filtrate was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 41 to 71%, retention time: 9 minutes) to obtain compound **81.** ¹H NMR (400 MHz, DMSO-*d*₆) δ 9.25 (s, 1H), 7.89 (d, *J* = 8.60 Hz, 1H), 7.01 (d, *J* = 2.11 Hz, 1H), 6.69-6.55 (m, 1H), 4.86-4.84 (m, 1H), 4.63-4.58 (m, 2H), 4.36-4.34 (m, 1H), 4.20-4.14 (m, 4H), 4.08-4.03 (m, 2H), 3.58-3.55 (m, 2H), 3.08-3.03 (m, 1H), 2.84-2.79 (m, 2H), 2.37-2.30 (m, 5H). ESI-MS calculated for C₂₂H₂₅N₆O₃ [M+H]⁺ = 421.2, found 421.2.

### Example 82

### Synthetic route:

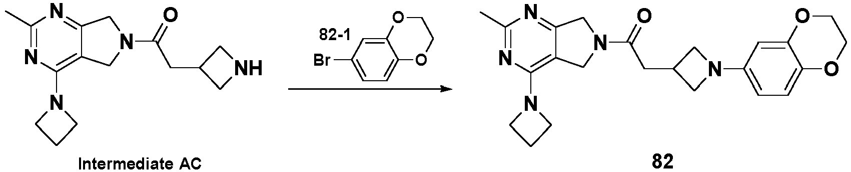

### Step 1

Intermediate **AC** (100 mg, 0.35 mmol), **82-1** (75 mg, 0.35 mmol), and cesium carbonate (342 mg, 1.05 mmol) were dissolved in 1,4-dioxane (5 mL), then methanesulfonato(2-dicyclohexylphosphino-2',4',6'-tri-i-propyl-1,1'-biphenyl)(2'-amino-1,1'-biphenyl-2-yl)palladium(II) (30 mg, 0.04 mmol) was added thereto, and the mixture was heated to 100°C under nitrogen atmosphere and stirred for 18 hours. After the reaction mixture was cooled to room temperature, the insoluble matter was removed by filtration, and the filtrate was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 25 to 55%, retention time: 9 minutes) to obtain compound **82.** ¹H NMR (400 MHz, DMSO-*d*₆) δ 6.70-6.60 (m, 1H), 5.93-5.89 (m, 2H), 4.84-4.82 (m, 1H), 4.61-4.56 (m, 2H), 4.34-4.32 (m, 1H), 4.21-4.07 (m, 8H), 3.88-3.84 (m, 2H), 3.39-3.34 (m, 2H), 2.99-2.89 (m, 1H), 2.77-2.72 (m, 2H), 2.34-2.28 (m, 5H). ESI-MS calculated for C₂₃H₂₈N₅O₃ [M+H]⁺ = 422.2, found 422.2.

### Example 83

### Synthetic route:

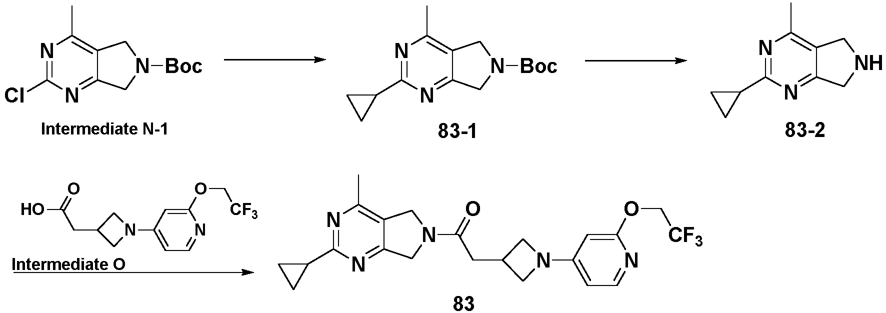

### Step 1

Intermediate **N-1** (300 mg, 1.11 mmol), cyclopropylboronic acid (95 mg, 1.11 mmol), potassium carbonate (461 mg, 3.34 mmol), and [1,1'-bis(diphenylphosphino)ferrocene]dichloropalladium(II) (82 mg, 0.11 mmol) were added to 1,4-dioxane (5 mL) and water (1 mL), and the reaction mixture was heated to 100°C under nitrogen atmosphere and stirred for 18 hours. The reaction mixture was cooled to room temperature, diluted with water (10 mL), and extracted with ethyl acetate (20 mL × 3). The organic phases were combined, washed with saturated brine (40 mL), dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **83-1.** ESI-MS calculated for C₁₅H₂₂N₃O₂ [M+H]⁺ = 276.2, found 276.2.

### Step 2

**83-1** (100 mg, 0.36 mmol) was dissolved in dichloromethane (5 mL), then trifluoroacetic acid (2 mL) was added thereto, and the mixture was stirred at room temperature for 2 hours. The reaction mixture was concentrated under reduced pressure to obtain a crude product containing **83-2,** which was directly used in the next reaction step. ESI-MS calculated for C₁₀H₁₄N₃ [M+H]⁺ = 176.1, found 176.2.

### Step 3

The **83-2** (60 mg, 0.34 mmol) and intermediate **O** (98 mg, 0.34 mmol) were dissolved in DMF (5 mL), then DIEA (132 mg, 1.03 mmol) was added thereto, and the mixture was stirred at room temperature for 30 minutes. HATU (156 mg, 0.41 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 52%, retention time: 9 minutes) to obtain compound **83.** ¹H NMR (400 MHz, DMSO-*d*₆) δ 7.76 (d, *J =* 5.80 Hz, 1H), 6.15 (dd, *J =* 5.76, 1.92 Hz, 1H), 5.78 (d, *J =* 1.90 Hz, 1H), 4.93-4.82 (m, 2H), 4.81-4.76 (m, 2H), 4.59-4.51 (m, 2H), 4.09-4.03 (m, 2H), 3.62-3.55 (m, 2H), 3.09-3.03 (m, 1H), 2.84-2.80 (m, 2H), 2.36-2.34 (m, 3H), 2.20-2.11 (m, 1H), 1.04-0.95 (m, 4H). ESI-MS calculated for C₂₂H₂₅F₃N₅O₂ [M+H]⁺ = 448.2, found 448.2.

### Example 84

### Synthetic route:

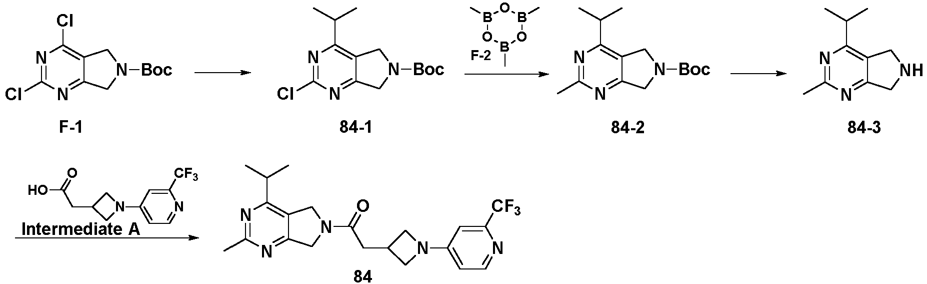

### Step 1

**F-1** (800 mg, 2.76 mmol) was dissolved in tetrahydrofuran (20 mL), followed by the addition of iron(III) acetylacetonate (292 mg, 0.83 mmol). The mixture was cooled to 0°C, and then a solution of isopropylmagnesium bromide in tetrahydrofuran (1.0 mol/L, 5.51 mL, 5.51 mmol) was added. The reaction mixture was stirred at room temperature for 18 hours. The reaction mixture was quenched with saturated ammonium chloride solution (20 mL) and extracted with ethyl acetate (20 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **84-1.** ESI-MS calculated for C₁₄H₂₁ClN₃O₂ [M+H]⁺ = 298.1, found 298.2.

### Step 2

**84-1** (1.0 g, 3.36 mmol), [1,1'-bis(diphenylphosphino)ferrocene]dichloropalladium(II) (270 mg, 0.34 mmol), potassium carbonate (1.39 g, 10.1 mmol), **F-2** (840 mg, 6.72 mmol), and 1,4-dioxane (10 mL) and water (1 mL) were heated to 90°C under nitrogen atmosphere and stirred for 18 hours. The reaction mixture was cooled to room temperature, filtered, and the filtrate was diluted with water (20 mL) and extracted with ethyl acetate (20 mL × 3). The organic phases were combined, washed with saturated brine (30 mL), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **84-2.** ESI-MS calculated for C₁₅H₂₄N₃O₂ [M+H]⁺ = 278.2, found 278.2.

### Step 3

**84-2** (100 mg, 0.36 mmol) was dissolved in dichloromethane (5 mL), then trifluoroacetic acid (1 mL) was added thereto, and the mixture was stirred at room temperature for 1 hour. The reaction mixture was concentrated under reduced pressure to obtain a crude product containing **84-3,** which was directly used in the next reaction step. ESI-MS calculated for C₁₀H₁₆N₃ [M+H]⁺ = 178.1, found 178.1.

### Step 4

**84-3** (50 mg, 0.28 mmol) and intermediate **A** (73 mg, 0.28 mmol) were dissolved in DMF (5 mL), then DIEA(219 mg, 1.69 mmol) was added thereto, and the mixture was stirred at room temperature for 30 minutes. HATU (129 mg, 0.34 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 32 to 62%, retention time: 9 minutes) to obtain compound **84.** ¹H NMR (400 MHz, DMSO-*d*₆) δ 8.21 (d, *J =* 5.68 Hz, 1H), 6.71 (t, *J* = 1.56 Hz, 1H), 6.55-6.51 (m, 1H), 4.94-4.79 (m, 2H), 4.69-4.54 (m, 2H), 4.18 (t, *J =* 8.24 Hz, 2H), 3.72-3.68 (m, 2H), 3.14-3.08 (m, 2H), 2.89-2.84 (m, 2H), 2.42-2.38 (m, 3H), 1.28-1.17 (m, 6H). ESI-MS calculated for C₂₁H₂₅F₃N₅O [M+H]⁺ = 420.2, found 420.2.

### Example 85

### Synthetic route:

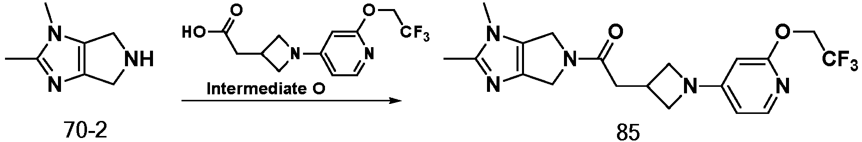

### Step 1

The **70-2** (50 mg, 0.36 mmol) and intermediate **O** (106 mg, 0.36 mmol) were dissolved in DMF (5 mL), then DIEA (141 mg, 1.09 mmol) was added thereto, and the mixture was stirred at room temperature for 30 minutes. HATU (180 mg, 0.47 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 31 to 61%, retention time: 9 minutes) to obtain compound **85.** ¹H NMR (400 MHz, DMSO-*d*₆) δ 7.75 (d, *J* = 5.76 Hz, 1H), 6.14 (m, *J =* 5.76 Hz, 1H), 5.77 (s, 1H), 4.92-4.84 (m, 2H), 4.63-4.61 (m, 1H), 4.44-4.39 (m, 2H), 4.23-4.21 (m, 1H), 4.10-4.02 (m, 2H), 3.63-3.57 (m, 2H), 3.50-3.48 (m, 3H), 3.08-3.02 (m, 1H), 2.78-2.74 (m, 2H), 2.28 (s, 3H). ESI-MS calculated for C₁₉H₂₃F₃N₅O₂ [M+H]⁺ = 410.2, found 410.2.

### Example 86

### Synthetic route:

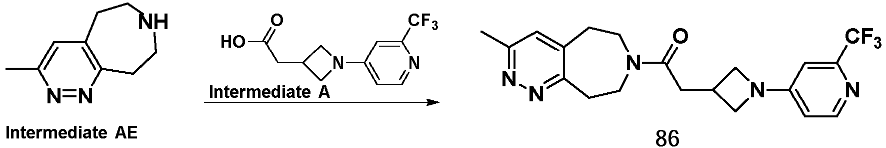

### Step 1

Intermediate **AE** (90 mg, 0.55 mmol) and intermediate **A** (143 mg, 0.55 mmol) were dissolved in DMF (5 mL), then DIEA (356 mg, 2.76 mmol) was added thereto, and the mixture was stirred at room temperature for 30 minutes. HATU (210 mg, 0.55 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 52%, retention time: 9 minutes) to obtain compound **86.** ¹H NMR (400 MHz, DMSO-*d*₆) δ 8.20 (d, *J= 5.62* Hz, 1H), 7.37-7.34 (m, 1H), 6.69 (s, 1H), 6.55-6.49 (m, 1H), 4.17-4.12 (m, 2H), 3.69-3.54 (m, 6H), 3.30-3.26 (m, 1H), 3.18-3.15 (m, 1H), 3.07-3.04 (m, 1H), 2.96-2.79 (m, 4H), 2.53 (s, 3H). ESI-MS calculated for C₂₀H₂₃F₃N₅O [M+H]⁺ = 406.2, found 406.2.

### Example 87

### Synthetic route:

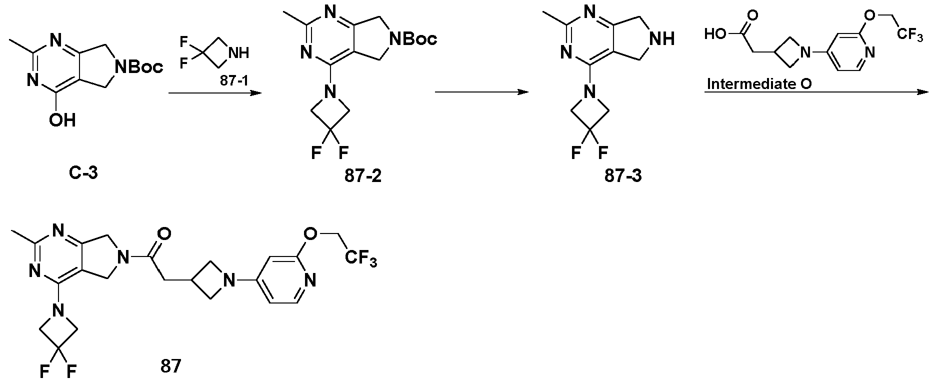

### Step 1

**C-3** (2.0 g, 7.96 mmol) and **87-1** (1.0 g, 7.96 mmol) were dissolved in DMF (10 mL), then DBU (2.6 g, 17.5 mmol) and PyBOP (6.2 g, 11.9 mmol) were sequentially added thereto, and the mixture was heated to 80°C and stirred for 18 hours. The reaction mixture was cooled to room temperature and concentrated under reduced pressure to obtain a crude product containing the target compound, which was purified by silica gel column chromatography (petroleum ether/ethyl acetate, 1/1, v/v) to obtain **87-2.** ¹H NMR (400 MHz, DMSO-*d*₆) δ 4.72-4.48 (m, 6H), 4.38-4.31 (m, 2H), 2.40 (s, 3H), 1.46 (s, 9H). ESI-MS calculated for C₁₅H₂₁F₂N₄O₂ [M+H]⁺ = 327.2, found 327.2.

### Step 2

**87-2** (600 mg, 1.84 mmol) was dissolved in dichloromethane (10 mL), then trifluoroacetic acid (10 mL) was added thereto, and the mixture was stirred at room temperature for 2 hours. The reaction mixture was concentrated under reduced pressure to obtain a crude product containing **87-3,** which was directly used in the next reaction step. ESI-MS calculated for C₁₀H₁₃F₂N₄ [M+H]⁺ = 227.1, found 227.2.

### Step 3

The **87-3** (200 mg, 0.88 mmol) and intermediate **O** (366 mg, 0.88 mmol) were dissolved in DMF (5 mL), then DIEA (340 mg, 2.65 mmol) was added thereto, and the mixture was stirred at room temperature for 30 minutes. HATU (500 mg, 1.33 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Bonnasil-BR-C18-10 µm-20 * 250 mm, mobile phase: acetonitrile-10 mmol/L ammonium bicarbonate aqueous solution, gradient: 22 to 52%, retention time: 9 minutes) to obtain compound **87.** ¹H NMR (400 MHz, DMSO-*d*₆) δ 7.76 (dd, *J =* 5.84, 1.80 Hz, 1H), 6.17-6.14 (m, 1H), 5.79 (dd, *J =* 6.02, 1.86 Hz, 1H), 4.95-4.83 (m, 3H), 4.71-4.59 (m, 6H), 4.43-4.40 (m, 1H), 4.10-4.05 (m, 2H), 3.62-3.58 (m, 2H), 3.10-3.05 (m, 1H), 2.85-2.80 (m, 2H), 2.42 (s, 3H). ESI-MS calculated for C₂₂H₂₄F₅N₆O₂ [M+H]⁺ = 499.2, found 499.2.

### Example 88

### Synthetic route:

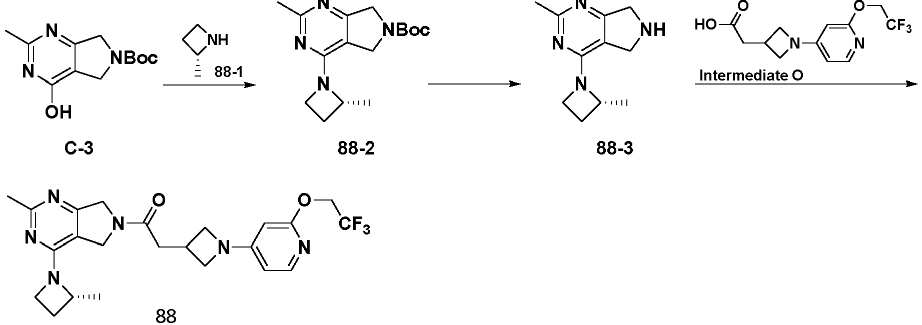

### Step 1

**C-3** (934 mg, 3.72 mmol) and **88-1** (400 mg, 3.72 mmol) were dissolved in DMF (10 mL), then DBU (1.42 g, 9.30 mmol) and PyBOP (2.32 g, 4.46 mmol) were sequentially added thereto, and the mixture was heated to 80°C and stirred for 4 hours. The reaction mixture was cooled to room temperature and concentrated under reduced pressure to obtain a crude product containing the target compound, which was purified by silica gel column chromatography (petroleum ether/ethyl acetate, 9/1, v/v) to obtain **88-2.** ESI-MS calculated for C₁₆H₂₅N₄O₂ [M+H]⁺ = 305.2, found 305.2.

### Step 2

**88-2** (200 mg, 0.66 mmol) was dissolved in dichloromethane (5 mL), then trifluoroacetic acid (2 mL) was added thereto, and the mixture was stirred at room temperature for 2 hours. The reaction mixture was concentrated under reduced pressure to obtain a crude product containing **88-3,** which was directly used in the next reaction step. ESI-MS calculated for C₁₁H₁₇N₄ [M+H]⁺ = 205.1, found 205.2.

### Step 3

**88-3** (74 mg, 0.36 mmol) and intermediate **O** (70 mg, 0.24 mmol) were dissolved in DMF (5 mL), then DIEA (93 mg, 0.72 mmol) was added thereto, and the mixture was stirred at room temperature for 30 minutes. HATU (138 mg, 0.36 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 39 to 69%, retention time: 9 minutes) to obtain compound **88.** ¹H NMR (400 MHz, DMSO-*d*₆) δ 7.75 (d, *J* = 5.80 Hz, 1H), 6.16-6.13 (m, 1H), 5.79-5.75 (m, 1H), 4.94-4.74 (m, 3H), 4.65-4.50 (m, 3H), 4.35-4.32 (m, 1H), 4.18-4.01 (m, 4H), 3.62-3.56 (m, 2H), 3.12-2.99 (m, 1H), 2.83-2.78 (m, 2H), 2.48-2.40 (m, 1H), 2.34 (s, 3H), 1.98-1.88 (m, 1H), 1.45 (d, *J* = 6.20 Hz, 3H). ESI-MS calculated for C₂₃H₂₈F₃N₆O₂ [M+H]⁺ = 477.2, found 477.2.

### Example 89

### Synthetic route:

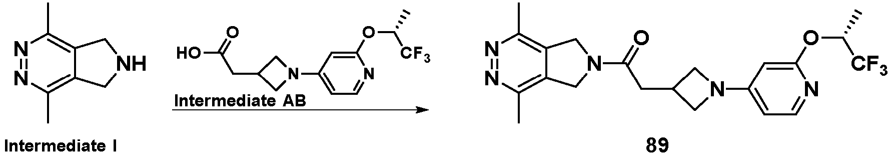

### Step 1

The trifluoroacetate salt of intermediate **I** (49 mg, 0.33 mmol) and intermediate **AB** (100 mg, 0.33 mmol) were dissolved in DMF (5 mL), then DIEA (127 mg, 0.99 mmol) was added thereto, and the mixture was stirred at room temperature for 30 minutes. HATU (175 mg, 0.46 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 35 to 65%, retention time: 11 minutes) to obtain compound **89.** ¹H NMR (400 MHz, DMSO-*d*₆) δ 7.75 (d, *J =* 5.80 Hz, 1H), 6.15-6.12 (m, 1H), 5.87-5.78 (m, 1H), 5.75-5.73 (m, 1H), 4.93-4.90 (m, 2H), 4.71-4.68 (m, 2H), 4.10-4.06 (m, 2H), 3.64-3.58 (m, 2H), 3.12-3.05 (m, 1H), 2.85-2.82 (m, 2H), 2.55-2.53 (m, 6H), 1.38 (d, *J* = 6.50 Hz, 3H). ESI-MS calculated for C₂₁H₂₅F₃N₅O₂ [M+H]⁺ = 436.2, found 436.2.

### Example 90

### Synthetic route:

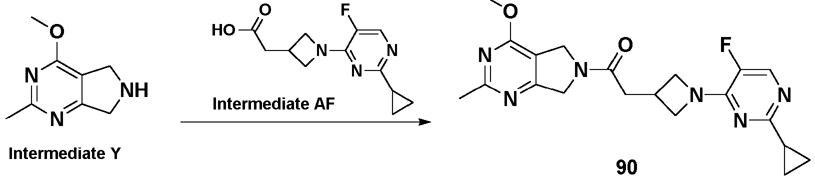

### Step 1

The trifluoroacetate salt of intermediate **Y** (40 mg, 0.24 mmol) and intermediate **AF** (60 mg, 0.24 mmol) were dissolved in DMF (5 mL), then DIEA (185 mg, 1.43 mmol) was added thereto, and the mixture was stirred at room temperature for 30 minutes. HATU (109 mg, 0.29 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 25 to 55%, retention time: 10 minutes) to obtain compound 90. ¹H NMR (400 MHz, DMSO-*d*₆) δ 7.99-7.97 (m, 1H), 4.79-4.71 (m, 2H), 4.56-4.47 (m, 2H), 4.34-4.27 (m, 2H), 3.97 (s, 3H), 3.93-3.87 (m, 2H), 3.11-3.01 (m, 1H), 2.86-2.83 (m, 2H), 2.55 (s, 3H), 1.97-1.90 (m, 1H), 0.88-0.84 (m, 4H). ESI-MS calculated for C₂₀H₂₄FN₆O₂ [M+H]⁺ = 399.2, found 399.2.

### Example 91

### Synthetic route:

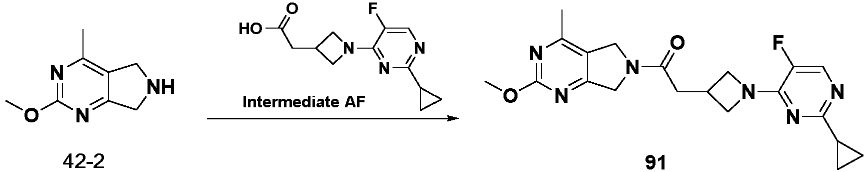

### Step 1

The trifluoroacetate salt of **42-2** (40 mg, 0.24 mmol) and intermediate **AF** (60 mg, 0.24 mmol) were dissolved in DMF (5 mL), then DIEA (185 mg, 1.43 mmol) was added thereto, and the mixture was stirred at room temperature for 30 minutes. HATU (109 mg, 0.29 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 25 to 55%, retention time: 10 minutes) to obtain compound 91. ¹H NMR (400 MHz, DMSO-*d₆*) δ 7.99-7.97 (m, 1H), 4.81-4.75 (m, 2H), 4.60-4.55 (m, 2H), 4.35-4.27 (m, 2H), 3.92-3.89 (m, 5H), 3.11-3.04 (m, 1H), 2.87-2.83 (m, 2H), 2.39-2.37 (m, 3H), 1.99-1.93 (m, 1H), 0.92-0.84 (m, 4H). ESI-MS calculated for C₂₀H₂₄FN₆O₂ [M+H]⁺ = 399.2, found 399.2.

### Example 92

### Synthetic route:

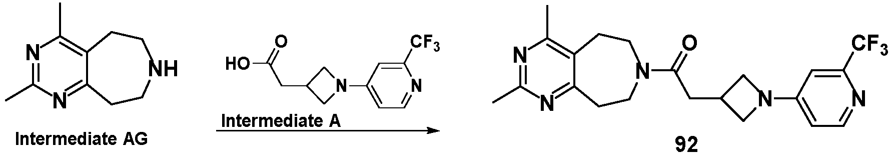

### Step 1

Intermediate **AG** (140 mg, 0.79 mmol) and intermediate **A** (205 mg, 0.79 mmol) were dissolved in DMF (5 mL), then DIEA (204 mg, 1.58 mmol) was added thereto, and the mixture was stirred at room temperature for 30 minutes. HATU (360 mg, 0.95 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 52%, retention time: 9 minutes) to obtain compound **92.** ¹H NMR (400 MHz, DMSO-*d₆*) δ 8.21-8.18 (m, 1H), 6.68 (d, *J =* 2.42 Hz, 1H), 6.52-6.48 (m, 1H), 4.16-4.09 (m, 2H), 3.66-3.57 (m, 6H), 3.10-2.93 (m, 4H), 2.86-2.80 (m, 3H), 2.47-2.45 (m, 3H), 2.42-2.40 (m, 3H). ESI-MS calculated for C₂₁H₂₅F₃N₅O [M+H]⁺ = 420.2, found 420.2.

### Example 93

### Synthetic route:

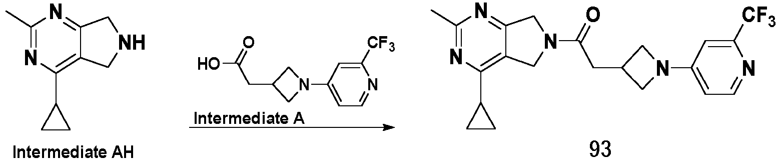

### Step 1

Intermediate **AH** (400 mg, 0.46 mmol) and intermediate **A** (119 mg, 0.46 mmol) were dissolved in DMF (5 mL), then DIEA (354 mg, 2.74 mmol) was added thereto, and the mixture was stirred at room temperature for 30 minutes. HATU (208 mg, 0.55 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 36 to 46%, retention time: 9 minutes) to obtain compound **93.** ¹H NMR (400 MHz, DMSO-*d*₆) δ 8.21 (d, *J =* 5.66 Hz, 1H), 6.71 (d, *J =* 2.24 Hz, 1H), 6.53 (dd, *J =* 5.70, 2.24 Hz, 1H), 4.98-4.76 (m, 2H), 4.72-4.54 (m, 2H), 4.22-4.15 (m, 2H), 3.74-3.68 (m, 2H), 3.14-3.04 (m, 1H), 2.89-2.84 (m, 2H), 2.50 (s, 3H), 2.11-1.80 (m, 1H), 1.12-1.05 (m, 4H). ESI-MS calculated for C₂₁H₂₃F₃N₅O [M+H]⁺ = 418.2, found 418.2.

### Example 94

### Synthetic route:

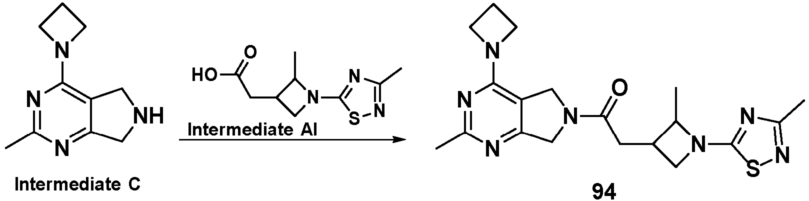

### Step 1

Intermediate **C** (80 mg, 0.42 mmol) and intermediate **AI** (80 mg, 0.35 mmol) were dissolved in DMF (5 mL), then DIEA (136 mg, 1.06 mmol) was added thereto, and the mixture was stirred at room temperature for 30 minutes. HATU (200 mg, 0.53 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 15 to 45%, retention time: 8 minutes) to obtain compound **94.** ¹H NMR (400 MHz, DMSO-*d₆*) δ 4.90-4.80 (m, 1H), 4.69-4.58 (m, 2H), 4.58-4.48 (m, 1H), 4.39-4.27 (m, 1H), 4.26-4.12 (m, 4H), 4.13-4.01 (m, 1H), 3.78-3.59 (m, 1H), 3.28-3.16 (m, 1H), 2.91-2.69 (m, 2H), 2.41-2.19 (m, 8H), 1.51-1.24 (m, 3H). ESI-MS calculated for C₁₉H₂₆N₇OS [M+H]⁺ = 400.2, found 400.2.

### Example 95

### Synthetic route:

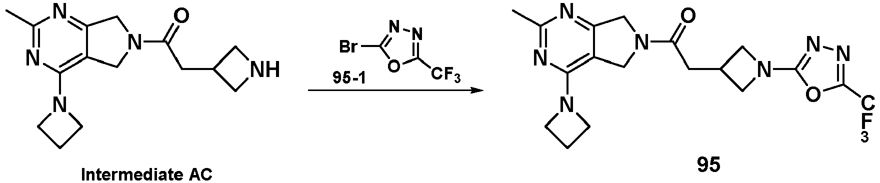

### Step 1

Intermediate **AC** (40 mg, 0.14 mmol), **95-1** (20 mg, 0.09 mmol) and cesium carbonate (90 mg, 0.28 mmol) were dissolved in DMF (5 mL), and the reaction mixture was heated to 100°C and stirred for 18 hours. After the reaction mixture was cooled to room temperature, the insoluble matter was removed by filtration, and the filtrate was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 24 to 64%, retention time: 9 minutes) to obtain compound 95. ¹H NMR (400 MHz, DMSO-*d*₆) δ 4.84-4.81 (m, 1H), 4.59-4.55 (m, 2H), 4.36-4.31 (m, 3H), 4.18-4.14 (m, 4H), 3.95-3.90 (m, 2H), 3.18-3.13 (m, 1H), 2.86-2.82 (m, 2H), 2.36-2.31 (m, 5H). ESI-MS calculated for C₁₈H₂₁F₃N₇O₂ [M+H]⁺ = 424.2, found 424.1.

### Example 96

### Synthetic route:

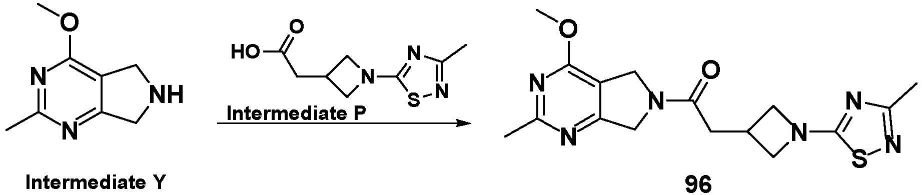

### Step 1

Intermediate **Y** (258 mg, 0.47 mmol) and intermediate **P** (100 mg, 0.47 mmol) were dissolved in DMF (5 mL), then DIEA (364 mg, 2.81 mmol) was added thereto, and the mixture was stirred at room temperature for 30 minutes. HATU (214 mg, 0.56 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 17 to 47%, retention time: 9 minutes) to obtain compound 96. ¹H NMR (400 MHz, DMSO-*d*₆) δ 4.78-4.74 (m, 2H), 4.53-4.48 (m, 2H), 4.23 (t, *J =* 8.30 Hz, 2H), 3.97 (s, 3H), 3.82-3.78 (m, 2H), 3.22-3.17 (m, 1H), 2.89-2.85 (m, 2H), 2.55 (s, 3H), 2.28 (s, 3H). ESI-MS calculated for C₁₆H₂₁N₆O₂S [M+H]⁺ = 361.1, found 361.1.

### Example 97

### Synthetic route:

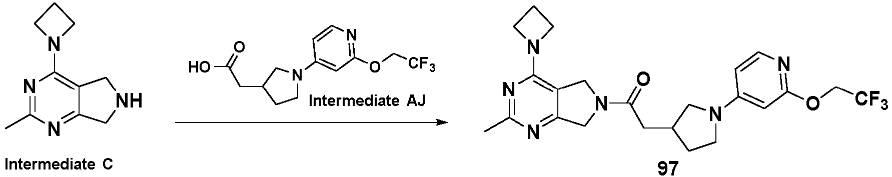

### Step 1

Intermediate **C** (125 mg, 0.33 mmol) and intermediate **AJ** (100 mg, 0.33 mmol) were dissolved in DMF (5 mL), then DIEA (255 mg, 1.97 mmol) was added thereto, and the mixture was stirred at room temperature for 30 minutes. HATU (150 mg, 0.39 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 35 to 60%, retention time: 9 minutes) to obtain compound **97.** ¹H NMR (400 MHz, DMSO-*d*₆) δ 7.74 (d, *J =* 5.92 Hz, 1H), 6.28-6.25 (m, 1H), 5.85 (t, *J =* 2.80 Hz, 1H), 4.94-4.86 (m, 2H), 4.83-4.80 (m, 1H), 4.65-4.54 (m, 2H), 4.39-4.35 (m, 1H), 4.22-4.13 (m, 4H), 3.55-3.51 (m, 1H), 3.41-3.35 (m, 1H), 3.29-3.21 (m, 1H), 2.99-2.94 (m, 1H), 2.68-2.60 (m, 1H), 2.58-2.51 (m, 2H), 2.38-2.26 (m, 5H), 2.21-2.16 (m, 1H), 1.73-1.67 (m, 1H). ESI-MS calculated for C₂₈H₂₃F₃N₆O₂ [M+H]⁺ = 477.2, found 477.2.

### Example 98

### Synthetic route:

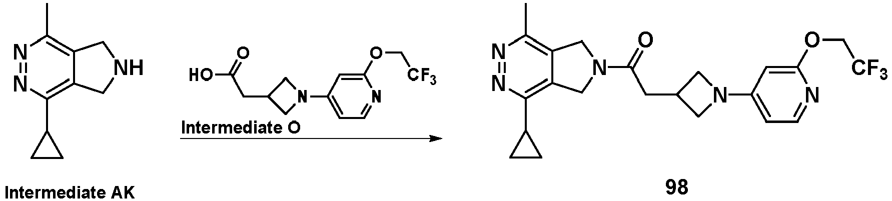

### Step 1

Intermediate **AK** (73 mg, 0.41 mmol) and intermediate **O** (80 mg, 0.28 mmol) were dissolved in DMF (5 mL), then DIEA (142 mg, 1.10 mmol) was added thereto, and the mixture was stirred at room temperature for 30 minutes. HATU (136 mg, 0.36 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 35 to 65%, retention time: 9 minutes) to obtain compound **98.** ¹H NMR (400 MHz, DMSO-*d*₆) δ 7.77 (d, *J =* 5.76 Hz, 1H), 6.16 (dd, *J =* 5.80, 1.96 Hz, 1H), 5.79 (d, *J =* 1.94 Hz, 1H), 5.06-5.03 (m, 1H), 4.95-4.86 (m, 3H), 4.79-4.69 (m, 2H), 4.09 (t, *J =* 8.04 Hz, 2H), 3.65-3.58 (m, 2H), 3.14-3.05 (m, 1H), 2.88-2.83 (m, 2H), 2.53 (s, 3H), 2.11-2.01 (m, 1H), 1.18-1.13 (m,2H), 1.09-1.04 (m, 2H). ESI-MS calculated for C₂₂H₂₅F₃N₅O₂ [M+H]⁺ = 448.2, found 448.2.

### Example 99

### Synthetic route:

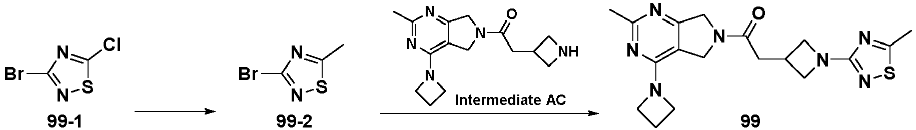

### Step 1

Zinc bromide (565 mg, 2.51 mmol) was added to tetrahydrofuran (5 mL) and cooled to -78°C under nitrogen atmosphere. Methylmagnesium bromide (3 mol/L 2-methyltetrahydrofuran solution, 0.84 mL, 2.51 mmol) was added dropwise thereto, stirred at - 78°C for 1 hour, and then warmed to 25°C and stirred for 1 hour. 99-1 (500 mg, 2.51 mmol) and tetrakis(triphenylphosphine)palladium (290 mg, 0.25 mmol) were added to the reaction system, heated to 50°C and stirred for 8 hours. After the reaction was completed, the reaction mixture was diluted with saturated ammonium chloride solution (30 mL) and extracted with ethyl acetate (50 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **99-2.** ESI-MS calculated for C₃H₄⁷⁹BrN₂S [M+H]⁺ = 178.9, found 178.8.

### Step 2

**99-2** (60 mg, 0.34 mmol), intermediate **AC** (140 mg, 0.34 mmol), cesium carbonate (546 mg, 1.68 mmol), palladium acetate (8 mg, 0.03 mmol), and 2,2'-bis(diphenylphosphino)-1,1'-binaphthyl (42 mg, 0.07 mmol) were added to 1,4-dioxane (5 mL), and the mixture was heated to 100°C under nitrogen atmosphere and stirred for 8 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 30 to 55%, retention time: 8 minutes) to obtain compound **99.** ¹H NMR (400 MHz, DMSO-*d*₆) δ 4.85-4.59 (m, 2H), 4.58-4.31 (m, 2H), 4.21-4.13 (m, 6H), 3.77-3.71 (m, 2H), 3.07-3.01 (m, 1H), 2.82-2.77 (m, 2H), 2.65 (s, 3H), 2.34 (s, 3H), 2.32-2.27 (m, 2H). ESI-MS calculated for C₁₈H₂₄N₇OS [M+H]⁺ = 386.2, found 386.2.

### Example 100

### Synthetic route:

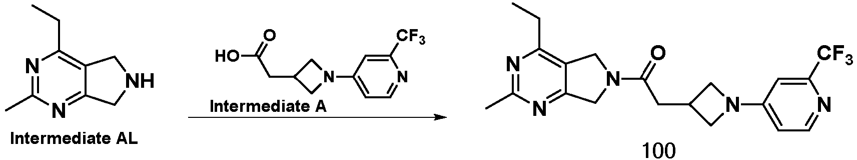

### Step 1

Intermediate **AL** (300 mg, 0.92 mmol) and intermediate A (341 mg, 0.92 mmol) were dissolved in DMF (5 mL), then DIEA (590 mg, 4.60 mmol) was added thereto, and the mixture was stirred at room temperature for 30 minutes. HATU (419 mg, 1.10 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 30 to 60%, retention time: 9 minutes) to obtain compound **100.** ¹H NMR (400 MHz, DMSO-*d*₆) δ 8.21 (d, *J* = 5.70 Hz, 1H), 6.71 (d, *J =* 2.24 Hz, 1H), 6.53 (dd, *J =* 5.74, 2.24 Hz, 1H), 4.94-4.75 (m, 2H), 4.65-4.57 (m, 2H), 4.18 (t, *J =* 8.22 Hz, 2H), 3.73-3.65 (m, 2H), 3.16-3.08 (m, 1H), 2.90-2.83 (m, 2H), 2.74-2.63 (m, 2H), 2.59 (s, 3H), 1.20 (t, *J =* 7.58 Hz, 3H). ESI-MS calculated for C₂₀H₂₃F₃N₅O [M+H]⁺ = 406.2, found 406.2.

### Example 101

### Synthetic route:

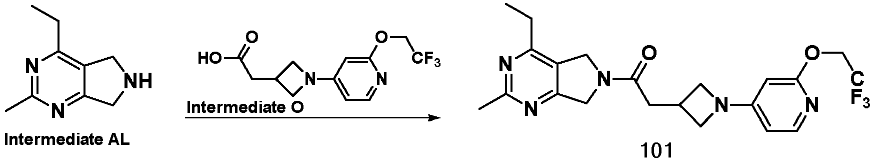

### Step 1

Intermediate **AL** (57 mg, 0.31 mmol) and intermediate **O** (100 mg, 0.31 mmol) were dissolved in DMF (5 mL), then DIEA (240 mg, 1.86 mmol) was added thereto, and the mixture was stirred at room temperature for 30 minutes. HATU (142 mg, 0.37 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 35 to 65%, retention time: 9 minutes) to obtain compound **101.** ¹H NMR (400 MHz, DMSO-*d*₆) δ 7.76 (d, *J* = 5.80 Hz, 1H), 6.15 (dd, *J =* 5.80, 1.94 Hz, 1H), 5.78 (d, *J =* 1.92 Hz, 1H), 4.95-4.79 (m, 4H), 4.65-4.55 (m, 2H), 4.08 (t, *J =* 8.02 Hz, 2H), 3.64-3.58 (m, 2H), 3.13-3.02 (m, 1H), 2.84 (t, *J =* 7.22 Hz, 2H), 2.71-2.63 (m, 2H), 2.59 (s, 3H), 1.22-1.18 (m, 3H). ESI-MS calculated for C₂₁H₂₅F₃N₅O₂ [M+H]⁺ = 436.2, found 436.2.

### Example 102

### Synthetic route:

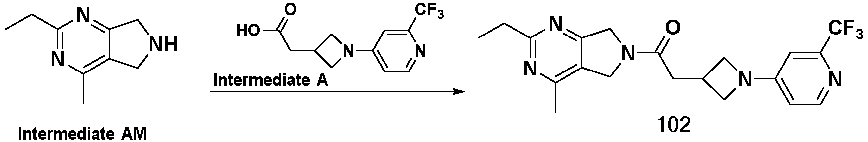

### Step 1

Intermediate **AM** (90 mg, 0.55 mmol) and intermediate **A** (205 mg, 0.55 mmol) were dissolved in DMF (5 mL), then DIEA (356 mg, 2.76 mmol) was added thereto, and the mixture was stirred at room temperature for 30 minutes. HATU (252 mg, 0.66 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 52%, retention time: 9 minutes) to obtain compound 102. ¹H NMR (400 MHz, DMSO-*d*₆) δ 8.21 (d, *J =* 5.60 Hz, 1H), 6.71 (d, *J =* 2.24 Hz, 1H), 6.56-6.48 (m, 1H), 4.88-4.79 (m, 2H), 4.64-4.57 (m, 2H), 4.22-4.14 (m, 2H), 3.74-3.67 (m, 2H), 3.15-3.09 (m, 1H), 2.88-2.81 (m, 4H), 2.40 (s, 3H), 1.28-1.20 (m, 3H). ESI-MS calculated for C₂₀H₂₃F₃N₅O [M+H]⁺ = 406.2, found 406.2.

### Example 103

### Synthetic route:

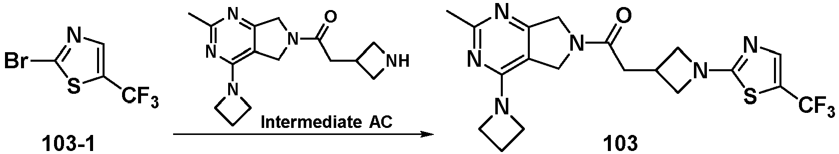

### Step 1

Intermediate **AC** (100 mg, 0.17 mmol), **103-1** (40 mg, 0.17 mmol) and cesium carbonate (170 mg, 0.52 mmol) were dissolved in DMF (5 mL), and the reaction mixture was heated to 100°C and stirred for 18 hours. After the reaction mixture was cooled to room temperature, the insoluble matter was removed by filtration, and the filtrate was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 29 to 69%, retention time: 9 minutes) to obtain compound **103.** ¹H NMR (400 MHz, DMSO-*d*₆) δ 7.71-7.68 (m, 1H), 4.85-4.80 (m, 1H), 4.61-4.55 (m, 2H), 4.38-4.35 (m, 1H), 4.30-4.10 (m, 6H), 3.86-3.75 (m, 2H), 3.25-3.10 (m, 1H), 2.88-2.83 (m, 2H), 2.35 (s, 3H), 2.33-2.28 (m, 2H). ESI-MS calculated for C₁₉H₂₂F₃N₆OS [M+H]⁺ = 439.1, found 439.2.

### Example 104

### Synthetic route:

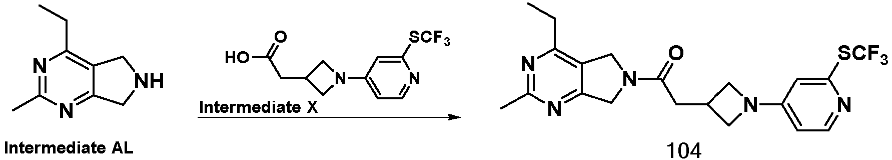

### Step 1

Intermediate **X** (30 mg, 0.10 mmol) and intermediate **AL** (17 mg, 0.10 mmol) were dissolved in DMF (5 mL), then DIEA (80 mg, 0.62 mmol) was added thereto, and the mixture was stirred at room temperature for 30 minutes. HATU (47 mg, 0.12 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 38 to 68%, retention time: 9 minutes) to obtain compound **104.** ¹H NMR (400 MHz, DMSO-*d*₆) δ 8.10 (d, *J =* 5.70 Hz, 1H), 6.59 (d, *J =* 2.14 Hz, 1H), 6.40 (dd, *J =* 5.78, 2.18 Hz, 1H), 4.91-4.80 (m, 2H), 4.66-4.55 (m, 2H), 4.13 (t, *J =* 8.24 Hz, 2H), 3.69-3.63 (m, 2H), 3.17-3.05 (m, 1H), 2.89-2.83 (m, 2H), 2.71-2.59 (m, 2H), 2.59 (s, 3H), 1.24-1.18 (m, 3H). ESI-MS calculated for C₂₀H₂₃F₃N₅OS [M+H]⁺ = 438.2, found 438.1.

### Example 105

### Synthetic route:

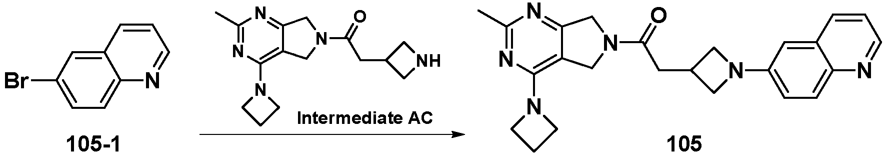

### Step 1

**105-1** (73 mg, 0.35 mmol), intermediate **AC** (100 mg, 0.35 mmol), cesium carbonate (342 mg, 1.05 mmol), and methanesulfonato(2-dicyclohexylphosphino-2',4',6'-tri-i-propyl-1,1'-biphenyl)(2'-amino-1,1'-biphenyl-2-yl)palladium(II) (30 mg, 0.04 mmol) were added to 1,4-dioxane (5 mL), and the mixture was heated to 100°C under nitrogen atmosphere and stirred for 18 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 16 to 46%, retention time: 9 minutes) to obtain compound **105.** ¹H NMR (400 MHz, DMSO-d6) δ 8.55 (dd, *J =* 4.16, 1.60 Hz, 1H), 8.06 (d, *J =* 8.32 Hz, 1H), 7.81 (d, *J =* 9.02 Hz, 1H), 7.34 (dd, *J =* 8.32, 4.18 Hz, 1H), 7.06 (dd, *J =* 8.86, 2.44 Hz, 1H), 6.66 (d, *J =* 2.46 Hz, 1H), 4.87-4.84 (m, 1H), 4.65-4.59 (m, 2H), 4.39-4.34 (m, 1H), 4.24-4.08 (m, 6H), 3.66-3.63 (m, 2H), 3.15-3.05 (m, 1H), 2.88-2.80 (m, 2H), 2.39-2.28 (m, 5H). ESI-MS calculated for C₂₄H₂₇N₆O [M+H]⁺ = 415.2, found 415.2.

### Example 106

### Synthetic route:

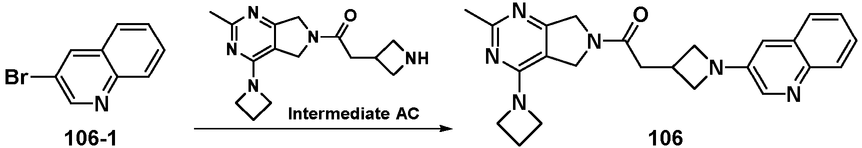

### Step 1

**106-1** (60 mg, 0.29 mmol), intermediate **AC** (125 mg, 0.43 mmol), cesium carbonate (235 mg, 0.72 mmol), and methanesulfonato(2-dicyclohexylphosphino-2',4',6'-tri-i-propyl-1,1'-biphenyl)(2'-amino-1,1'-biphenyl-2-yl)palladium(II) (25 mg, 0.03 mmol) were added to 1,4-dioxane (5 mL), and the mixture was heated to 100°C under nitrogen atmosphere and stirred for 18 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 52%, retention time: 9 minutes) to obtain compound **106.** ¹H NMR (400 MHz, DMSO-*d*₆) δ 8.38-8.35 (m, 1H), 7.84 (d, *J =* 8.04 Hz, 1H), 7.72 (dd, *J =* 7.92, 1.66 Hz, 1H), 7.46-7.38 (m, 2H), 7.08-7.05 (m, 1H), 4.87-4.84 (m, 1H), 4.64-4.59 (m, 2H), 4.38-4.35 (m, 1H), 4.23-4.15 (m, 6H), 3.73-3.67 (m, 2H), 3.18-3.12 (m, 1H), 2.88-2.82 (m, 2H), 2.37-2.32 (m, 5H). ESI-MS calculated for C₂₄H₂₇N₆O [M+H]⁺ = 415.2, found 415.2.

### Example 107

### Synthetic route:

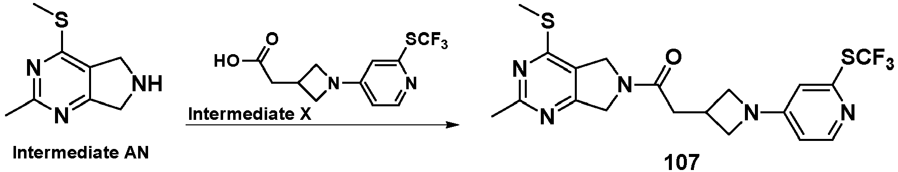

### Step 1

Intermediate **X** (50 mg, 0.17 mmol) and intermediate **AN** (37 mg, 0.21 mmol) were dissolved in DMF (5 mL), then DIEA (66 mg, 0.51 mmol) was added thereto, and the mixture was stirred at room temperature for 30 minutes. HATU (98 mg, 0.26 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 46 to 76%, retention time: 8 minutes) to obtain compound **107.** ¹H NMR (400 MHz, DMSO-*d*₆) δ 8.10 (d, *J =* 5.72 Hz, 1H), 6.63-6.56 (m, 1H), 6.42-6.36 (m, 1H), 4.81-4.68 (m, 2H), 4.53-4.44 (m, 2H), 4.15-4.09 (m, 2H), 3.71-3.61 (m, 2H), 3.13-3.02 (m, 1H), 2.88-2.78 (m, 2H), 2.65 (s, 3H), 2.53 (m, 3H). ESI-MS calculated for C₁₉H₂₁F₃N₅OS₂ [M+H]⁺ = 456.1, found 456.1.

### Example 108

### Synthetic route:

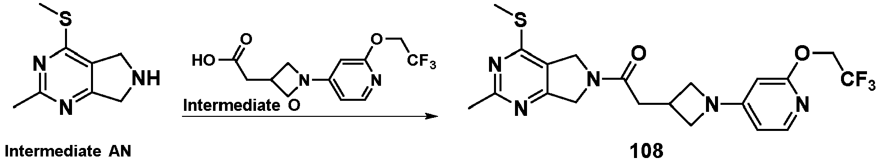

### Step 1

Intermediate **AN** (75 mg, 0.41 mmol) and intermediate **O** (100 mg, 0.34 mmol) were dissolved in DMF (5 mL), then DIEA (133 mg, 1.03 mmol) was added thereto, and the mixture was stirred at room temperature for 30 minutes. HATU (196 mg, 0.52 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 46 to 76%, retention time: 8 minutes) to obtain compound **108.** ¹H NMR (400 MHz, DMSO-*d*₆) δ 7.76 (d, *J =* 5.82 Hz, 1H), 6.17-6.12 (m, 1H), 5.80-5.75 (m, 1H), 4.94-4.85 (m, 2H), 4.78-4.71 (m, 2H), 4.55-4.44 (m, 2H), 4.10-4.02 (m, 2H), 3.65-3.55 (m, 2H), 3.11-3.02 (m, 1H), 2.88-2.79 (m, 2H), 2.64 (s, 3H), 2.51 (m, 3H). ESI-MS calculated for C₂₀H₂₃F₃N₅O₂S [M+H]⁺ = 454.1, found 454.1.

### Example 109

### Synthetic route:

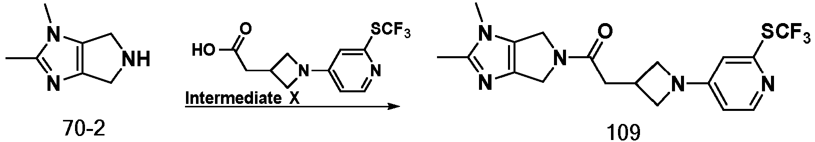

### Step 1

**70-2** (25 mg, 0.18 mmol) and intermediate **X** (53 mg, 0.18 mmol) were dissolved in DMF (5 mL), then DIEA (71 mg, 0.55 mmol) was added thereto, and the mixture was stirred at room temperature for 30 minutes. HATU (90 mg, 0.24 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 25 to 55%, retention time: 9 minutes) to obtain compound **109.** ¹H NMR (400 MHz, DMSO-*d*₆) δ 8.09 (d, *J =* 5.70 Hz, 1H), 6.58 (d, *J* = 2.16 Hz, 1H), 6.39 (dd, *J =* 5.72, 2.22 Hz, 1H), 4.63-4.60 (m, 1H), 4.44-4.39 (m, 2H), 4.24-4.21 (m, 1H), 4.16-4.09 (m, 2H), 3.68-3.63 (m, 2H), 3.52-3.49 (m, 3H), 3.12-3.03 (m, 1H), 2.82-2.77 (m, 2H), 2.28 (s, 3H). ESI-MS calculated for C₁₈H₂₁F₃N₅OS [M+H]⁺ = 412.1, found 412.1.

### Example 110

### Synthetic route:

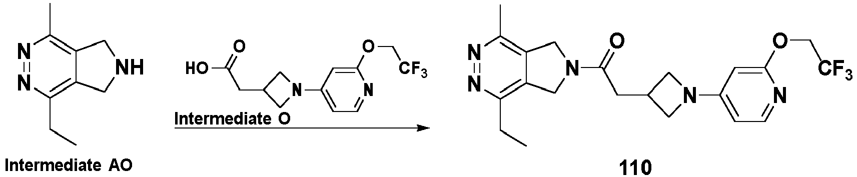

### Step 1

Intermediate **AO** (59 mg, 0.36 mmol) and intermediate **O** (70 mg, 0.24 mmol) were dissolved in DMF (5 mL), then DIEA (94 mg, 0.72 mmol) was added thereto, and the mixture was stirred at room temperature for 30 minutes. HATU (119 mg, 0.31 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 31 to 61%, retention time: 9 minutes) to obtain compound **110.** ¹H NMR (400 MHz, DMSO-*d*₆) δ 7.76 (d, *J* = 5.84 Hz, 1H), 6.15 (dd, *J =* 5.80, 1.96 Hz, 1H), 5.78 (d, *J =* 1.94 Hz, 1H), 5.02-4.85 (m, 4H), 4.74-4.67 (m, 2H), 4.11-4.05 (m, 2H), 3.64-3.57 (m, 2H), 3.12-3.04 (m, 1H), 2.90-2.82 (m, 4H), 2.55 (s, 3H), 1.26 (t, *J* = 7.54 Hz, 3H). ESI-MS calculated for C₂₁H₂₅F₃N₅O₂ [M+H]⁺ = 436.2, found 436.2.

### Example 111

### Synthetic route:

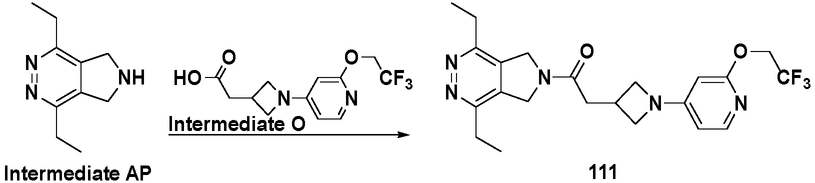

### Step 1

Intermediate **AP** (70 mg, 0.39 mmol) and intermediate **O** (115 mg, 0.39 mmol) were dissolved in DMF (5 mL), then DIEA (153 mg, 1.18 mmol) was added thereto, and the mixture was stirred at room temperature for 30 minutes. HATU (195 mg, 0.51 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 34 to 64%, retention time: 9 minutes) to obtain compound **111.** ¹H NMR (400 MHz, DMSO-*d*₆) δ 7.76 (d, *J* = 5.78 Hz, 1H), 6.15 (dd, *J =* 5.82, 1.94 Hz, 1H), 5.78 (d, *J =* 1.92 Hz, 1H), 4.97-4.83 (m, 4H), 4.73-4.70 (m, 2H), 4.10-4.06 (m, 2H), 3.64-3.57 (m, 2H), 3.13-3.05 (m, 1H), 2.91-2.83 (m, 6H), 1.27 (t, *J =* 7.54 Hz, 6H). ESI-MS calculated for C₂₂H₂₇F₃N₅O₂ [M+H]⁺ = 450.2, found 450.2.

### Example 112

### Synthetic route:

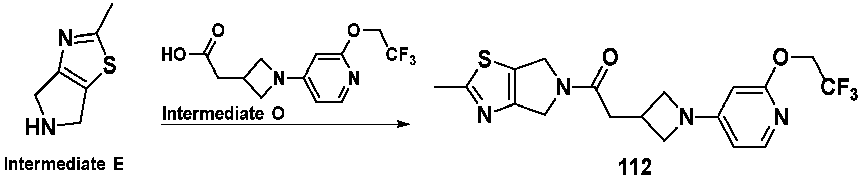

### Step 1

Intermediate **E** (24 mg, 0.17 mmol) and intermediate **O** (50 mg, 0.17 mmol) were dissolved in DMF (5 mL), then DIEA (52 mg, 0.52 mmol) was added thereto, and the mixture was stirred at room temperature for 30 minutes. HATU (78 mg, 1.20 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: XBndge C18 19 * 250 mm 10 µm, mobile phase: acetonitrile-10 mmol/L ammonium bicarbonate aqueous solution, gradient: 40 to 55%, retention time: 9.5 minutes) to obtain compound **112.** ¹H NMR (400 MHz, DMSO-*d*₆) δ 7.76 (d, *J =* 5.82 Hz, 1H), 6.15 (dd, *J=* 5.90, 2.00 Hz, 1H), 5.80-5.77 (m, 1H), 4.96-4.86 (m, 2H), 4.79-4.67 (m, 2H), 4.58-4.44 (m, 2H), 4.10-4.03 (m, 2H), 3.66-3.55 (m, 2H), 3.12-3.07 (m, 1H), 2.83-2.76 (m, 2H), 2.68 (s, 3H). ESI-MS calculated for C₁₈H₂₀F₃N₄O₂S [M+H]⁺ = 413.1, found 413.1.

### Example 113

### Synthetic route:

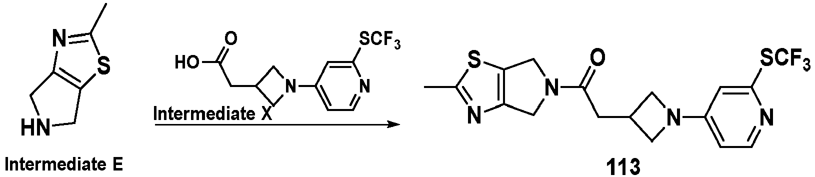

### Step 1

Intermediate **E** (24 mg, 0.17 mmol) and intermediate **X** (50 mg, 0.17 mmol) were dissolved in DMF (5 mL), then DIEA (52 mg, 0.52 mmol) was added thereto, and the mixture was stirred at room temperature for 30 minutes. HATU (78 mg, 0.21 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: XBndge C18 19 * 250 mm 10 µm, mobile phase: acetonitrile-10 mmol/L ammonium bicarbonate aqueous solution, gradient: 45 to 60%, retention time: 8.5 minutes) to obtain compound **113.** ¹H NMR (400 MHz, DMSO-*d*₆) δ 8.10 (d, J= 5.70 Hz, 1H), 6.59 (d, *J =* 2.22 Hz, 1H), 6.40 (dd, *J =* 5.76, 2.20 Hz, 1H), 4.84-4.65 (m, 2H), 4.80-4.44 (m, 2H), 4.16-4.09 (m, 2H), 3.69-3.63 (m, 2H), 3.13-3.06 (m, 1H), 2.85-2.78 (m, 2H), 2.68 (s, 3H). ESI-MS calculated for C₁₇H₁₈F₃N₄OS₂ [M+H]⁺ = 415.1, found 415.1.

### Example 114

### Synthetic route:

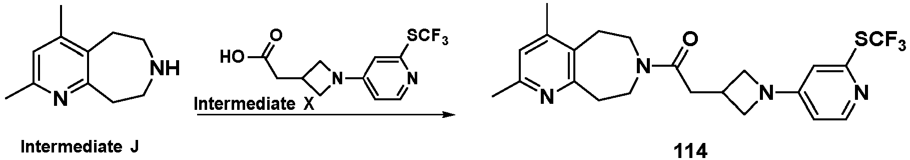

### Step 1

Intermediate **J** (80 mg, 0.45 mmol) and intermediate **X** (133 mg, 0.45 mmol) were dissolved in DMF (5 mL), then DIEA (176 mg, 1.36 mmol) was added thereto, and the mixture was stirred at room temperature for 30 minutes. HATU (259 mg, 0.68 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Bonnasil-BR-C18-10 µm-20 * 250 mm, mobile phase: acetonitrile-10 mmol/L ammonium bicarbonate aqueous solution, gradient: 35 to 65%, retention time: 11 minutes) to obtain compound **114.** ¹H NMR (400 MHz, DMSO-*d*₆) δ 8.10 (d, *J =* 5.72 Hz, 1H), 6.90 (d, *J =* 4.84 Hz, 1H), 6.57 (t, *J =* 2.60 Hz, 1H), 6.39-6.36 (m, 1H), 4.11-4.05 (m, 2H), 3.66-3.53 (m, 5H), 3.30-3.25 (m, 1H), 3.11-2.96 (m, 3H), 2.92-2.77 (m, 4H), 2.37-2.28 (m, 3H), 2.23 (s, 3H). ESI-MS calculated for C₂₂H₂₆F₃N₄OS [M+H]⁺ = 451.2, found 451.2.

### Example 115

### Synthetic route:

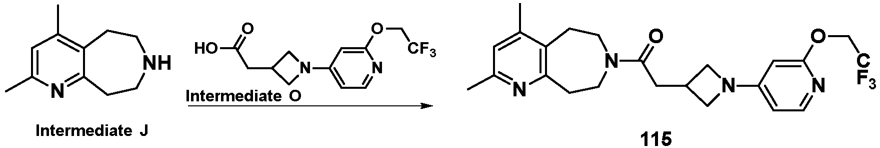

### Step 1

Intermediate **J** (80 mg, 0.45 mmol) and intermediate **O** (187 mg, 0.45 mmol) were dissolved in DMF (5 mL), then DIEA (176 mg, 1.36 mmol) was added thereto, and the mixture was stirred at room temperature for 30 minutes. HATU (259 mg, 0.68 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Bonnasil-BR-C18-10 µm-20 * 250 mm, mobile phase: acetonitrile-10 mmol/L ammonium bicarbonate aqueous solution, gradient: 44 to 74%, retention time: 9 minutes) to obtain compound **115.** ¹H NMR (400 MHz, DMSO-*d*₆) δ 7.76 (d, *J =* 5.76 Hz, 1H), 6.93-6.88 (m, 1H), 6.14-6.11 (m, 1H), 5.78-5.74 (m, 1H), 4.94-4.87 (m, 2H), 4.05-3.97 (m, 2H), 3.64-3.45 (m, 5H), 3.09-2.63 (m, 8H), 2.40-2.28 (m, 4H), 2.24-2.20 (m, 2H). ESI-MS calculated for C₂₃H₂₈F₃N₄O₂ [M+H]⁺ = 449.2, found 449.2.

### Example 116

### Synthetic route:

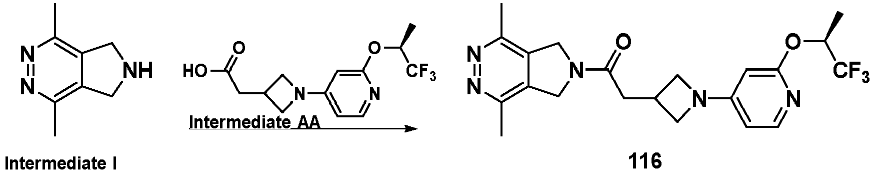

### Step 1

Intermediate **I** (66 mg, 0.44 mmol) and intermediate **AA** (90 mg, 0.30 mmol) were dissolved in DMF (5 mL), then DIEA (115 mg, 0.89 mmol) was added thereto, and the mixture was stirred at room temperature for 30 minutes. HATU (169 mg, 0.44 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile- 10 mmol/L ammonium bicarbonate aqueous solution, gradient: 32 to 62%, retention time: 9 minutes) to obtain compound **116.** ¹H NMR (400 MHz, DMSO-*d*₆) δ 7.76 (d, *J* = 5.80 Hz, 1H), 6.13 (dd, *J =* 5.84, 2.00 Hz, 1H), 5.87-5.80 (m, 1H), 5.74 (d, *J =* 1.96 Hz, 1H), 4.93-4.90 (m, 2H), 4.71-4.68 (m, 2H), 4.12-4.03 (m, 2H), 3.64-3.55 (m, 2H), 3.14-3.03 (m, 1H), 2.86-2.82 (m, 2H), 2.58-2.52 (m, 6H), 1.38 (d, *J* = 6.50 Hz, 3H). ESI-MS calculated for C₂₁H₂₅F₃N₅O₂ [M+H]⁺ = 436.2, found 436.2.

### Example 117

### Synthetic route:

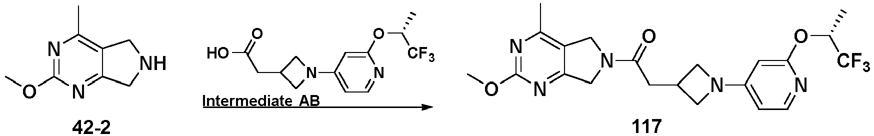

### Step 1

**42-2** (82 mg, 0.49 mmol) and intermediate **AB** (100 mg, 0.33 mmol) were dissolved in DMF (5 mL), then DIEA (170 mg, 1.32 mmol) was added thereto, and the mixture was stirred at room temperature for 30 minutes. HATU (175 mg, 0.46 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile- 10 mmol/L ammonium bicarbonate aqueous solution, gradient: 40 to 70%, retention time: 11 minutes) to obtain compound **117.** ¹H NMR (400 MHz, DMSO-*d*₆) δ 7.75 (d, *J* = 5.78 Hz, 1H), 6.13 (dd, *J =* 5.80, 1.96 Hz, 1H), 5.88-5.75 (m, 1H), 5.75-5.73 (m, 1H), 4.79 (d, *J =* 8.48 Hz, 2H), 4.58-4.50 (m, 2H), 4.11-4.04 (m, 2H), 3.89 (s, 3H), 3.64-3.56 (m, 2H), 3.14-3.03 (m, 1H), 2.85-2.80 (m, 2H), 2.39-2.35 (m, 3H), 1.38 (d, *J* = 6.52 Hz, 3H). ESI-MS calculated for C₂₁H₂₅F₃N₅O₃ [M+H]⁺ = 452.2, found 452.2.

### Example 118

### Synthetic route:

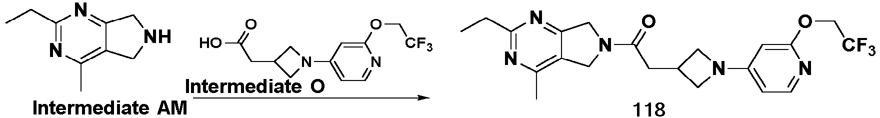

### Step 1

**AM** (90 mg, 0.55 mmol) and intermediate **O** (228 mg, 0.55 mmol) were dissolved in DMF (5 mL), then DIEA (214 mg, 1.65 mmol) was added thereto, and the mixture was stirred at room temperature for 30 minutes. HATU (251 mg, 0.66 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile- 10 mmol/L ammonium bicarbonate aqueous solution, gradient: 22 to 52%, retention time: 3 minutes) to obtain compound **118.** ¹H NMR (400 MHz, DMSO-*d*₆) δ 7.76 (d, *J* = 5.80 Hz, 1H), 6.15 (dd, *J* = 5.86, 2.02 Hz, 1H), 5.78 (d, *J =* 1.96 Hz, 1H), 4.93-4.82 (m, 4H), 4.63-4.57 (m, 2H), 4.10-4.04 (m, 2H), 3.63-3.58 (m, 2H), 3.13-3.07 (m, 1H), 2.91-2.77 (m, 4H), 2.42-2.38 (m, 3H), 1.25 (t, *J* = 7.56 Hz, 3H). ESI-MS calculated for C₂₁H₂₅F₃N₅O₂ [M+H]⁺ = 436.2, found 436.2.

### Example 119

### Synthetic route:

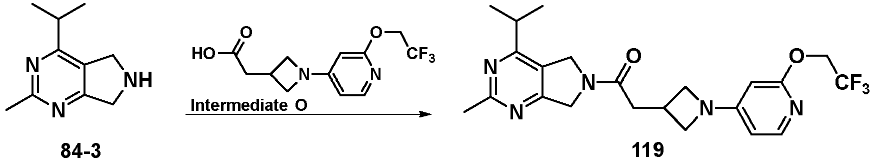

### Step 1

**84-3** (122 mg, 0.34 mmol) and intermediate **O** (100 mg, 0.34 mmol) were dissolved in DMF (5 mL), then DIEA (267 mg, 2.07 mmol) was added thereto, and the mixture was stirred at room temperature for 30 minutes. HATU (157 mg, 0.41 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile- 10 mmol/L ammonium bicarbonate aqueous solution, gradient: 41 to 71%, retention time: 9 minutes) to obtain compound **119.** ¹H NMR (400 MHz, DMSO-*d*₆) δ 7.76 (d, *J* = 5.74 Hz, 1H), 6.15 (dd, *J =* 5.80, 1.96 Hz, 1H), 5.78 (d, *J =* 1.94 Hz, 1H), 4.98-4.77 (m, 4H), 4.70-4.52 (m, 2H), 4.12-4.06 (m, 2H), 3.63-3.59 (m, 2H), 3.17 -2.92 (m, 2H), 2.87-2.82 (m, 2H), 2.63-2.60 (m, 1H), 2.42-2.39 (m, 2H), 1.28-1.16 (m, 6H). ESI-MS calculated for C₂₂H₂₇F₃N₅O₂ [M+H]⁺ = 450.2, found 450.2.

### Example 120

### Synthetic route:

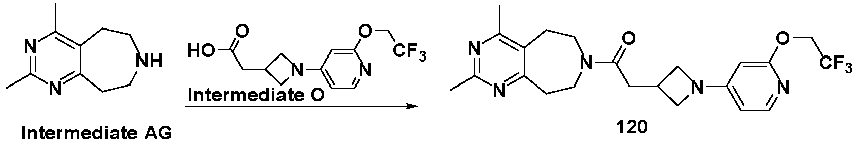

### Step 1

Intermediate **AG** (52 mg, 0.29 mmol) and intermediate **O** (85 mg, 0.29 mmol) were dissolved in DMF (5 mL), then DIEA (227 mg, 1.76 mmol) was added thereto, and the mixture was stirred at room temperature for 30 minutes. HATU (133 mg, 0.35 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile- 10 mmol/L ammonium bicarbonate aqueous solution, gradient: 30 to 70%, retention time: 9 minutes) to obtain compound **120.** ¹H NMR (400 MHz, DMSO-*d*₆) δ 7.75 (d, *J =* 5.80 Hz, 1H), 6.15-6.10 (m, 1H), 5.75 (t, *J =* 2.44 Hz, 1H), 4.92-4.85 (m, 2H), 4.04-3.98 (m, 2H), 3.65-3.49 (m, 5H), 3.34-3.29 (m, 1H), 3.10-2.82 (m, 7H), 2.49-2.44 (m, 3H), 2.41 (s, 3H). ESI-MS calculated for C₂₂H₂₇F₃N₅O₂ [M+H]⁺ = 450.2, found 450.2.

### Example 121

### Synthetic route:

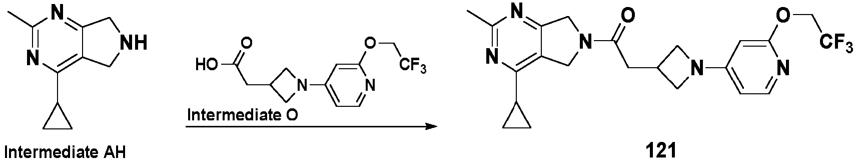

### Step 1

Intermediate **AH** (109 mg, 0.31 mmol) and intermediate **O** (100 mg, 0.31 mmol) were dissolved in DMF (5 mL), then DIEA (240 mg, 1.86 mmol) was added thereto, and the mixture was stirred at room temperature for 30 minutes. HATU (141 mg, 0.37 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile- 10 mmol/L ammonium bicarbonate aqueous solution, gradient: 38 to 68%, retention time: 9 minutes) to obtain compound **121.** ¹H NMR (400 MHz, DMSO-*d*₆) δ 7.76 (d, *J* = 5.80 Hz, 1H), 6.16 (dd, *J =* 5.86, 1.98 Hz, 1H), 5.79 (d, *J* = 1.94 Hz, 1H), 4.97-4.72 (m, 5H), 4.54-4.51 (m, 1H), 4.01-4.04 (m, 2H), 3.68-3.57 (m, 2H), 3.14-3.06 (m, 1H), 2.87-2.83 (m, 2H), 2.52 (s, 3H), 1.99-1.92 (m, 1H), 1.14-1.02 (m, 4H). ESI-MS calculated for C₂₂H₂₅F₃N₅O₂ [M+H]⁺ = 448.2, found 448.2.

### Example 122

### Synthetic route:

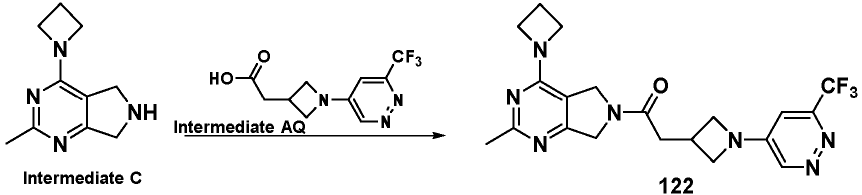

### Step 1

Intermediate **AQ** (68 mg, 0.26 mmol) and intermediate **C** (59 mg, 0.20 mmol) were dissolved in DMF (5 mL), then DIEA (101 mg, 0.78 mmol) was added thereto, and the mixture was stirred at room temperature for 30 minutes. HATU (148 mg, 0.39 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile- 10 mmol/L ammonium bicarbonate aqueous solution, gradient: 9 to 76%, retention time: 9 minutes) to obtain compound **122.** ¹H NMR (400 MHz, DMSO-*d*₆) δ 8.65-8.59 (m, 1H), 6.92-6.85 (m, 1H), 4.85-4.82 (m, 1H), 4.63-4.56 (m, 2H), 4.34-4.12 (m, 7H), 3.87-3.77 (m, 2H), 3.21-3.07 (m, 1H), 2.89-2.78 (m, 2H), 2.39-2.32 (m, 5H). ESI-MS calculated for C₂₀H₂₃F₃N₇O [M+H]⁺ = 434.2, found 434.1.

### Example 123

### Synthetic route:

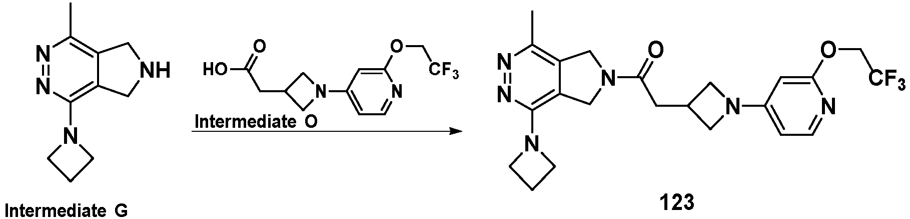

### Step 1

Intermediate **G** (33 mg, 0.17 mmol), HATU (78 mg, 0.20 mmol), DIEA(66 mg, 0.51 mmol), and intermediate **O** (50 mg, 0.17 mmol) were added to DMF (5 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 30 to 60%, retention time: 10 minutes) to obtain compound **123.** ¹H NMR (400 MHz, DMSO-*d*₆) δ 7.76 (d, *J* = 5.78 Hz, 1H), 6.14 (dd, *J =* 5.78, 1.82 Hz, 1H), 5.78 (d, *J =* 1.884 Hz, 1H), 4.95-4.76 (m, 4H), 4.65-4.57 (m, 2H), 4.13-4.05 (m, 6H), 3.61-3.58 (m, 2H), 3.12-3.01 (m, 1H), 2.84-2.81 (m, 2H), 2.40-2.32 (m, 5H). ESI-MS calculated for C₂₂H₂₆F₃N₆O₂ [M+H]⁺ = 463.2, found 463.0.

### Example 124

### Synthetic route:

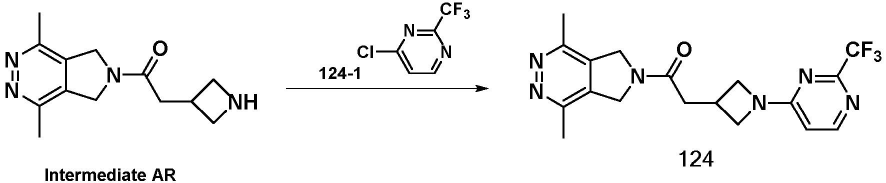

### Step 1

Intermediate **AR** (67 mg, 0.27 mmol), **124-1** (50 mg, 0.27 mmol), TEA (83 mg, 0.82 mmol), and cesium fluoride (62 mg, 0.42 mmol) were added to DMSO (5 mL), and the mixture was stirred at 100°C for 10 hours. The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 16 to 46%, retention time: 10 minutes) to obtain compound **124.** ¹H NMR (400 MHz, DMSO-*d*₆) δ 8.27 (d, *J =* 6.02 Hz, 1H), 6.59 (d, *J =* 6.02 Hz, 1H), 4.93-4.89 (m, 2H), 4.71-4.68 (m, 2H), 4.29-4.23 (m, 2H), 3.85-3.77 (m, 2H), 3.17-3.07 (m, 1H), 2.89-2.86 (m, 2H), 2.55-2.53 (m, 6H). ESI-MS calculated for C₁₈H₂₀F₃N₆O [M+H]⁺ = 393.2, found 393.2.

### Example 125

### Synthetic route:

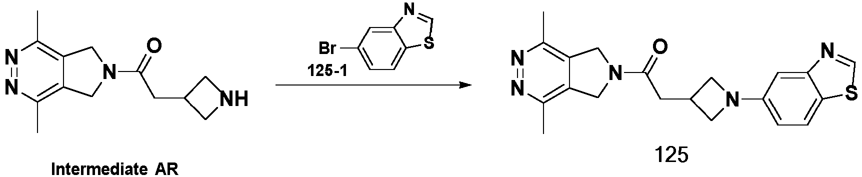

### Step 1

Intermediate **AR** (80 mg, 0.32 mmol), **125-1** (70 mg, 0.32 mmol), methanesulfonato(2-dicyclohexylphosphino-2',4',6'-tri-i-propyl-1,1'-biphenyl)(2'-amino-1,1'-biphenyl-2-yl)palladium(II) (28 mg, 0.03 mmol), and cesium carbonate (317 mg, 0.97 mmol) were added to 1,4-dioxane (5 mL), and the mixture was heated to 100°C under nitrogen atmosphere and stirred for 18 hours. The reaction mixture was cooled and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 18 to 48%, retention time: 10 minutes) to obtain compound **125.** ¹H NMR (400 MHz, DMSO-*d*₆) δ 9.25 (s, 1H), 7.89 (d, *J =* 8.62 Hz, 1H), 7.02 (d, *J* = 2.18 Hz, 1H), 6.68 (dd, *J =* 8.62, 2.24 Hz, 1H), 4.94-4.92 (m, 2H), 4.72-4.69 (m, 2H), 4.10-4.05 (m, 2H), 3.60-3.56 (m, 2H), 3.16-3.03 (m, 1H), 2.88-2.84 (m, 2H), 2.55 (s, 3H), 2.53 (s, 3H). ESI-MS calculated for C₂₀H₂₂N₅OS [M+H]⁺ = 380.2, found 380.0.

### Example 126

### Synthetic route:

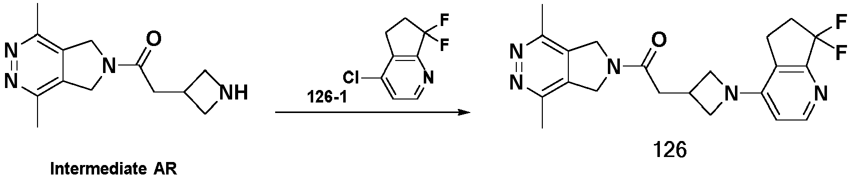

### Step 1

Intermediates **AR** (65 mg, 0.26 mmol), **126-1** (50 mg, 0.26 mmol), cesium fluoride (60 mg, 0.40 mmol), and TEA (80 mg, 0.79 mmol) were added to DMSO (5 mL). The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 15 to 45%, retention time: 10 minutes) to obtain compound **126.** ¹H NMR (400 MHz, DMSO-*d*₆) δ 8.11 (d, *J =* 5.50 Hz, 1H), 6.28 (d, *J =* 5.58 Hz, 1H), 4.94-4.91 (m, 2H), 4.70-4.67 (m, 2H), 4.32-4.28 (m, 2H), 3.86-3.82 (m, 2H), 3.09-2.98 (m, 3H), 2.87-2.84 (m, 2H), 2.56-2.53 (m, 8H). ESI-MS calculated for C₂₁H₂₄F₂N₅O [M+H]⁺ = 400.2, found 400.2.

### Example 127

### Synthetic route:

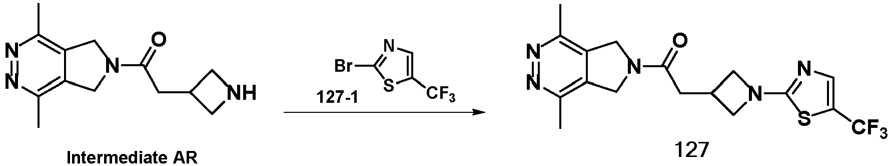

### Step 1

Intermediates **AR** (106 mg, 0.43 mmol), **127-1** (100 mg, 0.43 mmol), and cesium carbonate (351 mg, 1.08 mmol) were added to DMF (3 mL). The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 25 to 55%, retention time: 10 minutes) to obtain compound 127. ¹H NMR (400 MHz, DMSO-*d*₆) δ 7.70 (s, 1H), 4.92-4.89 (m, 2H), 4.71-4.68 (m, 2H), 4.28-4.21 (m, 2H), 3.84-3.78 (m, 2H), 3.22-3.16 (m, 1H), 2.91-2.88 (m, 2H), 2.56-2.53 (m, 6H). ESI-MS calculated for C₁₇H₁₉F₃N₅OS [M+H]⁺ = 398.1, found 398.1.

### Example 128

### Synthetic route:

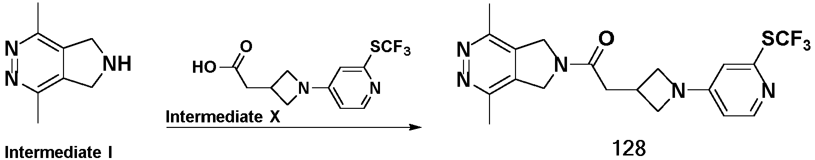

### Step 1

Intermediate **I** (26 mg, 0.17 mmol), HATU (78 mg, 0.20 mmol), DIEA(66 mg, 0.51 mmol), and intermediate **X** (50 mg, 0.17 mmol) were added to DMF (5 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 20 to 50%, retention time: 10 minutes) to obtain compound **128.** ¹H NMR (400 MHz, DMSO-*d*₆) δ 8.10 (d, *J =* 5.72 Hz, 1H), 6.59 (d, *J =* 2.20 Hz, 1H), 6.40 (dd, *J =* 5.72, 2.22 Hz, 1H), 4.93-4.90 (m, 2H), 4.70-4.67 (m, 2H), 4.16-4.12 (m, 2H), 3.68-3.64 (m, 2H), 3.17-3.03 (m, 1H), 2.88-2.85 (m, 2H), 2.55-2.52 (m, 6H). ESI-MS calculated for C₁₉H₂₁F₃N₅OS [M+H]⁺ = 424.1, found 424.1.

### Example 129

### Synthetic route:

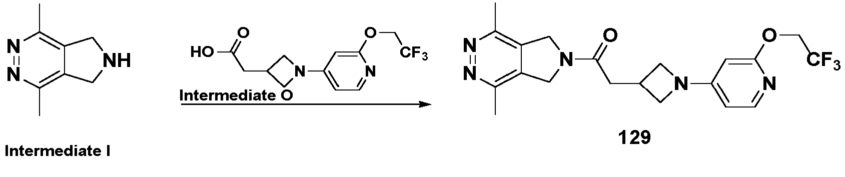

### Step 1

Intermediate **I** (26 mg, 0.17 mmol), HATU (78 mg, 0.20 mmol), DIEA (66 mg, 0.51 mmol), and intermediate **O** (50 mg, 0.17 mmol) were added to DMF (5 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 37 to 47%, retention time: 10 minutes) to obtain compound **129.** ¹H NMR (400 MHz, DMSO-*d*₆) δ 7.76 (d, *J* = 5.80 Hz, 1H), 6.15 (dd, *J* = 5.78, 1.98 Hz, 1H), 5.78 (d, *J =* 1.96 Hz, 1H), 4.99-4.83 (m, 4H), 4.71-4.68 (m, 2H), 4.10-4.06 (m, 2H), 3.62-3.59 (m, 2H), 3.12-3.05 (m, 1H), 2.86-2.83 (m, 2H), 2.56-2.53 (m, 6H). ESI-MS calculated for C₂₀H₂₃F₃N₅O₂ [M+H]⁺ = 422.2, found 422.0.

### Example 130

### Synthetic route:

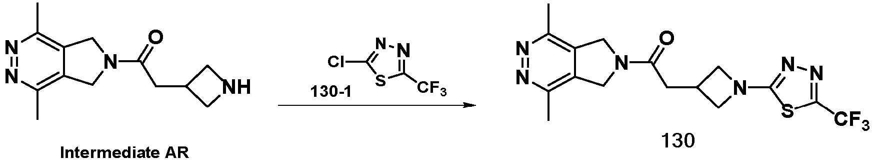

### Step 1

Intermediates **AR** (65 mg, 0.26 mmol), **130-1** (50 mg, 0.26 mmol), cesium fluoride (60 mg, 0.40 mmol), and TEA (80 mg, 0.79 mmol) were added to DMSO (5 mL). The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 18 to 45%, retention time: 9 minutes) to obtain compound **130.** ¹H NMR (400 MHz, DMSO-*d*₆) δ 4.93-4.89 (m, 2H), 4.72-4.68 (m, 2H), 4.38-4.32 (m, 2H), 3.96-3.88 (m, 2H), 3.29-3.18 (m, 1H), 2.92-2.88 (m, 2H), 2.55-2.52 (m, 6H). ESI-MS calculated for C₁₆H₁₈F₃N₆OS [M+H]⁺ = 399.1, found 399.1.

### Example 131

### Synthetic route:

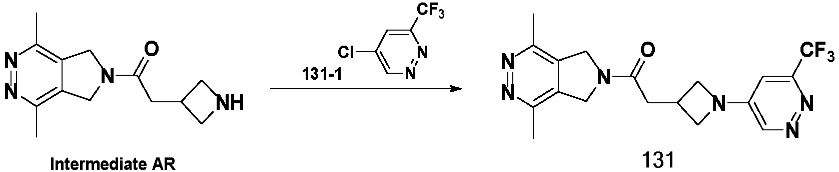

### Step 1

Intermediate **AR** (67 mg, 0.27 mmol), **131-1** (50 mg, 0.27 mmol), TEA (83 mg, 0.82 mmol), and cesium fluoride (42 mg, 0.27 mmol) were added to DMSO (5 mL), and the mixture was stirred at 70°C for 18 hours. The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 5 to 95%, retention time: 10 minutes) to obtain compound **131.** ¹H NMR (400 MHz, DMSO-*d*₆) δ 8.63 (d, *J* = 2.80 Hz, 1H), 6.91 (d, *J* = 2.80 Hz, 1H), 4.92-4.90 (m, 2H), 4.70-4.68 (m, 2H), 4.32-4.28 (m, 2H), 3.87-3.80 (m, 2H), 3.20-3.10 (m, 1H), 2.89-2.86 (m, 2H), 2.55-2.52 (m, 6H). ESI-MS calculated for C₁₈H₂₀F₃N₆O [M+H]⁺ = 393.2, found 392.9.

### Example 132

### Synthetic route:

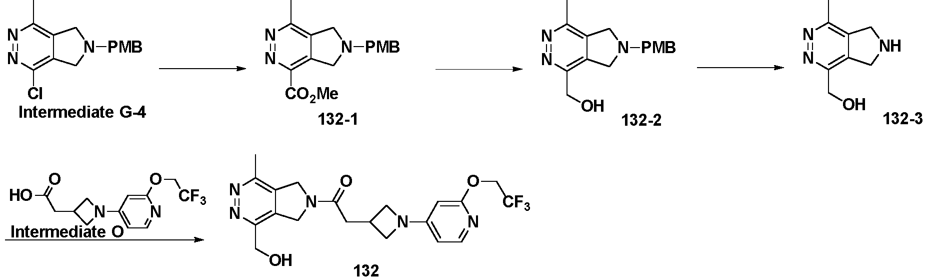

### Step 1

Intermediate **G-4** (500 mg, 1.60 mmol) was dissolved in methanol (20 mL), then [1,1'-bis(diphenylphosphino)ferrocene]dichloropalladium(II) dichloromethane complex (127 mg, 0.17 mmol) and TEA (524 mg, 5.18 mmol) were added thereto. The reaction system was replaced with carbon monoxide three times, then heated to 70°C, and stirred for 18 hours. The reaction mixture was filtered, and the filtrate was concentrated under reduced pressure and purified by silica gel column chromatography (petroleum ether/ethyl acetate, 1/4, v/v) to obtain **132-1.** ESI-MS calculated for C₁₇H₂₀N₃O₃ [M+H]⁺ =314.1, found 314.0.

### Step 2

**132-1** (200 mg, 0.64 mmol) was dissolved in tetrahydrofuran (10 mL), then lithium borohydride (14 mg, 0.64 mmol) was slowly added thereto at 0°C, and the mixture was stirred at room temperature for 1 hour. The reaction mixture was quenched with water (30 mL) and extracted with dichloromethane (20 mL × 3). The organic phases were combined, dried over anhydrous sodium sulfate, filtered, concentrated under reduced pressure, and purified by silica gel column chromatography (petroleum ether/ethyl acetate, 1/4, v/v) to obtain 132-2. ESI-MS calculated for C₁₆H₂₀N₃O₂ [M+H]⁺ = 286.2, found 286.2.

### Step 3

**132-2** (100 mg, 0.35 mmol) and trifluoroacetic acid (2 mL) were added to a 5 mL microwave tube, and the mixture was heated to 90°C and stirred for 8 hours. The reaction mixture was cooled to room temperature and concentrated under reduced pressure to obtain a crude product containing **132-3,** which was directly used in the next reaction step. ESI-MS calculated for C₈H₁₂N₃O [M+H]⁺ = 166.1, found 166.1.

### Step 4

**132-3** (56 mg, 0.34 mmol), HATU (98 mg, 0.26 mmol), DIEA (66 mg, 0.51 mmol), and intermediate **O** (50 mg, 0.17 mmol) were added to DMF (5 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 5 to 95%, retention time: 10 minutes) to obtain compound **132.** ¹H NMR (400 MHz, MeOD-*d*₄) δ 7.74-7.71 (m, 1H), 6.14-6.12 (m, 1H), 5.82-5.75 (m, 1H), 5.12-5.10 (m, 1H), 4.97-4.89 (m, 4H), 4.82-4.78 (m, 1H), 4.74-4.68 (m, 2H), 4.19-4.14 (m, 2H), 3.73-3.64 (m, 2H), 3.26-3.08 (m, 1H), 2.95-2.91 (m, 2H), 2.65 (s, 3H). ESI-MS calculated for C₂₀H₂₃F₃N₅O₃ [M+H]⁺ = 438.2, found 437.9.

### Activity assay 1: Evaluation of PAM activity of compounds on M4 receptor

### Experimental purpose:

The activity of compounds on M4 receptor was determined by FLIPR CALCIUM 6 ASSAY KIT using a stable transfected cell line expressing human M4 receptor (M4-Gα15-CHO).

### Experimental materials:

| **Reagents and consumables** | **Supplier** | **Catalog number** |
|---|---|---|
| F-12 medium | Hyclone | SH30026.01 |
| FLIPR CALCIUM 6 ASSAY KIT | Molecul Device | R8191 |
| Fetal bovine serum | AusGeneX | FBS500-S |
| Hygromycin B | Innochem | H8080-1g |
| Geneticin (G418) | Gibco | 10131035 |
| 0.25% trypsin | Gibco | 25200072 |
| Phosphate buffered saline (PBS) | Innochem | B46663 |
| Hanks' balanced salt solution (HBSS) | Gibco | 14025-092 |
| Dimethyl sulfoxide (DMSO) | Sigma | D4540 |
| 384-well loading plate | Nunc | 264573 |
| 96-well polypropylene plate | Biosen | P-0.36-BSA-96 |
| 384-well cell plate | Corning | 3764 |
| 2-(4-(2-Hydroxyethyl)piperazine)ethanesulfonic acid (HEPES) | Santa Cruz | SC-29097A |
| Sodium hydroxide (NaOH) | Innochem | A36865 |
| Probenecid | MCE | HY-B0545 |
| Acetylcholine | MCE | HY-B0282 |
| Pipette tip | Molecular Devices | 9000-0763 |

### Experimental instruments:

| **Instruments and equipment** | **Supplier** | **Catalog number** |
|---|---|---|
| Biosafety cabinet | ECSO | AC2-6S1-TC |
| Carbon dioxide cell incubator | ESCO | CCl-170B-8 |
| Inverted microscope | Olympus | CKX53 |
| Microplate low-speed centrifuge | Xiangzhi | TD5B |
| FLIPR^{®} Penta | Molecul Device | 5.0 |

### Cell treatment:

1. This study uses a CHO cell line stably expressing M4-Gα15 receptor, which expresses human CHRM4 and GNA15 genes, respectively.
2. Cell treatment: M4-Gα15-CHO cells were cultured in F-12 medium containing 10% fetal bovine serum, 0.4 mg/mL hygromycin B, and 0.8 mg/mL geneticin G418, with a culture temperature of 37°C and a CO₂ concentration of 5%. The old media was removed and the cells were washed once with phosphate buffered saline. 1 mL of trypsin solution was then added, and the mixture was incubated at 37°C for approximately 2 minutes. After the cells were detached from the dish, approximately 5 mL of complete media pre-warmed at 37°C was added. The cell suspension was gently pipetted to separate the aggregated cells. The cell suspension was transferred to a sterile centrifuge tube and centrifuged at 1000 rpm for 5 minutes.

### Experimental procedure:

1. Cell plating: M4-Gα15-CHO cells were digested and collected, then resuspended and counted, and seeded into a 384-well cell plate at a density of 1.2 × 10⁴ cells/25 µL/well. The cell plate was then incubated in a 5% CO₂ incubator at 37°C for approximately 20 hours.
2. Preparation of loading buffer according to the instructions of FLIPR CALCIUM 6 ASSAY KIT after 24 hours: Component A was freeze-thawed to room temperature, and diluted with assay buffer and probenecid solution to obtain a loading buffer, which was kept at room temperature for later use.
3. The medium was removed from the cell plate, and 35 µL of loading buffer was quickly added to each well. After centrifugation, the cell plate was incubated at 37°C in the dark for 120 minutes.
4. A working solution of the compound to be tested was prepared, 5 µL of which was transferred to the corresponding cell wells, and incubated at 37°C in the dark for 30 minutes.
5. 30 nM acetylcholine agonist working solution was prepared and transferred to a 384-well loading plate at 20 µL/well.
6. The cell plate, the loading plate, and the pipette tip were placed at the corresponding positions of the FLIPR instrument. 10 µL of the agonist diluted in step 5 was added to the experimental wells using FLIPR, and data were collected at a wavelength of 515 nm to 575 nm.
7. The signal value was plotted against compound concentration. Curve fitting and EC₅₀ calculation were performed by nonlinear regression method using GraphPad Prism software.

### Preparation of assay buffer and loading buffer:

1. 0.5 mol/L 2-(4-(2-hydroxyethyl)piperazine)ethanesulfonic acid buffer was mixed with Hanks' balanced salt solution (pH 7.4) at a volume ratio of 1:24 to prepare an assay buffer. 10 mL of assay buffer was added to a vial of CALCIUM 6 ASSAY KIT Component A in the form of lyophilized powder, and aliquoted and stored at -20°C.
2. Probenecid powder was dissolved in 1 mol/L sodium hydroxide solution to a final concentration of 250 mol/L. The assay buffer, freeze-thawed Component A, and probenecid solution were mixed at a volume ratio of 44:5:1 to prepare a loading buffer.

### Experimental results:

| **Compound** | **EC₅₀ (µM)** |
|---|---|
| Example 1 | 0.20 |
| Example 6 | 1.61 |
| Example 7 | 0.31 |
| Example 8 | 0.68 |
| Example 12 | 0.35 |
| Example 13 | 5.72 |
| Example 15 | > 20 |
| Example 19 | > 20 |
| Example 24 | 0.387 |
| Example 26 | 0.691 |
| Example 34 | 1.33 |
| Example 42 | 1.34 |
| Example 43 | 1.46 |
| Example 44 | 5.03 |
| Example 45 | 1.74 |
| Example 46 | 2.33 |
| Example 47 | 0.25 |
| Example 48 | 4.48 |
| Example 50 | 1.78 |
| Example 51 | 1.37 |
| Example 53 | 0.35 |
| Example 55 | 2.33 |
| Example 57 | 0.38 |
| Example 59 | 0.31 |
| Example 60 | 0.86 |
| Example 61 | 1.39 |
| Example 63 | 0.06 |
| Example 64 | 1.55 |
| Example 66 | 0.48 |
| Example 67 | 0.39 |
| Example 68 | 0.48 |
| Example 69 | 0.33 |
| Example 70 | 0.46 |
| Example 73 | 1.30 |
| Example 74 | 0.64 |
| Example 75 | 0.40 |
| Example 76 | 0.19 |
| Example 78 | 0.34 |
| Example 79 | 0.39 |
| Example 81 | 0.38 |
| Example 82 | 0.67 |
| Example 83 | 0.34 |
| Example 85 | 0.28 |
| Example 86 | 6.34 |
| Example 88 | 0.85 |
| Example 89 | 0.29 |
| Example 90 | 0.42 |
| Example 91 | 0.80 |
| Example 92 | 1.90 |
| Example 94 | 0.52 |
| Example 95 | 2.23 |
| Example 96 | 0.85 |
| Example 98 | 1.13 |
| Example 99 | 1.39 |
| Example 100 | 3.27 |
| Example 101 | 0.61 |
| Example 102 | 1.89 |
| Example 103 | 0.41 |
| Example 104 | 0.84 |
| Example 105 | 0.51 |
| Example 106 | 0.34 |
| Example 107 | 0.22 |
| Example 109 | 0.28 |
| Example 110 | 0.90 |
| Example 112 | 0.47 |
| Example 113 | 0.84 |
| Example 114 | 1.46 |
| Example 116 | 0.58 |
| Example 117 | 0.31 |
| Example 118 | 0.36 |
| Example 122 | 0.44 |

.

### Experimental conclusion:

The experimental samples (compounds) were prepared from the corresponding examples, and the results are shown in the table above. The compounds of the present disclosure show agonistic activity on the M4 receptor in this experimental system.

### Activity assay 2: Evaluation of pharmacokinetic properties of compounds in vivo in mice

### Experimental purpose:

The compounds obtained in the examples of the present disclosure were evaluated for their pharmacokinetic properties *in vivo* in CD-1 mice.

### Experimental materials:

| **Material** | **Supplier** | **Catalog number** |
|---|---|---|
| CD-1 mice (male, 20 to 40 g, 6 to 9 weeks old) | Beijing Vital River Laboratory Animal Technology Co., Ltd. | M-LOT20220246 |
| EDTA2K anticoagulant tube | Jiangsu Kangjian Medical Apparatus Co., Ltd. | 20020301 |

### Experimental procedure:

The compounds were tested for their pharmacokinetic characteristics in rodents after intravenous injection and oral administration following standard protocols. In the experiment, the candidate compound was prepared into a clear solution or suspension with a specified vehicle, and administered to three mice via single-dose intravenous injection and oral administration, respectively. The vehicle for both intravenous injection and oral administration was 5% dimethyl sulfoxide + 95% (10% polyethylene glycol (15)-hydroxystearate in water). Whole blood samples within 24 hours were collected into a commercial EDTA2K anticoagulant tube, followed by centrifugation to obtain the upper plasma sample, and acetonitrile solution containing internal standard was added to precipitate proteins. After centrifugation, the supernatant was mixed with an equal volume of water, and after another centrifugation, the supernatant was taken for injection. Plasma concentration was quantitatively analyzed using LCMS/MS analysis method, and pharmacokinetic parameters were calculated.

### Experimental method:

| Test sample | Dose |
|---|---|
| Example 1 | Intravenous injection: 1.0 mg/kg |
| | Oral administration: 2.0 mg/kg |
| Example 8 | Intravenous injection: 1.0 mg/kg |
| | Oral administration: 2.0 mg/kg |
| Example 12 | Intravenous injection: 1.0 mg/kg |
| | Oral administration: 2.0 mg/kg |
| Example 42 | Intravenous injection: 1.0 mg/kg |
| | Oral administration: 2.0 mg/kg |
| Example 57 | Intravenous injection: 1.0 mg/kg |
| | Oral administration: 2.0 mg/kg |
| Example 66 | Intravenous injection: 1.0 mg/kg |
| | Oral administration: 2.0 mg/kg |
| Example 67 | Intravenous injection: 1.0 mg/kg |
| | Oral administration: 2.0 mg/kg |
| Example 89 | Intravenous injection: 1.0 mg/kg |
| | Oral administration: 2.0 mg/kg |
| Example 90 | Intravenous injection: 1.0 mg/kg |
| | Oral administration: 2.0 mg/kg |

### Experimental results:

| Test sample | Peak concentration Cₘₐₓ (ng/mL) | Half-life T_{1/2}, po (hr) | Volume of distribution at steady state Vdₛₛ (L/kg) | Clearance CL (mL/min/kg) | Area under the curve AUC₀₋ₗₐₛₜ, po (hr*ng/mL) | Bioavailability F (%) |
|---|---|---|---|---|---|---|
| Example 1 | 437 | 1.12 | 1.69 | 40.1 | 925 | 110 |
| Example 8 | 710 | 2.25 | 1.76 | 11.4 | 2262 | 95.4 |
| Example 12 | 614 | 1.13 | 2.30 | 17.1 | 1693 | 86.6 |
| Example 42 | 1037 | 2.43 | 1.36 | 9.5 | 4754 | 148 |
| Example 57 | 1008 | 0.83 | 0.74 | 17.6 | 1912 | 124 |
| Example 66 | 435 | 2.44 | 1.48 | 20.2 | 1653 | 106 |
| Example 67 | 827 | 4.78 | 1.53 | 3.32 | 8039 | 82.7 |
| Example 89 | 345 | 4.65 | 3.72 | 7.65 | 3474 | 80.6 |
| Example 90 | 1263 | 1.63 | 1.04 | 13.7 | 3585 | 147 |

### Experimental conclusion:

The test samples were prepared from the corresponding examples. The results show that the compounds of the present disclosure exhibit good pharmacokinetic properties.

### Activity assay 3: Evaluation of pharmacokinetic properties of compounds in vivo in rats

### Experimental purpose:

The compounds obtained in the examples of the present disclosure were evaluated for their pharmacokinetic properties *in vivo* in SD rats.

### Experimental materials:

| **Material** | **Supplier** | **Catalog number** |
|---|---|---|
| SD rats (male, 150 to 400 g, 6 to 9 weeks old) | Beijing Vital River Laboratory Animal Technology Co., Ltd. | R-LOT20230032 |
| EDTA2K anticoagulant tube | Jiangsu Kangjian Medical Apparatus Co., Ltd. | 20020301 |

### Experimental procedure:

The compounds were tested for their pharmacokinetic characteristics in rodents after intravenous injection and oral administration following standard protocols. In the experiment, the candidate compound was prepared into a clear solution or suspension with a specified vehicle, and administered to three rats via single-dose intravenous injection and oral administration, respectively. The vehicle for intravenous injection was 5% dimethyl sulfoxide + 95% (10% polyethylene glycol (15)-hydroxystearate in water). The vehicle for oral administration was 5% dimethyl sulfoxide + 95% (10% polyethylene glycol (15)-hydroxystearate in water) or 0.5% methyl cellulose + 0.2% Tween 80 + 99.3% water. Whole blood samples within 24 hours were collected into a commercial EDTA2K anticoagulant tube, followed by centrifugation to obtain the upper plasma sample, and acetonitrile solution containing internal standard was added to precipitate proteins. After centrifugation, the supernatant was mixed with an equal volume of water, and after another centrifugation, the supernatant was taken for injection. Plasma concentration was quantitatively analyzed using LCMS/MS analysis method, and pharmacokinetic parameters were calculated.

The test samples were prepared from the corresponding examples. Some of the compounds of the present disclosure have a bioavailability of 80% or more, a clearance of 16 mL/min/kg or less, a half-life (T_{1/2}) of 2 to 10 hours, and an area under the curve (AUC₀₋ₗₐₛₜ) of 5000 to 100000 hr*ng/mL. The compounds of the present disclosure exhibit good pharmacokinetic properties.

### Activity assay 4: Evaluation of pharmacokinetic properties of compounds in vivo in dogs

### Experimental purpose:

The compounds obtained in the examples of the present disclosure were evaluated for their pharmacokinetic properties *in vivo* in Beagles.

### Experimental materials:

| **Material** | **Supplier** | **Catalog number** |
|---|---|---|
| Beagles (male, 5 to 12 kg, ≥ 5 months old) | Jiangsu Masi Biotechnology Co., Ltd. | Lot_NJ_D20230206_M30F10 |
| EDTA2K anticoagulant tube | Jiangsu Kangjian Medical Apparatus Co., Ltd. | 20020301 |

### Experimental procedure:

The compounds were tested for their pharmacokinetic characteristics in Beagles after intravenous injection and oral administration following standard protocols. In the experiment, the candidate compound was prepared into a clear solution or suspension with a specified vehicle, and administered to two Beagles via single-dose intravenous injection and oral administration, respectively. The vehicle for intravenous injection was 5% dimethyl sulfoxide + 95% (10% polyethylene glycol (15)-hydroxystearate in water). The vehicle for oral administration was 0.5% methyl cellulose + 0.2% Tween 80 + 99.3% water. Whole blood samples within 24 hours were collected into a commercial EDTA2K anticoagulant tube, followed by centrifugation to obtain the upper plasma sample, and acetonitrile solution containing internal standard was added to precipitate proteins. After centrifugation, the supernatant was mixed with an equal volume of water, and after another centrifugation, the supernatant was taken for injection. Plasma concentration was quantitatively analyzed using LCMS/MS analysis method, and pharmacokinetic parameters were calculated.

The test samples were prepared from the corresponding examples. Some of the compounds of the present disclosure have a bioavailability of 80% or more, a clearance of 16 mL/min/kg or less, a half-life (T_{1/2}) of 2 to 10 hours, and an area under the curve (AUC₀₋ₗₐₛₜ) of 2000 to 20000 hr*ng/mL. The compounds of the present disclosure exhibit good pharmacokinetic properties.

### Activity assay 5: Efficacy evaluation of compounds in prepulse inhibition model in mice

### Experimental purpose:

The compounds obtained in the examples of the present disclosure were evaluated for their efficacy in a prepulse inhibition model in C57 mice.

### Experimental materials:

| **Material** | **Supplier** | **Catalog number or model number** |
|---|---|---|
| C57BL/6J mice (male, 20 to 22 g, 6 to 9 weeks old) | Zhejiang Vital River Laboratory Animal Technology Co., Ltd. | 1820230403129018 |
| Prepulse inhibition test chamber | Shanghai Xinruan Information Technology Co., Ltd. | XR-XZ208-8 |

### Experimental procedure:

Animals were randomly divided into five groups of 10 each based on body weight prior to administration, with group 1 being the vehicle control group, group 2 being the model group, and groups 3, 4, and 5 being the administration groups. The animals in groups 1 and 2 were intragastrically administered with vehicle C. The animals in group 3 were intragastrically administered with the compound of the present disclosure at a dose of 5 mg/kg. The animals in groups 4 and 5 were intragastrically administered with the compound of the present disclosure at a dose of 10 mg/kg. 30 minutes after the administration, the animals in group 1 were intraperitoneally injected with vehicle B (saline), and the animals in groups 2, 3, 4, and 5 were intraperitoneally injected with Dizocilpine (MK-801) at a dose of 0.3 mg/kg. 30 minutes after the injection, the animals were placed in a prepulse inhibition test chamber to start the experiment, and their peak of response was recorded by the instrument. First, the background noise of the prepulse inhibition test chamber parameters was set to 67 dB, and the animals were gently placed in a restraining cage and allowed to adapt for 10 minutes. Then, pre-test parameters were set: the animals were subjected to five random stimuli at 120 dB within a 5-minute period. Formal test parameters were set: at the beginning of the experiment, the animals were subjected to ten shock stimuli: five stimuli at 120 dB and five stimuli at 81+120 dB, with an average interval of 7 to 21 seconds between each stimulus by selecting a random function. The background noise of 67 dB was maintained throughout the experiment. Percentage of prepulse inhibition in animals = 1 - [(peak of response during stimulation at 81+120 dB) / peak of response during stimulation at 120 dB] × 100%.

The test samples were prepared from the corresponding examples. The percentage of prepulse inhibition in mice was observed to be approximately 40 to 80%. Some of the compounds of the present disclosure can effectively reverse the damage caused by MK-801 to the function of prepulse inhibition in mice, and exhibit good *in vivo* efficacy in the prepulse inhibition model in C57 mice.

### Activity assay 6: Efficacy evaluation of compounds in forced swimming model in mice

### Experimental purpose:

The compounds obtained in the examples of the present disclosure were evaluated for their efficacy in a forced swimming model in C57 mice.

### Experimental materials:

| **Material** | **Supplier** | **Catalog number or model number** |
|---|---|---|
| C57BL/6J mice (male, 20 to 25 g, 6 to 8 weeks old) | Zhejiang Vital River Laboratory Animal Technology Co., Ltd. | 1820230403129018 |
| Forced swimming test frame and analysis software | Vistrack | XR-VT |

### Experimental procedure:

Animals were randomly divided into seven groups of 8 each based on body weight prior to administration, with group 1 being the vehicle control group and groups 2 to 7 being the administration groups. The animals in group 1 were intragastrically administered with vehicle C. The animals in groups 2 to 4 were intragastrically administered with the compound of the present disclosure at doses of 2, 5, and 10 mg/kg respectively. The animals in groups 5 to 7 were intragastrically administered with the compound of the present disclosure at doses of 2, 5, and 10 mg/kg respectively. After administration, the animals were returned to their original feeding cages. After 1 hour, the animals were gently taken out of their feeding cages, soothed for 1 to 3 minutes until they were not stressed, and individually placed in a glass cylinder of 40 cm in height and 13 cm in diameter containing water at a depth of 26 cm and maintained at 23°C. Then, the immobility time of the animals within a 4-minute period of forced swimming was automatically recorded using analysis software.

The test samples were prepared from the corresponding examples. The immobility time of the mice was observed to be approximately 50 to 150 seconds. Some of the compounds of the present disclosure can significantly reduce the immobility time of the mice during forced swimming, and exhibit good antidepressant efficacy in the forced swimming model in C57 mice.

### Activity assay 7: Efficacy evaluation of compounds in tail suspension model in mice

### Experimental purpose:

The compounds obtained in the examples of the present disclosure were evaluated for their efficacy in a tail suspension model in C57 mice.

### Experimental materials:

| **Material** | **Supplier** | **Catalog number or model number** |
|---|---|---|
| C57BL/6J mice (male, 20 to 25 g, 6 to 8 weeks old) | Zhejiang Vital River Laboratory Animal Technology Co., Ltd. | |
| Tail suspension test frame and analysis software | Vistrack | XR-VT |

### Experimental procedure:

Animals were randomly divided into five groups of 8 each based on body weight prior to administration, with group 1 being the vehicle control group and groups 2 to 5 being the administration groups. The animals in group 1 were intragastrically administered with vehicle C. The animals in groups 2 to 4 were intragastrically administered with the compound of the present disclosure at doses of 3, 5, and 10 mg/kg. The animals in group 5 were intragastrically administered with the compound of the present disclosure at a dose of 5 mg/kg. After administration, the animals were returned to their original feeding cages. After 1 hour, the animals were gently taken out of their feeding cages, soothed for 1 to 3 minutes until they were not stressed, wrapped with medical tape around the tail tip at 1/3, and suspended in a tail suspension box. Then, the immobility time of the animals within a 4-minute period was automatically recorded using analysis software.

The test samples were prepared from the corresponding examples. The immobility time of the mice was observed to be 150 seconds or less. Some of the compounds of the present disclosure exhibit good antidepressant efficacy in the tail suspension model in C57 mice.

## Claims

1. A compound of formula I or a pharmaceutically acceptable salt thereof, 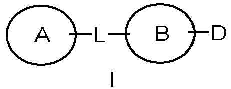 wherein 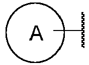 is 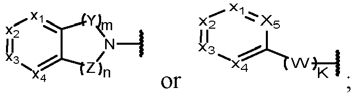
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, O, S, 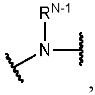 or a chemical bond;
R^{N-1} is hydrogen, C₁-C₆ alkyl, C₁-C₆ alkoxy, or 3- to 7-membered cycloalkyl, wherein the C₁-C₆ alkyl and C₁-C₆ alkoxy are optionally and independently substituted by 1, 2, 3, or 4 R^{N-2};
each R^{N-2} is independently halogen;
Y and Z are independently carbonyl (CO), -(CR²R³)ᵣ-, or a chemical bond; r is a natural number of 0 to 5;
each W is independently carbonyl (CO), -O-, -(CR⁴R⁵)-, -NR⁶-, or a chemical bond;
each R¹ is independently hydrogen, halogen, cyano, hydroxyl, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a}; or two R¹ together with the atom to which they are attached form a 3- to 7-membered cycloalkyl group or a 3- to 7-membered heterocycloalkyl group;
each R^{a} is independently halogen, cyano, hydroxyl, C₁-C₃ alkyl, C₁-C₆ alkoxy, or -NR¹⁻⁴R¹⁻⁵; or two R^{a} together with the atom to which they are attached form a 3- to 7-membered cycloalkyl group or a 3- to 7-membered heterocycloalkyl group;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently halogen, cyano, hydroxyl, C₁-C₆ alkoxy, 3- to 7-membered cycloalkyl, 3- to 7-membered heterocycloalkyl, C₁-C₃ alkyl, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently halogen, hydroxyl, or cyano;
each R^{b-2} is independently halogen, C₁-C₆ alkyl, or cyano;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl, wherein the C₁-C₃ alkyl is optionally and independently substituted by 1, 2, 3, or 4 R¹⁻¹⁻¹⁻¹;
R¹⁻⁴⁻¹ and R¹⁻¹⁻¹⁻¹ are each independently halogen, hydroxyl, or cyano;
R² and R³ are independently hydrogen, halogen, cyano, hydroxyl, C₁-C₆ alkoxy, or NR²⁻¹R²⁻²; or R² and R³ together with the atom to which they are attached form a 3- to 7-membered cycloalkyl group or a 3- to 7-membered heterocycloalkyl group;
R²⁻¹ and R²⁻² are independently hydrogen or C₁-C₆ alkyl, wherein the C₁-C₆ alkyl is optionally and independently substituted by 1, 2, 3, or 4 R^{d}; or R²⁻¹ and R²⁻² together with the atom to which they are attached form a 3- to 7-membered heterocycloalkyl group;
each R^{d} is independently halogen, cyano, hydroxyl, C₁-C₆ alkoxy, 3- to 7-membered cycloalkyl, 3- to 7-membered heterocycloalkyl, or C₁-C₃ alkyl;
R⁴, R⁵, and R⁶ are independently hydrogen, halogen, cyano, hydroxyl, C₁-C₆ alkoxy, C₁-C₆ alkyl, or NR⁴⁻¹R⁴⁻², wherein the C₁-C₆ alkyl is optionally and independently substituted by 1, 2, 3, or 4 R^{e};
each R^{e} is independently halogen, cyano, hydroxyl, C₁-C₃ alkyl, C₁-C₃ alkoxy, or -NR⁴⁻⁴R⁴⁻⁵;
R⁴⁻¹ and R⁴⁻² are independently hydrogen or C₁-C₆ alkyl, wherein the C₁-C₆ alkyl is optionally and independently substituted by 1, 2, 3, or 4 R^{f}; or R⁴⁻¹ and R⁴⁻² together with the atom to which they are attached form a 3- to 7-membered heterocycloalkyl group;
each R^{f} is independently halogen, cyano, hydroxyl, 3- to 7-membered cycloalkyl, 3- to 7-membered heterocycloalkyl, C₁-C₃ alkyl, C₁-C₃ alkoxy, or -NR⁴⁻¹⁻¹R⁴⁻²⁻¹;
R⁴⁻⁴ and R⁴⁻⁵ are independently hydrogen or C₁-C₃ alkyl; or R⁴ and R⁵ together with the atom to which they are attached form a 3- to 7-membered cycloalkyl group or a 3- to 7-membered heterocycloalkyl group;
R⁴⁻¹⁻¹ and R⁴⁻²⁻ are independently hydrogen or C₁-C₃ alkyl;
L is 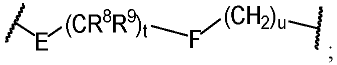
t is a natural number of 0 to 3;
u is a natural number of 0 to 3;
E is carbonyl, 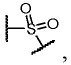 -NHCO-, or a chemical bond;
F is carbonyl, 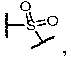 -O-, -NH-, or a chemical bond;
R⁸ and R⁹ are independently hydrogen, halogen, cyano, hydroxyl, or C₁-C₆ alkyl, wherein the C₁-C₆ alkyl is optionally and independently substituted by 1, 2, 3, or 4 R⁸; or R⁸ and R⁹ together with the atom to which they are attached form a 3- to 7-membered cycloalkyl group or a 3- to 7-membered heterocycloalkyl group;
each R^{g} is independently halogen, cyano, hydroxyl, C₁-C₃ alkyl, or C₁-C₃ alkoxy;
B is 3- to 7-membered cycloalkyl, 4- to 6-membered heterocycloalkyl, 6- to 10-membered aryl, or 5- to 12-membered heteroaryl; wherein the 3- to 7-membered cycloalkyl, 4- to 6-membered heterocycloalkyl, 6- to 10-membered aryl, and 5- to 12-membered heteroaryl are optionally and independently substituted by 1, 2, or 3 Rⁱ;
each Rⁱ is independently hydrogen, halogen, hydroxyl, C₁-C₃ alkyl, 3- to 7-membered cycloalkyl, 3- to 7-membered heterocycloalkyl, cyano, -NR¹¹⁻¹R¹¹⁻², -OR¹¹⁻³, or -SR¹¹⁻⁴; wherein the C₁-C₃ alkyl is optionally and independently substituted by 1, 2, 3 or 4 Rⁱ⁻¹, or two Rⁱ on the same atom together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group;
each Rⁱ⁻¹ is independently C₁-C₃ alkyl, halogen, cyano, or hydroxyl;
R¹¹⁻¹ and R¹¹⁻² are independently hydrogen, C₁-C₃ alkyl, 3- to 7-membered cycloalkyl, or 3- to 7-membered heterocycloalkyl; or R¹¹⁻¹ and R¹¹⁻² together with the atom to which they are attached form a 3- to 7-membered cycloalkyl group or a 3- to 7-membered heterocycloalkyl group;
R¹¹⁻³ and R¹¹⁻⁴ are independently C₁-C₃ alkyl, 3- to 7-membered cycloalkyl, or 3- to 7-membered heterocycloalkyl;
in R¹¹⁻¹, R¹¹⁻², R¹¹⁻³, and R¹¹⁻⁴, the C₁-C₃ alkyl, 3- to 7-membered cycloalkyl, and 3- to 7-membered heterocycloalkyl are optionally and independently substituted by 1, 2, or 3 Rⁱ⁻²;
each Rⁱ⁻² is independently C₁-C₃ alkyl, 3- to 7-membered cycloalkyl, 3- to 7-membered heterocycloalkyl, halogen, cyano, or hydroxyl;
D is hydrogen, halogen, cyano, hydroxyl, C₁-C₆ alkyl, 3- to 7-membered cycloalkyl, 3- to 7-membered heterocycloalkyl, 6- to 10-membered aryl, or 5- to 12-membered heteroaryl; wherein the C₁-C₆ alkyl, 3- to 7-membered cycloalkyl, 3- to 7-membered heterocycloalkyl, 6-to 10-membered aryl, and 5- to 12-membered heteroaryl are optionally and independently substituted by 1, 2, or 3 R^{j};
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻³, and R¹²⁻⁴ are independently hydrogen, C₁-C₃ alkyl, 3- to 7-membered cycloalkyl, or 3- to 7-membered heterocycloalkyl; wherein the C₁-C₃ alkyl, 3- to 7-membered cycloalkyl, and 3- to 7-membered heterocycloalkyl are optionally and independently substituted by 1, 2, or 3 R^{k-1}; or R¹²⁻¹ and R¹²⁻² together with the atom to which they are attached form a 3- to 7-membered heterocycloalkyl group;
each R^{k} is independently halogen, cyano, hydroxyl, C₁-C₃ alkyl, 3- to 7-membered cycloalkyl, or 3- to 7-membered heterocycloalkyl;
each R^{k-1} is independently halogen, cyano, hydroxyl, C₁-C₃ alkyl, 3- to 7-membered cycloalkyl, or 3- to 7-membered heterocycloalkyl;
when A is 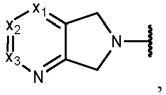
and X₁, X₂, and X₃ are CR¹, then the compound of formula I satisfies any one of the following conditions:
(1) E is -NHCO- or a chemical bond, and F is -O-, -NH-, or a chemical bond,
(2) E is carbonyl, and F is -NH-,
(3) L is -(CH₂)-, -(CH₂)₂-, 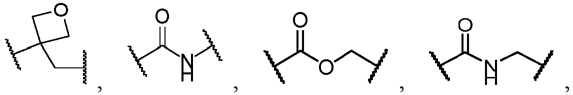 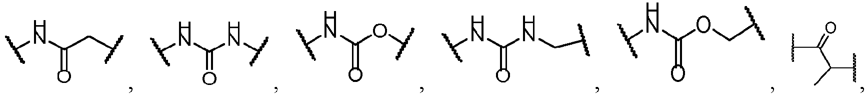 or 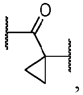
(4) when E or F is carbonyl, B is 4- to 7-membered cycloalkyl, 6- to 10-membered aryl, 5- to 12-membered heteroaryl, or 4- to 6-membered heterocycloalkyl substituted by 1, 2, or 3 Rᵢ, wherein two Rᵢ on the same atom together with the atom to which they are attached form a 3- to 7-membered cycloalkyl group or a 3- to 7-membered heterocycloalkyl group; or, two adjacent Rᵢ together with the atom to which they are attached form a 3- to 7-membered cycloalkyl group or a 3- to 7-membered heterocycloalkyl group (wherein the 3- to 7-membered cycloalkyl group or the 3- to 7-membered heterocycloalkyl group forms a fused ring with B); wherein the 4- to 7-membered cycloalkyl, 6- to 10-membered aryl, and 5- to 12-membered heteroaryl are optionally and independently substituted by 1, 2 or 3 Rᵢ,
(5) 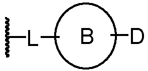 is 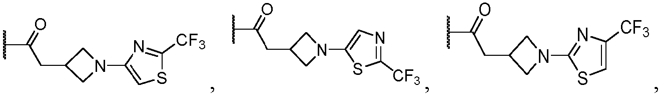 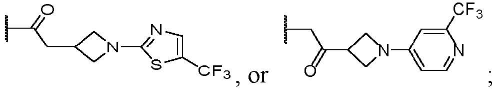
when A is 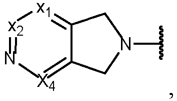 the number of heteroatoms in X₁, X₂, and X₄ is 1 or 2;
when A is 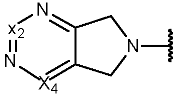
and L is carbonyl, then u is a natural number of 1 to 3, B is 4- to 6-membered heterocycloalkyl, and the 4- to 6-membered heterocycloalkyl is optionally and independently substituted by 1, 2, or 3 Rⁱ;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 5.

2. The compound of formula I or the pharmaceutically acceptable salt thereof according to claim 1, wherein 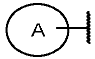 is 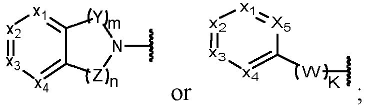
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, O, S, 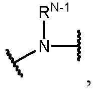 or a chemical bond;
R^{N-1} is hydrogen, C₁-C₆ alkyl, C₁-C₆ alkoxy, or 3- to 7-membered cycloalkyl, wherein the C₁-C₆ alkyl and C₁-C₆ alkoxy are optionally and independently substituted by 1, 2, 3, or 4 R^{N-2};
each R^{N-2} is independently halogen;
Y and Z are independently carbonyl (CO), -(CR²R³)ᵣ-, or a chemical bond; r is a natural number of 0 to 5;
each W is independently carbonyl (CO), -O-, -(CR⁴R⁵)-, -NR⁶-, or a chemical bond;
each R¹ is independently hydrogen, halogen, cyano, hydroxyl, C₁-C₆ alkyl, C₁-C₆ alkoxy, 3- to 7-membered cycloalkyl, 3- to 7-membered heterocycloalkyl, or -NR¹⁻¹R¹⁻², wherein the C₁-C₆ alkyl and C₁-C₆ alkoxy are optionally and independently substituted by 1, 2, 3, or 4 R^{a}; or two R¹ together with the atom to which they are attached form a 3- to 7-membered cycloalkyl group or a 3- to 7-membered heterocycloalkyl group;
each R^{a} is independently halogen, cyano, hydroxyl, C₁-C₃ alkyl, C₁-C₆ alkoxy, or -NR¹⁻⁴R¹⁻⁵; or two R^{a} together with the atom to which they are attached form a 3- to 7-membered cycloalkyl group or a 3- to 7-membered heterocycloalkyl group;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently halogen, cyano, hydroxyl, C₁-C₆ alkoxy, 3- to 7-membered cycloalkyl, 3- to 7-membered heterocycloalkyl, C₁-C₃ alkyl, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently halogen, hydroxyl, or cyano;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl, wherein the C₁-C₃ alkyl is optionally and independently substituted by 1, 2, 3, or 4 R¹⁻¹⁻¹⁻¹;
R¹⁻⁴⁻¹ and R¹⁻¹⁻¹⁻¹ are each independently halogen, hydroxyl, or cyano;
R² and R³ are independently hydrogen, halogen, cyano, hydroxyl, C₁-C₆ alkoxy, or NR²⁻¹R²⁻²; or R² and R³ together with the atom to which they are attached form a 3- to 7-membered cycloalkyl group or a 3- to 7-membered heterocycloalkyl group;
R²⁻¹ and R²⁻² are independently hydrogen or C₁-C₆ alkyl, wherein the C₁-C₆ alkyl is optionally and independently substituted by 1, 2, 3, or 4 R^{d}; or R²⁻¹ and R²⁻² together with the atom to which they are attached form a 3- to 7-membered heterocycloalkyl group;
each R^{d} is independently halogen, cyano, hydroxyl, C₁-C₆ alkoxy, 3- to 7-membered cycloalkyl, 3- to 7-membered heterocycloalkyl, or C₁-C₃ alkyl;
R⁴, R⁵, and R⁶ are independently hydrogen, halogen, cyano, hydroxyl, C₁-C₆ alkoxy, C₁-C₆ alkyl, or NR⁴⁻¹R⁴⁻², wherein the C₁-C₆ alkyl is optionally and independently substituted by 1, 2, 3, or 4 R^{e};
each R^{e} is independently halogen, cyano, hydroxyl, C₁-C₃ alkyl, C₁-C₃ alkoxy, or -NR⁴⁻⁴R⁴⁻⁵;
R⁴⁻¹ and R⁴⁻² are independently hydrogen or C₁-C₆ alkyl, wherein the C₁-C₆ alkyl is optionally and independently substituted by 1, 2, 3, or 4 R^{f}; or R⁴⁻¹ and R⁴⁻² together with the atom to which they are attached form a 3- to 7-membered heterocycloalkyl group;
each R^{f} is independently halogen, cyano, hydroxyl, 3- to 7-membered cycloalkyl, 3- to 7-membered heterocycloalkyl, C₁-C₃ alkyl, C₁-C₃ alkoxy, or -NR⁴⁻¹⁻¹R⁴⁻²⁻¹;
R⁴⁻⁴ and R⁴⁻⁵ are independently hydrogen or C₁-C₃ alkyl; or R⁴ and R⁵ together with the atom to which they are attached form a 3- to 7-membered cycloalkyl group or a 3- to 7-membered heterocycloalkyl group;
R⁴⁻¹⁻¹ and R⁴⁻²⁻ are independently hydrogen or C₁-C₃ alkyl;
L is 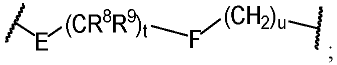
t is a natural number of 0 to 3;
u is a natural number of 0 to 3;
E is carbonyl, -NHCO-, or a chemical bond;
F is carbonyl, -O-, -NH-, or a chemical bond;
R⁸ and R⁹ are independently hydrogen, halogen, cyano, hydroxyl, or C₁-C₆ alkyl, wherein the C₁-C₆ alkyl is optionally and independently substituted by 1, 2, 3, or 4 R^{g}; or R⁸ and R⁹ together with the atom to which they are attached form a 3- to 7-membered cycloalkyl group or a 3- to 7-membered heterocycloalkyl group;
each R^{g} is independently halogen, cyano, hydroxyl, C₁-C₃ alkyl, or C₁-C₃ alkoxy;
B is 3- to 7-membered cycloalkyl, 4- to 6-membered heterocycloalkyl, 6- to 10-membered aryl, or 5- to 12-membered heteroaryl; wherein the 3- to 7-membered cycloalkyl, 4- to 6-membered heterocycloalkyl, 6- to 10-membered aryl, and 5- to 12-membered heteroaryl are optionally and independently substituted by 1, 2, or 3 Rⁱ;
each Rⁱ is independently hydrogen, halogen, hydroxyl, C₁-C₃ alkyl, 3- to 7-membered cycloalkyl, 3- to 7-membered heterocycloalkyl, cyano, -NR¹¹⁻¹R¹¹⁻², -OR¹¹⁻³, or -SR¹¹⁻⁴; wherein the C₁-C₃ alkyl is optionally and independently substituted by 1, 2, 3 or 4 Rⁱ⁻¹, or two Rⁱ on the same atom together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group;
each Rⁱ⁻¹ is independently C₁-C₃ alkyl, halogen, cyano, or hydroxyl;
R¹¹⁻¹ and R¹¹⁻² are independently hydrogen, C₁-C₃ alkyl, 3- to 7-membered cycloalkyl, or 3- to 7-membered heterocycloalkyl; or R¹¹⁻¹ and R¹¹⁻² together with the atom to which they are attached form a 3- to 7-membered cycloalkyl group or a 3- to 7-membered heterocycloalkyl group;
R¹¹⁻³ and R¹¹⁻⁴ are independently C₁-C₃ alkyl, 3- to 7-membered cycloalkyl, or 3- to 7-membered heterocycloalkyl;
in R¹¹⁻¹, R¹¹⁻², R¹¹⁻³, and R¹¹⁻⁴, the C₁-C₃ alkyl, 3- to 7-membered cycloalkyl, and 3- to 7-membered heterocycloalkyl are optionally and independently substituted by 1, 2, or 3 Rⁱ⁻²;
each Rⁱ⁻² is independently C₁-C₃ alkyl, 3- to 7-membered cycloalkyl, 3- to 7-membered heterocycloalkyl, halogen, cyano, or hydroxyl;
D is hydrogen, halogen, cyano, hydroxyl, C₁-C₆ alkyl, 3- to 7-membered cycloalkyl, 3- to 7-membered heterocycloalkyl, 6- to 10-membered aryl, or 5- to 12-membered heteroaryl; wherein the C₁-C₆ alkyl, 3- to 7-membered cycloalkyl, 3- to 7-membered heterocycloalkyl, 6-to 10-membered aryl, and 5- to 12-membered heteroaryl are optionally and independently substituted by 1, 2, or 3 R^{j};
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻³, and R¹²⁻⁴ are independently hydrogen, C₁-C₃ alkyl, 3- to 7-membered cycloalkyl, or 3- to 7-membered heterocycloalkyl; wherein the C₁-C₃ alkyl, 3- to 7-membered cycloalkyl, and 3- to 7-membered heterocycloalkyl are optionally and independently substituted by 1, 2, or 3 R^{k-1}; or R¹²⁻¹ and R¹²⁻² together with the atom to which they are attached form a 3- to 7-membered heterocycloalkyl group;
each R^{k} is independently halogen, cyano, hydroxyl, C₁-C₃ alkyl, 3- to 7-membered cycloalkyl, or 3- to 7-membered heterocycloalkyl;
each R^{k-1} is independently halogen, cyano, hydroxyl, C₁-C₃ alkyl, 3- to 7-membered cycloalkyl, or 3- to 7-membered heterocycloalkyl;
when A is 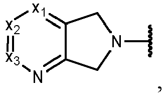 and X₁, X₂, and X₃ are CR¹, then the compound of formula I satisfies any one of the following conditions:
(1) E is -NHCO- or a chemical bond, and F is -O-, -NH-, or a chemical bond,
(2) E is carbonyl, and F is -NH-,
(3) L is -(CH₂)-, -(CH₂)₂-, 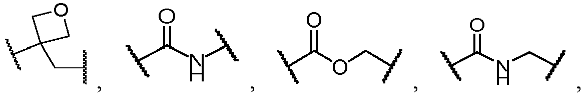 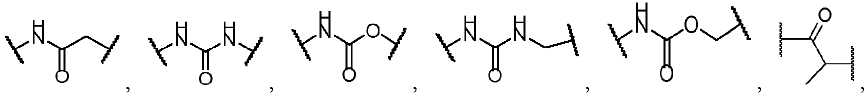 or 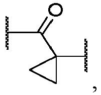
(4) when E or F is carbonyl, B is 4- to 7-membered cycloalkyl, 6- to 10-membered aryl, 5- to 12-membered heteroaryl, or 4- to 6-membered heterocycloalkyl substituted by 1, 2, or 3 Rᵢ, wherein two Rᵢ on the same atom together with the atom to which they are attached form a 3- to 7-membered cycloalkyl group or a 3- to 7-membered heterocycloalkyl group; or, two adjacent Rᵢ form a 3- to 7-membered cycloalkyl group or a 3- to 7-membered heterocycloalkyl group; wherein the 4- to 7-membered cycloalkyl, 6- to 10-membered aryl, and 5- to 12-membered heteroaryl are optionally and independently substituted by 1, 2 or 3 Rᵢ,
(5) 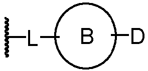 is 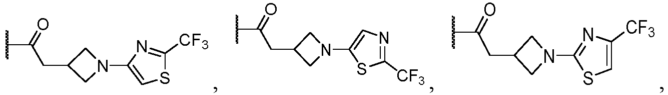 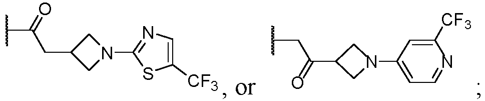
when A is 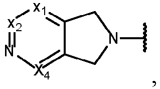 the number of heteroatoms in X₁, X₂, and X₄ is 1 or 2;
when A is 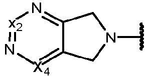 and L is carbonyl, then u is a natural number of 1 to 3, B is 4-to 6-membered heterocycloalkyl, and the 4- to 6-membered heterocycloalkyl is optionally and independently substituted by 1, 2, or 3 Rⁱ;
the heteroatom in the 3- to 7-membered heterocycloalkyl, 4- to 6-membered heterocycloalkyl, and 5- to 12-membered heteroaryl is N, O, or S, and the number of heteroatoms is 1 to 5; or, the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 5.

3. The compound of formula I or the pharmaceutically acceptable salt thereof according to claim 1 or 2, which satisfies one or more of the following conditions:
(1) in R^{N-1}, the C₁-C₆ alkyl is C₁-C₃ alkyl, for example, methyl, ethyl, *n*-propyl, or isopropyl; for another example, methyl, ethyl, or isopropyl;
(2) in R^{N-1}, the C₁-C₆ alkoxy is C₁-C₃ alkoxy, for example, methoxy, ethoxy, *n*-propoxy, or isopropoxy;
(3) in R^{N-1}, the 3- to 7-membered cycloalkyl is 3- to 6-membered cycloalkyl, for example, cyclopropyl, cyclobutyl, or cyclopentyl;
(4) in R^{N-2}, the halogen is F, Cl, Br, or I, for example, F or Cl;
(5) in R¹, the halogen is F, Cl, Br, or I, for example, F or Cl; for another example, Cl;
(6) in R¹, the C₁-C₆ alkyl is C₁-C₃ alkyl, for example, methyl, ethyl, n-propyl, or isopropyl; for another example, methyl, ethyl, or isopropyl;
(7) in R¹, the C₁-C₆ alkoxy is C₁-C₃ alkoxy, for example, methoxy, ethoxy, n-propoxy, or isopropoxy; for another example, methoxy or ethoxy;
(8) in R¹, the 3- to 7-membered cycloalkyl is 3- to 6-membered cycloalkyl, for example, cyclopropyl, cyclobutyl, or cyclopentyl; for another example, cyclopropyl;
(9) in R¹, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, for example, 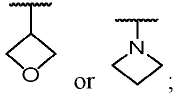
(10) in R^{a}, the halogen is F, Cl, Br, or I, for example, F or Cl; for another example, F;
(11) in R^{a}, the C₁-C₃ alkyl is methyl, ethyl, n-propyl, or isopropyl;
(12) in R^{a}, the C₁-C₆ alkoxy is C₁-C₃ alkoxy, for example, methoxy, ethoxy, n-propoxy, or isopropoxy; for another example, methoxy;
(13) in R^{a}, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, for example, 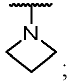
(14) in R^{a}, the 3- to 7-membered cycloalkyl is 3- to 6-membered cycloalkyl, for example, cyclopropyl, cyclobutyl, or cyclopentyl;
(15) in R¹⁻¹ and R¹⁻², the C₁-C₆ alkyl is C₁-C₃ alkyl, for example, methyl, ethyl, n-propyl, or isopropyl; for another example, methyl or ethyl, preferably methyl;
(16) in R¹⁻¹ and R¹⁻², the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is, for example, 4-membered heterocycloalkyl; for another example, 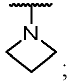
(17) in R^{b}, the halogen is F, Cl, Br, or I, for example, F or Cl;
(18) in R^{b}, the C₁-C₆ alkoxy is C₁-C₃ alkoxy, for example, methoxy, ethoxy, n-propoxy, or isopropoxy;
(19) in R^{b}, the 3- to 7-membered cycloalkyl is 3- to 6-membered cycloalkyl, for example, cyclopropyl, cyclobutyl, or cyclopentyl; for another example, cyclopropyl;
(20) in R^{b}, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(21) in R^{b}, the C₁-C₃ alkyl is preferably methyl, ethyl, *n*-propyl, or isopropyl, for example, methyl;
(22) in R^{b-1}, the halogen is F, Cl, Br, or I, for example, F or Cl;
(23) in R¹⁻⁴ and R¹⁻⁵, the C₁-C₃ alkyl is methyl, ethyl, *n*-propyl, or isopropyl, for example, methyl, ethyl, or *n*-propyl; for another example, methyl;
(24) in R¹⁻⁴ and R¹⁻⁵, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, for example, 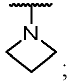
(25) in R¹⁻¹⁻¹ and R¹⁻²⁻¹, the C₁-C₃ alkyl is methyl, ethyl, *n*-propyl, or isopropyl;
(26) in R¹⁻⁴⁻¹ and R¹⁻¹⁻¹⁻¹, the halogen is F, Cl, Br, or I, for example, F or Cl; for another example, F;
(27) in R² and R³, the halogen is F, Cl, Br, or I, for example, F or Cl;
(28) in R² and R³, the C₁-C₆ alkoxy is C₁-C₃ alkoxy, for example, methoxy, ethoxy, *n-*propoxy, or isopropoxy;
(29) in R² and R³, the 3- to 7-membered cycloalkyl is 3- to 6-membered cycloalkyl, for example, cyclopropyl, cyclobutyl, or cyclopentyl;
(30) in R² and R³, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(31) in R²⁻¹ and R²⁻², the C₁-C₆ alkyl is C₁-C₃ alkyl, for example, methyl, ethyl, *n*-propyl, or isopropyl; for another example, methyl or ethyl;
(32) in R²⁻¹ and R²⁻², the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(33) in R^{d}, the halogen is F, Cl, Br, or I, for example, F or Cl;
(34) in R^{d}, the C₁-C₆ alkoxy is C₁-C₃ alkoxy, for example, methoxy, ethoxy, n-propoxy, or isopropoxy;
(35) in R^{d}, the 3- to 7-membered cycloalkyl is 3- to 6-membered cycloalkyl, for example, cyclopropyl, cyclobutyl, or cyclopentyl;
(36) in R^{d}, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(37) in R^{d}, the C₁-C₃ alkyl is methyl, ethyl, *n*-propyl, or isopropyl;
(38) in R⁴, R⁵, and R⁶, the halogen is F, Cl, Br, or I, for example, F or Cl;
(39) in R⁴, R⁵, and R⁶, the C₁-C₆ alkoxy is C₁-C₃ alkoxy, for example, methoxy, ethoxy, *n*-propoxy, or isopropoxy;
(40) in R⁴, R⁵, and R⁶, the C₁-C₆ alkyl is C₁-C₃ alkyl, for example, methyl, ethyl, n-propyl, or isopropyl;
(41) in R^{e}, the halogen is F, Cl, Br, or I, for example, F or Cl;
(42) in R^{e}, the C₁-C₃ alkyl is methyl, ethyl, n-propyl, or isopropyl;
(43) in R^{e}, the C₁-C₃ alkoxy is methoxy, ethoxy, *n*-propoxy, or isopropoxy;
(44) in R⁴⁻¹ and R⁴⁻², the C₁-C₆ alkyl is C₁-C₃ alkyl, for example, methyl, ethyl, *n*-propyl, or isopropyl;
(45) in R⁴⁻¹ and R⁴⁻², the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(46) in R^{f}, the halogen is F, Cl, Br, or I, for example, F or Cl;
(47) in R^{f}, the 3- to 7-membered cycloalkyl is 3- to 6-membered cycloalkyl, for example, cyclopropyl, cyclobutyl, or cyclopentyl;
(48) in R^{f}, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(49) in R^{f}, the C₁-C₃ alkyl is methyl, ethyl, *n*-propyl, or isopropyl;
(50) in R^{f}, the C₁-C₃ alkoxy is methoxy, ethoxy, *n*-propoxy, or isopropoxy;
(51) in R⁴⁻⁴ and R⁴⁻⁵, the C₁-C₃ alkyl is methyl, ethyl, *n*-propyl, or isopropyl;
(52) t is a natural number of 0 to 3, for example, 0, 1, 2, or 3; for another example, 0, 1, or 2;
(53) u is a natural number of 0 to 3, for example, 0, 1, 2, or 3; for another example, 0, 1, or 2;
(54) in R⁸ and R⁹, the halogen is F, Cl, Br, or I, for example, F or Cl;
(55) in R⁸ and R⁹, the C₁-C₆ alkyl is C₁-C₃ alkyl, for example, methyl, ethyl, *n*-propyl, or isopropyl; for another example, methyl;
(56) in R⁸ and R⁹, the 3- to 7-membered cycloalkyl is 3- to 6-membered cycloalkyl, for example, cyclopropyl, cyclobutyl, or cyclopentyl; for another example, cyclopropyl;
(57) in R⁸ and R⁹, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is, for example, 4-membered heterocycloalkyl; for another example, oxetanyl;
(58) in R^{g}, the halogen is F, Cl, Br, or I, for example, F or Cl;
(59) in R^{g}, the C₁-C₃ alkyl is methyl, ethyl, *n*-propyl, or isopropyl, for example, methyl;
(60) in R^{g}, the C₁-C₃ alkoxy is methoxy, ethoxy, *n*-propoxy, or isopropoxy;
(61) in B, the 3- to 7-membered cycloalkyl is 3- to 6-membered cycloalkyl, for example, cyclopropyl, cyclobutyl, or cyclopentyl;
(62) in B, the 4- to 7-membered cycloalkyl is 3- to 6-membered cycloalkyl, for example, cyclobutyl or cyclopentyl;
(63) in B, the 4- to 6-membered heterocycloalkyl is 4-membered heterocycloalkyl, 5-membered heterocycloalkyl, or 6-membered heterocycloalkyl; the heteroatom in the 4-membered heterocycloalkyl, 5-membered heterocycloalkyl, and 6-membered heterocycloalkyl is preferably N; the number of heteroatoms in the 4- to 6-membered heterocycloalkyl is preferably 1 or 2; for example, the 4- to 6-membered heterocycloalkyl is 4-membered azacycloalkyl or 5-membered azacycloalkyl; the 4-membered azacycloalkyl may be 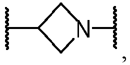 and the 5-membered azacycloalkyl may be 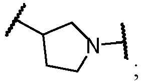
(64) in B, the 6- to 10-membered aryl is phenyl or naphthyl, for example, phenyl;
(65) in Rⁱ, the halogen is F, Cl, Br, or I, for example, F or Cl; for another example, F;
(66) in Rⁱ, the C₁-C₃ alkyl is methyl, ethyl, *n*-propyl, or isopropyl, for example, methyl;
(67) in Rⁱ, the 3- to 7-membered cycloalkyl is 3- to 6-membered cycloalkyl, for example, cyclopropyl, cyclobutyl, or cyclopentyl; for another example, cyclopropyl or cyclobutyl, for example, cyclopropyl;
(68) in Rⁱ, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(69) in Rⁱ⁻¹, the halogen is F, Cl, Br, or I, for example, F or Cl;
(70) in Rⁱ⁻¹, the C₁-C₃ alkyl is ethyl, n-propyl, or isopropyl;
(71) in R¹¹⁻¹, R¹¹⁻², R¹¹⁻³, and R¹¹⁻⁴, the C₁-C₃ alkyl is methyl, ethyl, n-propyl, or isopropyl;
(72) in R¹¹⁻³ and R¹¹⁻⁴, the 3- to 7-membered cycloalkyl is 3- to 6-membered cycloalkyl, for example, cyclopropyl, cyclobutyl, or cyclopentyl;
(73) in R¹¹⁻³ and R¹¹⁻⁴, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(74) in R¹¹⁻¹ and R¹¹⁻², the 3- to 7-membered cycloalkyl is 3- to 6-membered cycloalkyl, for example, cyclopropyl, cyclobutyl, or cyclopentyl;
(75) in R¹¹⁻¹ and R¹¹⁻², the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(76) in Rⁱ⁻², the halogen is F, Cl, Br, or I, for example, F or Cl;
(77) in Rⁱ⁻², the C₁-C₃ alkyl is methyl, ethyl, *n*-propyl, or isopropyl;
(78) in Rⁱ⁻², the 3- to 7-membered cycloalkyl is 3- to 6-membered cycloalkyl, for example, cyclopropyl, cyclobutyl, or cyclopentyl;
(79) in Rⁱ⁻², the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(80) in D, the halogen is F, Cl, Br, or I, for example, F or Cl;
(81) in D, the C₁-C₆ alkyl is C₁-C₃ alkyl, for example, methyl, ethyl, n-propyl, or isopropyl; for another example, methyl;
(82) in D, the 3- to 7-membered cycloalkyl is 3- to 6-membered cycloalkyl, for example, cyclopropyl, cyclobutyl, or cyclopentyl; for another example, cyclopropyl;
(83) in D, the 6- to 10-membered aryl is phenyl or naphthyl;
(84) in R^{j}, the halogen is F, Cl, Br, or I, for example, F or Cl; for another example, F;
(85) in R^{j}, the C₁-C₃ alkyl is methyl, ethyl, n-propyl, or isopropyl, for example, methyl, ethyl, or isopropyl; for another example, methyl;
(86) in R^{j}, the 3- to 7-membered cycloalkyl is 3- to 6-membered cycloalkyl, for example, cyclopropyl, cyclobutyl, or cyclopentyl; for another example, cyclopropyl;
(87) in R^{j}, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(88) in R¹²⁻¹, R¹²⁻², R¹²⁻³, and R¹²⁻⁴, the C₁-C₃ alkyl is methyl, ethyl, *n*-propyl, or isopropyl, for example, methyl or ethyl;
(89) in R¹²⁻¹, R¹²⁻², R¹²⁻³, and R¹²⁻⁴, the 3- to 7-membered cycloalkyl is 3- to 6-membered cycloalkyl, for example, cyclopropyl, cyclobutyl, or cyclopentyl;
(90) in R¹²⁻¹, R¹²⁻², R¹²⁻³, and R¹²⁻⁴, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(91) in R^{k}, the halogen is F, Cl, Br, or I, for example, F or Cl; for another example, F;
(92) in R^{k}, the C₁-C₃ alkyl is methyl, ethyl, *n*-propyl, or isopropyl;
(93) in R^{k}, the 3- to 7-membered cycloalkyl is 3- to 6-membered cycloalkyl, for example, cyclopropyl, cyclobutyl, or cyclopentyl;
(94) in R^{k}, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(95) in R^{k-1}, the halogen is F, Cl, Br, or I, for example, F or Cl; for another example, F;
(96) in R^{k-1}, the C₁-C₃ alkyl is methyl, ethyl, n-propyl, or isopropyl, for example, methyl;
(97) in R^{k-1}, the 3- to 7-membered cycloalkyl is 3- to 6-membered cycloalkyl, for example, cyclopropyl, cyclobutyl, or cyclopentyl;
(98) in R^{k-1}, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2;
(99) in R¹, the C₁-C₆ alkylthio is preferably C₁-C₃ alkylthio, for example, methylthio, ethylthio, n-propylthio, or isopropylthio; for another example, methylthio;
(100) in R^{b-2}, the halogen is preferably F, Cl, Br, or I, for example, F;
(101) in R^{b-2}, the C₁-C₆ alkyl is preferably C₁-C₃ alkyl, for example, methyl, ethyl, *n-*propyl, or isopropyl; for another example, methyl.

4. The compound of formula I or the pharmaceutically acceptable salt thereof according to claim 1 or 2, which satisfies one or more of the following conditions:
(1) m is a natural number of 0 to 3, for example, 0, 1, 2, or 3; for another example, 1, 2, or 3;
(2) n is a natural number of 1 to 3, for example, 1, 2, or 3; for another example, 1 or 2;
(3) k is a natural number of 0 to 3, for example, 0, 1, 2, or 3; for another example, 1, 2, or 3;
(4) r is a natural number of 0 to 5, for example, 0, 1, 2, 3, 4, or 5; for another example, 1 or 2;
(5) R^{N-1} is C₁-C₆ alkyl;
(6) each W is independently -(CR⁴R⁵)-, -O-, -NR⁶-, or a chemical bond;
(7) Y and Z are independently carbonyl (CO) or -(CR²R³)ᵣ-;
(8) each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²;
(9) each R^{a} is independently hydroxyl, C₁-C₃ alkoxy, or NR¹⁻⁴R¹⁻⁵;
(10) each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹;
(11) R^{b-1} is independently hydroxyl;
(12) R¹⁻⁴⁻¹ is halogen;
(13) R² and R³ are independently hydrogen or NR²⁻¹R²⁻²; preferably, R² and R³are independently hydrogen;
(14) R⁴, R⁵, and R⁶ are independently hydrogen;
(15) R^{e} is -NR⁴⁻⁴R⁴⁻⁵;
(16) R⁴⁻⁴ and R⁴⁻⁵ are independently hydrogen or C₁-C₃ alkyl;
(17) E is carbonyl or a chemical bond;
(18) F is -NH-, -O-, or a chemical bond;
(19) R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group; preferably, R⁸ and R⁹ are independently C₁-C₆ alkyl; or R⁸ and R⁹ together with the atom to which they are attached form a 3- to 7-membered cycloalkyl group or a 3- to 7-membered heterocycloalkyl group;
(20) B is 4- to 6-membered heterocycloalkyl, 6- to 10-membered aryl, or 5- to 12-membered heteroaryl, wherein the 5- to 12-membered heteroaryl, 6- to 10-membered aryl, and 4- to 6-membered heterocycloalkyl are optionally and independently substituted by 1, 2, or 3 Rⁱ; preferably, B is 4- to 6-membered heterocycloalkyl or 5- to 12-membered heteroaryl, wherein the 5- to 12-membered heteroaryl and 4- to 6-membered heterocycloalkyl are optionally and independently substituted by 1, 2, or 3 Rⁱ;
(21) Rⁱ is hydrogen, halogen, hydroxyl, or C₁-C₃ alkyl; or two Rⁱ together with the atom to which they are attached form a 3- to 7-membered cycloalkyl group or a 3- to 7-membered heterocycloalkyl group;
(22) D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j}; or, D is hydrogen, 3- to 7-membered cycloalkyl, C₁-C₆ alkyl, or 5- to 6-membered heteroaryl; wherein the C₁-C₆ alkyl and 5- to 6-membered heteroaryl are optionally and independently substituted by 1, 2, or 3 R^{j};
(23) R^{j} is halogen, C₁-C₃ alkyl, OR¹²⁻³, or SR¹²⁻⁴; preferably, R^{j} is halogen or C₁-C₃ alkyl;
(24) R¹²⁻³ and R¹²⁻⁴ are independently C₁-C₃ alkyl;
(25) R^{k} is independently halogen;
(26) R^{k-1} is independently halogen;
(27) the heteroatom in the 3- to 7-membered heterocycloalkyl, 5- to 6-membered heteroaryl, 4- to 6-membered heterocycloalkyl, and 5- to 12-membered heteroaryl is N, O, or S, and the number of heteroatoms is 1 to 3.

5. The compound of formula I or the pharmaceutically acceptable salt thereof according to claim 1 or 2, which satisfies one or more of the following conditions:
(1) X₁ and X₃ are independently N; X₄ and X₂ are independently -CR¹-; preferably, when X₁ and X₃ are independently N; and X₄ and X₂ are independently -CR¹-, then each R¹ is independently C₁-C₆ alkyl, C₁-C₆ alkoxy, C₁-C₆ alkylthio, 3- to 7-membered heterocycloalkyl, or -NR¹⁻¹R¹⁻²; further preferably, each R¹ is independently C₁-C₆ alkyl or C₁-C₆ alkoxy;
or, X₂ and X₃ are independently N; X₄ and X₁ are independently -CR¹-; preferably, when X₂ and X₃ are independently N; and X₄ and X₁ are independently -CR¹-, then B is azetidinyl, and the azetidinyl is, for example, 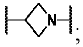 and/or, when X₂ and X₃ are independently N; and X₄ and X₁ are independently -CR¹-, then D is 6-membered heteroaryl; wherein the 6-membered heteroaryl is optionally and independently substituted by 1, 2, or 3 R^{j}; the 6-membered heteroaryl is, for example, 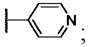 and/or, when X₂ and X₃ are independently N; and X₄ and X₁ are independently -CR¹-, then each R¹ is independently C₁-C₆ alkyl or 3- to 7-membered heterocycloalkyl;
or, X₁ and X₄ are independently N; X₂ and X₃ are independently -CR¹-; preferably, when X₂ is independently -CR¹-, R¹ is C₁-C₆ alkyl or C₁-C₆ alkoxy;
or, 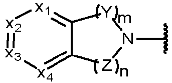 is 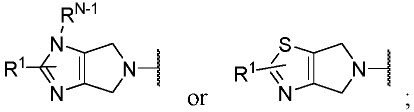 preferably, when 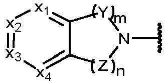 is 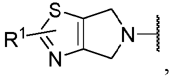 each R^{j} is independently OR¹²⁻³ or SR¹²⁻⁴;
or, X₁ is N; X₂, X₃, and X₄ are -CR¹-; Y and Z are independently -(CH₂)-; preferably, when X₁ is N; X₂, X₃, and X₄ are -CR¹-; Y and Z are independently -(CH₂)-, then m and n are 2, and R^{j} is trifluoromethyl; further preferably, R¹ is C₁-C₆ alkyl or C₁-C₆ alkoxy;
(2) R^{N-1} is hydrogen, C₁-C₆ alkyl, or 3- to 7-membered cycloalkyl;
(3) each W is independently -(CR⁴R⁵)-, -NR⁶-, or a chemical bond;
(4) each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a}; for example, each R¹ is independently hydrogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻²; for another example, C₁-C₆ alkyl, C₁-C₆ alkoxy, C₁-C₆ alkylthio, or -NR¹⁻¹R¹⁻²; preferably, each R¹ is independently C₁-C₆ alkyl; further preferably, when D is pyrimidinyl, each R¹ is independently halogen or 3- to 7-membered cycloalkyl;
(5) R¹⁻¹ and R¹⁻² are independently hydrogen or C₁-C₆ alkyl; or R¹⁻¹ and R¹⁻² together with the atom to which they are attached form a 3- to 7-membered heterocycloalkyl group; for example, R¹⁻¹ and R¹⁻² together with the atom to which they are attached form a 3- to 7-membered heterocycloalkyl group;
(6) each R^{a} is independently halogen, hydroxyl, C₁-C₆ alkoxy, or -NR¹⁻⁴R¹⁻⁵; for example, each R^{a} is independently hydroxyl, C₁-C₆ alkoxy, or -NR¹⁻⁴R¹⁻⁵; for another example, hydroxyl;
(7) each R^{b} is independently hydroxyl, 3- to 7-membered cycloalkyl, or C₁-C₃ alkyl; for example, each R^{b} is independently hydroxyl or 3- to 7-membered cycloalkyl; for another example, each R^{b} is independently 3- to 7-membered cycloalkyl;
(8) each R^{b-2} is independently halogen or C₁-C₆ alkyl;
(9) R¹⁻⁴ and R¹⁻⁵ are independently C₁-C₃ alkyl;
(10) each R¹⁻⁴⁻¹ is independently halogen;
(11) R²⁻¹ and R²⁻² are independently hydrogen or C₁-C₆ alkyl, for example, hydrogen;
(12) R⁴, R⁵, and R⁶ are independently hydrogen or NR⁴⁻¹R⁴⁻²;
(13) R⁴⁻¹ and R⁴⁻² are independently hydrogen;
(14) E is carbonyl, 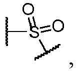 -NHCO-, or a chemical bond;
(15) F is carbonyl, 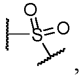 -O-, -NH-, or a chemical bond;
(16) R⁸ and R⁹ are independently hydrogen or C₁-C₆ alkyl; or R⁸ and R⁹ together with the atom to which they are attached form a 3- to 7-membered cycloalkyl group;
(17) when B is 6- to 10-membered aryl or 5- to 12-membered heteroaryl, D is hydrogen, halogen, cyano, hydroxyl, C₁-C₆ alkyl, 3- to 7-membered cycloalkyl, or 3- to 7-membered heterocycloalkyl, wherein the C₁-C₆ alkyl, 3- to 7-membered cycloalkyl, and 3- to 7-membered heterocycloalkyl are optionally and independently substituted by 1, 2, or 3 R^{j}; preferably, D is hydrogen or C₁-C₆ alkyl;
(18) each Rⁱ is independently hydrogen, halogen, hydroxyl, or C₁-C₃ alkyl; or two Rⁱ together with the atom to which they are attached form a 3- to 7-membered cycloalkyl group;
for example, Rⁱ is hydrogen or C₁-C₃ alkyl;
preferably, Rⁱ is C₁-C₃ alkyl;
(19) each R^{j} is independently hydrogen, halogen, C₁-C₃ alkyl, 3- to 7-membered cycloalkyl, -OR¹²⁻³, or -SR¹²⁻⁴; the C₁-C₃ alkyl and 3- to 7-membered cycloalkyl are optionally and independently substituted by 1, 2, or 3 R^{k};
for example, each R^{j} is independently hydrogen, halogen, C₁-C₃ alkyl, 3- to 7-membered cycloalkyl, -OR¹²⁻³, or -SR¹²⁻⁴;
for another example, each R^{j} is independently halogen, C₁-C₃ alkyl, OR¹²⁻³, or SR¹²⁻⁴;
for yet another example, each R^{j} is independently C₁-C₃ alkyl, OR¹²⁻³, or SR¹²⁻⁴, and the C₁-C₃ alkyl is optionally and independently substituted by 1, 2, or 3 R^{k}; preferably, each R^{j} is independently OR¹²⁻³ or SR¹²⁻⁴, for example, OR¹²⁻³;
further preferably, each R^{j} is independently halogen or C₁-C₃ alkyl;
further, when X₁ and X₃ are -CR¹-, and R¹ is C₁-C₆ alkyl, then each R^{j} is independently - OR¹²⁻³ or -SR¹²⁻⁴; or, when X₁ or X₄ is -CR¹-, and R¹ is independently 3- to 7-membered heterocycloalkyl or -NR¹⁻¹R¹⁻², then each R^{j} is independently OR¹²⁻³ or SR¹²⁻⁴;
(20) each R^{k} is independently halogen;
(21) each R^{k-1} is independently halogen;
(22) the heteroatom in the 3- to 7-membered heterocycloalkyl, 4- to 6-membered heterocycloalkyl, 5- to 6-membered heteroaryl, and 5- to 12-membered heteroaryl is selected from one, two, or three kinds of N, O, or S, and the number of heteroatoms is 1, 2, or 3;
(23) when A is 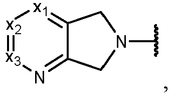 and X₁, X₂, and X₃ are CR¹, then E or F in the compound of formula I is 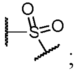
(24) R¹²⁻¹, R¹²⁻², R¹²⁻³, and R¹²⁻⁴ are independently C₁-C₃ alkyl, wherein the C₁-C₃ alkyl is optionally and independently substituted by 1, 2, or 3 R^{k-1}; for example, R¹²⁻¹, R¹²⁻², R¹²⁻³, and R¹²⁻⁴ are independently C₁-C₃ alkyl;
(25) the heteroatom in the 3- to 7-membered heterocycloalkyl, 4- to 6-membered heterocycloalkyl, 5- to 6-membered heteroaryl, and 5- to 12-membered heteroaryl is selected from one, two, or three kinds of N, O, or S, and the number of heteroatoms is 1, 2, or 3; for example, the heteroatom in the 3- to 7-membered heterocycloalkyl, 4- to 6-membered heterocycloalkyl, and 5- to 12-membered heteroaryl is N, O, or S, and the number of heteroatoms is 1 to 5;
preferably, the 3- to 7-membered heterocycloalkyl may be 3- to 6-membered heterocycloalkyl, wherein the heteroatom is, for example, one or two kinds of N and O, and the number of heteroatoms is, for example, 1 or 2;
the 4- to 6-membered heterocycloalkyl may be 4-membered heterocycloalkyl, wherein the heteroatom is, for example, one or two kinds of N and O, and the number of heteroatoms is, for example, 1;
the 5- to 6-membered heteroaryl may be 6-membered heteroaryl, wherein the heteroatom is, for example, one or two kinds of N and O, and the number of heteroatoms is, for example, 1;
the 5- to 12-membered heteroaryl may be 5- to 10-membered heterocycloalkyl, wherein the heteroatom is, for example, one or two kinds of N and O, and the number of heteroatoms is, for example, 1.

6. The compound of formula I or the pharmaceutically acceptable salt thereof according to claim 1 or 2, wherein the compound of formula I is a compound of formula I-1, a compound of formula I-2, a compound of formula I-3, a compound of formula I-4, or a compound of formula I-5; 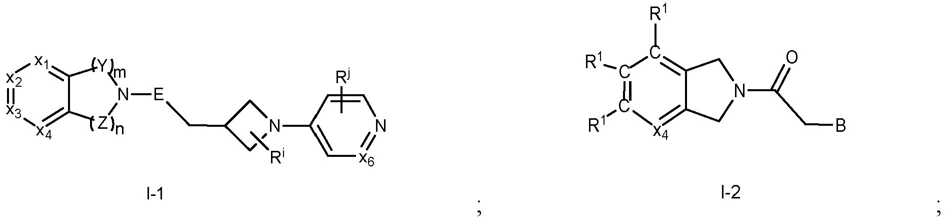 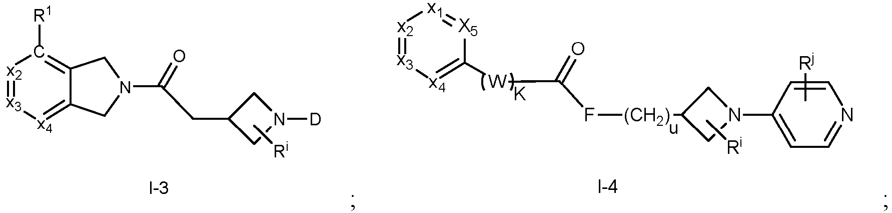 or 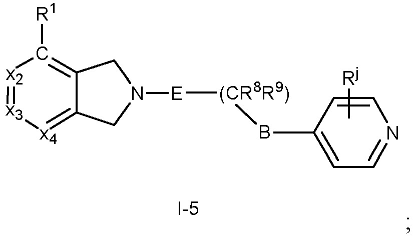 wherein X₆ is N or CH, and X₁, Rⁱ, X₂, X₃, X₄, X₅, Y, Z, m, n, E, R^{j}, R¹, B, D, W, k, F, u, R⁸, and R⁹ are as defined in claim 1 or 2.

7. The compound of formula I or the pharmaceutically acceptable salt thereof according to claim 6, wherein the compound of formula I-1 is a compound of formula I-1-1, a compound of formula I-1-2, a compound of formula I-1-3, or a compound of formula 1-1-4; 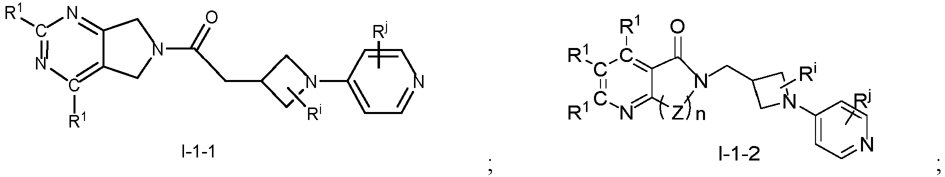 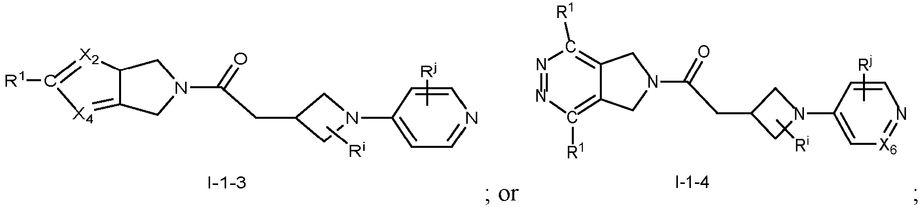 or, the compound of formula I-5 is a compound of formula I-5-1; 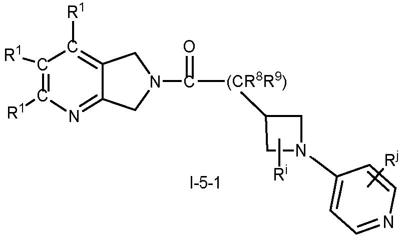 or, the compound of formula I-4 is a compound of formula I-4-1; 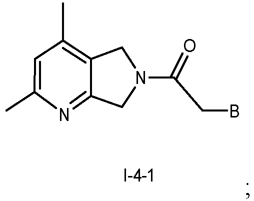 wherein R¹, R^{j}, R⁸, R⁹, B, Z, n, X₆, X₂, and X₄ are as defined in claim 6.

8. The compound of formula I or the pharmaceutically acceptable salt thereof according to claim 1 or 2, which satisfies one or more of the following conditions:
(1) X₁, X₂, X₃, X₄, and X₅ are independently -CR¹-, N, O, S, 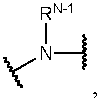 or a chemical bond, and the number of heteroatoms in X₁, X₂, X₃, X₄, and X₅ is 0, 1, 2, or 3;
(2) X₁, X₂, X₃, X₄, and X₅ are independently -CR¹-, N, S, 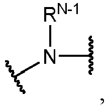 or a chemical bond, and the number of heteroatoms in X₁, X₂, X₃, X₄, and X₅ is 1 or 2;
(3) in 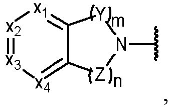 X₁, X₂, X₃, and X₄ are independently -CR¹- or N; the number of heteroatoms is 0, 1, or 2;
(4) in 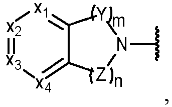 Y and Z are independently carbonyl (CO) or -(CR²R³)ᵣ-; r is 1, R² and R³ are independently H, -(CH₂)-, -(NHCH₂)-, -(NHCH₂CH₂)-, or 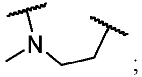
(5) in 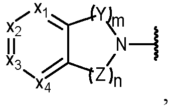 Y and Z are independently -(CR²R³)ᵣ-; m is 2, n is 2, r is 1, R² and R³ are independently H;
(6) 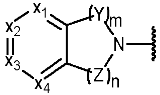 is phenyl-fused 5-membered heterocycloalkyl, wherein the heteroatom in the 5-membered heterocycloalkyl is N, and the number of heteroatoms is 1, for example, 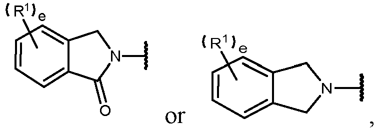 wherein e is independently 0, 1, 2, or 3; for another example, 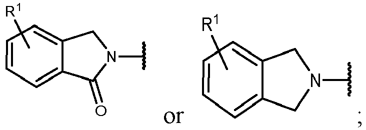
(7) 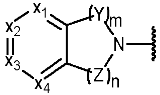 is phenyl-fused 7-membered heterocycloalkyl, wherein the heteroatom in the 7-membered heterocycloalkyl is N, and the number of heteroatoms is 1 or 2, for example, 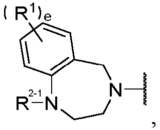 wherein e is independently 0, 1, 2, or 3; for another example, 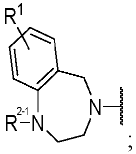
(8) 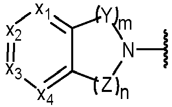 is 6-membered heteroaryl-fused 5-membered heterocycloalkyl, wherein the heteroatom in the 6-membered heteroaryl is N, and the number of heteroatoms is 1 or 2; preferably, the heteroatom in the 5-membered heterocycloalkyl is N, and the number of heteroatoms is 1, for example, 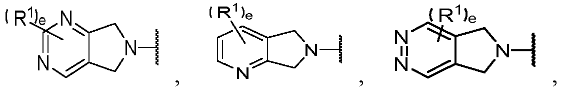 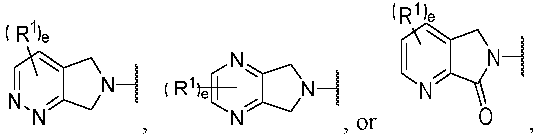 wherein e is independently 0, 1, or 2; for another example, 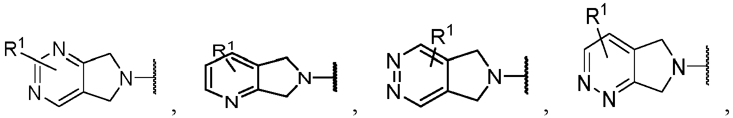 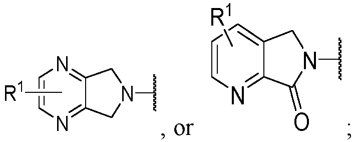
(9) 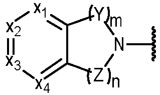 is 6-membered heteroaryl-fused 5-membered heterocycloalkyl, wherein the heteroatom in the 6-membered heteroaryl is N, and the number of heteroatoms is 1, for example, 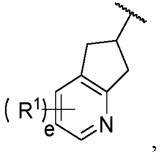 wherein e is independently 0, 1, 2, or 3; for another example, 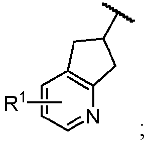
(10) 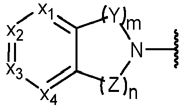 is 6-membered heteroaryl-fused 6-membered heterocycloalkyl, wherein the heteroatom in the 6-membered heteroaryl is N, and the number of heteroatoms is 1; preferably, the heteroatom in the 6-membered heterocycloalkyl is N, and the number of heteroatoms is 1, for example, 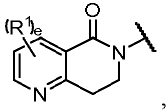 wherein e is independently 0, 1, 2, or 3; for another example, 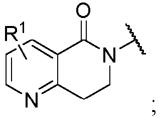
(11) 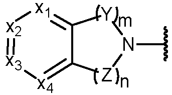 is 6-membered heteroaryl-fused 7-membered heterocycloalkyl, wherein the heteroatom in the 6-membered heteroaryl is N, and the number of heteroatoms is 1 or 2; preferably, the heteroatom in the 7-membered heterocycloalkyl is N, and the number of heteroatoms is 1, for example, 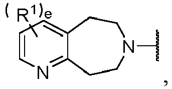 wherein e is independently 0, 1, 2, or 3; for another example, 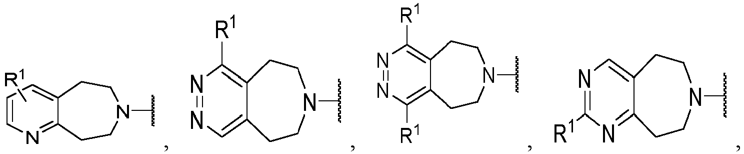 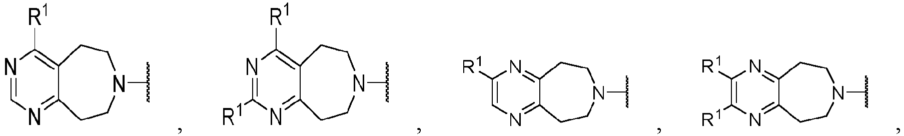 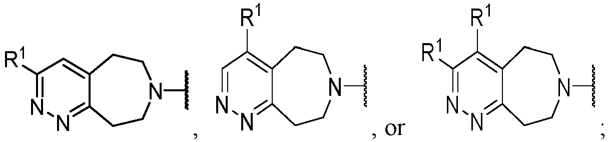
(12) 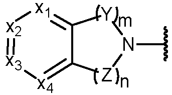 is 5-membered heteroaryl-fused 5-membered heterocycloalkyl, wherein the heteroatom in the 5-membered heteroaryl is N and/or S, and the number of heteroatoms is 1 or 2; for example, the heteroatom in the 5-membered heteroaryl is N or S, and the number of heteroatoms is 1 or 2; preferably, the heteroatom in the 5-membered heterocycloalkyl is N, and the number of heteroatoms is 1, for example, 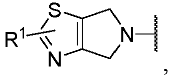 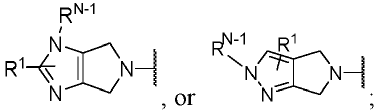
(13) 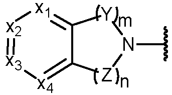 is 5-membered heteroaryl-fused 6-membered heterocycloalkyl, wherein the heteroatom in the 5-membered heteroaryl is N, and the number of heteroatoms is 1 or 2; for example, the heteroatom in the 5-membered heteroaryl is N, and the number of heteroatoms is 1; preferably, the heteroatom in the 6-membered heterocycloalkyl is N, and the number of heteroatoms is 1, for example, 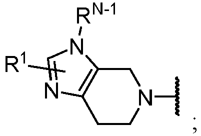 for another example, 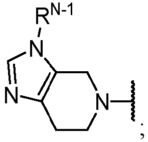
(14) in 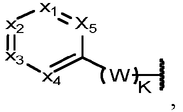 X₁, X₂, X₃, X₄, and X₅ are independently -CR¹-, N, S, 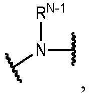 or a chemical bond, and the number of heteroatoms in X₁, X₂, X₃, X₄, and X₅ is 0, 1, or 2;
(15) 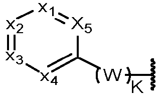 is C-(W)ₖ-, wherein C is 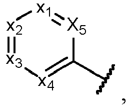 C is 5-membered heteroaryl, 6-membered heteroaryl, or phenyl; preferably, the heteroatom in the 5-membered heteroaryl is N, and the number of heteroatoms is 1 or 2, or the heteroatom in the 6-membered heteroaryl is N, and the number of heteroatoms is 1; for example, the C is pyridyl, phenyl, or 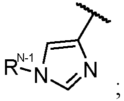
(16) in 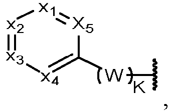 W is -O-, -CH₂-, or -NH-;
(17) in B, the 5- to 12-membered heteroaryl is 5- to 6-membered heteroaryl, phenyl-fused 5- to 6-membered heteroaryl, 5- to 7-membered cycloalkyl-fused phenyl, 5- to 7-membered cycloalkyl-fused 5- to 6-membered heteroaryl, 5- to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused 5- to 6-membered aryl; further preferably 5- to 6-membered heteroaryl, phenyl-fused 5- to 6-membered heteroaryl, 5- to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused phenyl;
(18) in B, the heteroatom in the 4- to 6-membered heterocycloalkyl is N, and the number of heteroatoms is 1, for example, 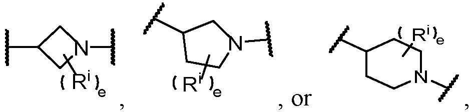 wherein e is independently 0, 1, 2, or 3; for another example, 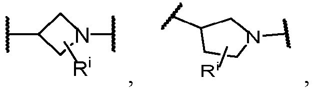 or 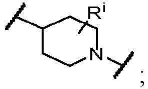
(19) B is 4- to 6-membered heterocycloalkyl substituted by two Rⁱ; two Rⁱ on the same atom together with the atom to which they are attached form a 3- to 7-membered cycloalkyl group, and the 3- to 7-membered cycloalkyl is preferably 3- to 6-membered cycloalkyl; for example, B is 4-membered heterocycloalkyl, and two Rⁱ form a 3- to 4-membered cycloalkyl group; for another example, B is 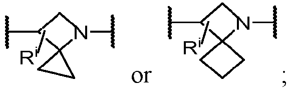
(20) B is 4- to 6-membered heterocycloalkyl substituted by two Rⁱ at adjacent positions; two adjacent Rⁱ together with the atom to which they are attached form a 3- to 7-membered cycloalkyl group, and the 3- to 7-membered cycloalkyl is preferably 3- to 6-membered cycloalkyl; for example, B is 5-membered heterocycloalkyl, and two Rⁱ form a 3-membered cycloalkyl group; for another example, B is 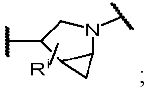
(21) B is 5-membered heteroaryl, wherein the heteroatom in the 5-membered heteroaryl is N and/or S, and the number of heteroatoms is 1 or 2; for example, the heteroatom in the 5-membered heteroaryl is N or S, and the number of heteroatoms is 1 or 2, for example, 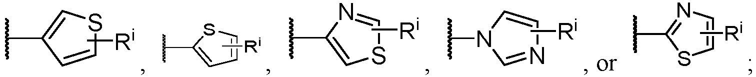
(22) B is 6-membered heteroaryl, wherein the heteroatom in the 6-membered heteroaryl is N, and the number of heteroatoms is 1 or 2, for example, 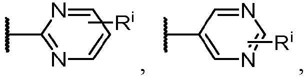 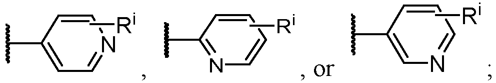
(23) in B, the 6- to 10-membered aryl is, for example, phenyl;
(24) B is phenyl-fused 5-membered heterocycloalkyl, wherein the heteroatom in the 5-membered heterocycloalkyl is N, and the number of heteroatoms is 1, for example, 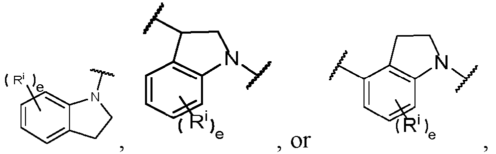 wherein e is independently 0, 1, 2, or 3; for another example, 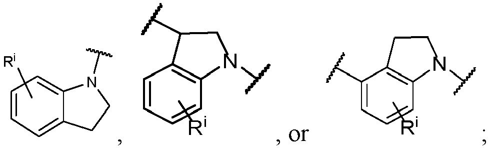
(25) B is phenyl-fused 6-membered heteroaryl, wherein the heteroatom in the 6-membered heteroaryl is N, and the number of heteroatoms is 1 or 2, for example, 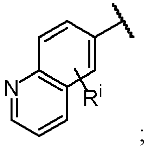
(26) B is phenyl-fused 6-membered heterocycloalkyl, wherein the heteroatom in the 6-membered heterocycloalkyl is N and/or O, and the number of heteroatoms is 1 or 2; for example, the heteroatom in the 6-membered heterocycloalkyl is N or O, and the number of heteroatoms is 1 or 2, for example, 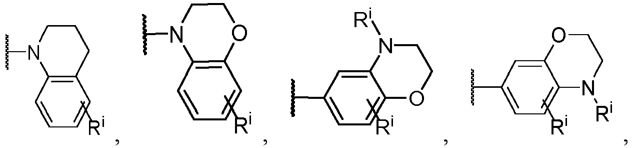 or 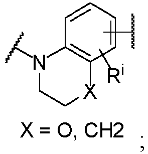
(27) B is 6-membered heteroaryl-fused 5-membered heteroaryl, wherein the heteroatom in the 6-membered heteroaryl is N, and the number of heteroatoms is 1; preferably, the heteroatom in the 5-membered heteroaryl is N or S, and the number of heteroatoms is 1, for example, 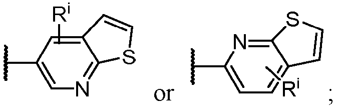
(28) B is 6-membered heteroaryl-fused 6-membered heterocycloalkyl, wherein the heteroatom in the 6-membered heteroaryl is N, and the number of heteroatoms is 1; preferably, the heteroatom in the 6-membered heterocycloalkyl is O, and the number of heteroatoms is 1, for example, 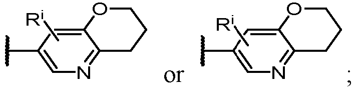
(29) in D, the 5- to 12-membered heteroaryl is 5- to 6-membered heteroaryl, phenyl-fused 5- to 6-membered heteroaryl, 5- to 7-membered cycloalkyl-fused phenyl, 5- to 7-membered cycloalkyl-fused 5- to 6-membered heteroaryl, 5- to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused 5- to 6-membered aryl; preferably 5- to 6-membered heteroaryl, phenyl-fused 5- to 6-membered heteroaryl, 5- to 7-membered cycloalkyl-fused 5- to 6-membered heteroaryl, 5- to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused phenyl; further preferably 5- to 6-membered heteroaryl, phenyl-fused 5- to 6-membered heteroaryl, 5- to 7-membered cycloalkyl-fused 5-to 6-membered heteroaryl, 5- to 6-membered heteroaryl-fused 5- to 6-membered heteroaryl, 5-to 7-membered heterocycloalkyl-fused 5- to 6-membered heteroaryl, or 5- to 7-membered heterocycloalkyl-fused phenyl, wherein the heteroatom in the 5- to 6-membered heteroaryl and 5- to 7-membered heterocycloalkyl is preferably selected from one, two, or three kinds of N, O, and S, and the number of heteroatoms is preferably 1, 2, or 3;
(30) in D, the 5- to 6-membered heteroaryl is 5-membered heteroaryl or 6-membered heteroaryl;
(31) D is hydrogen, methyl, ethyl, isopropyl, cyclopropyl, or trifluoromethyl;
(32) D is 6-membered heteroaryl, wherein the heteroatom in the 6-membered heteroaryl is N, and the number of heteroatoms is 1 or 2; for example, 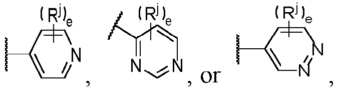 wherein e is independently 0, 1, 2, or 3; for another example, 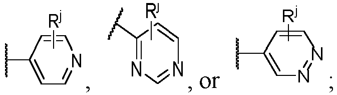
(33) D is 5-membered heteroaryl, wherein the heteroatom in the 5-membered heteroaryl is N or S, and the number of heteroatoms is 1, 2, or 3, for example, 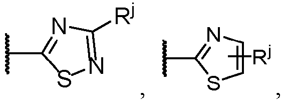 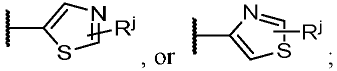
(34) D is 6-membered heteroaryl-fused 5-membered heterocycloalkyl, wherein the heteroatom in the 6-membered heteroaryl is N, and the number of heteroatoms is 1; preferably, the heteroatom in the 5-membered heterocycloalkyl is N or O, and the number of heteroatoms is 1, for example, 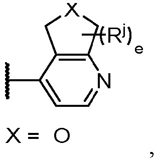 wherein e is independently 0, 1, 2, or 3; for another example, 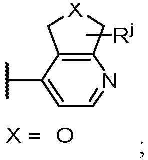
(35) D is 6-membered heteroaryl-fused 5-membered cycloalkyl, wherein the heteroatom in the 6-membered heteroaryl is N, and the number of heteroatoms is 1, for example, 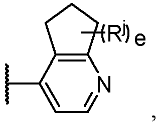 wherein e is independently 0, 1, 2, or 3; for another example,

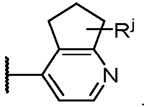

9. The compound of formula I or the pharmaceutically acceptable salt thereof according to claim 1 or 2, wherein the compound of formula I satisfies any of the following conditions:
(1) 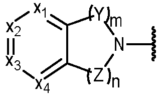 is 6-membered heteroaryl-fused 5-membered heterocycloalkyl, wherein the heteroatom in the 6-membered heteroaryl is N, and the number of heteroatoms is 2; the heteroatom in the 5-membered heterocycloalkyl may be N, and the number of heteroatoms is 1, for example, 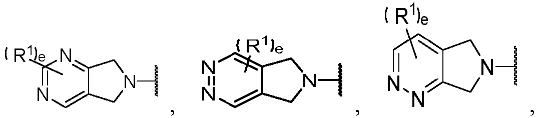 or 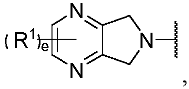 wherein e is independently 0, 1, or 2;
(2) B is 4- to 6-membered heterocycloalkyl substituted by two Rⁱ; two Rⁱ on the same atom together with the atom to which they are attached form a 3- to 7-membered cycloalkyl group, and the 3- to 7-membered cycloalkyl is preferably 3- to 6-membered cycloalkyl; for example, B is 4-membered heterocycloalkyl or 5-membered heterocycloalkyl, and two Rⁱ form a 3- to 4-membered cycloalkyl group; for another example, B is 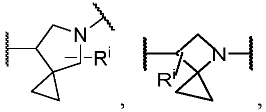 or 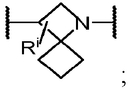
(3) D is 5-membered heteroaryl, wherein the heteroatom in the 5-membered heteroaryl is one or more kinds of N, S, and O, and the number of heteroatoms is 1, 2, or 3, for example, 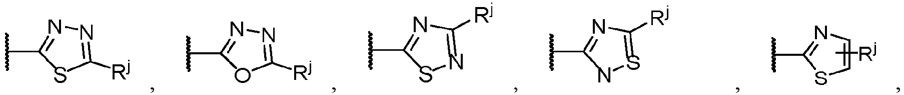 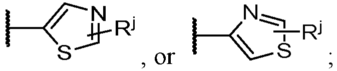
(4) D is phenyl-fused 5-membered heteroaryl, wherein the heteroatom in the 5-membered heteroaryl is N and/or S, and the number of heteroatoms is 2, for example, 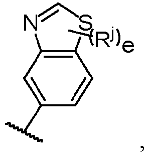 wherein e is independently 0, 1, 2, or 3; for another example, 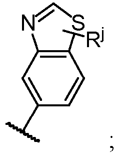
(5) D is 5- to 6-membered heteroaryl-fused 5- to 6-membered heteroaryl, wherein the heteroatom in the 5- to 6-membered heteroaryl is N and/or S, and the number of heteroatoms is 1, 2, or 3, for example, 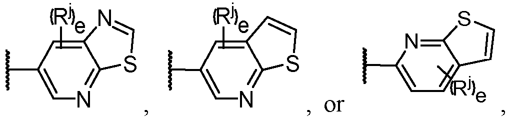 wherein e is independently 0, 1, 2, or 3;
(6) D is 5- to 6-membered heteroaryl-fused 5- to 6-membered heterocycloalkyl, wherein the heteroatom in the 5- to 6-membered heteroaryl is N, and the number of heteroatoms is 1, 2, or 3; preferably, the heteroatom in the 5- to 6-membered heterocycloalkyl is O, and the number of heteroatoms is 1, 2, or 3, for example, 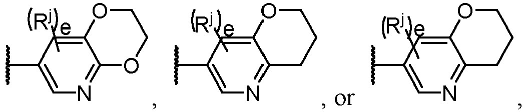 wherein e is independently 0, 1, 2, or 3;
(7) D is phenyl-fused 5- to 6-membered heterocycloalkyl, wherein the heteroatom in the 5- to 6-membered heterocycloalkyl is one or more kinds of O, N, and O, and the number of heteroatoms is 1, 2, or 3, for example, 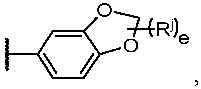 wherein e is independently 0, 1, 2, or 3;
(8) D is phenyl-fused 5- to 6-membered heteroaryl, wherein the heteroatom in the 5- to 6-membered heteroaryl is N, and the number of heteroatoms is 1, 2, or 3, for example, 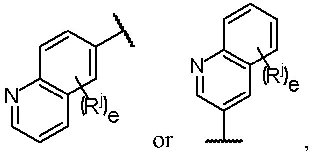 wherein e is independently 0, 1, 2, or 3.

10. The compound of formula I or the pharmaceutically acceptable salt thereof according to claim 1 or 2, wherein the compound of formula I satisfies any of the following conditions:
scheme 1: 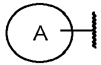 is 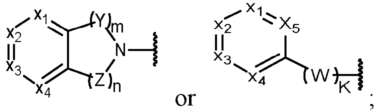
m is a natural number of 1 to 3;
n is a natural number of 1 to 3;
k is a natural number of 1 to 3;
X₁, X₂, X₃, X₄, and X₅ are independently -CR¹-, N, O, S, 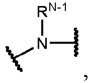 or a chemical bond, and the number of heteroatoms in X₁, X₂, X₃, X₄, and X₅ is 0, 1, 2, or 3;
R^{N-1} is hydrogen or C₁-C₆ alkyl;
Y and Z are independently carbonyl (CO) or -(CR²R³)ᵣ-; r is a natural number of 1 to 2;
each W is independently -O-, -(CR⁴R⁵)-, -NR⁶-, or a chemical bond;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl; wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
each R¹⁻⁴⁻¹ is independently halogen;
R¹⁻¹⁻¹ and R¹⁻²⁻¹ are independently hydrogen or C₁-C₃ alkyl;
R² and R³ are independently hydrogen or NR²⁻¹R²⁻²;
R²⁻¹ and R²⁻² are independently hydrogen or C₁-C₆ alkyl;
R⁴, R⁵, and R⁶ are independently hydrogen;
L is 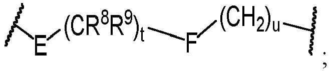
t is a natural number of 0 to 2;
u is a natural number of 0 to 2;
E is carbonyl, -NHCO-, or a chemical bond;
F is -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl; wherein the 5- to 12-membered heteroaryl, 6- to 10-membered aryl, and 4- to 6-membered heterocycloalkyl are optionally and independently substituted by 1, 2, or 3 Rⁱ;
each Rⁱ is independently hydrogen, halogen, hydroxyl, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group;
D is hydrogen, 3- to 7-membered cycloalkyl, C₁-C₆ alkyl, or 5- to 12-membered heteroaryl; wherein the C₁-C₆ alkyl or 5- to 12-membered heteroaryl are optionally and independently substituted by 1, 2, or 3 R^{j};
each R^{j} is independently halogen, C₁-C₃ alkyl, -OR¹²⁻³, or -SR¹²⁻⁴; the C₁-C₃ alkyl is optionally and independently substituted by 1, 2, or 3 R^{k}; each R^{k} is independently halogen;
R¹²⁻³ and R¹²⁻⁴ are independently C₁-C₃ alkyl; the C₁-C₃ alkyl is optionally and independently substituted by 1, 2, or 3 R^{k}; each R^{k} is independently halogen;
the heteroatom in the 3- to 7-membered heterocycloalkyl, 4- to 6-membered heterocycloalkyl, and 5- to 12-membered heteroaryl is N, O, or S, and the number of heteroatoms is 1 to 3; or, the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3;
scheme 2: 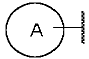 is 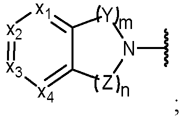
m is a natural number of 1 to 3;
n is a natural number of 1 to 3;
X₁, X₂, X₃, and X₄ are independently -CR¹-, N, S, 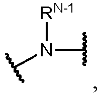 or a chemical bond, and the number of heteroatoms in X₁, X₂, X₃, X₄, and X₅ is 1 or 2;
R^{N-1} is C₁-C₆ alkyl;
Y and Z are independently carbonyl (CO) or -(CR²R³)ᵣ-; r is a natural number of 1 to 2;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl;
each R¹⁻⁴⁻¹ is independently halogen;
R² and R³ are independently hydrogen;
L is 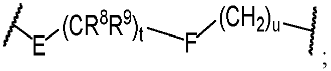
t is a natural number of 0 to 2;
u is a natural number of 0 to 2;
E is carbonyl or a chemical bond;
F is -O-, -NH-, or a chemical bond;
R⁸ and R⁹ are independently C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl or 5- to 12-membered heteroaryl, wherein the 5-to 12-membered heteroaryl and 4- to 6-membered heterocycloalkyl are optionally and independently substituted by 1, 2, or 3 Rⁱ;
each Rⁱ is independently hydrogen, halogen, hydroxyl, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group;
D is hydrogen, 3- to 7-membered cycloalkyl, C₁-C₆ alkyl, or 5- to 6-membered heteroaryl; wherein the C₁-C₆ alkyl and 5- to 6-membered heteroaryl are optionally and independently substituted by 1, 2, or 3 R^{j};
each R^{j} is independently halogen or C₁-C₃ alkyl; the C₁-C₃ alkyl is optionally and independently substituted by 1, 2, or 3 R^{k}; each R^{k} is independently halogen;
the heteroatom in the 3- to 7-membered heterocycloalkyl, 4- to 6-membered heterocycloalkyl, 5- to 6-membered heteroaryl, and 5- to 12-membered heteroaryl is N, O, or S, and the number of heteroatoms is 1 to 3; or, the heteroatom in the 3- to 7-membered heterocycloalkyl, 4- to 6-membered heterocycloalkyl, 5- to 6-membered heteroaryl, and 5- to 12-membered heteroaryl is one or more kinds of N, O, and S, and the number of heteroatoms is 1 to 3;
scheme 3: 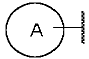 is 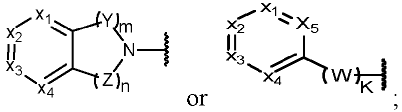
m is a natural number of 1 to 2;
n is a natural number of 1 to 2;
k is a natural number of 1 to 3;
X₁, X₂, X₃, X₄, and X₅ are independently -CR¹-, N, O, S, 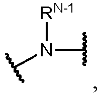 or a chemical bond, and the number of heteroatoms in X₁, X₂, X₃, X₄, and X₅ is 0, 1, 2, or 3;
R^{N-1} is C₁-C₆ alkyl;
each W is independently -(CR⁴R⁵)-, -O-, -NR⁶-, or a chemical bond; Y and Z are independently carbonyl (CO) or -(CR²R³)ᵣ-; r is a natural number of 1 to 2;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl;
each R¹⁻⁴⁻¹ is independently halogen;
R² and R³ are independently hydrogen or NR²⁻¹R²⁻²;
R²⁻¹ and R²⁻² are independently hydrogen or C₁-C₆ alkyl;
R⁴, R⁵*,* and R⁶ are independently hydrogen; L is 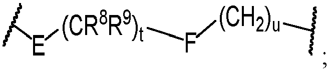
t is a natural number of 0 to 2;
u is a natural number of 0 to 2;
E is carbonyl, -NHCO-, or a chemical bond;
F is -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl; wherein the 5- to 12-membered heteroaryl, 6- to 10-membered aryl, and 4- to 6-membered heterocycloalkyl are optionally and independently substituted by 1, 2, or 3 Rⁱ;
each Rⁱ is independently hydrogen, hydroxyl, halogen, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group;
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently halogen, C₁-C₃ alkyl, OR¹²⁻³, or SR¹²⁻⁴; the C₁-C₃ alkyl is optionally and independently substituted by 1, 2, or 3 R^{k}; each R^{k} is independently halogen;
R¹²⁻³ and R¹²⁻⁴ are independently C₁-C₃ alkyl, wherein the C₁-C₃ alkyl is optionally and independently substituted by 1, 2, or 3 R^{k-1}; each R^{k-1} is independently halogen; the heteroatom in the 3- to 7-membered heterocycloalkyl, 4- to 6-membered heterocycloalkyl, and 5- to 12-membered heteroaryl is N, O, or S, and the number of heteroatoms is 1 to 3; or, the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3;
scheme 4: 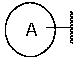 is 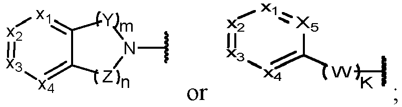
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, S, 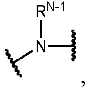 or a chemical bond;
R^{N-1} is hydrogen, C₁-C₆ alkyl, or 3- to 7-membered cycloalkyl;
Y and Z are independently carbonyl (CO) or -(CR²R³)ᵣ-; r is a natural number of 0 to 5;
each W is independently -O-, -(CR⁴R⁵)-, -NR⁶-, or a chemical bond;
each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a};
each R^{a} is independently halogen, hydroxyl, C₁-C₆ alkoxy, or -NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently hydroxyl, 3- to 7-membered cycloalkyl, or C₁-C₃ alkyl;
each R^{b-2} is independently halogen or C₁-C₆ alkyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
each R¹⁻⁴⁻¹ is independently halogen;
R² and R³ are independently hydrogen or NR²⁻¹R²⁻²;
R²⁻¹ and R²⁻² are independently hydrogen or C₁-C₆ alkyl;
R⁴, R⁵*,* and R⁶ are independently hydrogen or NR⁴⁻¹R⁴⁻²;
R⁴⁻¹ and R⁴⁻² are independently hydrogen;
L is 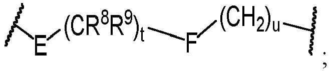
t is a natural number of 0 to 3;
u is a natural number of 0 to 3;
E is carbonyl, 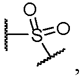 -NHCO-, or a chemical bond;
F is carbonyl, 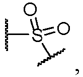 -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl; wherein the 4- to 6-membered heterocycloalkyl, 6- to 10-membered aryl, or 5- to 12-membered heteroaryl are optionally and independently substituted by 1, 2, or 3 Rⁱ;
each Rⁱ is independently hydrogen, halogen, hydroxyl, or C₁-C₃ alkyl; or two Rⁱ on the same atom together with the atom to which they are attached form a 3- to 7-membered cycloalkyl group; or two adjacent Rⁱ together with the atom to which they are attached form a 3- to 7-membered cycloalkyl group;
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently hydrogen, halogen, C₁-C₃ alkyl, 3- to 7-membered cycloalkyl, - OR¹²⁻³, or -SR¹²⁻⁴; the C₁-C₃ alkyl and 3- to 7-membered cycloalkyl are optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen;
each R^{k-1} is independently halogen;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3;
scheme 5: 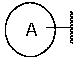 is 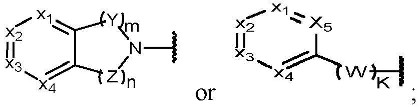
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, S, 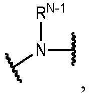 or a chemical bond;
R^{N-1} is C₁-C₆ alkyl;
Y and Z are independently carbonyl (CO) or -(CR²R³)ᵣ-; r is a natural number of 0 to 5;
each W is independently -(CR⁴R⁵)-, -NR⁶-, or a chemical bond;
each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a};
each R^{a} is independently hydroxyl, C₁-C₆ alkoxy, or -NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently 3- to 7-membered cycloalkyl;
each R^{b-2} is independently halogen or C₁-C₆ alkyl;
R¹⁻⁴ and R¹⁻⁵ are independently C₁-C₃ alkyl;
R² and R³ are independently hydrogen or NR²⁻¹R²⁻²;
R²⁻¹ and R²⁻² are independently hydrogen;
R⁴, R⁵*,* and R⁶ are independently hydrogen;
L is 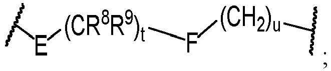
t is a natural number of 0 to 3;
u is a natural number of 0 to 3;
E is carbonyl, 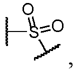 -NHCO-, or a chemical bond;
F is a chemical bond;
R⁸ and R⁹ are independently hydrogen or C₁-C₆ alkyl; or R⁸ and R⁹ together with the atom to which they are attached form a 3- to 7-membered cycloalkyl group;
B is 4- to 6-membered heterocycloalkyl, 6- to 10-membered aryl, or 5- to 12-membered heteroaryl; wherein the 4- to 6-membered heterocycloalkyl, 6- to 10-membered aryl, or 5- to 12-membered heteroaryl are optionally and independently substituted by 1, 2, or 3 Rⁱ;
each Rⁱ is independently hydrogen, halogen, hydroxyl, or C₁-C₃ alkyl; or two Rⁱ on the same atom together with the atom to which they are attached form a 3- to 7-membered cycloalkyl group;
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently hydrogen, halogen, C₁-C₃ alkyl, 3- to 7-membered cycloalkyl, - OR¹²⁻³, or -SR¹²⁻⁴; the C₁-C₃ alkyl and 3- to 7-membered cycloalkyl are optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen;
each R^{k-1} is independently halogen;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3;
scheme 6:
wherein 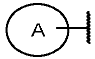 is 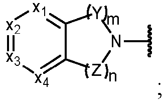
m is 1;
n is 1;
X₁, X₂, X₃, and X₄ are independently -CR¹- or N, and the number of heteroatoms in X₁, X₂, X₃, and X₄ is 2;
Y and Z are independently -(CR²R³)ᵣ-; r is 1;
each Rⁱ is independently C₁-C₆ alkyl or C₁-C₆ alkoxy;
R² and R³ are independently hydrogen;
L is 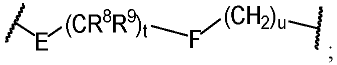
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen;
D is 5- to 12-membered heteroaryl; wherein the 5- to 12-membered heteroaryl is optionally and independently substituted by 1, 2, or 3 R^{j};
each R^{j} is independently -OR¹²⁻³;
R¹²⁻³ is C₁-C₃ alkyl; the C₁-C₃ alkyl is optionally and independently substituted by 1, 2, or 3 R^{k}; each R^{k} is independently halogen;
the heteroatom in the 4- to 6-membered heterocycloalkyl and 5- to 12-membered heteroaryl is N, O, or S, and the number of heteroatoms is 1 to 3; or, the heteroatom in the 4-to 6-membered heterocycloalkyl and 5- to 12-membered heteroaryl is one or more kinds of N, O, and S, and the number of heteroatoms is 1 to 3;
when A is 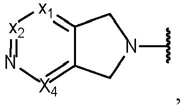 the number of heteroatoms in X₁, X₂, and X₄ is 1 or 2;
when A is 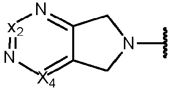 and L is carbonyl, then u is a natural number of 1 to 3, B is 4- to 6-membered heterocycloalkyl, and the 4- to 6-membered heterocycloalkyl is optionally and independently substituted by 1, 2, or 3 Rⁱ;
scheme 7:
wherein 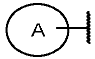 is 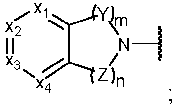
m is 1;
n is 1;
X₂ and X₃ are N;
X₁ and X₄ are independently -CR¹-;
Y and Z are independently -(CR²R³)ᵣ-; r is 1;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
R¹⁻¹ and R¹⁻² are independently hydrogen or C₁-C₆ alkyl; or R¹⁻¹ and R¹⁻² together with the atom to which they are attached form a 3- to 7-membered heterocycloalkyl group;
each R^{a} is independently hydroxyl, C₁-C₆ alkoxy, or NR¹⁻⁴R¹⁻⁵;
R¹⁻⁴ and R¹⁻⁵ are independently hydrogen or C₁-C₃ alkyl;
R² and R³ are independently hydrogen;
L is 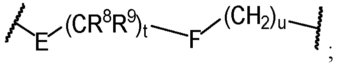
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen, halogen, hydroxyl, or C₁-C₃ alkyl;
D is 5- to 12-membered heteroaryl; wherein the 5- to 12-membered heteroaryl is optionally and independently substituted by 1, 2, or 3 R^{j}; preferably, D is 5- to 6-membered heteroaryl; wherein the 5- to 6-membered heteroaryl is optionally and independently substituted by 1, 2, or 3 R^{j}; the heteroatom in the 5- to 6-membered heteroaryl is selected from one, two, or three kinds of N, S, and O, and the number of heteroatoms is 1, 2, or 3;
each R^{j} is independently H, halogen, C₁-C₃ alkyl, OR¹²⁻³, or SR¹²⁻⁴; wherein the C₁-C₃ alkyl is optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen or C₁-C₃ alkyl;
each R^{k-1} is independently halogen or C₁-C₃ alkyl;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3;
scheme 8:
wherein 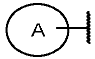 is 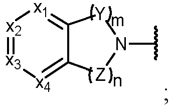
m is 1;
n is 1;
X₁ and X₃ are N;
X₂ and X₄ are independently -CR¹-;
Y and Z are independently -(CR²R³)ᵣ-; r is 1;
each R¹ is independently hydrogen, halogen, cyano, hydroxyl, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkyl, C₁-C₆ alkoxy, and C₁-C₆ alkylthio are optionally and independently substituted by 1, 2, 3, or 4 R^{a};
R¹⁻¹ and R¹⁻² are independently hydrogen or C₁-C₆ alkyl; or R¹⁻¹ and R¹⁻² together with the atom to which they are attached form a 3- to 7-membered heterocycloalkyl group;
each R^{a} is independently hydroxyl, C₁-C₆ alkoxy, or NR¹⁻⁴R¹⁻⁵;
R¹⁻⁴ and R¹⁻⁵ are independently hydrogen or C₁-C₃ alkyl;
R² and R³ are independently hydrogen;
L is 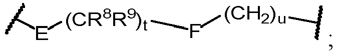
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen, halogen, hydroxyl, or C₁-C₃ alkyl; or two Rⁱ together with the atom to which they are attached form a 3- to 7-membered cycloalkyl group;
D is 5- to 12-membered heteroaryl; wherein the 5- to 12-membered heteroaryl is optionally and independently substituted by 1, 2, or 3 R^{j}; preferably, D is 5- to 6-membered heteroaryl; wherein the 5- to 6-membered heteroaryl is optionally and independently substituted by 1, 2, or 3 R^{j}; the heteroatom in the 5- to 6-membered heteroaryl is selected from one, two, or three kinds of N, S, and O, and the number of heteroatoms is 1, 2, or 3;
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻⁴, and R¹²⁻³ are independently C₁-C₃ alkyl; the C₁-C₃ alkyl is optionally and independently substituted by 1, 2, or 3 R^{k-1}
each R^{k} is independently halogen or C₁-C₃ alkyl;
each R^{k-1} is independently halogen or C₁-C₃ alkyl;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

11. The compound of formula I or the pharmaceutically acceptable salt thereof according to claim 1 or 2, wherein the compound of formula I satisfies one or more of the following conditions:
(1) 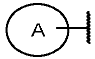 is 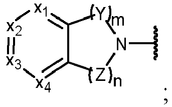
(2) m is 1;
(3) n is 1;
(4) X₁, X₂, X₃, and X₄ are independently -CR¹- or N, and the number of heteroatoms in X₁, X₂, X₃, and X₄ is 2;
(5) Y and Z are independently -(CR²R³)ᵣ-; r is 1;
(6) each R¹ is independently C₁-C₆ alkyl or C₁-C₆ alkoxy;
(7) t is 0;
(8) u is 1;
(9) E is carbonyl;
(10) F is a chemical bond;
(11) B is 4- to 6-membered heterocycloalkyl; wherein the 4- to 6-membered heterocycloalkyl is optionally and independently substituted by 1, 2, or 3 Rⁱ;
(12) each Rⁱ is independently hydrogen;
(13) D is 5- to 12-membered heteroaryl; wherein the 5- to 12-membered heteroaryl is optionally and independently substituted by 1, 2, or 3 R^{j}; preferably, D is 5- to 6-membered heteroaryl; wherein the 5- to 6-membered heteroaryl is optionally and independently substituted by 1, 2, or 3 R^{j}; the heteroatom in the 5- to 6-membered heteroaryl is selected from one, two, or three kinds of N, S, and O, and the number of heteroatoms is 1, 2, or 3;
(14) each R^{j} is independently halo-OR¹²⁻³;
(15) R¹²⁻³ is C₁-C₃ alkyl; the C₁-C₃ alkyl is optionally and independently substituted by 1, 2, or 3 R^{k}; each R^{k} is independently halogen.

12. The compound of formula I or the pharmaceutically acceptable salt thereof according to claim 1 or 2, which satisfies one or more of the following conditions:
(1) each R¹ is independently hydrogen, methyl, 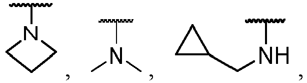 ethyl, chlorine, 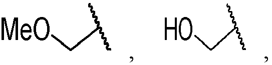 methoxy, 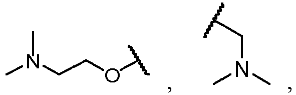 isopropyl, 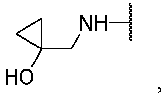 amino (-NH₂), 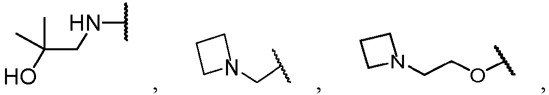 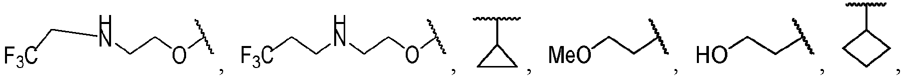 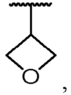 trifluoromethyl, 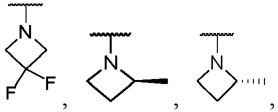 or methylthio; for example, each R¹ is independently hydrogen, methyl, methoxy, 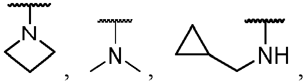 ethyl, chlorine, 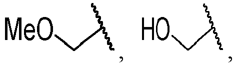 methoxy, 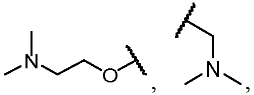 isopropyl, 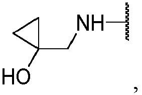 amino, 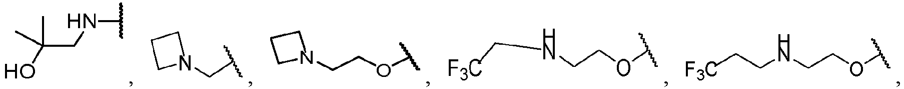 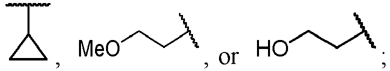 preferably, each R¹ is independently methyl, methoxy, 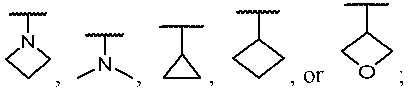
(2) 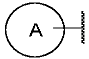 is 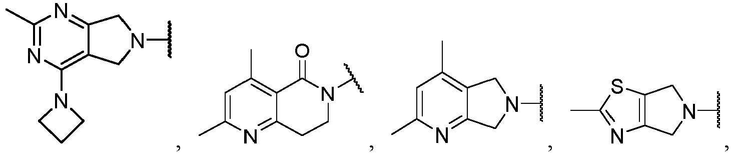 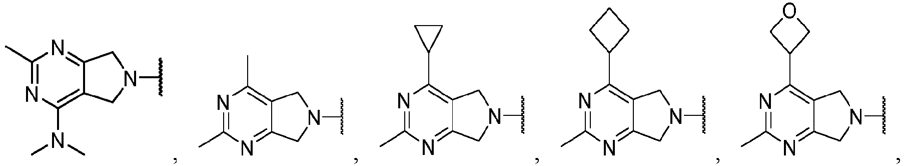 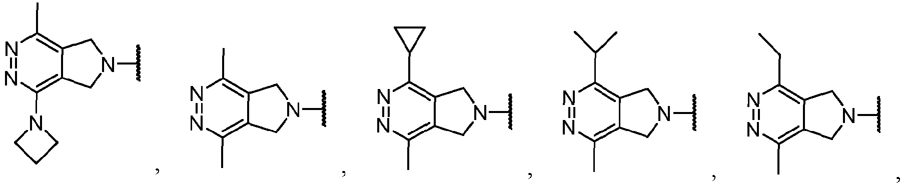 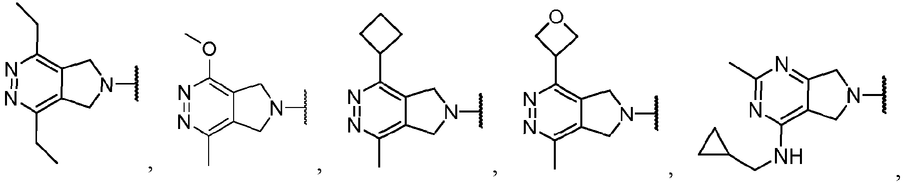 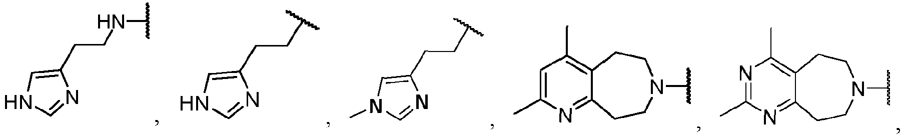 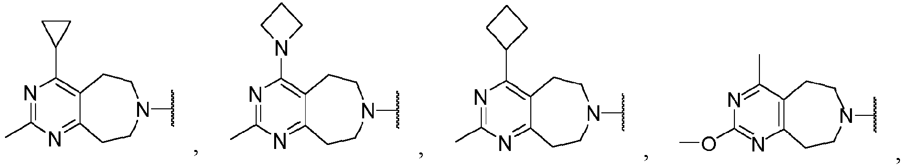 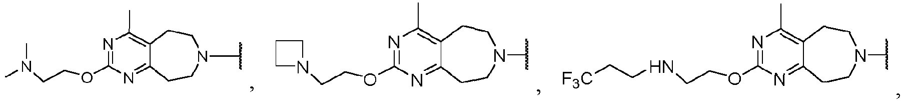 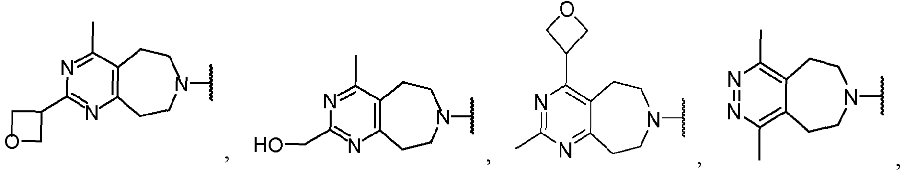 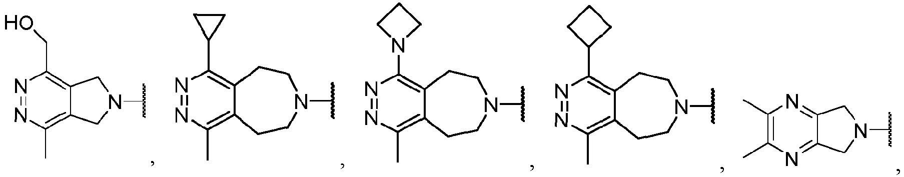 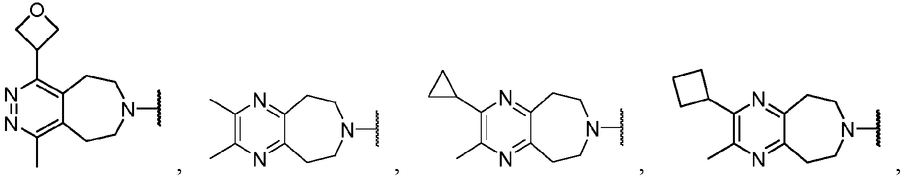 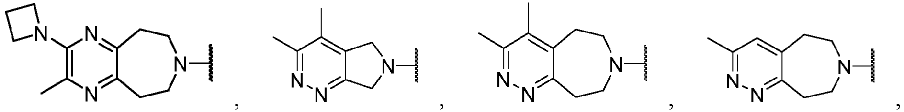 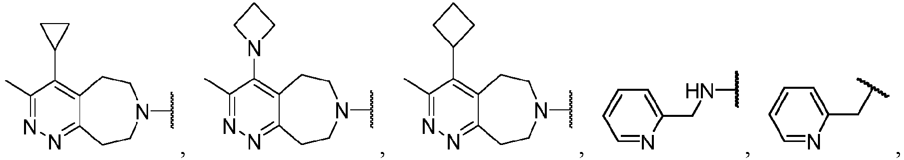 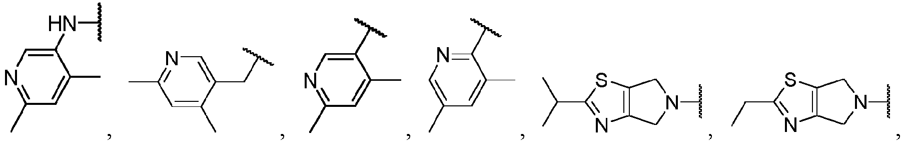 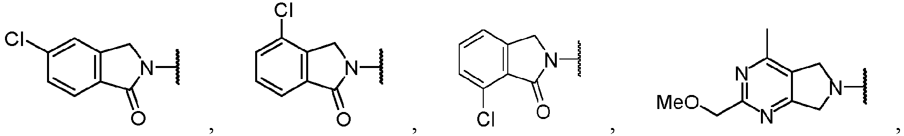 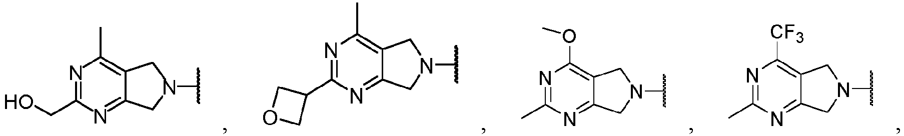 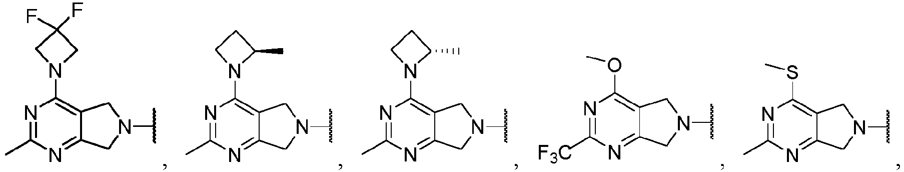 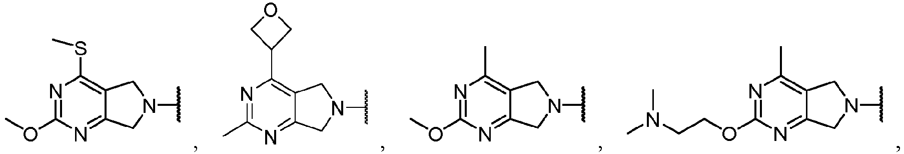 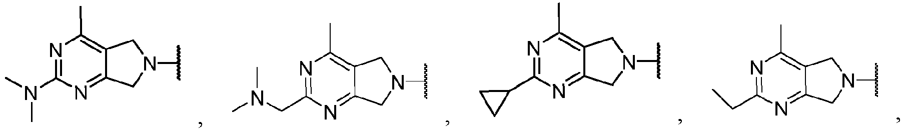 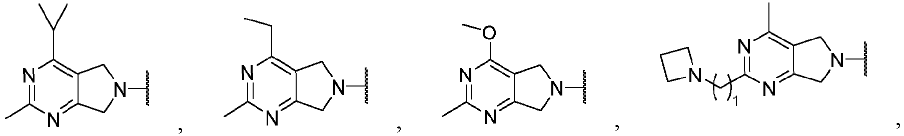 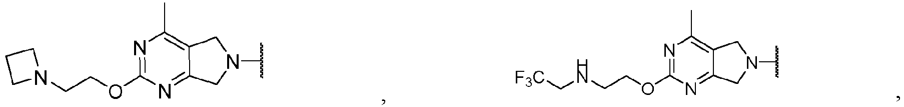 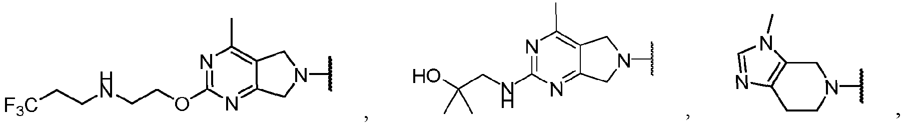 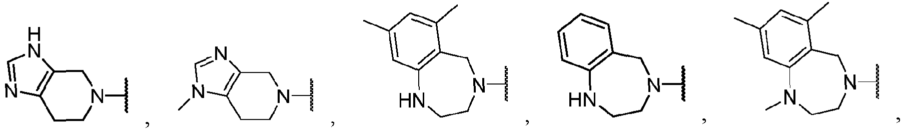 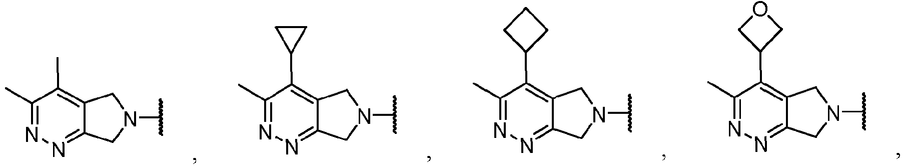 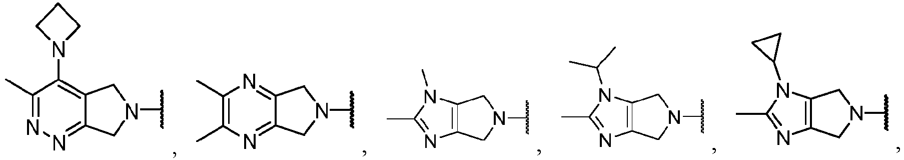 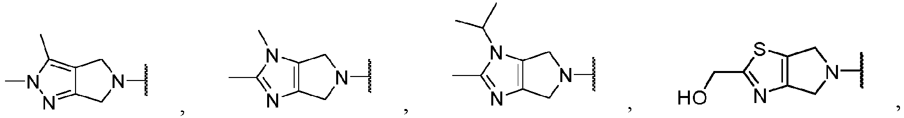 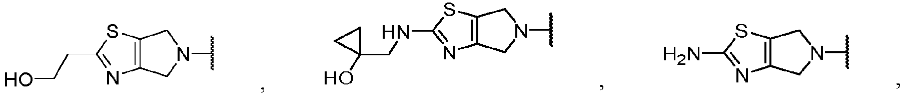 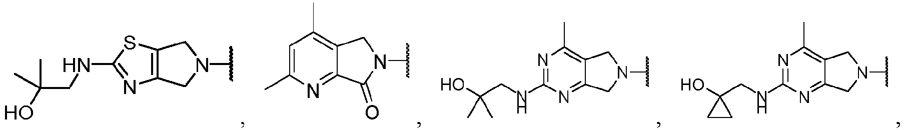 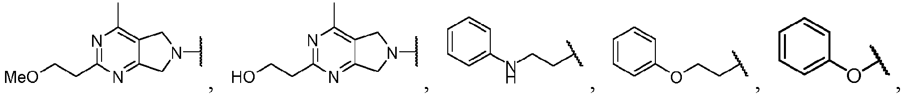 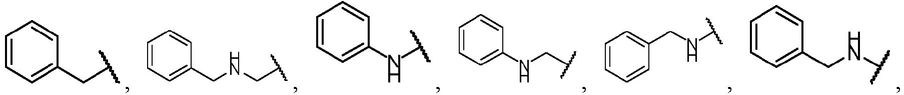 or 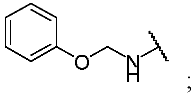 for example, 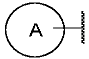 is 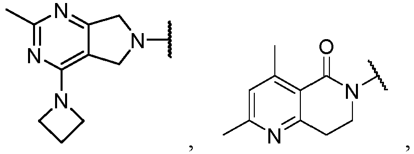 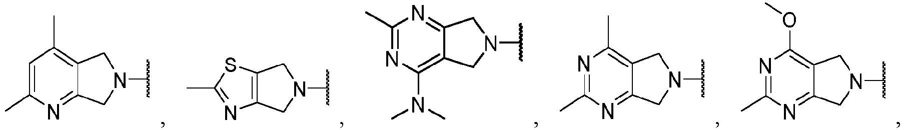 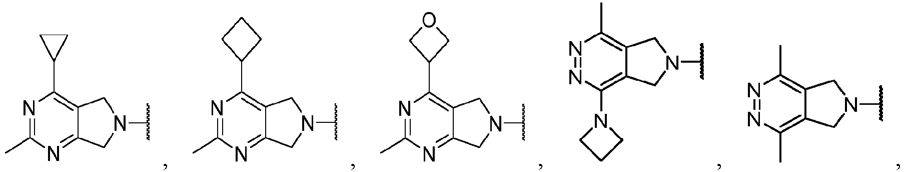 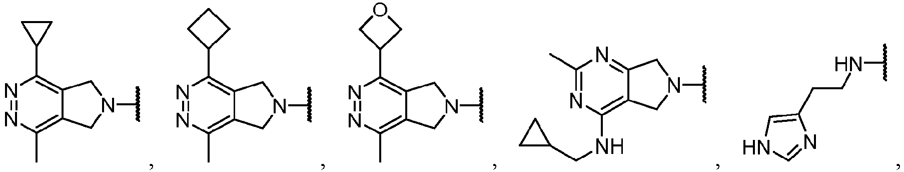 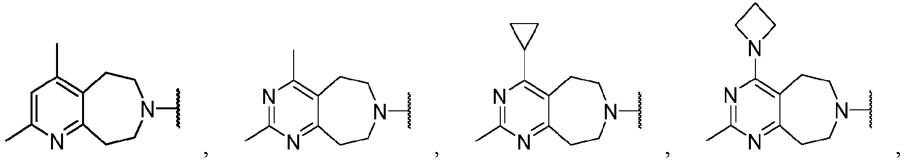 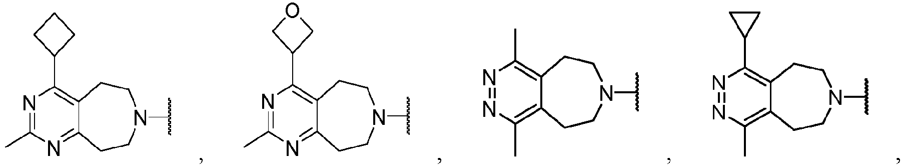 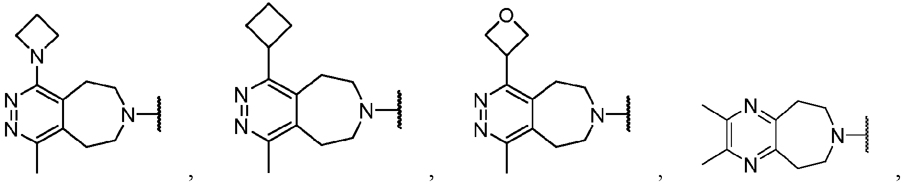 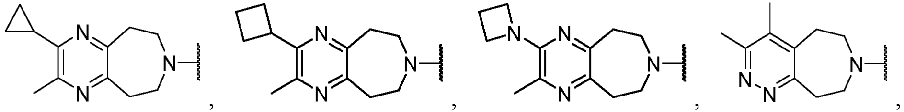 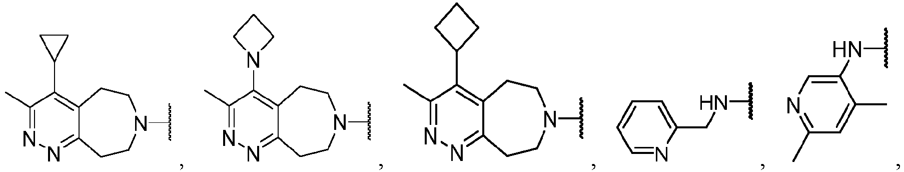 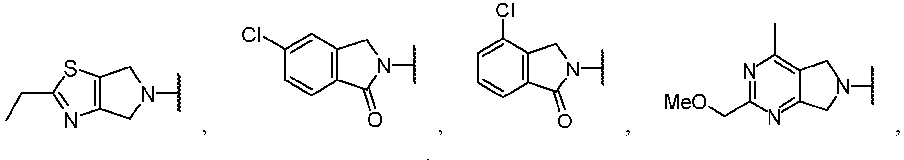 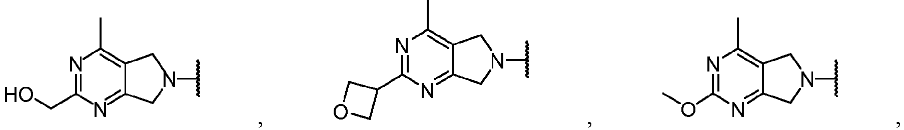 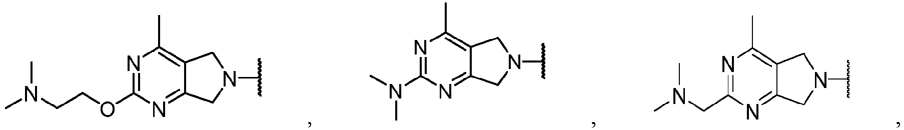 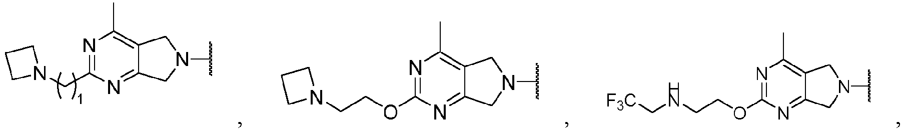 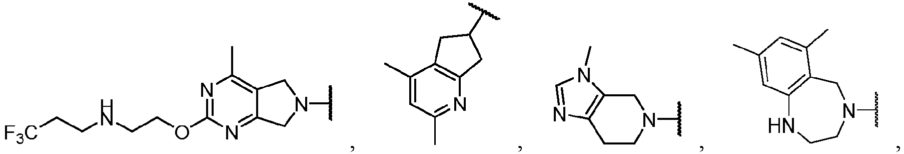 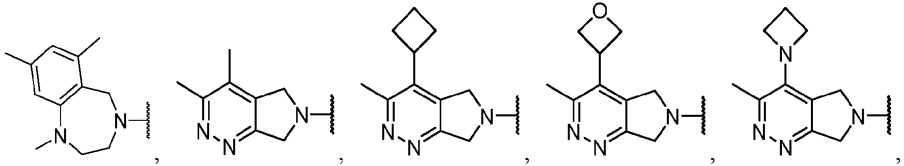 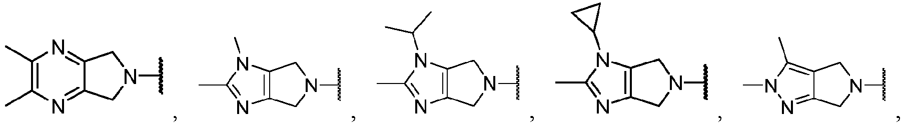 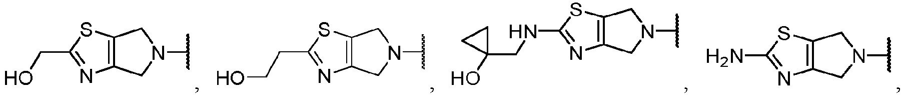 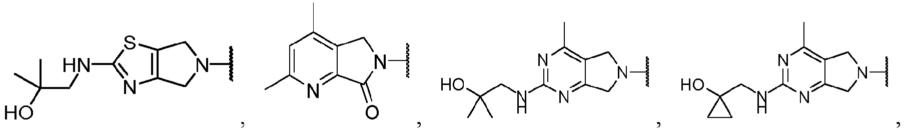 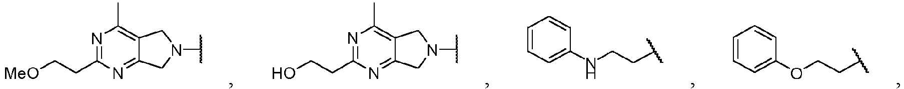 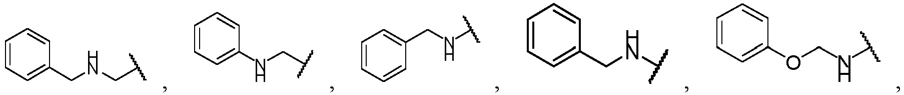 or 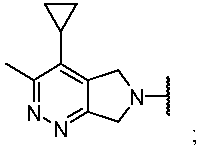 preferably, 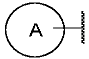 is 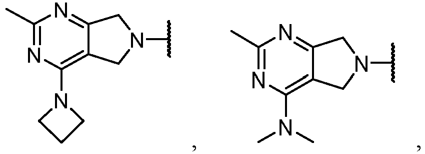 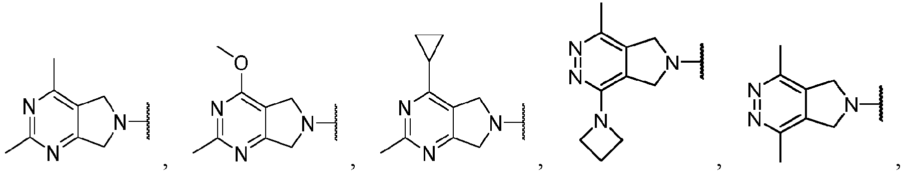 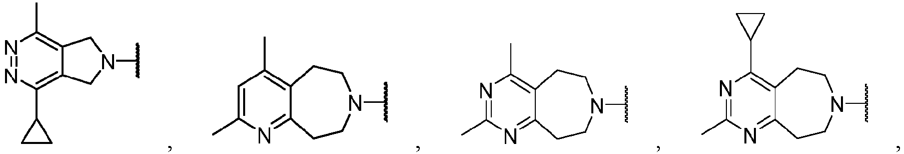 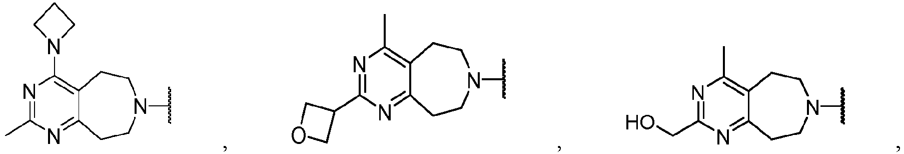 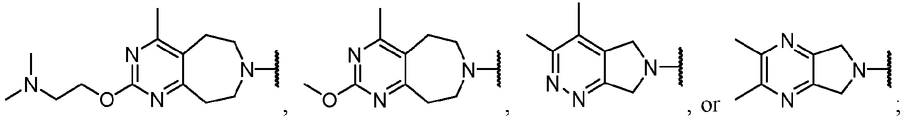
(3) L is -(CH₂)-, -(CH₂)₂-, 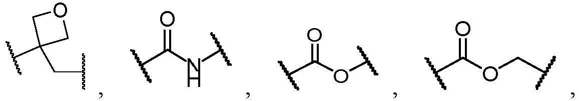 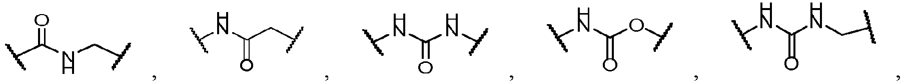 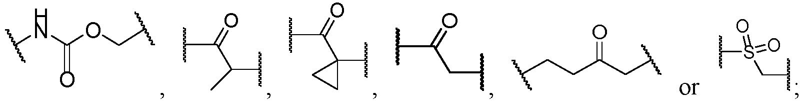 preferably, L is 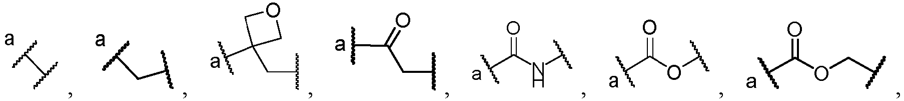 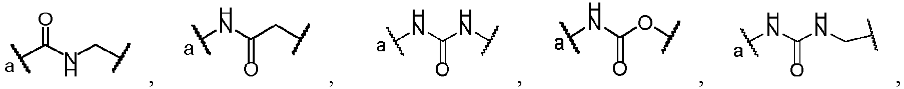 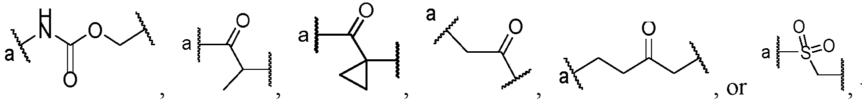 wherein " 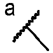 " represents the site connected to A, more preferably, L is -(CH₂)-, -(CH₂)₂-, 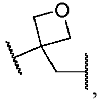 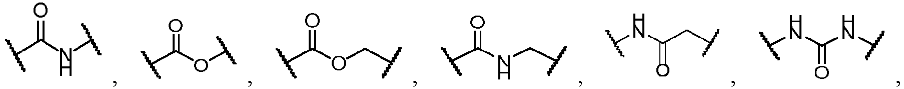 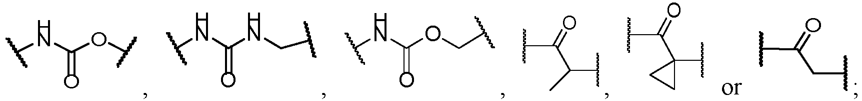 further, L is 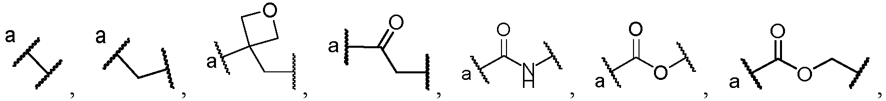 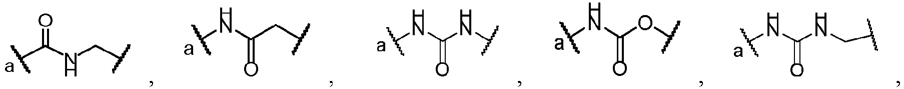 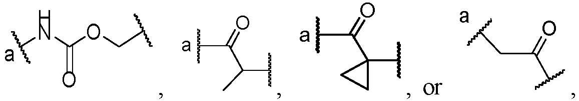 wherein " 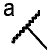 "represents the site connected to A; further, preferably, L is 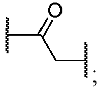
(4) B is 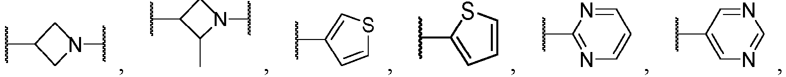 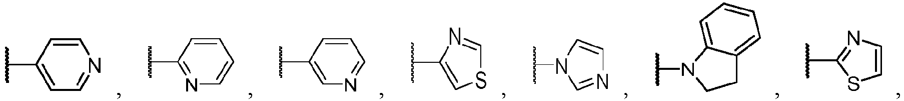 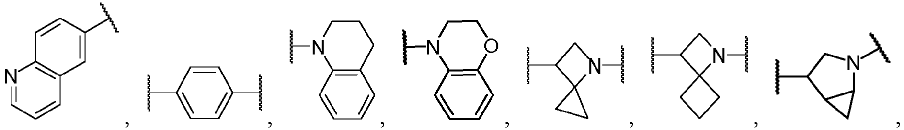 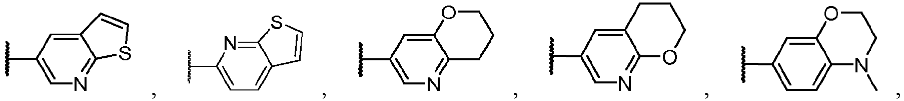 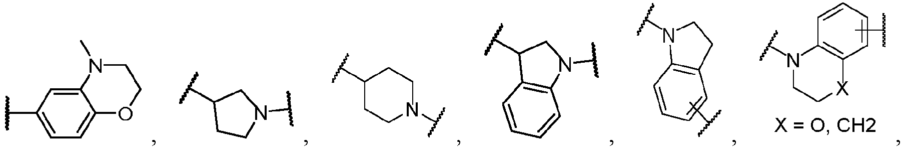 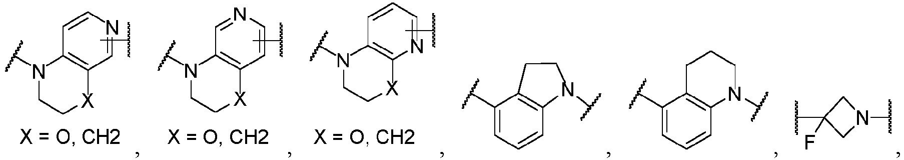 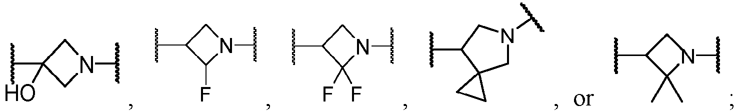 for example, B is 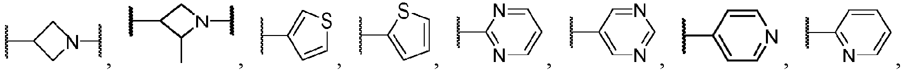 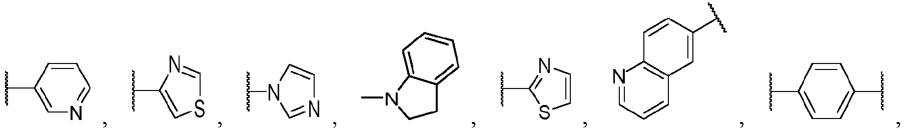 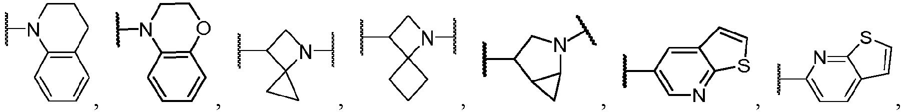 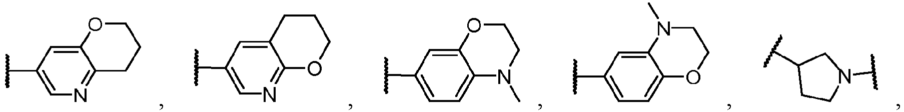 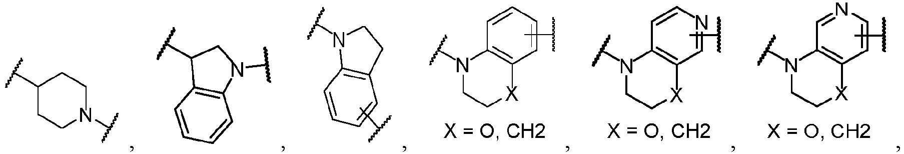 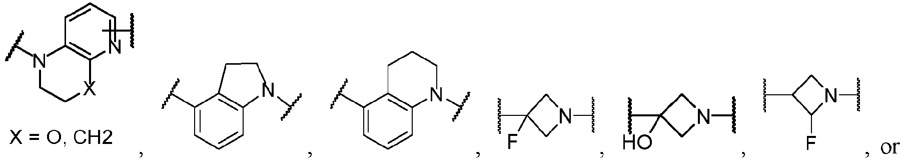 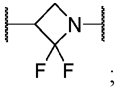 preferably, B is 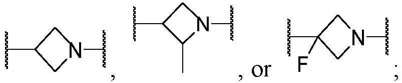
(5) D is hydrogen, methyl, 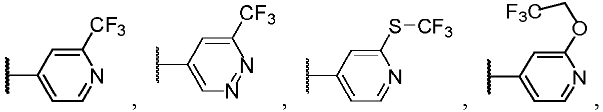 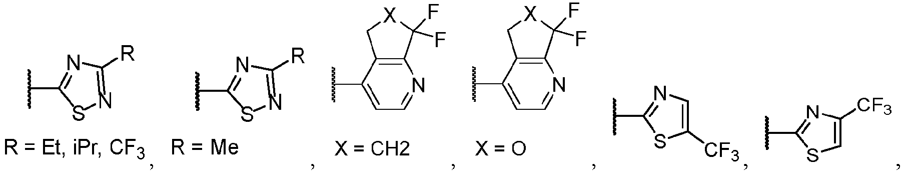 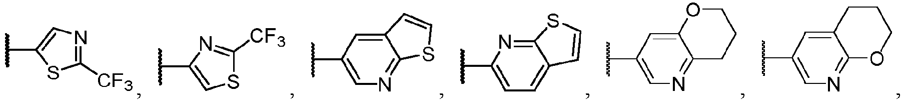 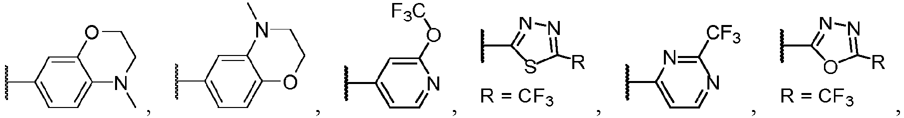 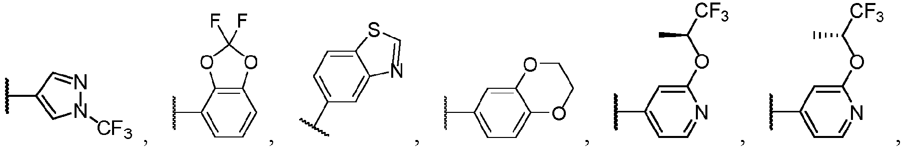 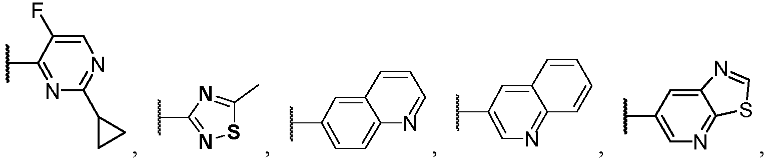 ethyl, isopropyl, cyclopropyl, trifluoromethyl, or 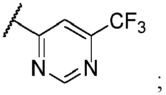 for example, D is hydrogen, methyl, 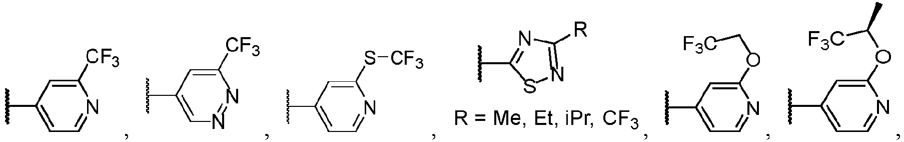 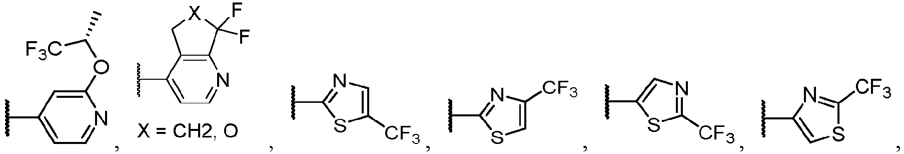 ethyl, isopropyl, cyclopropyl, trifluoromethyl, or 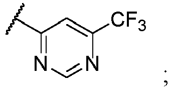 preferably, D is

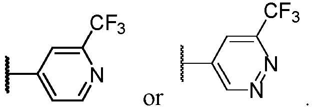

13. The compound of formula I or the pharmaceutically acceptable salt thereof according to claim 1 or 2, which is any one of the following compounds:

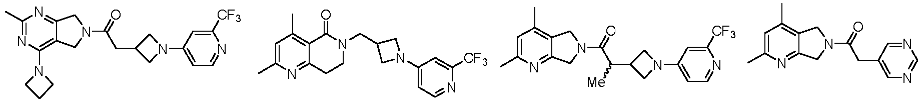

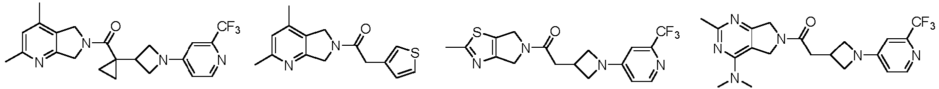

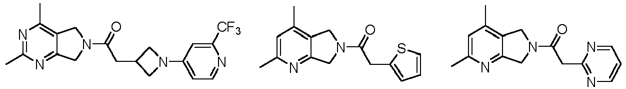

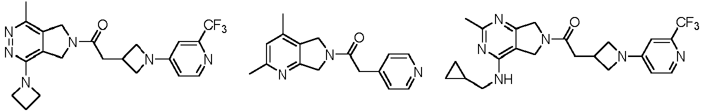

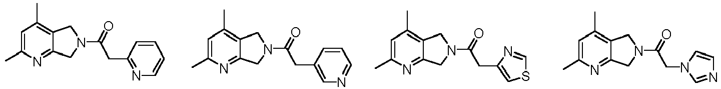

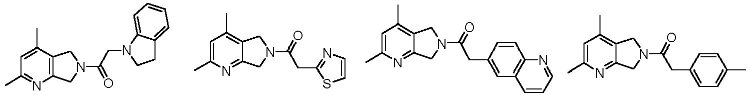

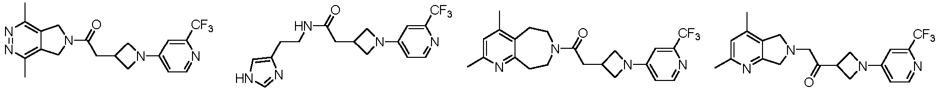

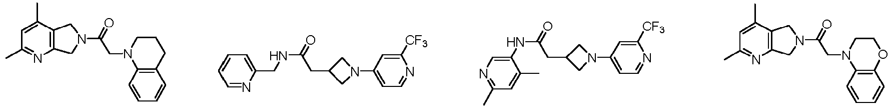

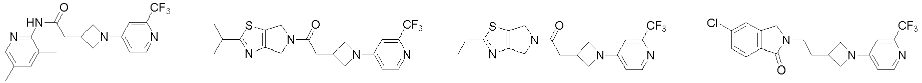

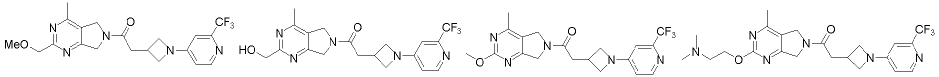

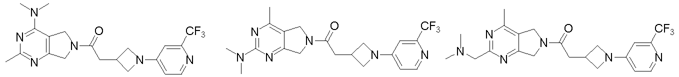

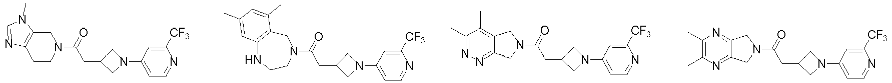

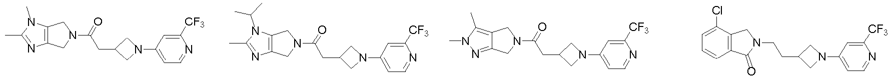

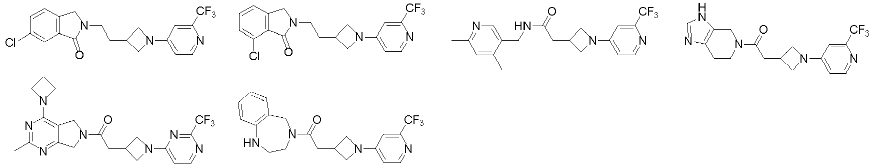

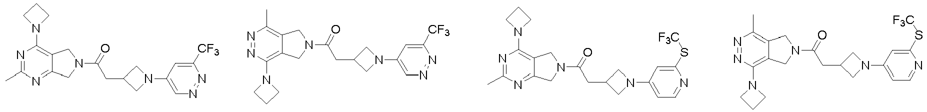

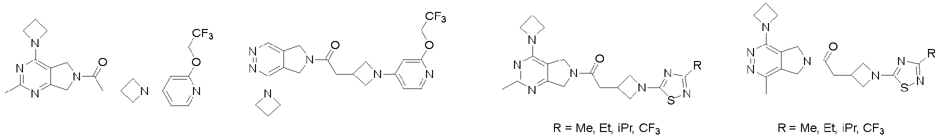

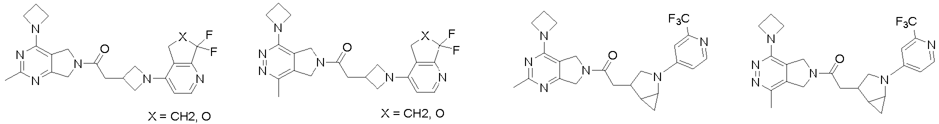

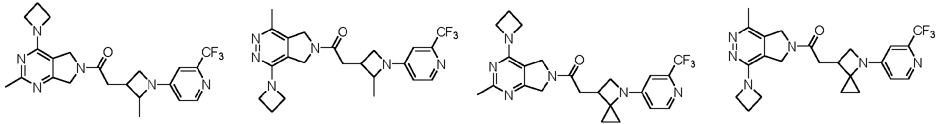

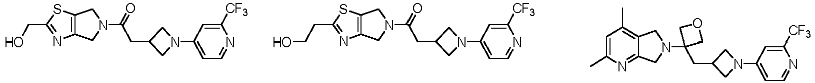

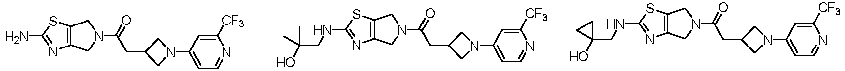

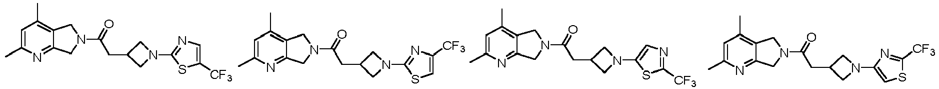

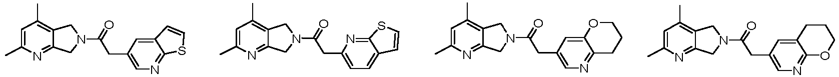

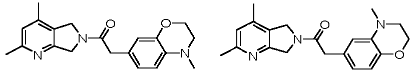

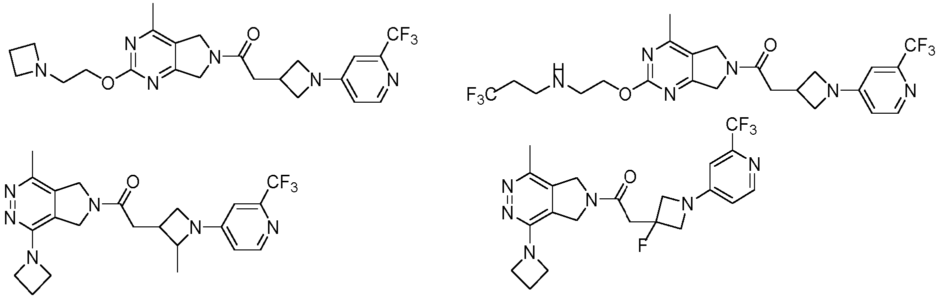

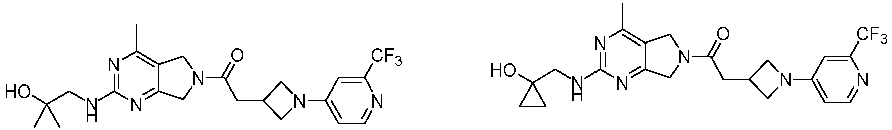

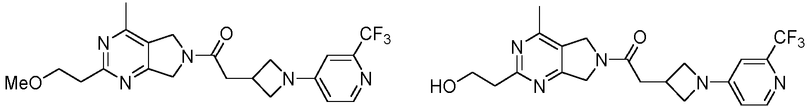

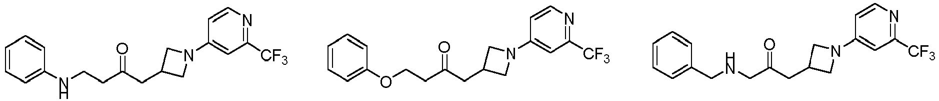

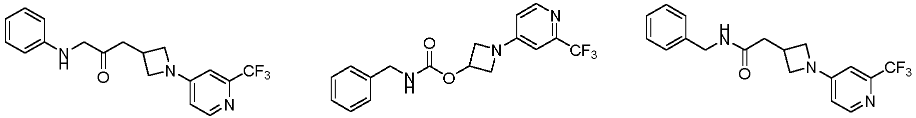

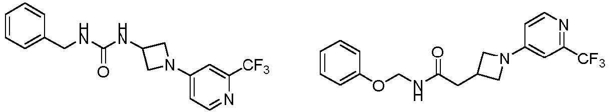

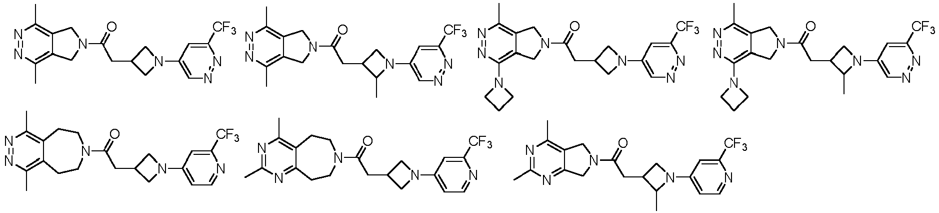

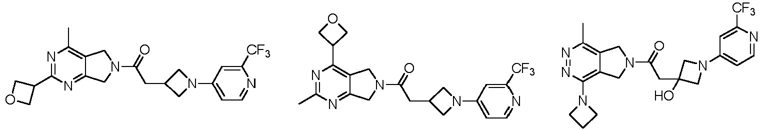

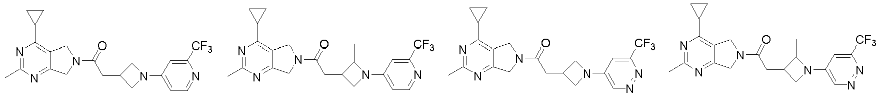

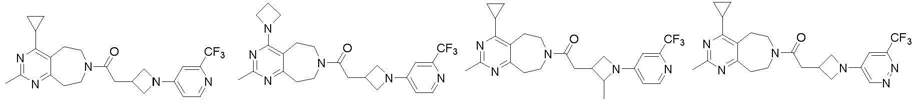

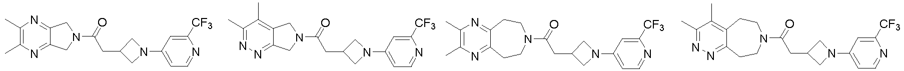

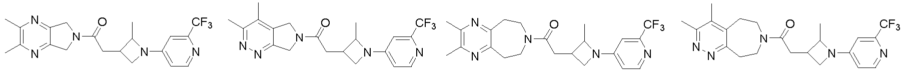

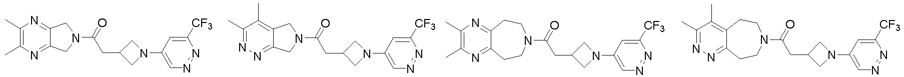

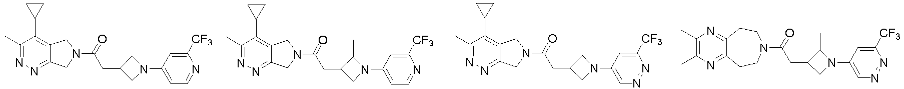

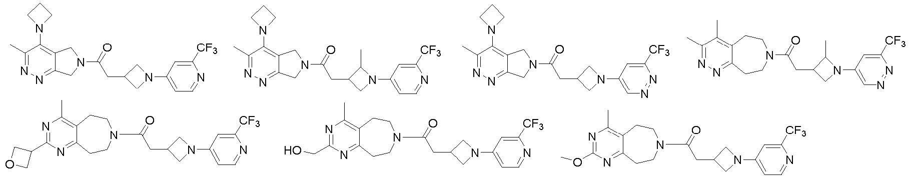

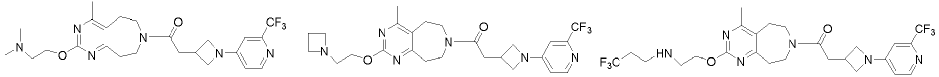

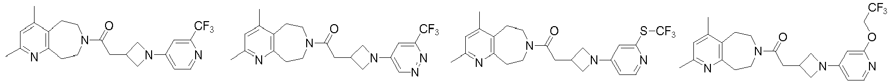

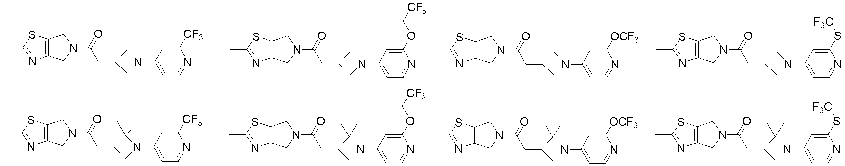

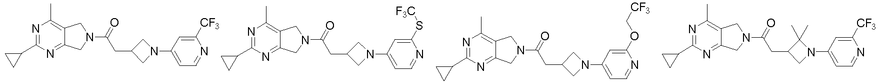

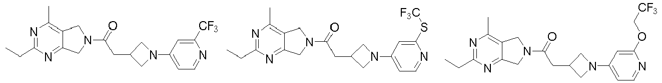

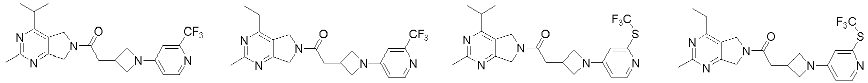

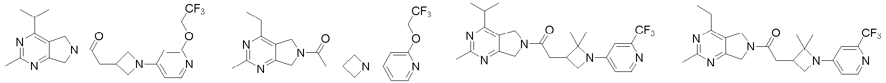

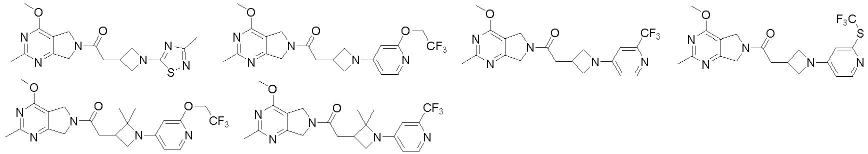

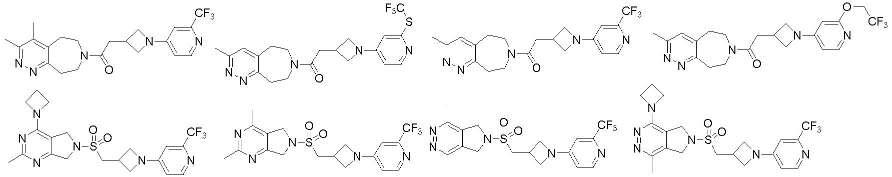

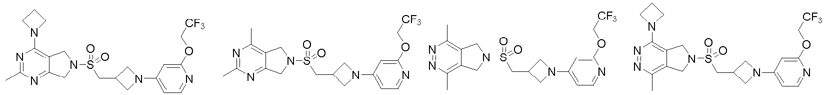

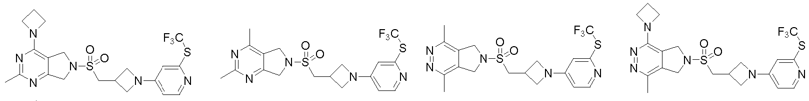

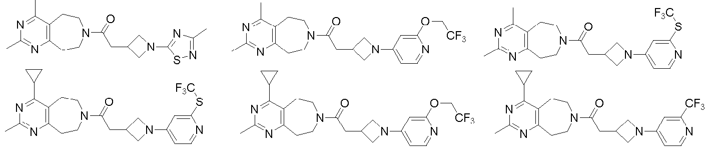

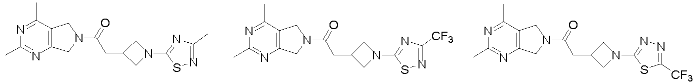

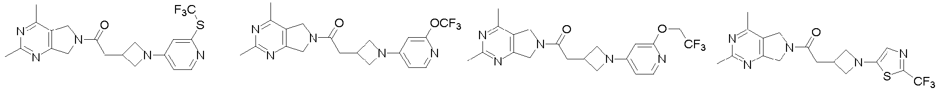

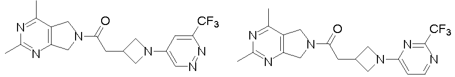

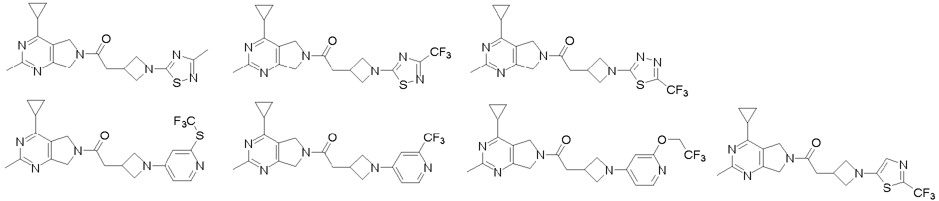

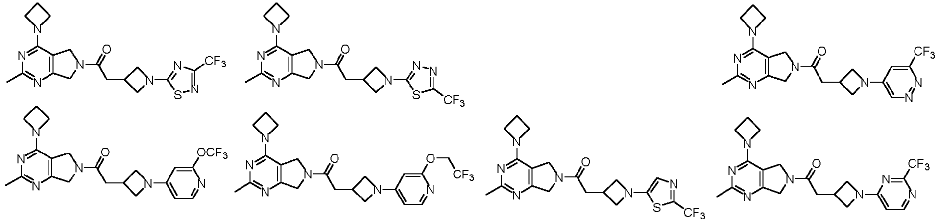

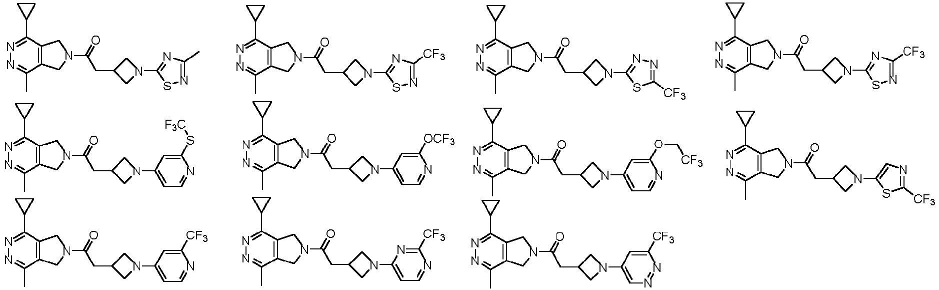

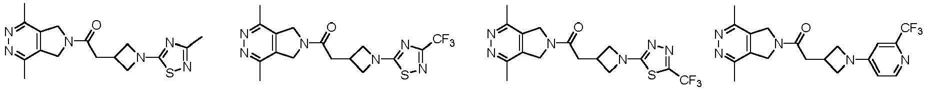

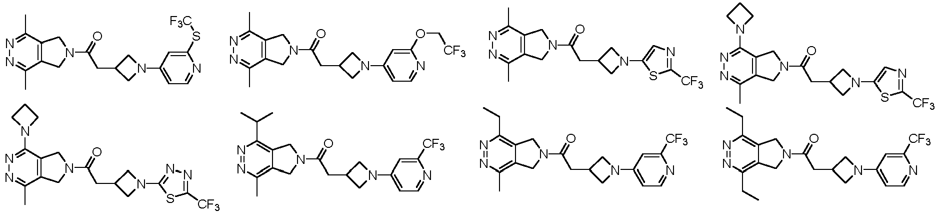

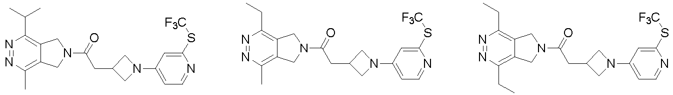

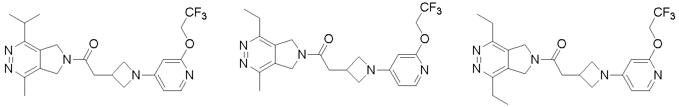

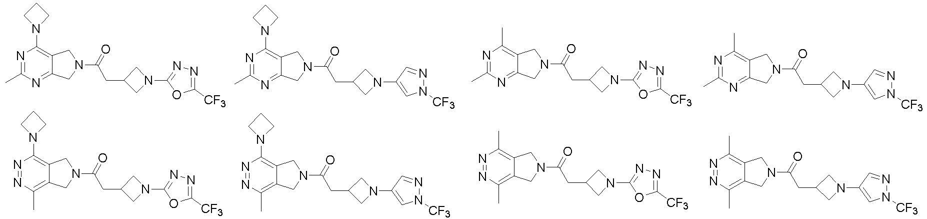

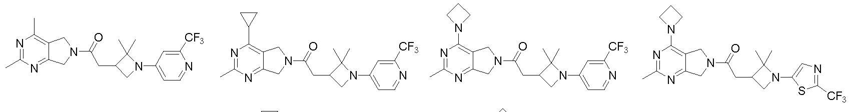

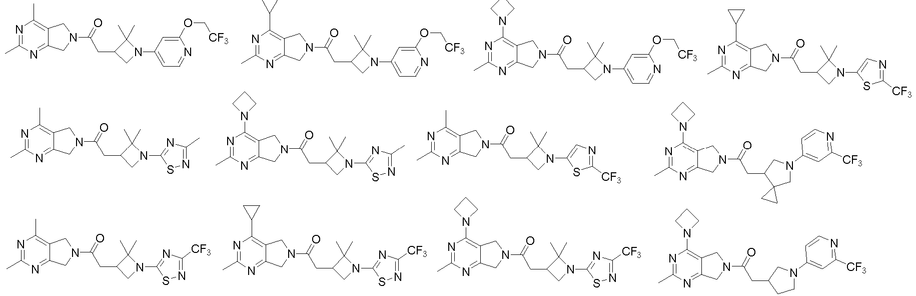

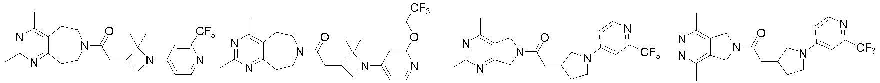

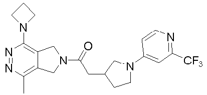

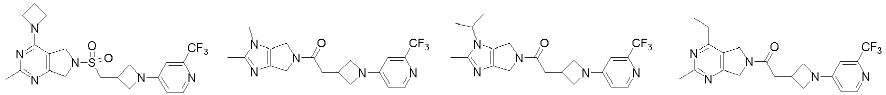

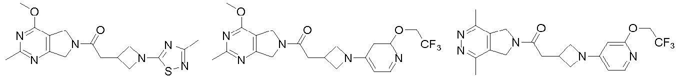

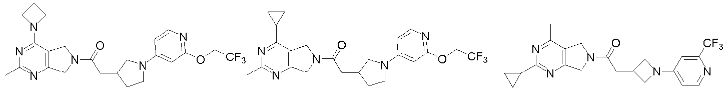

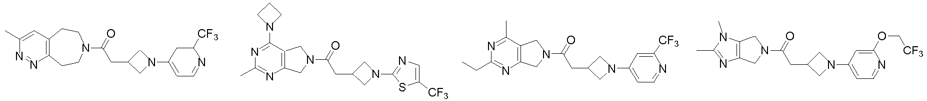

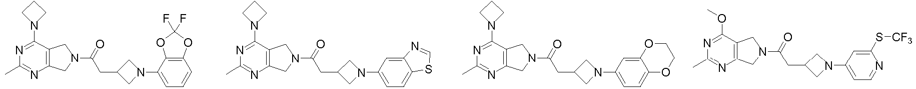

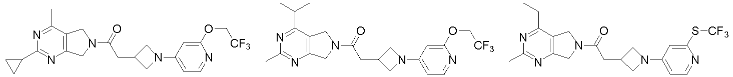

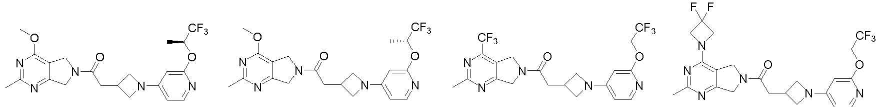

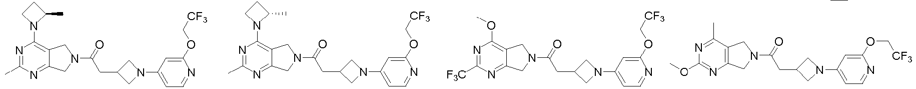

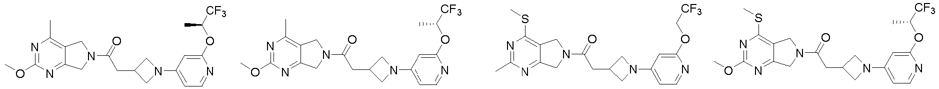

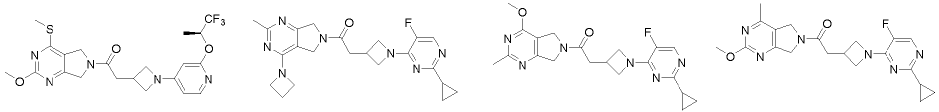

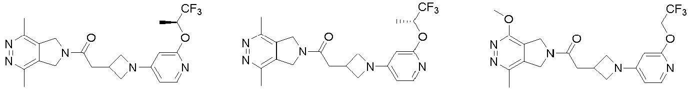

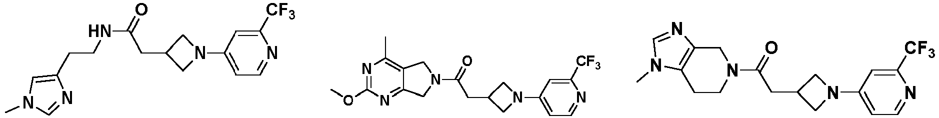

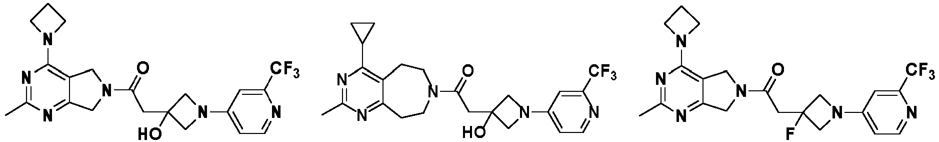

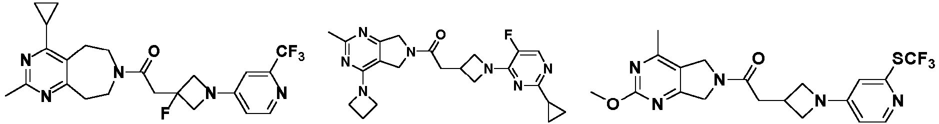

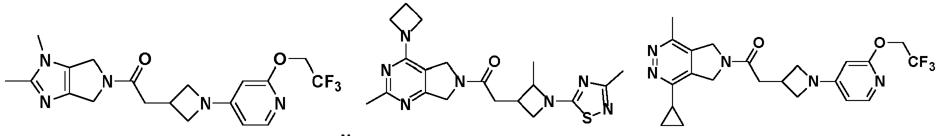

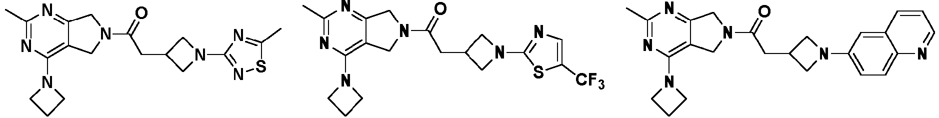

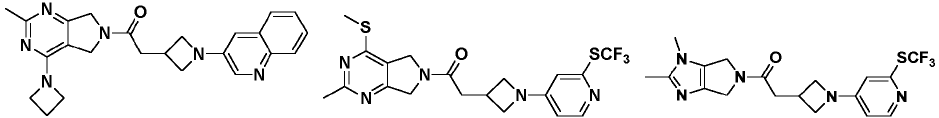

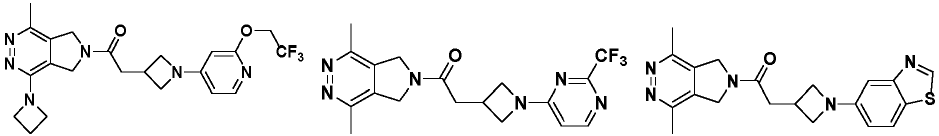

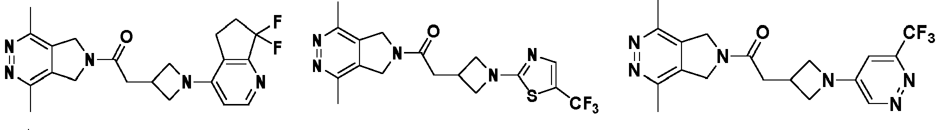

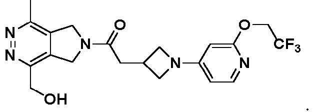

14. The present disclosure provides a preparation method for the compound of formula I or the pharmaceutically acceptable salt thereof according to any one of claims 1 to 13, wherein the preparation method is any one of the following schemes:
scheme (a): in an organic solvent, in the presence of a catalyst, performing a condensation reaction between a compound of formula II and a compound of formula III as follows to obtain the compound of formula I; 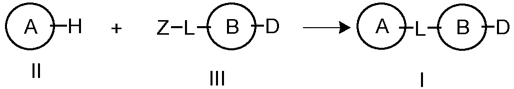
scheme (b): in an organic solvent, in the presence of a catalyst, performing a condensation reaction between a compound of formula IV and a compound of formula V as follows to obtain the compound of formula I; 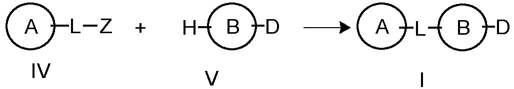
scheme (C): in an organic solvent, in the presence of a catalyst, performing a cyclization reaction between a compound of formula VI and a compound of formula VII as follows to obtain the compound of formula I; 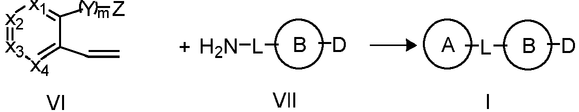
scheme (D): in an organic solvent, in the presence of a catalyst, performing a cyclization reaction between a compound of formula VIII and a compound of formula VIIII as follows to obtain the compound of formula I; 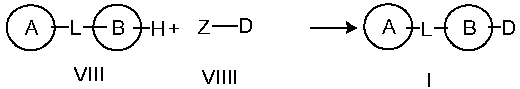
wherein Z is halogen, TsO-, hydroxyl, methoxy, ethoxy, n-propoxy, or isopropoxy; the halogen is, for example, chlorine or bromine; preferably, Z is hydroxyl, methoxy, ethoxy, n-propoxy, or isopropoxy;
A, L, B, D, X₁, X₂, X₃, X₄, Y, and m are as defined in any one of claims 1 to 13.

15. A pharmaceutical composition comprising a therapeutically effective amount of substance A and a pharmaceutical excipient; the substance A is the compound of formula I or the pharmaceutically acceptable salt thereof according to any one of claims 1 to 13.

16. A use of substance A in the manufacture of a positive allosteric modulator of a muscarinic receptor; the substance A is the compound of formula I or the pharmaceutically acceptable salt thereof according to any one of claims 1 to 12, or the pharmaceutical composition according to claim 15.

17. A use of substance A in the manufacture of a medicament for treating and/or preventing a muscarinic receptor-mediated disease; the substance A is the compound of formula I or the pharmaceutically acceptable salt thereof according to any one of claims 1 to 13, or the pharmaceutical composition according to claim 15; preferably, the disease is Parkinson's disease, Alzheimer's disease, Huntington's disease, schizophrenia, or drug addiction.
